# EUROPEAN PATENT SPECIFICATION

(11) **EP 1 620 102 B1**
(45) Date of publication and mention of the grant of the patent: **24.12.2008**
(21) Application number: 04750987.2
(22) Date of filing: 30.04.2004
(51) Int. Cl.: A61K 31/496, C07C 255/19, C07D 295/185, C07D 213/74, C07D 211/82, C07D 211/18, C07D 209/08, C07D 217/16, C07D 205/04, C07D 215/14, A61K 31/44, A61P 29/00

(54) **3-(1-NAPHTHYL)-2-CYANOPROPANOIC ACID DERIVATIVES AS ESTROGEN RECEPTOR LIGANDS**
3-(1-NAPHTHYL)-2-CYANOPROPANSÄUREDERIVATE ALS ÖSTROGEN REZEPTOR LIGANDEN
DERIVES D'AMIDES ET D'ESTERS D'ACIDE 2-CYANOPROPANOIQUE ET METHODES D'UTILISATION DESDITS DERIVES

(30) Priority: 30.04.2003 US 466567 P; 28.04.2004 US 833678
(43) Date of publication of application: 01.02.2006
(73) Proprietor: Wyeth, Madison, New Jersey 07940 (US)
(72) Inventor: CAGGIANO, Thomas, J., Morrisville, PA 19067 (US); BRAZZALE, Antony, A., Loxahatchee, Florida 33470 (US); MOORE, William, Jay, Collegeville, PA 19426 (US); KIM, Callain, Younghee, Collegeville, PA 19426 (US)
(74) Representative: Wileman, David Francis
(86) International application number: PCT/US2004/013360
(87) International publication number: WO 2004/099150

(56) References cited:
- EP-A- 0 733 620
- WO-A-99/07377
- DATABASE CA [Online] CHEMICAL ABSTRACTS SERVICE, COLUMBUS, OHIO, US; LATIF, N. ET AL: "Naphthalenes. Naphthylcyanoalkanoates, -cyano alcohols and -malonamic acids of anticipated broad spectrum biological activity" XP002301014 retrieved from STN Database accession no. 1977:405689 & EGYPTIAN JOURNAL OF CHEMISTRY , VOLUME DATE 1974, 17(6), 879-87 CODEN: EGJCA3; ISSN: 0449-2285, 1976,
- DATABASE CA [Online] CHEMICAL ABSTRACTS SERVICE, COLUMBUS, OHIO, US; PITTA DA ROCHA, IVAN ET AL: "Esters of some cinnamic acid derivatives. IV. 2-Cyanohydrocinnamates substituted" XP002301015 retrieved from STN Database accession no. 1975:155672 & EUROPEAN JOURNAL OF MEDICINAL CHEMISTRY , 9(4), 462-4 CODEN: EJMCA5; ISSN: 0223-5234, 1974,

## Description

### BACKGROUND

This invention relates to ligands for the estrogen receptor (ER), and specifically relates to 2-cyanopropanoic acid amide and ester derivatives useful for the treatment of the inflammatory component of diseases and are particularly useful in treating atherosclerosis, myocardial infarction, congestive heart failure, inflammatory bowel disease, arthritis, type 11 diabetes, and autoimmune diseases such as multiple sclerosis and rheumatoid arthritis.

The ability of ligands for the estrogen receptor to inhibit inflammatory gene expression causing a reduction of cytokines, chemokines, adhesion molecules and inflammatory enzymes provides a means to treat the inflammatory component of diseases such as atherosclerosis, myocardial infarction (MI), congestive heart failure (CHF), inflammatory bowel disease and arthritis. Other potential therapeutic indications for these type of molecules include type II diabetes (Cefalu, J Womens Health & Gender-based Med., 2001, 10, 241 & Yuan et al., Science, 2001, 293, 1673), osteoarthritis (Pelletier et al., Arthr. & Rheum., 2001, 44:1237 and Felson et al., Curr Opinion Rheum, 1998, 10, 269) asthma (Chin-Chi Lin et.al., Immunol. Lett., 2000, 73, 57), Alzheimer's disease (Roth, A. et. al.,; J. Neurosci. Res., 1999, 57, 399) and autoimmune diseases such as multiple sclerosis and rheumatoid arthritis.

A common component of these chronic inflammatory conditions is polymorphonuclear leukocyte and monocyte infiltration into the site of damage through increased expression of cytokines and adhesion molecules responsible for their recruitment. Overproduction of the cytokine interleukin (IL-6) has been associated with states of chronic inflammation (Bauer M. A., Herrmann F., Ann. Hematol., 1991, 62, 203). Synthesis of the IL-6 gene is induced by the transcription factor, nuclear factor κB (NF-κB). Interference at this step in the inflammatory process can effectively regulate the uncontrolled proliferative process that occurs in these chronic conditions.

In endothelial cells, 17β-estradiol (E2) inhibits IL-1β induced NF-κB reporter activity and IL-6 expression in an ER dependent fashion (Kurebayashi S. et. al., J. Steroid Biochem. Molec. Biol., 1997, 60, 11). This correlates with anti-inflammatory action of E2 *in vivo* as confirmed in different animal models of inflammation. In models of atherosclerosis, E2 was shown to protect endothelial cell integrity and function and to reduce leukocyte adhesion and intimal accumulation (Adams, M. R. et al., Arterio., 1990, ,1051, Sullivan, T. R. et al. J. Clin. Invst., 1995, 96, 2482, Nathan, L. et. al., Circ. Res., 1999, 85, 377). Similar effects of estrogen on the vascular wall have also been demonstrated in animal models of myocardial infarction (Delyani, J. A. et al., J. Molec. Cell. Cardiol., 1996, 28, 1001) and congestive heart failure. Clinically, estrogen replacement therapy (ERT) has been demonstrated to reduce the risk of mortality in patients with both CHF (Reis et. al., J. Am. Coll. Cardio., 2000, 36, 529) and MI (Grodstein, F. et. al., Ann. Int. Med., 2000, 133, 933, Alexander et. al., J. Am. Co/l. Cardio., 2001, 38, 1 and Grodstein F. et. al., Ann. Int. Med, 2001, 135,1). In ERT, clinical studies demonstrated an influence of E2 on the decrease in the production of β-amyloid 1-42 (Aβ42), a peptide central for the formation of senile plaques in Alzheimer's disease (Schonknecht, P. et. al., Neurosci. Lett., 2001, 307, 122).

However, 17-β-estradiol also strongly stimulates creatine kinase expression. Thus, in ERT some potential unwanted side effects, such as an increase risk of cardiovascular events in the first year of use, have been demonstrated (Hulley, S. et. al., J. Am. Med. Assoc., 1998, 280, 605) as well as proliferative effects on uterine and breast tissue.

### SUMMARY OF THE INVENTION

In one embodiment, the invention is directed to use of compounds of formula I: 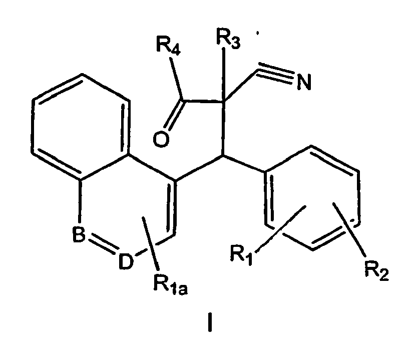 wherein
B and D are independently CH or N, provided that B and D are not both N;
R₁, R₁ₐ, R₂ are each, independently, hydrogen, halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
or R₁ and R₂ together with carbon atoms to which they are attached form a fused benzene ring, the naphthalene ring so formed being optionally substituted by halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
R₃ is hydrogen, alkyl of 1-6 carbon atoms, arylalkyl having 1-6 carbon atoms in the alkyl moiety, alkenyl of 2-7 carbon atoms, cycloalkylmethyl of 3-8 carbon atoms in the cycloalkyl moiety, arylalkoxyalkyl, alkoxyalkyl, dialkylaminoalkyl having 1-6 carbon atoms in the alkyl moieties, or Het-alkyl having 1-6 carbon atoms in the alkyl moiety;
R₄ is-NR₅R₆, -OR₆, 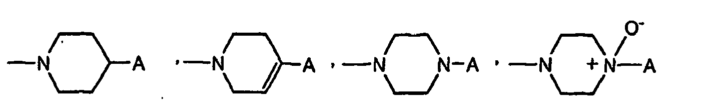 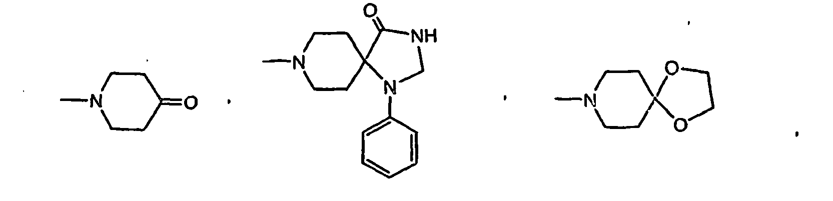 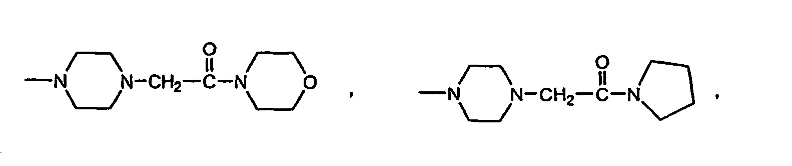 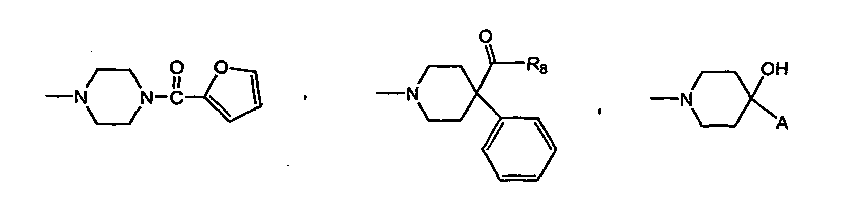 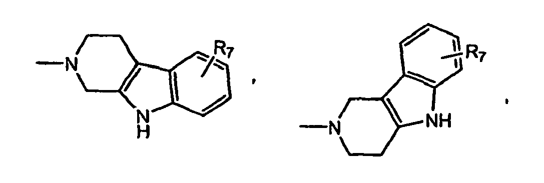
or A, wherein any phenyl ring in R₄ is optionally substituted with R₇;
R₅ and R₆ are each, independently, hydrogen, alkyl of 1-6 carbon atoms, aryl, arylalkyl having 1-6 carbon atoms in the alkyl moiety, Het-alkyl having 1-6 carbon atoms in the alkyl moiety, hydroxyalkyl of 1-6 carbon atoms, dihydroxyalkyl of 1-6 carbon atoms, or cycloalkyl of 3-8 carbon atoms;
R₇ is alkyl of 1-6 carbon atoms, alkoxy of 1-6 carbon atoms, halogen, nitro, cyano, alkylthio of 1-6 carbon atoms, thioalkyl of 1-6 carbon atoms, CF₃, or -OCF₃;
R₈ is alkyl of 1-6 carbon atoms;
A is hydrogen, cycloalkyl of 3-8 carbon atoms, alkoxyalkyl having 1-6 carbon atoms in the alkyl and alkoxy moieties, dialkylaminoalkyl having 1-6 carbon atoms in the alkyl moieties, aryl, Het, hydroxyalkyl of 1-6 carbon atoms, dihydroxyalkyl of 1-6 carbon atoms, Het-alkyl having 1-6 carbon atoms in the alkyl moiety, arylalkyl having 1-6 carbon atoms in the alkyl moiety, or 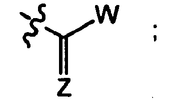
W is aryl, -Y-aryl, or Het or -Y-Het;
Y is -O- or -NH-;
Z is O or S;
Het is a saturated, unsaturated, or partially unsaturated heterocyclic ring or ring system having 4-12 ring atoms and 1-3 heteroatoms selected from N, O, or S, that may be optionally substituted with 1-3 R₇ groups;
aryl is an aromatic ring or ring system having 6-14 carbon atoms in the ring or ring system, that may be optionally substituted with 1-3 R₇ groups;
with the proviso that at least one of the R₁, R₁ₐ, or R₂ groups is not hydrogen;
or a pharmaceutically acceptable salt thereof; in the preparation of a medicament for treating the inflammatory component of a disease.

In another embodiment, the invention is directed to pharmaceutical compositions, comprising:
a pharmaceutical carrier; and
a compound of formula I or a pharmaceutically acceptable salt thereof.

In further embodiments, the invention is directed to the use of a compound of formula I for the preparation of a medicament useful for treating the inflammatory component of a disease, comprising the step of:
administering an effective amount of a compound of formula I or a pharmaceutically acceptable salt thereof.

The disease include atherosclerosis, myocardial infarction, congestive heart failure, inflammatory bowel disease, arthritis, type II diabetes, and autoimmune disease (such as multiple sclerosis or rheumatoid arthritis).

In yet further embodiments, the invention is directed to processes for preparing a compound of formula I.

In yet other embodiments, the invention is directed to process for preparing a substantially pure enantiomer of a compound of formula I.

In yet further embodiments this invention provides novel compounds of formula I as defined above wherein R₆ is hydrogen aryl, arylalkyl having 1-6 carbon atoms in the alkyl moiety, Het-alkyl having 1-6 carbon atoms in the alkyl moiety, hydroxyalkyl of 1-6 carbon atoms, dihydroxyalkyl of 1-6 carbon atoms, or cycloalkyl of 3-8 carbon atoms, or a pharmaceutically acceptable salt thereof.

### DESCRIPTION OF THE INVENTION

The invention provides substituted 2-cyanopropanoic acid amide and ester derivatives represented by formula I that are useful for the treatment of the inflammatory component of diseases and are particularly useful in treating atherosclerosis, myocardial infarction, congestive heart failure, inflammatory bowel disease, arthritis, type II diabetes, and autoimmune diseases such as multiple sclerosis and rheumatoid arthritis.

This invention provides use of compounds of formula I having the structure 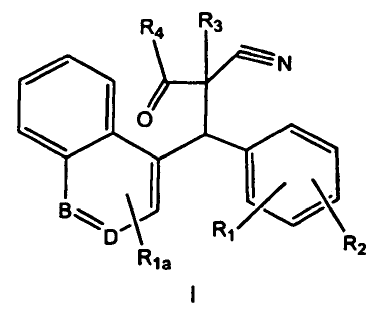 wherein
B and D are independently CH or N, provided that B and D are not both N;
R₁, R₁ₐ, R₂ are each, independently, hydrogen, halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
or R₁ and R₂ together with carbon atoms to which they are attached form a fused benzene ring, the naphthalene ring so formed being optionally substituted by halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
R₃ is hydrogen, alkyl of 1-6 carbon atoms, arylalkyl having 1-6 carbon atoms in the alkyl moiety, alkenyl of 2-7 carbon atoms, cycloalkylmethyl of 3-8 carbon atoms in the cycloalkyl moiety, arylalkoxyalkyl, alkoxyalkyl, dialkylaminoalkyl having 1-6 carbon atoms in
the alkyl moieties, or Het-alkyl having 1-6 carbon atoms in the alkyl moiety;
R₄ is-NR₅R₆, -OR₆, 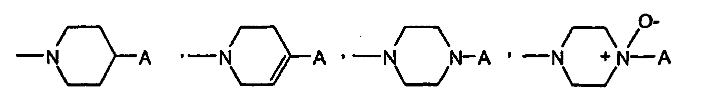 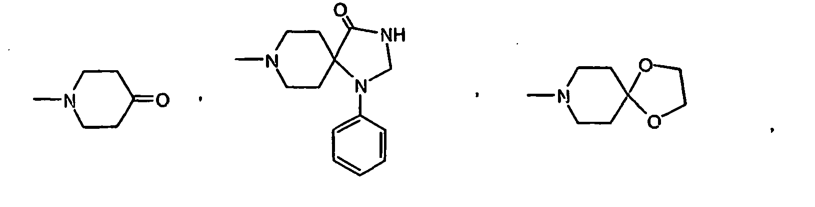 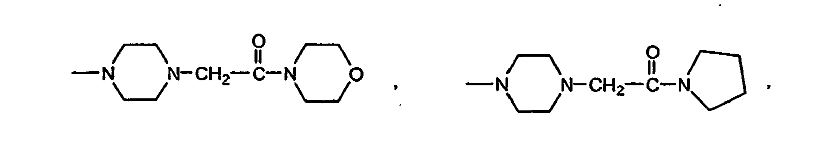 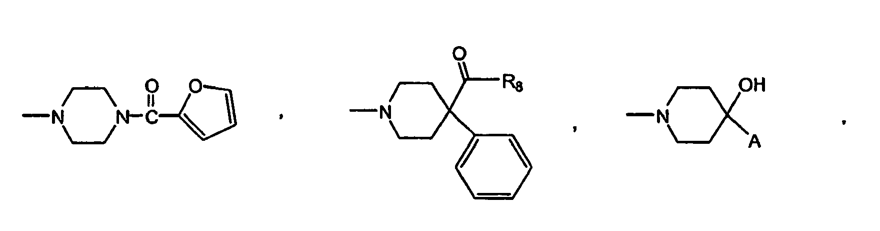 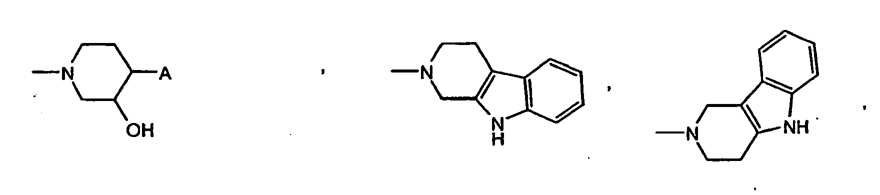 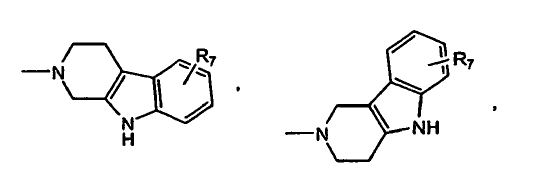
or A, wherein any phenyl ring in R₄ is optionally substituted with R₇;
R₅ and R₆ are each, independently, hydrogen, alkyl of 1-6 carbon atoms, aryl, arylalkyl having 1-6 carbon atoms in the alkyl moiety, Het-alkyl having 1-6 carbon atoms in the alkyl moiety, hydroxyalkyl of 1-6 carbon atoms, Dihydroxyalkyl of 1-6 carbon atoms, or cycloalkyl of 3-8 carbon atoms;
R₇ is alkyl of 1-6 carbon atoms, alkoxy of 1-6 carbon atoms, halogen, nitro, cyano, alkylthio of 1-6 carbon atoms, thioalkyl of 1-6 carbon atoms, CF₃, or-OCF₃;
R₈ is alkyl of 1-6 carbon atoms;
A is hydrogen, cycloalkyl of 3-8 carbon atoms, alkoxyalkyl having 1-6 carbon atoms in the alkyl and alkoxy moieties, dialkylaminoalkyl having 1-6 carbon atoms in the alkyl moieties, aryl, Het, hydroxyalkyl of 1-6 carbon atoms, dihydroxyalkyl of 1-6 carbon atoms, Het-alkyl having 1-6 carbon atoms in the alkyl moiety, arylalkyl having 1-6 carbon atoms in the alkyl moiety, or 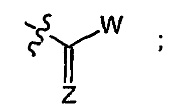
W is aryl, -Y-aryl, or Het or -Y-Het;
Y is -O- or -NH-;
Z is O or S;
Het is a saturated, unsaturated, or partially unsaturated heterocyclic ring or ring system having 4-12 ring atoms and 1-3 heteroatoms selected from N, O, or S, that may be optionally substituted with 1-3 R₇ groups;
aryl is an aromatic ring or ring system having 6-14 carbon atoms in the ring or ring system, that may be optionally substituted with 1-3 R₇ groups;
with the proviso that at least one of the R₁, R₁ₐ, or R₂ groups is not hydrogen;
or a pharmaceutically acceptable salt thereof.

The term "alkyl", employed alone, is defined herein as, unless otherwise stated, either a (C₁-C₈) straight chain or branched-chain monovalent saturated hydrocarbon moiety. It is preferred that the alkyl moiety contains 1-6 carbon atoms. Examples of saturated hydrocarbon alkyl moieties include, but are not limited to, chemical groups such as methyl, ethyl, *n*-propyl, isopropyl, *n*-butyl, *tert*-butyl, isobutyl, sec-butyl; higher homologs such as *n*-pentyl, *n*-hexyl, *n*-heptyl, *n*-octyl, and the like.

The term "cycloalkyl", employed alone or in combination with other terms, is defined herein as, unless otherwise stated, a monocyclic, bicyclic, tricyclic, fused, bridged, or spiro monovalent saturated hydrocarbon moiety of 3-8 carbon atoms. Any suitable ring position of the cycloalkyl moiety may be covalently linked to the defined chemical structure. Examples of cycloalkyl moieties include, but are not limited to, chemical groups such as cyclopropyl, cyclopropylmethyl, cyclobutyl, cyclopentyl, cyclohexyl, cyclohexylmethyl, cyclohexylethyl, cycloheptyl, norbornyl, adamantyl, spiro[4.5]decanyl, and homologs, isomers, and the like.

The terms "halo" or "halogen", employed alone or in combination with other terms, is defined herein as, unless otherwise stated, a fluorine, chlorine, bromine, or iodine atom.

The term "aryl", employed alone or in combination with other terms, is defined herein as, unless otherwise stated, an aromatic carbocyclic moiety of up to 14 carbon atoms, which may be a single ring (monocyclic) or multiple rings (bicyclic, up to three rings) fused together or linked covalently. Any suitable ring position of the aryl moiety may be covalently linked to the defined chemical structure. Examples of aryl moieties include, but are not limited to, chemical groups such as phenyl, 1-naphthyl, 2-naphthyl, dihydronaphthyl, tetrahydronaphthyl, biphenyl, anthryl, phenanthryl, fluorenyl, indanyl, biphenylenyl, acenaphthenyl, acenaphthylenyl, and the like.

The term "arylalkyl", employed alone or in combination with other terms, is defined herein as, unless otherwise stated, an aryl, as herein before defined, suitably substituted on any open ring position with an alkyl moiety wherein the alkyl chain is either a (C₁-C₆) saturated hydrocarbon moiety. Examples of arylalkyl moieties includes, but are not limited to, chemical groups such as benzyl, 1-phenylethyl, 2-phenylethyl, diphenylmethyl, 3-phenylpropyl, 2-phenylpropyl, fluorenylmethyl, and homologs, isomers, and the like.

The term "Het", employed alone or in combination with other terms, is defined herein as, unless otherwise stated, an heterocyclic ring system having 4-14 ring atoms, which may be a single ring (monocyclic) or multiple rings (bicyclic, up to three rings) fused together or linked covalently. The rings may contain from one to four hetero atoms selected from nitrogen (N), oxygen (O), and sulfur (S), wherein the nitrogen or sulfur atom(s) are optionally oxidized, or the nitrogen atom(s) are optionally quartemized. Any suitable ring position of the heteroaryl moiety may be covalently linked to the defined chemical structure. Examples of unsaturated Het moieties include, but are not limited to, heterocycles such as furan, thiophene, pyrrole, N-methylpyrrole, pyrazole, N-methylpyrazole, imidazole, N-methylimidazole, oxazole, isoxazole, thiazole, isothiazole, 1H-tetrazole, 1-methyltetrazole, 1,3,4-oxadiazole, 1H-1,2,4-triazole, 1-methyl-1,2,4-triazole 1,3,4-triazole, 1-methyl-1,3,4-triazole, pyridine, pyrimidine, pyrazine, pyridazine, benzoxazole, benzisoxazole, benzothiazole, benzofuran, benzothiophene, thianthrene, dibenzo[b,d]furan, dibenzo[b,d]thiophene, benzimidazole, N-methylbenzimidazole, indole, indazole, quinoline, isoquinoline, quinazoline, quinoxaline, purine, pteridine, 9H-carbazole, β-carboline, and the like.

Het, as defined herein, also includes saturated or partial saturated heterocyclic rings of 4-14 ring atoms, and 1-4 heteroatoms selected from N, O, and S. Examples of saturated or partially saturated heteroaryl moieties include, but are not limited to, chemical groups such as azetidinyl, 1,4-dioxanyl, hexahydroazepinyl, piperazinyl, piperidinyl, pyrrolidinyl, morpholinyl, thiomorpholinyl, dihydrobenzimidazolyl, dihydrobenzofuranyl, dihydrobenzothienyl, dihydrobenzoxazolyl, dihydrobenzoimidazolonyl, dihydrofuranyl, dihydroimidazolyl, dihydroindolyl, dihydroisooxazolyl, dihydroisothiazolyl, dihydrooxadiazolyl, dihydrooxazolyl, dihydropyrrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydro-1,4-dioxanyl, tetrahydrofuranyl, tetrahydrothienyl, tetrahydroquinolinyl, tetrahydroisoquinolinyl, and the like.

The term "Het-alkyl", employed alone or in combination with other terms, is defined herein as, unless otherwise stated, a heteroaryl, as herein before defined, suitably substituted on any open ring position with an alkyl moiety, wherein the alkyl chain is either a (C₁-C₆) straight or branched-chain saturated hydrocarbon moiety. Examples of heteroarylalkyl moieties include, but are not limited to, chemical groups such as furanylmethyl, thienylethyl, indolylmethyl, and the like.

The term "hydroxyalkyl", employed alone or in combination with other terms, is defined herein as, unless otherwise stated, a (C₁-C₆) straight chain hydrocarbon, terminally substituted with a hydroxyl group. Examples of hydroxyalkyl moieties include chemical groups such as -CH₂OH -CH₂CH₂OH, -CH₂CH₂CH₂OH, and higher homologs. Similarly, dihydroxyalkyl indicates an alkyl moiety that is substituted by two hydroxyl groups.

The term "alkoxy", employed alone or in combination with other terms, is defined herein as, unless otherwise stated, either a (C₁-C₆) straight chain or branched-chain hydrocarbon covalently bonded to an oxygen atom. Examples of alkoxy moieties include, but are not limited to, chemical groups such as methoxy, ethoxy, isopropoxy, *sec-*butoxy, *tert*-butoxy, and homologs, isomers, and the like.

The term "alkylthio", employed alone or in combination with other terms, is defined herein as, unless otherwise stated, either a (C₁-C₆) straight or branched-chain hydrocarbon covalently bonded to a sulfur atom. Examples of alkylthio moieties include, but are not limited to, chemical groups such as methylthio, ethylthio, isopropylthio, *sec*-butylthio, *tert*-butylthio, and homologs, isomers, and the like.

The term "thioalkyl", employed alone or in combination with other terms, is defined herein as, unless otherwise stated, a sulfhydryl group covalently bonded to either a (C₁-C₆) straight or branched-chain hydrocarbon. Examples of thioalkyl moieties include, but are not limited to, chemical groups such as thiomethyl, thioethyl, thioisopropyl, and homologs, isomers, and the like.

The term "alkoxyalkyl" is defined herein as, unless otherwise stated, an alkyl, as herein before defined, substituted by an alkoxy group, as herein before defined. An example of an alkoxyalkyl moiety is methoxyethyl.

The term "arylalkoxyalkyl" is defined herein as, unless otherwise stated, an alkyl, as herein before defined, substituted by an alkoxy group, as herein before defined, wherein the alkoxy is substituted by an aryl, as herein before defined. An example of an arylalkyloxyalkyl moiety is phenylmethoxymethyl.

The term "dialkylaminoalkyl" is defined herein as, unless otherwise stated, an alkyl as herein before defined, substituted by an amino group independently disubstituted with alkyl, as herein before defined. An example of a dialkylaminoalkyl group is dimethylaminoethyl.

Unless otherwise indicated, each of the above terms (e.g., alkyl, aryl, Het) are meant to include unsubstituted, monosubstituted, and polysubstituted forms of the indicated radical or moiety. As described above, the moiety R₇ represents typical substituents for the radicals or moieties.

The compounds of the present invention may contain an asymmetric atom, and some of the compounds may contain one or more asymmetric atoms or centers, which may thus give rise to optical isomers (enantiomers) and diastereomers. While shown without respect to the stereochemistry in Formula (I), the present invention includes such optical isomers (enantiomers) and diastereomers (geometric isomers); as well as the racemic and resolved, enantiomerically pure *R* and *S* stereoisomers; as well as other mixtures of the *R* and *S* stereoisomers and pharmaceutically acceptable salts thereof. Optical isomers may be obtained in pure form by standard procedures known to those skilled in the art, and include, but are not limited to, diasteromeric salt formation, kinetic resolution, and asymmetric synthesis. It is also understood that this invention encompasses all possible regioisomers, and mixtures thereof, which may be obtained in pure form by standard separation procedures known to those skilled in the art, and include, but are not limited to, column chromatography, thin-layer chromatography, and high-performance liquid chromatography.

The compounds of the present invention may contain isotopes of atoms for diagnostic, therapeutic, or metabolic purposes. Such isotopes may or may not be radioactive.

The compounds of this invention include racemates, enantiomers, geometric isomers, or pro-drugs of the compounds shown by formula I.

Pharmaceutically acceptable salts of the compounds of formula I with an acidic moiety can be formed from organic and inorganic bases. Suitable salts with bases are, for example, metal salts, such as alkali metal or alkaline earth metal salts, for example sodium, potassium, or magnesium salts; or salts with ammonia or an organic amine, such as morpholine, thiomorpholine, piperidine, pyrrolidine, a mono-, di- or tri-lower alkylamine, for example ethyl-tert-butyl-, diethyl-, diisopropyl-, triethyl-, tributyl- or dimethylpropylamine, or a mono-, di-, or trihydroxy lower alkylamine, for example mono-, di- or triethanolamine. Internal salts may furthermore be formed. Similarly, when a compound of the present invention contains a basic moiety, salts can be formed from organic and inorganic acids. For example salts can be formed from acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known pharmaceutically acceptable acids.

As used in accordance with this invention, the term "providing," with respect to providing a compound or substance covered by this invention, means either directly administering such a compound or substance, or administering a pro-drug, derivative, or analog that will form the effective amount of the compound or substance within the body. This invention also covers providing the compounds of this invention to treat the disease states disclosed herein that the compounds are useful for treating.

Preferred compounds useful in this invention include those of formula I in which:
R₄ is -NR₅R₆, -OR₆, 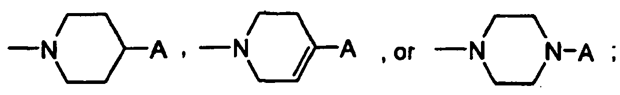 and
A is hydrogen, aryl, or Het; or
a pharmaceutically acceptable salt thereof.

More preferred compounds useful in this invention include those of formula I in which:
R₄ is 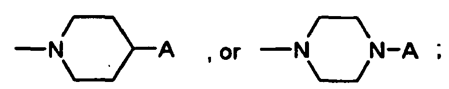 and
A is hydrogen, aryl, or Het; or
a pharmaceutically acceptable salt thereof.

In certain preferred embodiments, B and D are both CH. In certain other preferred embodiments, one of B or D is N. In yet other preferred embodiments, B is N and D is CH. In yet further embodiments, B is CH and D is N.

Preferred compounds of formula I useful in this invention include:
ethyl 2-cyano-3-(2,6-dimethoxyphenyl)-3-(1-naphthyl)propanoate;
ethyl 2-cyano-3-(2,6-dichlorophenyl)-3-(1-naphthyl)propanoate;
ethyl 2-cyano-3-[4-(dimethylamino)phenyl]-3-(1-naphthyl)propanoate;
ethyl 2-cyano-3-(1-naphthyl)-3-[2(trifluoromethyl)phenyl]propanoate;
ethyl 2-cyano-3-(2-isopropylpheny)-3-(1-naphthyl)propanoate;
ethyl 2-cyano-3-(2,4-dimethoxyphenyl)-3-(1-naphthyl)propanoate;
ethyl 2-cyano-3-(2,5-dimethoxyphenyl)-3-(1-naphthyl)propanoate;
*tert-*butyl 2-cyano-3-(1-naphthyl)-3-[2-(trifluoromethyl)phenyl]propanoate;
ethyl 2-cyano-3-(2-methoxyphenyl)-3-(1-naphthyl)propanoate;
ethyl (*RR,SS*)-2-cyano-3-(2-methoxyphenyl)-3-(1-naphthyl)propanoate;
*tert*-butyl 2-cyano-3-(2-isopropylphenyl)-3-(1-naphthyl)propanoate;
*tert*-butyl 2-cyano-3-(2-methoxyphenyl)-3-(1-naphthyl)propanoate;
*tert*-butyl 2-cyano-3-(1-naphthyl)-3-[2-(trifluoromethoxy)phenyl]propanoate;
ethyl 2-cyano-3-(1-naphthyl)-3-(2-nitrophenyl)propanoate;
*tert*-butyl 2-cyano-3-(2,6-dimethylphenyl)-3-(1-naphthyl)propanoate;
*tert*-butyl (*RR*,*SS*)-2-cyano-3-(1-naphthyl)-3-[2-(trifluoromethyl)phenyl] propanoate;
*tert*-butyl (*RR*,*SS*)-2-cyano-3-(2-methoxyphenyl)-2-methyl-3-(1-naphthyl)propanoate;
(-) ethyl (*SS*)-2-cyano-3-(2-methoxyphenyl)-2-methyl-3-(1-naphthyl)propanoate;
(+) ethyl (*R,R*)-2-cyano-3-(2-methoxyphenyl)-2-methyl-3-(1-naphthyl)propanoate;
ethyl (*RR*,*SS*)-2-cyano-2-[(2-methoxyphenyl)(1-naphthyl)methyl]-4-pentenoate;
ethyl (*RR*,*SS*)-2-benzyl-2-cyano-3-(2-methoxyphenyl)-3-(1-naphthyl) propanoate;
*tert*-butyl (*RR*,*SS*)-2-cyano-3-(2-isopropylphenyl)-2-methyl-3-(1-naphthyl)propanoate;
*tert*-butyl (*RS*,*SR*)-2-cyano-2-methyl-3-(1-naphthyl)3-[2-(trifluoromethyl)phenyl]propanoate;
*tert*-butyl (*RS*,*SR*)-2-cyano-3-(2,6-dimethylphenyl)2-methyl-3-(1-naphthyl)propanoate;
*tert*-butyl (*RR*,*SS*)-2-benzyl-2-cyano-3-(2-methoxyphenyl)-3-(1-naphthyl)propanoate;
*tert*-butyl (*RR*, *SS*)-2-(3-chlorobenzyl)-2-cyano-3-(2-methoxyphenyl)-3-(1-naphthyl)propanoate;
*tert*-butyl (*RR*,*SS*)-2-(2-bromobenzyl)-2-cyano-3-(2-methoxyphenyl)-3-(1-naphthyl)propanoate;
*tert*-butyl (*RR*,*SS*)-2-(2-chlorobenzyl)-2-cyano-3-(2-methoxyphenyl)-3-(1-naphthyl)propanoate;
*tert*-butyl (*RR*,*SS*)-2-cyano-2-(2,6-dichlorobenzyl)-3-(2-methoxyphenyl)-3-(1-naphthyl)propanoate;
ethyl (*RR*,*SS*)-2-cyano-3-(2,4-dimethoxyphenyl)-2-methyl-3-(1-naphthyl)propanoate;
*tert*-butyl (*RS*,*SR*)-2-cyano-2-methyl-3-(1-naphthyl)-3-[2-(trifluoromethoxy)phenyl]propanoate;
ethyl 2-cyano-3-(3-methoxyphenyl)-3-(1-naphthyl)propanoate;
ethyl 2-cyano-3-(4-methylphenyl)-3-(1-naphthyl)propanoate;
ethyl 2-cyano-3-(2-methylphenyl)-3-(1-naphthyl)propanoate;
ethyl 2-cyano-3-(1-naphthyl)-3-(2-naphthyl)propanoate;
ethyl 2-cyano-3-(4-fluoro-1-naphthyl)-3-(1-naphthyl)propanoate;
ethyl 2-cyano-3-[4-(methylthio)phenyl]-3-(1-naphthyl)propanoate;
ethyl 3-[1,1'-biphenyl]-4-yl-2-cyano-3-(1-naphthyl)propanoate;
ethyl 3-[1,1'-biphenyl]-2-yl-2-cyano-3-(1-naphthyl)propanoate;
ethyl 3-(4-chlorophenyl)-2-cyano-3-(1-naphthyl)propanoate;
ethyl 2-cyano-3-[2-(methylthio)phenyl]-3-(1-naphthyl)propanoate;
ethyl-(*RR*,*SS*)-2-cyano-3-(2-methoxyphenyl)-2-methyl-3-(1-naphthyl)propanoate;
ethyl (*RR*,*SS*)-2-cyano-2-methyl-3-[2-(methylthio)phenyl]-3-(1-naphthyl)propanoate;
(*RR*,*SS*)-2-cyano-3-(2-methoxyphenyl)-2-methyl-3-(1-naphthyl)propanoic acid;
(*RR*,*SS*)-2-cyano-3-(2-isopropylphenyl)-2-methyl-3-(1-naphthyl)propanoic acid;
(*RS*,*SR*)-2-cyano-2-methyl-3-(1-naphthyl)-3-[2-(trifluoromethyl)phenyl]propanoic acid;
(*RR*,*SS*)-2-benzyl-2-cyano-3-(2-methoxyphenyl)-3-(1-naphthyl)propanoic acid;
*tert*-butyl (*R,R*)-2-cyano-3-(2-isopropytphenyl)-2-methyl-3-(1-naphthyl)propanoate;
*tert*-butyl (*S*,*S*)-2-cyano-3-(2-isopropylpenyl)-2-methyl-3-(1-naphthyl)propanoate;
*tert*-butyl (*S,S*)-2-cyano-3-(2-methoxyphenyl)-2-methyl-3-(1-naphthyl)propanoate;
*tert*-butyl (*R*,*R*)-2-cyano-3-(2-methoxyphenyl)-2-methyl-3-(1 - naphthyl)propanoate;
(*RR*,*SS*)-3-(2-methoxyphenyl)-2-methyl-3-(1-naphthyl)-2-(piperazin-1-ylcarbonyl)propanenitrile;
*RR,SS*)(3-[4-(3-chloro-2-methylphenyl)piperazin-1-yl]-2-[(2-methoxyphenyl)(1-naphthyl)methyl]-2-methyl-3-oxopropanenitrile);
(*RR*,*S*,*S*)-3-(2-methoxyphenyl)-2-methyl-2-{[4-(2-methylphenyl)piperazin-1-yl]carbonyl}-3-(1-naphthyl)propanenitrile;
(*SS*)-3-(2-methoxyphenyl)-2-methyl-2-{[4-(2-methylphenyl)-1-piperazinyl]carbonyl}-3-(1-naphthyl)propanenitrile;
(*R*,*R*)-3-(2-methoxyphenyl)-2-methyl-2-{[4-(2-methylphenyl)piperazin-1-y)]carbonyt}-3-(1-naphthyl)propanenitrile;
(*S*,*S*)-3-(2-methoxyphenyl)-2-methyl-2-{[4-(3-methylphenyl)piperazin-1-yl]carbonyl}-3-(1-naphthyl)propanenitrile;
(*S*)-3-[4-(3,5-dimethoxyphenyl)piperazin-1-yl]-2-[(*S*)-(2-methoxyphenyl)(1-naphthyl)methyl]-2-methyl-3-oxopropanenitrile;
(*S*)3-(4-indan-4-yl-piperazin-1-yl)-2-[(*S*)(2-methoxy-phenyl)-naphthalen-1-yl-methyl]-2-methyl-3-oxo-propionitrile;
(*S*,*S*)-3-(2-methoxyphenyl)-2-methyl-3-(1-naphthyl)-2-{[4-(1-naphthyl)piperazin-1-yl]carbonyl}propanenitrile;
(*S*)-3-[4-(3,4-dimethylphenyl)piperazin-1-yl]-2-[(*S*)-(2-methoxyphenyl)(1-naphthyl)methyl]-2-methyl-3-oxopropanenitrile;
(*RR*,*SS*) 3-[4-(*H-*indol-4-yl)piperazin-1-yl]-2-[(2-methoxyphenyl)(1-naphthyl)methyl]-2-methyl-3-oxopropanenitrile;
(*S*)-3-[4-(3-chlorophenyl)piperazin-1-yl]-2-[(*S*)-(2-methoxyphenyl)(1-naphthyl)methyl]-2-methyl-3-oxopropanenitrile;
(*S*)-3-[4-(2,3-dimethylphenyl)piperazin-1-yl]-2-[(*S*)-(2-methoxyphenyl)(1-naphthyl)methyl]-2-methyl-3-oxopropanenitrile;
(*S*)-3-[4-(4-chlorophenyl)piperazin-1-yl]-2-[(*S*)-(2-methoxyphenyl)(1 - naphthyl)methyl]-2-methyl-3-oxopropanenitrile;
(*S*)-3-[4-(1*H*-Indol-4-yl)-piperazin-1-yl]-2-[(*S*)-(2-methoxy-phenyl)-naphthalen-1-yl-methyl]-2-methyl-3-oxo-propionitrile;
(*S,S*)-3-(2-methoxyphenyl)-2-methyl-3-(1-naphthyl)-2-({4-[3-(trifluoromethyl)phenyl] piperidin-1-yl}carbonyl)propanenitrile;
(*S*)-3-[4-(4-chloro-phenyl)-3,6-dihydro-2*H*-pyridin-1-yl]-2-[(*S*)-(2-methoxyphenyl)-naphthalen-1-yl-methyl]-2-methyl-3-oxo-propionitrile;
(*S,S*)-3-(2-methoxy-phenyl)-2-methyl-3-naphthalen-1-yl-2-[4-(3-trifluoromethyl-phenyl)-3,6-dihydro-2*H-*pyridine-1-carbonyl]-propionitrile;
(*SS*)2-[4-(4-Chloro-phenyl)-piperidine-1-carbonyl]-3-(2-methoxy-phenyl)-2-methyl-3-naphthalen-1-yl-propionitrile;
(*RR*,*SS*)-3-(2-methoxyphenyl)-2-methyl-3-(1-naphthyl)-2-({4-[3-(trifluoromethyl)phenyl]piperidin-1-yl}carbonyl)propanenitrile;
(*RR*,*SS*)-3-(2-methoxyphenyl)-2-methyl-3-(1-naphthyl)-2-(piperidin-1-ylcarbonyl)propanenitrile;
(*RR*,*SS*)-2-cyano-3-(2-methoxyphenyl)-2-methyl-3-(1-naphthyl)propanamide;
(*RR*,*SS*)-2-cyano-N-ethyl-3-(2-methoxyphenyl)-2-methyl-3-(1-naphthyl)propanamide;
(*RR*,*SS*)-N-(*tert*-butyl)-2-cyano-3-(2-methoxyphenyl)-2-methyl-3-(1-naphthyl)propanamide;
(*RR*,*SS*)-2-cyano-3-(2-methoxyphenyl)-N,N,2-trimethyl-3-(1-naphthyl)propanamide;
(*RR*,*SS*)-2-cyano-N-methoxy-3-(2-methoxyphenyl)-N,2-dimethyl-3-(1-naphthyl)propanamide;
(*RR*,*SS*)-2-benzyl-3-[4-(3,5-dichloro-4-pyridinyl)-1-piperazinyl-2-[(2-methoxyphenyl)(1-naphthyl)methyl]-3-oxopropanenitrile;
(*R*,*S*)-3-[4-(2,3-dimethylphenyl)piperazin-1-yl]-2-[(*R*,*S*)-(2-methoxyphenyl)(1-naphthyl)methyl]-2-methyl-3-oxopropanenitrile;
(*R,S*)-3-[4-(3-isopropylphenyl)piperazin-1-yl]-2-[(*R,S*)-(2-methoxyphenyl)(1-naphthyl)methyl]-2-methyl-3-oxopropanenitrile;
(*RR*,*SS*)-3-[4-(3,5-dichloropyridin-4-yl)piperazin-1-yl]-2-[(2-methoxyphenyl)(1-naphthyl)methyl]-2-methyl-3-oxopropanenitrile;
(2*SS*)-3-[4-(3-chloro-2-methylphenyl)piperazin-1-yl]-2-[(2-methoxyphenyl)(1-naphthyl)methyl]-2-methyl-3-oxopropanenitrile;
(*S*)-3-[4-(2-fluorophenyl)piperazin-1-yl]-2-[(*S*)-(2-methoxyphenyl)(1-naphthyl)methyl]-2-methyl-3-oxopropanenitrile;
(*S*)-3-[4-(2-chlorophenyl)piperazin-1-yl]-2-[(*S*)-(2-methoxyphenyl)(1-naphthyl)methyl]-2-methyl-3-oxopropanenitrile;
(*S,S*)-3-(2-methoxyphenyl)-2-{[4-(3-methoxyphenyl)piperazin-1-yl]carbonyl}-2-methyl-3-(1-naphthyl)propanenitrile;
(*R*,*S*)-3-[4-(3-chloropyndin-4-yl)piperazin-1-yl]-2-[(*R*,*S*)-(2-methoxyphenyl)(1-naphthyl)methyl]-2-methyl-3-oxopropanenitrile;
(*S*)-3-[4-(2,3-dichlorophenyl)piperazin-1-yl]-2-[(*S*)-(2-methoxyphenyl)(1-naphthyl)methyl]-2-methyl-3-oxopropanenitrile;
(2*S*)-3-[4-[4-chloro-3-(trifluoromethyl)phenyl]-3,6-dihydropyridin-1(2*H*)-yl]-2-[(*S*)-(2-methoxyphenyl)(1-naphthyl)methyl]-2-methyl-3-oxopropanenitrile;
(2*S*)-3-{4-[4-chloro-3-(trifluoromethyl)phenyl]piperidin-1-yl}-2-[(*S*)-(2-methoxyphenyl)(1-naphthyl)methyl]-2-methyl-3-oxopropanenitrile;
(*RR*/*SS*)-3-(2-methoxy-phenyl)-2-methyl-3-naphthalen-1-yl-2-(4-oxy-4-o-tolyl-piperazine-1-carbonyl)-propionitrile;
(*RR*,*SS*)-3-(2-methoxyphenyl)-2-methyl-3-(1-naphthyl)-2-({4-[3-(trifluoromethoxy)phenyl]piperazin-1-yl}carbonyl)propanenitrile;
(*R*,*S*)-3-[4-(2,3-difluorophenyl)piperazin-1-yl]-2-[(*R*,*S*)-(2-methoxyphenyl)(1-naphthyl)methyl]-2-methyl-3-oxopropanenitrile;
(*R*,*S*)-3-[4-(3-fluorophenyl)piperazin-1-yl]-2-[(*R*,*S*)-(2-methoxyphenyl)(1-naphthyl)methyl]-2-methyl-3-oxopropanenitrile;
(*RR*,*SS*)-3-(2-methoxyphenyl)-2-methyl-3-(1-naphthyl)-2-[(4-pyridin-3-ylpiperazin-1-yl)carbonyl]propanenitrile;
(*RR*,*SS*)-3-[4-(2,3-dichlorophenyl)piperazin-1-yl]-2-[(2-methoxyphenyl)(1-naphthyl)methyl]-2-methyl-3-oxopropanenitrile;
(*RR*,*SS*)-3-(2-methoxyphenyl)-2-methyl-2-{[4-(3-methylphenyl)piperazin-1-y1]carbonyl}-3-(1-naphthyl)propanenitrile;
(*RR*,*SS*)-3-(2-methoxyphenyl)-2-methyl-3-(1-naphthyl)-2-(pyrrolidin-1-ylcarbonyl)propanenitrile;
(*RR*,*SS*)-3-(2-methoxyphenyl)-2-methyl-2-(morpholin-4-ylcarbonyl)-3-(1-naphthyl)propanenitrile;
(*RR*,*SS*)-3-[4-(2-hydroxyethyl)piperazin-1-yl]-2-[(2-methoxphenyl)(1-naphthyl)methyl]-2-methyl-3-oxopropanenitrile;
(*RR*,*SS*)-3-(2,6-dimethylmorpholin-4-yl)-2-[(2-methoxyphenyl)(1-naphthyl)methyl]-2-methyt-3-oxopropanenitrile;
(*RR*,*SS*)-2-cyano-N,N-diethyl-3-(2-methoxyphenyl)-2-methyl-3-(1-naphthyl)propanamide;
(*RR*,*SS*)-2-cyano-N-[2-hydroxy-1-(hydroxymethyl)ethyl]-3-(2-methoxyphenyl)-2-methyl-3-(1-naphthyl)propanamide;
(*RR*,*SS*)-3-azetidin-1-yl-2-[(2-methoxyphenyl)(1-naphthyl)methyl]-2-methyl-3-oxopropanenitrile;
(*RR*,*SS*)-2-cyano-N,N-diisopropyl-3-(2-methoxyphenyl)-2-methyl-3-(1 naphthyl)propanamide;
(*RR*,*SS*)-3-(2-methoxyphenyl)-2-methyl-3-(1-naphthyl)-2-[(3,3,5-trimethylazepan-1-yl)carbonyl]propanenitrile;
(*RR*,*SS*)-3-(2,3-dihydro-*H*-indol-1-yl)2-[(2-methoxyphenyl)(1-naphthyl)methyl]-2-methyl-3-oxopropanenitrile;
(*RR*,*SS*)-3-(2-methoxyphenyl)-2-methyl-3-(1-naphthyl)-2-(thiomorpholin-4-ylcarbonyl)propanenitrile;
((*RR*,*SS*)-3-azepan-1-yl-2-[(2-methoxyphenyl)(1-naphthyl)methyl]-2-methyl-3-oxopropanenitrile;
(*RR*,*SS*)-2-cyano-N-cyclohexyl-3-(2-methoxyphenyl)-N,2-dimethyl-3-(1-naphthyl)propanamide;
(*RR*,*SS*)-2-cyano-3-(2-methoxyphenyl)-N,2-dimethyl-3-(1-naphthyl)propanamide;
(*RR*,*SS*)-3-(4-benzylpiperazin-1-yl)-2-[(2-methoxyphenyl)(1-naphthyl)methyl]-2-methyl-3-oxopropanenitrile;
(*RR*,*SS*)-3-(3,4-dihydroisoquinolin-2(*H*)-yl)2-[(2-methoxyphenyl)(1-naphthyl)methyl]-2-methyl-3-oxopropanenitrile;
(*RR*,*SS*)-3-(2-methoxyphenyl)-2-methyl-2-[4-(4-methylphenyl)piperazin-1-yl]carbonyl}-3-(1-naphthyl)propanenitrile;
(*RR,SS*)-N,N-dibenzyl-2-cyano-3-(2-methoxyphenyl)-2-methyl-3-(1-naphthyl)propanamide;
(*RR*,*SS*)-3-azocan-1-yl-2-[(2-methoxyphenyl)(1-naphthyl)methyl]-2-methyl-3-oxopropanenitrile;
4-chlorophenyl 4-[(*RR*,*SS*)-2-cyano-3-(2-methoxyphenyl)-2-methyl-3-(1-naphthyl)propanoyl]piperazine-1-carboxylate;
2-nitrophenyl 4-[(*RR*,*SS*)-2-cyano-3-(2-methoxyphenyl)-2-methyl-3-(1-naphthyl)propanoyl]piperazine-1-carboxylate;
4-(methoxycarbonyl)phenyl 4-[(*RR*,*SS*)-2-cyano-3-(2-methoxyphenyl)-2-methyl-3-(1-naphthyl)propanoyl]piperazine-1-carboxylate;
4-methylphenyl 4-[(*RR*,*SS*)-2-cyano-3-(2-methoxyphenyl)-2-methyl-3-(1-naphthyl)propanoyl]piperazine-1-carboxylate;
4-[(*RR*,*SS*)-2-cyano-3-(2-methoxyphenyl)-2-methyl-3-(1-naphthyl)propanoyl]-N-(2-methylphenyl)piperazine-1-carboxamide;
4-[(*RR*,*SS*)-2-cyano-3-(2-methoxyphenyl)-2-methyl-3-(1-naphthyl)propanoyl]-N-[2-(trifluoromethyl)phenyl]piperazine-1-carboxamide;
4-[(*RR*,*SS*)-2-cyano-3-(2-methoxyphenyl)-2-methyl-3-(1-naphthyl)propanoyl]-N-(3-methoxyphenyl)piperazine-1-carboxamide;
4-[(*RR*,*SS*)-2-cyano-3-(2-methoxyphenyl)-2-methyl-3-(1-naphthyl)propanoyl]-N-(4-ethoxyphenyl)piperazine-1-carboxamide;
N-(2-bromophenyl)-4-[(*RR*,*SS*)-2-cyano-3-(2-methoxyphenyl)-2-methyl-3-(1-naphthyl)propanoyl]piperazine-1-carboxamide;
4-[(*RR*,*SS*)-2-cyano-3-(2-methoxyphenyl)-2-methyl-3-(1-naphthyl)propanoyl]-N-(4-methylphenyl)piperazine-1-carboxamide;
4-fluorophenyl 4-[(*RR*, *SS*)-2-cyano-3-(2-methoxyphenyl)-2-methyl-3-(1-naphthyl)propanoyl]piperazine-1-carboxylate;
phenyl 4-[(*RR*,*SS*)-2-cyano-3-(2-methoxyphenyl)-2-methyl)-3-(1-naphthyl)propanoyl]piperazine-1-carboxylate;
(*RR*,*SS*)-3-[4-(4-bromobenzoyl)piperazin-1-yl]-2-[(2-methoxyphenyl)(1-naphthyl)methyl]-2-methyl-3-oxopropanenitrile;
N-(4-chlorophenyl)-4-[(RR,SS)-2-cyano-3-(2-methoxyphenyl)-2-methyl-3-(1-naphthyl)propanoyl]piperazine-1-carboxamide;
methyl (2*E*)-2-cyano-3-(quinolin-3-yl)prop-2-enoate; and
pharmaceutically acceptable salts thereof.

In a further aspect this invention provides the aforementioned compounds excepting ethyl 2-cyano-3-(4-methylphenyl)-3-(1-naphthyl)propanoate or ethyl 2-cyano-3-(1-naphthyl)-3-(2-naphthyl)propanoate.

This invention also provides processes for preparing the novel compounds of formula I as defined herein, which processes comprise steps (a)-(d) or (e), (f), or (g):
(a) reacting a compound of formula 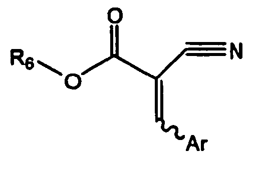 wherein R₆ is as defined hereinabove in connection with novel compounds of formula I excepting hydrogen and Ar is a group of formula (A), (B), or (C): 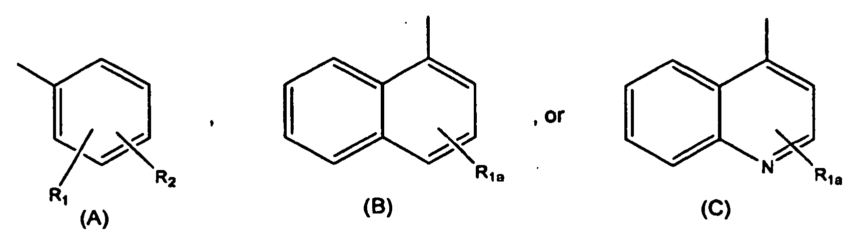 where R₁, R₂ and R₁ₐ are as defined hereinabove;
   with a compound of formula Ar₁MX wherein M is a metal such as Mg, X is a halogen such as Cl or Br, and Ar₁ is a group of formula (A) or (B) providing Ar and Ar₁ are not both of formula (A) or (B) to give a compound of formula I wherein R₄ is OR₆ where R₆ is defined immediately above excepting hydrogen and R₃ is hydrogen;
(b) alkylating a compound of formula: 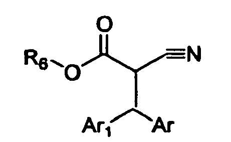 wherein R₆, Ar₁ and Ar are as defined above providing R₆ is other than hydrogen, with an alkylating agent of formula R₃L where L is a leaving group and R₃ is as defined in claim 1 excepting hydrogen to give a corresponding compound of formula I,
(c) hydrolyzing an ester of formula 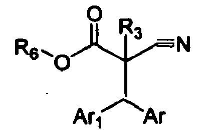 wherein Ar, Ar₁, R₃ and R₆ are as defined hereinabove providing R₆ is other than hydrogen, to give a corresponding compound of formula I wherein R₄ is OR₆ where R₆ is hydrogen;
(d) reacting an activated acid compound of formula: 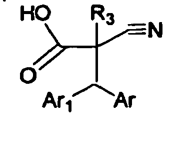 wherein Ar, Ar₁, and R₃ are as defined hereinabove, with an amine of formula HNR₅R₆, 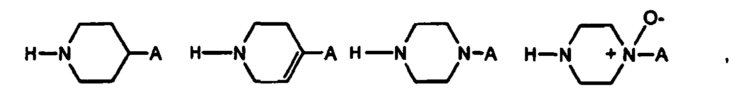 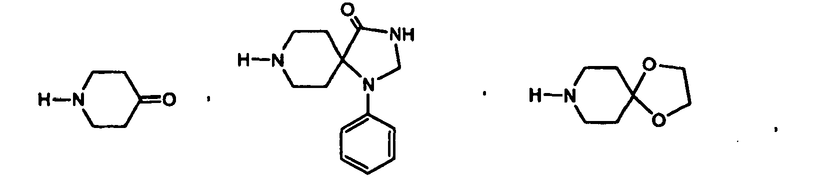 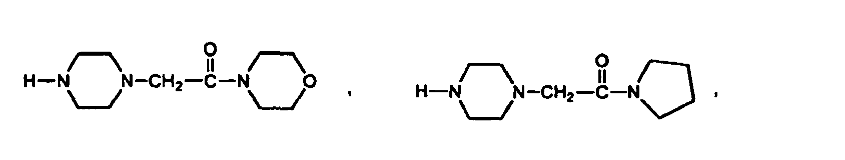 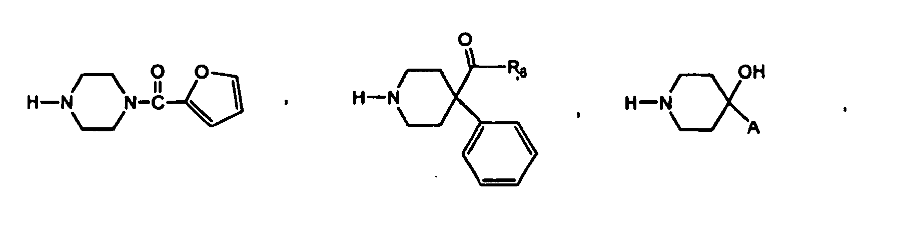 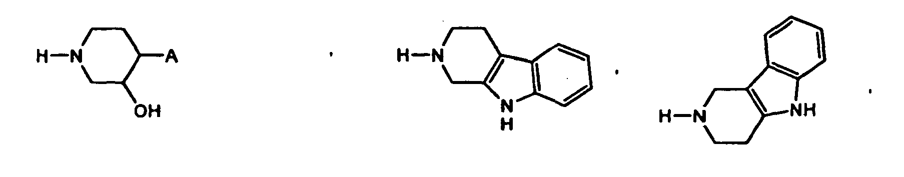 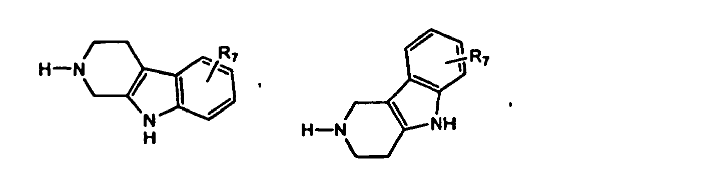 or
(e) reacting a compound of formula 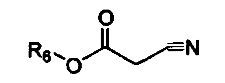 wherein R₆ is as defined hereinabove excepting hydrogen, in the presence of strong base with an halide of formula

   Ar₁ArCHX

   where Ar and Ar₁ are as defined herein and X is halogen to give a corresponding compound of formula I wherein R₃ is hydrogen,
   or
(f) converting a compound of formula I as defined hereinabove having a reactive substituent group or site to give a different compound of formula I; or
(g) converting a compound of formula I to a pharmaceutically acceptable salt thereof.

The compounds of the present invention can be prepared from commercially available starting materials, compounds known in the literature, or readily prepared intermediates, by employing standard synthetic methods and procedures known to those skilled in the art. Standard synthetic methods and procedures for the preparation of organic molecules and functional group transformations and manipulations can be readily obtained from the relevant scientific literature or from standard textbooks in the field. Although not limited to any one or several sources, classic texts such as Smith, M. B.; March, J. March's Advanced Organic Chemistry: Reactions, Mechanisms, and Structure, 5th ed.; John Wiley & Sons: New York, 2001; and Greene, T. W.; Wuts, P. G. M. Protective Groups in Organic Synthesis, 3rd ed.; John Wiley & Sons: New York, 1999 are useful and recognized reference textbooks of organic synthesis known to those in the art. The following synthetic schemes are designed to illustrate general procedures for the preparation of compounds of formula I.

The compounds used in the present invention may be prepared by the method described in *Scheme 1.*

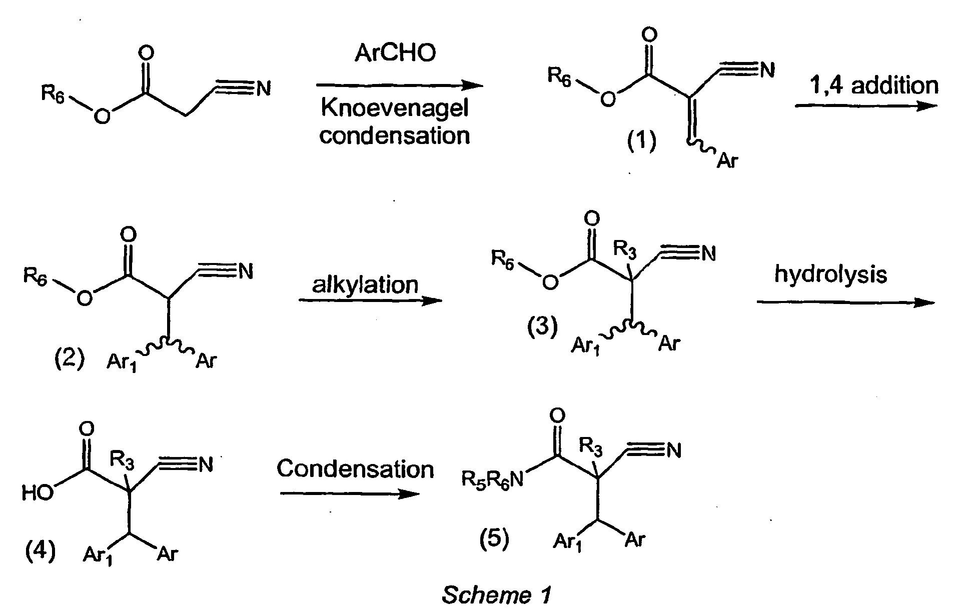

Condensation of a malonic acid derivative with an optionally substituted aromatic or heteroaromatic aldehyde or ketone in the presence of a catalyst provides the intermediate (1). This reaction is known to one skilled in the art as the Knoevenagel reaction. The catalyst can be an ammonium salt derived from an amine and a carboxylic acid. (G. Jones, Organic Reactions (1967) 15, 204; Y. Sumida, Polymer Journal (1981) 13, 521), Lewis acid such as titanium tetrachloride in pyridine (W. Lehnert, Tetrahedron Lett. (1970), 54, 4723) or a amine immobilized on a resin (J. Simpson, Tetrahedron Lett., (1999), 40, 7031). Michael addition of an aryl Grignard or Gilman reagent (N. Laitif, Egypt J. Chem (1974) 17, 879; C. Cativieia, Tetrahedron, (1994) 50, 9837) provides 2-cyano propionate (2). Alkylation of the anion of (2) with an alkyl halide or sulfonate (AA Fadda, Ind. J. Chem (1990) 29B, 171) provides ester (3). Subsequent hydrolysis of the ester under either acidic or basic conditions provides acid (4). Activation of the acid using reagents familiar to one skilled in the art, such as thionyl or oxalyl chloride, cyanuric chloride, pivaloyl chloride, diphenylphosphoryl azide, diethyl cyanophosphate, diethyl azodicarboxylate/triphenylphosphine, dicyclohexylcarbodimide, hydroxbenzotriazole, and subsequent reaction with an amine provides an amide.

In the case where the amine is piperazine an optionally substituted aryl or optionally substituted heteroaryl group can be attached to the piperazine ring using a Pd or Ni catalyst, with or without a ligand, a base such as sodium t-butoxide in a solvent and an aryl chloride, bromide, iodide sulfonate or siloxane (D. Baranano, Current Org. Chem. (1997) 1, 287; B. H. Yang, J. Organomet. Chem. (1999), 376, 125; E. Brenner, Tetrahedron (1999) 55, 12829). Alternatively a Cu catalyst, a base and an aryl chloride, bromide, iodide sulfonate, an aryl boronic acid or aryl siloxane (P.Y.S. Lam, J. Am Chem. Soc (2000)122, 7600; D.J. Cundy, Tetrahedron Letters (1998)39, 7979) can be used. The N-oxide of the substiruted piperazines can be prepared by oxidation of the basic nitrogen with reagents known to one skilled in the art (*e.g*., 3-chloro perbenzoic acid, trifluoroacetic peracid).

The amide can also be prepared directly from ester (3) by a modification of the Bodroux reaction in which the ester is converted directly into an amide by reaction with the magnesium salt of an amine (Dolling, Ulf H.; EP-A-599376) as shown in *Scheme 2*

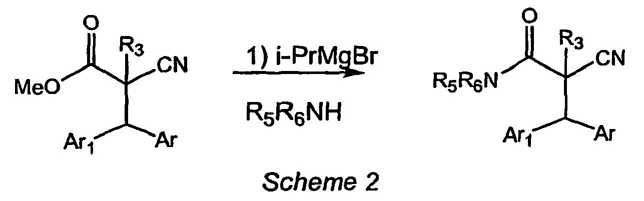

Another route to the substituted piperazines amides involves the synthesis of the piperazine amide and using a metal catalyzed coupling with a Pd or Ni catalyst, with or without a ligand, a base such as sodium t-butoxide in a solvent and an aryl chloride, bromide, iodide sulfonate or siloxane (D. Baranano, Current Org. Chem. (1997) 1, 287; B. H. Yang, J. Organomet. Chem (1999), 376, 125; E. Brenner, Tetrahedron (1999) 55, 12829). Alternatively a Cu catalyst, a base and an aryl chloride, bromide, iodide sulfonate, an aryl boronic acid or aryl siloxane (P.Y.S. Lam, J. Am Chem. Soc. (2000)122, 7600; D.J. Cundy, Tetrahedron Letters (1998)39, 7979) can be used.

In those cases where the amine used in the condensation reaction with acid, 4 , is an acylated piperazine, the piperazine, if not commercially available, was prepared by monoacylation of an excess of piperazine with an acid chloride, chloroformate or isocyanate in the presence of a base using methods known to one skilled in the art. Alternatively, the piperazine amide can be prepared by condensation of acid 4 with an excess of piperazine to generate a monoacyl piperazine. Subsequent reaction with activated (optionally substituted) (heterro) aryl acid derivatives, isocyanates, isothiocyanates or (optionally substituted) (hetero) aryl chloroformates under conditions familiar to one skilled in the art produces the disubstituted amide. This route is illustrated in *Scheme 3.*

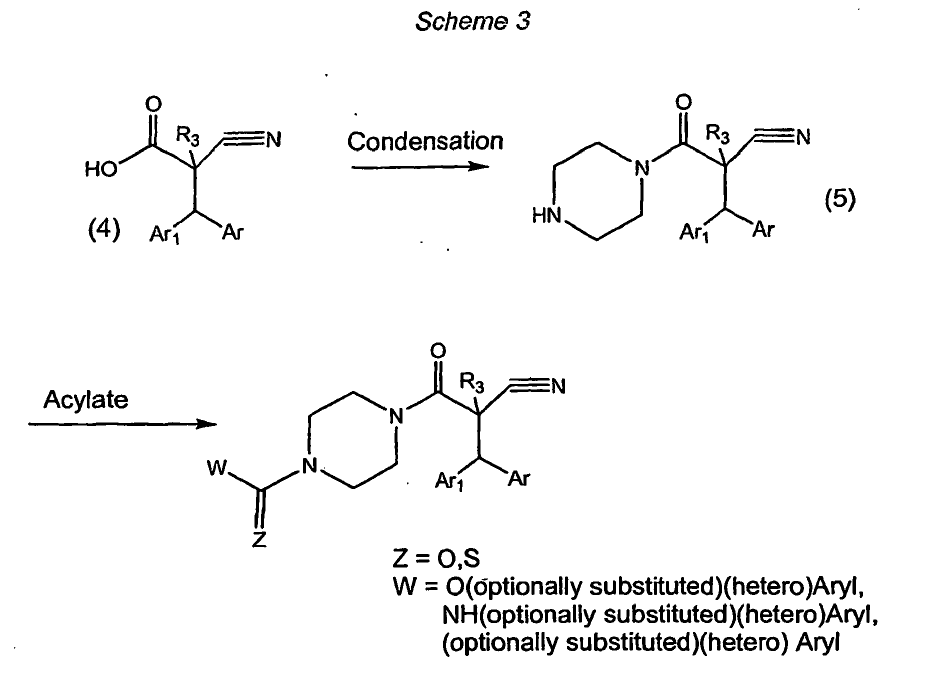

An alternative synthesis of the 3-(1-naphthyt)-3-phenyl-2-cyanopropanoic acid ester (intermediate 2), involves the synthesis of a naphthyl aryl methyl halide and its use in a subsequent alkylation of a cyanoacetic acid derivative. This route, based on the work of L. Voegtli (Helv. Chim Acta. 38 (1955) 46, is shown in *Scheme 4.*

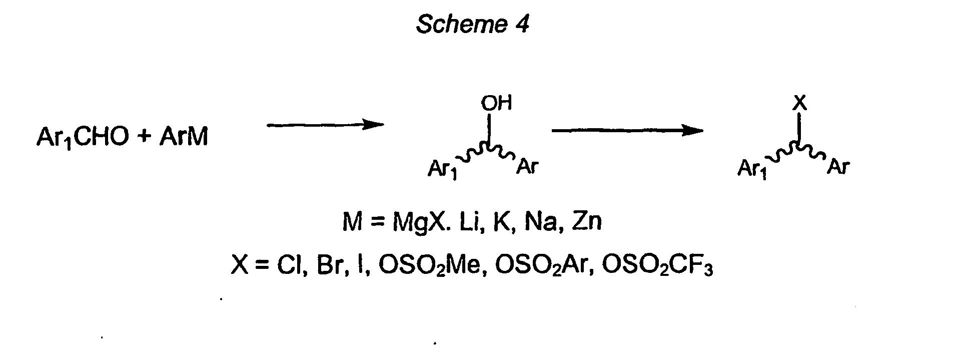

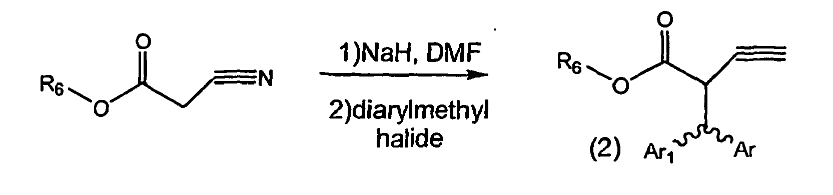

The alcohol could be converted into a leaving group familiar to one skilled in the art using methods familiar to one skilled in the art (eg Ph₃P/CCl₄, Ph₃P/CBr₄, methyltriphenoxy- phosphonium iodide, TsCl/Pyr, MsCI/TEA, Ac₂O/Pyr).

The substantially pure enantiomers of the compounds of formula I may be resolved by forming a ephedrine, cinchonidine, or quinidine salt of a compound of claim 1 or a pharmaceutically acceptable salt thereof; and extracting said ephedrine, cinchonidine, or quinidine salt with solvent. As used herein, "substantially pure enantiomer" means an enantiomer present it a mixture with other enantiomer at a proportion of at least about 90%, by weight, based on the total weight of all enantiomers of the compound, preferably at least about 95%, by weight, and, more preferably at least about 99%, by weight.

The compounds of this invention are useful in the treatment of the inflammatory component of diseases and are therefore particularly useful in treating atherosclerosis, myocardial infarction, congestive heart failure, arthritis, inflammatory bowel disease, type II diabetes, osteoarthritis, asthma and any other autoimmune disease in humans or other mammals which comprises administering to a human or other mammal an antiinflammatory effective amount of a compound of the present invention.

Representative compounds of this invention were evaluated in the following standard pharmacological test procedures that demonstrated the antiinflammatory activity for the compounds of this invention. The test procedures used and the results obtained are briefly described below.

### Test procedures:

### Cells

T-175 flasks of 100% confluent HAECT-1 cells (immortalized human aortic endothelial cells) were washed with 8 mL of HBSS (HEPES buffered saline solution) and infected for four hours with 6 mL of a 1:10 dilution of Ad5-wt-hERα virus (an adenovirus transfection vector that mediates CMV promoter driven expression of human ERα) in phenol red free Endothelial Cell Basal medium (Clonetics, San Diego CA, Catalog # CC-3129) containing 0.25% bovine serum albumin (EBM-BSA). After four hours, cells were washed with EBM-BSA and incubated overnight in the same medium. Following overnight incubation, cells were washed with EBM-BSA and infected for 2 hours with 6 mL of a 1:10 dilution of Ad5-3x(NFκB).Luc virus (Adenovirus luciferase expression vector driven by 3 repeats of the MHC NFκb site 5' to the thymidine kinase promoter) in EBM-BSA. After two hours, cells were washed and incubated at 34°C for 1 hour. Cells were then washed, trypsinized, counted and resuspended in 95%FBS / 5% dimethylsulfoxide at a concentration of 4x10⁶ cells/mL, frozen as 1 or 5 mL aliquots in cryo-vials and stored at -150 °C. Control (no ER infection) cells were processed as above without Ad5-wt-hERα virus infection.

A representative compound selected from the compounds of Examples 1-130 was evaluated in the test procedure described below.

### IL-6 and Creatine Kinase (CK)Test Procedure

ERα infected HAECT-1 cells or control cells were thawed, diluted 42x in warm EBM-BSA, plated into 96-well plates at 0.1 mL/well and incubated for 4 hours at 34 °C. Test compounds were added to the cells as 2x stocks in EBM-BSA containing 2 ng/mL IL-1β (R&D Systems) and the 96-well plates were returned to the incubator (34 °C). After 15-20 hours, 100 µL aliquots of media were removed from the cells and assayed for IL-6 content using a BioSource human IL-6 ELISA Kit. Cells were subsequently washed with 300 µL of Dulbecco's phosphate buffered saline and lysed in 50 µL of Cell Culture Lysis Reagent (Promega). Luciferase was determined on a Wallac Victor Luminometer (Gaithersburg, MD) using 10 µL of lysate and mixing with 100 µL of Promega Luciferase Assay reagent. Creatine kinase was determined from the rate of increase in A₃₄₀ following addition of 100 µL of CK assay reagent (Sigma, cat. No 47-10) to the remainder of the cell lysate.

### Data Analyses

For IC₅₀ and EC₅₀ calculations, mean IL-6, luciferase or CK values versus log₁₀ of the compound concentration were fitted to a four parameter logistic equation. The IC₅₀/ EC₅₀ value, 'Hill slope', upper and lower limits of the curve were iteratively estimated.

### Mice

Ovariectomized C57BU6 mice (16-20 g) (Taconic) were separated into groups of eight mice each. After 5-7 days of recuperation, the mice were fed a chow diet or an atherogenic diet (15.75% fat, 1.25% cholesterol and 0.5% sodium cholate) (Purina diet #21539). EE or test compound was administered once daily by gavage in a methylcellulose/tween vehicle (0.1 ml per mouse) for 5 weeks. At the end of the experimental period, the liver was collected and uterine wet weight was recorded.

### RNA Analysis

Liver total RNA was prepared by using Trizol reagent (BRL). Estrogen and compound regulation of NF-κB target genes were verified by real time reverse transcriptase-polymerase chain reaction (RT-PCR) using an ABI PRISM 7700 Sequence Detection System according to the manufacturer's protocol (Applied Biosystems). The data was analyzed using the Sequence Detector v1.7 software (Applied Biosystems) and normalized to GAPDH using the Applied Biosystems primer set.

The following table summarizes the results obtained in the standard pharmacological test procedures described above using a representative compound of this invention.

**Table 1:**

| Effects of tested compounds on ER/NF-κB, IL-6 and CK expression in Ad5-wt-ERα infected HAECT-1 cells | | | | | | |
|---|---|---|---|---|---|---|
| Example | ER/NFκB | | Interleukin-6 | | Creatine Kinase | |
| # | IC₅₀ (nM) | Efficacy (%)* | IC₅₀ (nM) | Efficacy (%) | EC₅₀ (nM) | Efficacy (%) |
| 67 | 43 | 90 | 35 | 45 | 222 | 41 |
| | 101 | 92 | | | 173 | 58 |
| | 153 | 100 | | | 84 | 41 |
| | 92 | 86 | | | | |
| | 222 | 100 | | | | |

| | | | | | | |
|---|---|---|---|---|---|---|
| *Efficacy values are relative to the maximal inhibition (ER/NF-κB or IL-6 test procedure) or stimulation (CK test procedure) observed with E2. | | | | | | |

E2 inhibits NF-κB and IL-6 expression in Ad5-wt-ER infected HAECT-1 cells with an IC₅₀ value around 1 nM and induces expression of creatine kinase in the same cells with similar potency (5.8 nM) (Table 1). In contrast, compounds of the present invention potently and efficaciously inhibit NF-_{K}B and IL-6 expression in Ad5-wt-ER infected HAECT-1 cells but do not induce CK expression (Table 1) in an ER-dependent manner. The ability of compounds of the present invention to inhibit NF-xB and IL-6 expression without inducing CK activity (as shown for a representative compound in Table 1) is demonstrates anti-inflammatory activity in the absence of classic estrogenic activity.

Representative compounds selected from the compounds of Examples 131-230 were evaluated in the test procedure described below.

### Test procedures:

### Cells

T-175 flasks of 100% confluent HAECT-1 cells (immortalized human aortic endothelial cells) were washed with 8 ml of HBSS (HEPES buffered saline solution) and infected for fours hours with 6 ml of a 1:10 dilution of Ad5-wt-hERα virus (an adenovirus transfection vector that mediates CMV promoter driven expression of human ERα) in phenol red free Endothelial Cell Basal medium (Clonetics, San Diego, CA, Catalog # CC-3129) containing 0.25% bovine serum albumin (EDM-BSA). After four hours, cells were washed with EDM-BSA and incubated overnight in the same medium. Following overnight incubation, cells were washed with EDM-BSA and infected for 2 hours with 6 ml of a 1:10 dilution of Ad5-3x(NFκB).Luc virus (Adenovirus luciferase expression vector driven by three repeats of the MHC NFκb sites 5' to the thymidine kinase promoter) in EDM-BSA. After two hours, cells were washed and incubated at 34 °C for one hour. Cells were then washed, trypsinized, counted, and resuspended in 95% FBS/5% dimethylsulfoxide at a concentration of 4x10⁸ cells/ml, frozen as 1 or 5 ml aliquots in cryo-vials and stored at -150°C. Control (no ER infection) cells were processed as above without Ad5-wt-hERα virus infection.

### IL-6 and Creatine Kinase Assays

ERα infected HAECT-1 cells or control cells were thawed, diluted 42x in warm EBM-BSA, plated into 96-well plates at 0.1 ml/well and incubated for 4 hours at 34. °C. Test compounds were added to the cells at 2x stocks in EBM-BSA containing 2 ng/ml IL-1*β* Ad6-IL-6(1250bp).Luc virus and plates were returned to the incubator (34 °C). After 15 to 10 hours, cells were lysed with 50 µl of Promega Cell Culture Lysis reagenet for about 5 minutes at room temperature on a shaker. After lysing, 15 µl of lysate is transferred to luminometer plates for luciferase determination. Luciferase activity is evaluated using a Perkin Elmer Victor2 1420 multilabel counter. Creatine kinase was determined from the rate of increase in A₃₄₀ following addition of 100 µl of CK assay reagent (Sigma catalog no. 47-10) to the remainder of the cell lysate.

### Data Analyses

For IC₅₀ and EC₅₀ calculations, mean IL-6, luciferase or CK values versus log₁₀ of the compound concentration were fitted to a four parameter logistic equation. The IC₅₀/EC₅₀ value, 'Hill slope,' upper and lower limits of the curve were iteratively estimated.

| Endothelial Cell ER/IL-6 Assay | | | | |
|---|---|---|---|---|
| | IL6luc | IL6luc | CK | CK |
| Example | IC₅₀ (nM) | %Efficacy (%) | EC₅₀ (nM) | %Efficacy (%) . |
| 131 | 15 | 119 | 212 | |
| 132 | 122 | 92 | 298 | 57 |
| 133 | 206 | 81 | | |
| 134 | 612 | 85 | | |
| 135 | 65 | 119 | | |
| 136 | 63 | 142 | | |
| 137 | 33 | 163 | | |
| 138 | 15 | 131 | 34 | 49 |
| 139 | 113 | 98 | | |
| 140 | 324 | 125 | | |
| 141 | 3.2 | 123 | 20 | 39 |
| 142 | 69 | 103 | | |
| 143 | 39 | 91 | 74 | 38 |
| 144 | 7 | 103 | 19 | 29 |
| 145 | 9.1 | 102 | | |
| 146 | 5.5 | 135 | | |
| 147 | 12 | | | |
| 148 | 257 | | | |
| 149 | 18 | 102 | | |
| 150 | | | | |
| 150 | 64 | | | |
| 151 | 5.5 | 141 | 13.5 | 30 |
| 152 | | | | |
| 152 | 19 | 130 | 85 | 14 |
| 153 | 149 | 150 | | 15 |
| 154 | 6 | 87 | | |
| 155 | 2 | 105 | 6 | 33 |
| 156 | 312 | 73 | | |
| 157 | 23 | 110 | | -100 |
| 158 | 4.5 | 121 | 55 | 23 |
| 159 | 273 | 95 | | |
| 162 | 546 | 126 | | -4 |
| 163 | 2.95 | -32 | | -12 |
| 165 | | 21 | | -22 |
| 166 | 353 | 77 | | -13 |
| 167 | 1229 | 72 | | -16 |
| 168 | 37 | 68 | | |
| 169 | 331 | 97 | | -8 |
| 170 | 577 | 89 | | |
| 171 | 337 | 54 | | |
| 172 | 114 | 149 | | -13 |
| 173 | 72 | 124 | | -13 |
| 174 | 1815 | 49 | | 34 |
| 175 | | | | |
| 176 | 413 | 71 | | |
| 177 | 1152 | 91 | | -32 |
| 178 | 94 | 137 | | -100 |
| 179 | 151 | 128 | | |
| 180 | 200 | 119 | | -6 |
| 181 | 389 | 104 | | 2 |
| 182 | 45 | 74 | | |
| 183 | 361 | 196 | 481 | 63 |
| 184 | 445 | 88 | | |
| 185 | 288 | 116 | | |
| 186 | 2461 | 60 | | 11 |
| 187 | 21 | 134 | 50 | 48 |
| 188 | 63 | 107 | | 39 |
| 189 | 348 | 103 | | |
| 190 | 113 | | | |
| 191 | 586 | | | |
| 192 | 44 | 176 | 415 | 61 |
| 193 | | | | |
| 194 | 132 | 150 | | |
| 195 | 710 | 74 | | |
| 196 | 70 | 142 | | |
| 197 | 39 | 46 | | |
| 198 | | | | |
| 199 | | | | |
| 200 | 157 | 162 | 320 | 13 |
| 201 | 1093 | 45 | | |
| 202 | | | | 9 |
| 202 | | 43 | | |
| 203 | | | | -26 |
| 203 | 9.93 | 28 | | |
| 204 | | | | -34 |
| 204 | 117 | 23 | | |
| 205 | 334 | 58 | | -35 |
| 206 | 345 | 83 | | |
| 207 | 4002 | 63 | | -16 |
| 208 | 13 | 50 | | |
| 209 | 87 | 116 | 311 | 2 |
| 210 | 16 | 132 | | |
| 211 | 268 | 78 | | |
| 212 | 59 | 113 | 138 | 22 |
| 213 | 5.6 | 103 | 64 | 26 |
| 214 | 276 | 136 | 315 | 12 |
| 215 | | | | |
| 216 | | 51 | | -21 |
| 217 | 203 | 12 | | -37 |
| 218 | 103 | 101 | | -12 |
| 219 | 1405 | 74 | | |
| 220 | 344 | 128 | | |
| 221 | 170 | 245 | 38 | 12 |
| 221 | 92 | 174 | 82 | 33 |
| 221 | 62 | 159 | | |
| 222 | | | | |
| 222 | 304 | 170 | 33 | 27 |
| 223 | 38 | 154 | 318 | 24 |
| 224 | 832 | 77 | | -14 |
| 225 | 1598 | 83 | | -12 |
| 226 | 220 | 115 | 138 | 18 |
| 160 | 1255 | 82 | | -10 |
| 161 | 798 | 122 | | 5 |
| 164 | 32 | 48 | | |

Based on the results obtained in the standard pharmacological test procedures, the compounds of this invention are selective antiinflammatory compounds described herein useful for the treatment and prevention of chronic inflammatory diseases without stimulating uterine and breast cell proliferation as found with classic estrogens.

Accordingly, the compounds of this invention are useful in treating or inhibiting osteoporosis and in the inhibition of bone demineralization, which may result from an imbalance in an individual's formation of new bone tissues and the resorption of older tissues, leading to a net loss of bone. Such bone depletion results in a range of individuals, particularly in post-menopausal women, women who have undergone bilateral oophorectomy, those receiving or who have received extended corticosteroid therapies, those experiencing gonadal dysgenesis, and those suffering from Cushing's syndrome. Special needs for bone, including teeth and oral bone, replacement can also be addressed using these compounds in individuals with bone fractures, defective bone structures, and those receiving bone-related surgeries and/or the implantation of prosthesis. In addition to those conditions described above, these compounds can be used in treatment or inhibition of osteoarthritis, hypocalcemia, hypercalcemia, Paget's disease, osteomalacia, osteohalisteresis, multiple myeloma and other forms of cancer having deleterious effects on bone tissues.

The compounds of this invention are also active in the brain and are therefore useful for inhibiting or treating Alzheimer's disease, cognitive decline, decreased libido, senile dementia, neurodegenerative disorders, depression, anxiety, insomnia, schizophrenia, and infertility. The compounds of this invention are also useful in treating or inhibiting benign or malignant abnormal tissue growth including, glomerulosclerosis, prostatic hypertrophy, uterine leiomyomas, breast cancer, scleroderma, fibromatosis, endometriosis, endometrial cancer, polycystic ovary syndrome, endometrial polyps, benign breast disease, adenomyosis, ovarian cancer, melanoma, prostate cancer, cancers of the colon, CNS cancers, such as glioma or astioblastomia.

The compounds of this invention are cardioprotective and are antioxidants, and are useful in lowering cholesterol, triglycerides, Lipoprotein (a) (Lp(a)), and low density lipoprotein (LDL) levels; inhibiting or treating hypercholesteremia, hyperlipidemia, cardiovascular disease, atherosclerosis, peripheral vascular disease, restenosis, and vasospasm, and inhibiting vascular wall damage from cellular events leading toward immune mediated vascular damage.

The compounds of this invention are also useful in treating disorders associated with inflammation or autoimmune diseases, including inflammatory bowel disease (Crohn's disease, ulcerative colitis, indeterminate colitis), arthritis (rheumatoid arthritis, spondyloarthropathies, osteoarthritis), pleurisy, ischemia/reperfusion injury (e.g. stroke, transplant rejection, myocardial infarction, etc.), asthma, giant cell arteritis, prostatitis, uveitis, psoriasis, multiple sclerosis, systemic lupus erythematosus and sepsis.

The compounds of this invention are also useful in treating or inhibiting ocular disorders including cataracts, uveitis, and macular degeneration and in treating skin conditions such as aging, alopecia, and acne.

The compounds of this invention are also useful in treating or inhibiting metabolic disorders such as disorders of lipid metabolism, appetite (e.g. anorexia nervosa and bulimia), or type-II diabetes.

Compounds in this invention are also useful in treating or inhibiting bleeding disorders such as hereditary hemorrhagic telangiectasia, dysfunctional uterine bleeding, and combating hemorrhagic shock.

The compounds of this invention are useful in disease states where amenorrhea is advantageous, such as leukemia, endometrial ablations, chronic renal or hepatic disease or coagulation diseases or disorders.

It is understood that the effective dosage of the active compounds of this invention may vary depending upon the particular compound utilized, the mode of administration, the condition, and severity thereof, of the condition being treated, as well as the various physical factors related to the individual being treated. It is projected that compounds of this invention will be administered at an oral daily dosage of from about 0.05 mg to about 30 mg per kilogram of body weight, preferably administered in divided doses two to six times per day, or in a sustained release form. For most large mammals, the total daily dosage is from about 3.5 mg to about 2100 mg, preferably from about 3.5 to about 5 mg. In the case of a 70 kg human adult, the total daily dose will generally be from about 3.5 mg to about 2100 mg and may be adjusted to provide the optimal therapeutic result.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances that may also act as flavoring agents, sweetening agents, lubricants, solubilizers, suspending agents, fillers, glidants, compression aids, binders, or tablet-disintegrating agents; it can also be an encapsulating material. In powders, the carrier is a finely divided solid that is in admixture with the finely divided active ingredient.

Solid dosage unit forms or compositions such as tablets, troches, pills, capsules, powders, and the like, may contain a solid carrier binder such as gum tragacanth, acacia, corn starch or gelatin; excipients such as dicalcium ,phosphate; a disintegrating agent such as corn starch, potato starch, alginic acid; a lubricant such as magnesium stearate; and a sweetening agent such as sucrose, lactose, or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both.

Liquid carriers are used in preparing liquid dosage forms such as solutions, suspensions, dispersions, emulsions, syrups, elixirs and pressurized compositions. The active ingredient can be dissolved or suspended in a pharmaceutically acceptable liquid carrier such as water, an organic solvent, a mixture of both, or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers, or osmo-regulators. Suitable examples of liquid carriers for oral and parenteral administration include water (partially containing additives as above, e.g. cellulose derivatives, preferably sodium carboxymethy, cellulose solution); alcohols, including monohydric alcohols such as ethanol and polyhydric alcohols such as glycols and their derivatives; lethicins, and oils such as fractionated coconut oil and arachis oil. For parenteral administration, the liquid carrier can also be an oily ester such as ethyl oleate and isopropyl myristate. Sterile liquid carriers are useful in sterile liquid form compositions for parenteral administration. The liquid carrier for pressurized compositions can be a halogenated hydrocarbon or other pharmaceutically acceptable propellant.

A liquid pharmaceutical composition such as a syrup or elixir may contain, in addition to one or more liquid carriers and the active ingredients, a sweetening agent such as sucrose, preservatives such as methyl and propyl parabens, a pharmaceutically acceptable dye or coloring agent, or a flavoring agent such as cherry or orange flavoring.

Liquid pharmaceutical compositions that are sterile solutions or suspensions can be administered intraocularly or parenterally, for example, by intramuscular, intraperitoneal or subcutaneous injection. Sterile solutions can also be administered intravenously. The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy injectability exists. It must be stable under the conditions of manufacture and storage and must be preserved against the contaminating action of microorganisms such as bacteria and fungi. The carrier can be a solvent or dispersion medium containing a liquid carrier, for example, water, ethanol, polyol (e.g. glycerol, propylene glycol and liquid polyethylene glycol), suitable mixtures thereof, and vegetable oils. The liquid carrier may be suitably mixed with a surfactant such as hydroxypropylcellulose.

The compounds of the present invention may also be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may be administered topically, or also transdermally through the use of a transdermal patch containing the active compound and a carrier that is inert to the active compound, which is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The following describes the preparation of representative compounds useful in this invention. Compounds described as homogeneous were determined to be 98% or greater a single peak (exclusive of enantiomers) by analytical reverse phase chromatographic analysis with 254 nM UV detection. Melting points are reported as uncorrected in degrees centigrade. The infrared data is reported as wavenumbers at maximum absorption, *vₘₐₓ*, in reciprocal centimeters, cm⁻¹. Mass spectral data is reported as the mass-to-charge ratio, m/z; and for high resolution mass spectral data, the calculated and experimentally found masses, [M+H]⁺, for the neutral formulae M are reported. Nuclear magnetic resonance data is reported as δ in parts per million (ppm) downfield from the standard, tetramethylsilane; along with the solvent, nucleus, and field strength parameters. The spin-spin homonuclear coupling constants are reported as J values in hertz; and the multiplicities are reported as a: s, singlet; d, doublet; t, triplet; q, quartet; quintet; or br, broadened. Italicized elements or groups are those responsible for the chemical shifts. "C NMR chemical shift assignments were made by reasonable comparison to a full chemical shift assignment determination for Example 1, Step a. The yields given below are for informational purposes and may vary according to experimental conditions or individual techniques.

### Example 1, Part1

### Ethyl (E)-2-cyano-3-(2,6-dimethoxyphenyl)prop-2-enoate

A solution of ethyl cyanoacetate (817mg,7.22mmol), 2,6-(dimethoxy)benzaldehyde (1g, 6.0 mmol), piperidinium acetate (0.430 mg, 3.0 mmol) in toluene (25mL) is refluxed for four hours under a Dean Stark trap. The reaction is cooled, diluted with isopropyl acetate (25mL) and washed with 1N HCI, saturated bicarbonate solution, and brine. The sample was evaporated to a solid. Recrystallization from ethanol provided the title compound as white crystals
mp 99-101 °C;
¹H NMR (DMSO-D6): δ 0.79 (t, *J*=7.08 Hz, 3H), 3.84 (s, 6H), 4.29 (q, *J*=7.08 Hz, 2 H), 6.77 (d, *J*=8.55 Hz, 2 H), 7.50 (t, *J*=8.55 Hz, 1H), 8.28 (s,1H)
MS (APCI) m/z [M+H]+ (262);
Anal. calcd for C₁₄H₁₅NO₄: C:64.36 H:5.79 N:5.36 Found: C:64.13 H:5.71 N:5.31.

### Example 1 Part 2

### Ethyl 2-cyano-3-(2,6-dimethoxyphenyl)-3-(1-naphthyl)propanoate

Ethyl (E)-2-cyano-3-(2,6-dimethoxyphenyl)prop-2-enoate (522 mg, 2 mmol) was disolved in 20 mL dry tetrahydrofuran (THF) and stirred under argon at room temperature while 0.25 M 1-naphthyl magnesium bromide (9.6 mL, 2.4 mmol) was added dropwise. The reaction was stirred 2 hrs., quenched with 1 N HCl, taken up in ethyl acetate, washed with NaHCO₃, brine, dried with MgSO₄, filtered, and evaporated. The crude reaction mixture was recrystallized from ethyl acetate/hexanes to yield 390 mg.
1H NMR (400 MHz, DMSO-D6) δ 0.79 (t, *J*=7.08 Hz, 1 H) 0.90 (t, *J*=7.08 Hz, 1H) 3.79 (d, *J*=10.49 Hz, 4 H) 3.92 (m, 1H) 5.18 (d, *J*=11.23 Hz, 1H) 5.26 (d, *J*=11.23 Hz, 1H) 5.84 (d, *J*=11.47 Hz, 1H) 5.94 (d, *J*=11.23 Hz, 1H) 6.60 (d, *J*=8.54 Hz, 1. H) 6.67 (d, *J*=8.30 Hz, 1H) 7.17 (t, *J*=8.30 Hz, 1H) 7.22 (t, *J*=8.42 Hz, 1H) 7.49 (m, 2 H) 7.72 (d, *J*=6.59 Hz, 1H) 7.80 (dd, *J*=17.33, 8.30 Hz, 1H) 7.89 (m, 1H) 8.30 (d, *J*=8.79 Hz, 1H)
MS (APCI) m/z 390 ([M+H]+);
MS (APCI) m/z 407 ([M+NH4]+);
Anal. calcd for C₂₄H₂₃NO₄: C:74.02 H:5.95 N:3.60 Found: C:73.73 H:5.78 N:3.57.

### Example 2

### Ethyl 2-cyano-3-(2,6-dichlorophenyl)-3-(1-naphthyl)propanoate

Ethyl (E)-2-cyano-3-(2,6-dichlorophenyl)prop-2-enoate (540 mg, 2 mmol) was dissolved in 20 mL. dry THF and stirred under argon at room temperature while 0.25 M 1-naphthyl magnesium bromide (9.6 mL, 2.4 mmol) was added dropwise. The reaction was stirred 2 hours quenched with 1 N HCI, taken up in ethyl acetate, washed with NaHCO₃, brine, dried with MgSO₄, filtered, and evaporated. The crude reaction mixture was purified on silica gel (20% ethyl acetate/hexanes) to yield 450 mg the title compound as a yellowish oil.
1H NMR (400 MHz, DMSO-D6) δ 0.97 (t, *J*=7.08 Hz, 1 H) 1.07 (t, *J*=7.08 Hz, 1H) 4.04 (m, 1H) 4.16 (dd, *J*=7.08, 4.15 Hz, 1H) 5.41 (d, *J*=9.27 Hz, 1H) 5.58 (d, *J*=11.23 Hz, 1H) 6.01 (d, *J*=9.03 Hz, 1H) 6.10 (d, *J*=11.47 Hz, 1H) 7.36 (m, 1H) 7.47 (m, 2H) 7.53 (d, *J*=7.81 Hz, 1H) 7.59 (t, *J*=7.81 Hz, 1H) 7.66 (d, *J*=8.30 Hz, 1 H) 7.74 (d, *J*=7.57 Hz, 1H) 7.81 (m, 1H) 7.90 (d, *J*=8.05 Hz, 1H) 7.95 (m, 1H)
MS (APCI) m/z 398 ([M+H]+);
MS (APCI) m/z 415 ([M+NH4]+);
Anal. calcd for C₂₂H₁₇Cl₂NO₂: C:66.34 H:4.30 N:3.52 Found: C:66.18 H:4.35 N:3.48.

### Example 3

### Ethyl 2-cyano-3-[4-(dimethylamino)phenyl]-3-(1-naphthyl)propanoate

Ethyl (E)-2-cyano-3-[4-(dimethylamino)phenyl)]prop-2-enoate (489 mg, 2 mmol) was disolved in 20 mL dry THF and stirred under argon at room temperature while 0.25 M 1-naphthyl magnesium bromide (9.6 mL, 2.4 mmol) was added dropwise. The reaction was stirred 2 hrs., quenched with 1 N HCl, taken up in ethyl acetate, washed with NaHCO₃, brine, dried with MgSO₄, filtered, and evaporated. The crude reaction mixture was purified on silica gel (20% ethyl acetate/hexanes) to yield 125 mg the title compound as a yellowish oil which became a hard foam upon high vaccum.
¹H NMR (400 MHz, DMSO-D6) δ 0.92 (dt, *J*=12.93, 7.08 Hz, 3 H) 2.79 (s, 3H) 2.82 (s, 3H) 4.01 (m, 1H) 5.19 (d, *J*=9.76 Hz, 0.5 H) 5.26 (d, *J*=9.27 Hz, 0.5 H) 5.38 (m, 1H) 6.58 (d, *J*=8.79 Hz, 1 H) 6.62 (d, *J*=8.79 Hz, 1H) 7.15 (d, *J*=8.79 Hz, 1H) 7.29 (m, 1.33 H) 7.49 (m, 3H) 7.58 (d, *J*=7.32 Hz, 0.5 H) 7.69 (d, *J*=7.08 Hz, 0.5 H) 7.88 (m, 3H) 8.12 (m, 0.66 H) 8.23 (d, J=8.30 Hz, 0.5 H)
MS (APCI) m/z 373 ([M+H]+);
Anal. calcd for C₂₄H₂₄N₂O₂: C:77.39 H:6.49 N:7.52 Found: C:77.31 H:5.90 N:6.90.

### Example 4

### Ethyl 2-cyano-3-(1-naphthyl)-3-[2-(trifluoromethyl)phenyl]propanoate

2-(trifluoromethyl)phenyl magnesium bromide (550 mg, 2 mmol) taken up in 10 mL dry THF with magnesium (59 mg, 2 mmol) overnight. To this was added ethyl (E)-2-cyano-3-(1-naphthyl)prop-2-enoate (502 mg, 2 mmol) in THF (10 mL). The reaction mixture allowed to stir overnight. The reaction was stirred 2 hrs., quenched with 1 N HCI, taken up in ethyl acetate, washed with NaHCO₃, brine, dried with MgSO₄, filtered, and evaporated. The crude reaction mixture was purified on silica gel (20% ethyl acetate/hexanes) to yield 692 mg of the title compound as a yellow oil.
¹H NMR (400 MHz, DMSO-D6) δ 0.76 (t, *J*=7.08 Hz, 1H) 0.92 (t, *J*=7.08 Hz, 1H) 3.87 (m, 1H) 4.02 (q, *J*=7.08 Hz, 0.5 H) 4.07 (q, *J*=7.08 Hz, 0.5 H) 5.23 (d, *J*=7.32 Hz, 0.5 H) 5.36 (d, *J*=8.30 Hz, 0.5 H) 5.87 (d, *J*=7.32 Hz, 0.5 H) 5.94 (d, *J*=8.30 Hz, 0.5 H) 7.27 (d, *J*=6.59 Hz, 0.5 H) 7.59 (m, 5H) 7.80 (m, 1.5 H) 7.94 (m, 3H) 8.13 (d, *J*=8.79 Hz, 0.5 H) 8.18 (d, *J*=7.81 Hz, 0.5 H).
MS (APCI) m/z 398 ([M+H]+);
Anal. calcd for C₂₃H₁₈F₃NO₂: C:69.52 H:4.57 N:3.52 Found: C:68.50 H:4.09 N:3.22.

### Example 5

### Ethyl 2-cyano-3-(2-isopropylphenyl)-3-(1-naphthyl)propanoate

2-(isopropyl)phenyl magnesium bromide (502mg, 2 mmol) taken up in 10 mL dry THF with magnesium (59 mg, 2 mmol) overnight. To this was added ethyl (E)-2-cyano-3-(1-naphthyl)prop-2-enoate (502 mg, 2 mmol) in THF (10 mL). The reaction mixture allowed to stir overnight. The reaction was stirred 2 hours, quenched with 1 N HCl, taken up in ethyl acetate, washed with NaHCO₃, brine, dried with MgSO₄, filtered, and evaporated. The crude reaction mixture was purified on silica gel (20% ethyl acetate/hexanes) to yield 302 mg of the title compound as a yellow oil. ¹H NMR (400 MHz, DMSO-D6) δ 0.66 (d, *J*=6.59 Hz, 1.66 H) 0.83 (t, *J*=7.08 Hz, 1.66 H) 0.93 (t, *J*=7.08 Hz, 0.33 H) 1.00 (d, *J*=6.59 Hz,0.33 H) 1.20 (d, *J*=6.83 Hz, 2.33 H) 1.26 (d, *J*=6.83 Hz, 0.66 H) 1.34 (t, *J*=7.20 Hz, 0.66 H) 2.94 (dt, *J*=13.54, 6.89 Hz, 0.66 H) 3.93 (qd, *J*=7.12, 1.59 Hz, 1.33 H) 4.05 (q, *J*=7.08 Hz, 0.66 H) 4.37 (q, *J*=7.08 Hz, 0.33 H) 5.14 (d, *J*=7.81 Hz, 0.33 H) 5.23 (d, *J*=8.05 Hz, 0.66 H) 5.82 (t, *J*=7.69 Hz, 1H) 7.11 (t, *J*=8.18 Hz, 0.33 H) 7.29 (m, 4H) 7.44 (t, *J*=7.81 Hz, 0.66 H) 7.55 (m, 2H) 7.69 (m, 2.33 H) 7.87 (t, *J*=8.42 Hz, 1H) 7.95 (d, *J*=8.30 Hz, 0.33 H) 7.99 (d, *J*=7.81 Hz, 1H) 8.07 (d, *J*=7.81 Hz, 0.66 H) 8.14 (d, *J*=7.32 Hz, 0.33 H) 8.20 (m, 1H)
MS (APCI) m/z 370 ([M-H]-);
Anal. calcd for C₂₅H₂₅NO₂: C:80.83 H:6.78 N:3.77 Found: C:79.13 H:6.07 N:3.94.

### Example 6

### Ethyl 2-cyano-3-(2,4-dimethoxyphenyl)-3-(1-naphthyl)propanoate

A solution of 2-cyano-3-(2,4-dimethoxy-phenyl)-acrylic acid ethyl ester (522 mg, 2 mmol) in THF (20mL) was treated dropwise with 1-naphthyl magnesium bromide (9.6mL, 2.4mmol) at room temperature. The reaction was stirred for 3 hours, quenched with 1 N HCl and diluted with ethyl acetate (40mL). The organic layer was separated and was washed sequentially with saturated aqueous sodium bicarbonate solution and brine. The organic layer was dried over Na₂SO₄, filtered and was concentrated *in vacuo.* Chromatography over silica gel (20% ethyl acetate/hexanes) yielded 421 mg of the title compound as an off white solid.
¹H NMR (400 MHz, DMSO-D6) δ 0.90 (td, *J*=7.08, 1.71 Hz, 3H) 3.68 (s, 1.5 H) 3.71 (s, 1.5 H) 3.84 (s, 1.5 H) 3.94 (s, 1.5 H) 4.00 (m, 2 H) 5.08 (d, J=8.30 Hz, 0.5 H) 5.20 (d, *J*=9.27 Hz, 0.5 H) 5.74 (d, *J*=8.54 Hz, 0.5 H) 5.78 (d, J=9.27 Hz, 0.5 H) 6.36 (dd, *J*=8.54, 2.44 Hz, 0.5 H) 6.45 (dd, *J*=8.66, 2.32 Hz, 0.5 H) 6.60 (dd, *J*=8.18, 2.32 Hz, 1H) 6.80 (d, *J*=8.54. Hz, 0.5 H) 7.20 (d, *J*=8.54 Hz, 0.5 H) 7.51 (m, 3 .5H) 7.64 (d, *J*=6.83 Hz, 0.5 H) 7.88 (m, 3.5 H) 8.06 (d, *J*=8.54 Hz, 0.5 H)
MS (APCI) m/z 390 ([M+H]+);
Anal. calcd for C₂₄H₂₃NO₄: C:74.02 H:5.95 N:3.60 Found: C:73.84 H:5.91 N:3.54.

### Example 7

### Ethyl 2-cyano-3-(2,5-dimethoxyphenyl)-3-(1-naphthyt)propanoate

The title compound was prepared according to example 6 using 2-cyano-3-(2,5-dimethoxy-phenyl)-acrylic acid ethyl ester as the starting material. This provided 309 mg of the title compound as a yellow powder.
1H NMR (400 MHz, DMSO-D6) δ 0.87 (t, *J*=7.20 Hz, 1.5 H) 0.91 (t, *J*=7.20 Hz, 1.5 H) 3.54 (s, 1.5 H) 3.62 (s, 1.5 H) 3.79 (s, 1.5 H) 3.88 (s, 1.5 H) 3.99 (m, 2 H) 5.16 (d, *J*=8.79 Hz, 0.5 H) 5.30 (d, *J*=9.52 Hz, 0.5 H) 5.81 (d, *J*=8.79 Hz, 0.5 H) 5.85 (d, *J*=9.76 Hz, 0.5 H) 6.54 (d, *J*=2.93 Hz, 0.5 H) 6.79 (ddd, *J*=8.79, 5.13, 3.17 Hz, 1 H) 6.97 (m, 1.5 H) 7.54 (m, 3 H) 7.67 (d, *J*=6.83 Hz, 0.5 H) 7.90 (m, 3 H) 8.13 (d, *J*=8.30 Hz, 0.5 H)
MS (APCI) m/z 390 ([M+H]+);
Anal. calcd for C₂₄H₂₃NO₄: C:74.02 H:5.95 N:3.60 Found: C:73.79 H:6.09 N:3.85.

### Example 8 - Part 1

### tert-Butyl (E)-2-cyano-3-[2-(trifluoromethyl)phenyl]-2-propenoate

A mixture of 2-trifluoromethyl benzaldehyde (13.19mL, 100mmol), t-butylcyanoacetate and piperazine resin (350mg) in toluene was heated under a Dan Stark trap for 2 hours. The reaction was cooled, diluted with ethyl acetate and filtered. The solution was concentrated *in vacuo* to yield the title compound as a clear oil.
¹H NMR (500 MHz,DMSO-D6 δ 1.52 (s, 9H), 7.78 (t, J=7.63 Hz, 1H), 7.88 (t, J= 7.79Hz,1H), 7.92 (d, J=7.79Hz,1H), 8.05 (d, J=7.63Hz, 1 H), 8.46 (d, J=1.36Hz,1H) MS (ESI) m/z 315 ([M+NH4]+);
Anal. calcd for C₁₅H₁₄F₃NO₂: C:60.61 H:4.75 N:4.71 Found: C:60.59 H:4.68 N:4.72.

### Example 8 - Part 2

### tert-Butyl 2-cyano-3-(1-naphthyl)-3-[2-(trifluoromethyl)phenyl]propanoate

The title compound was prepared according to example 6 using *tert*-butyl (E)-2-cyano-3-[2-(trifluoromethyl)phenyl]-2-propenoate prepared in part A as the starting material. This provided the title compound as a white foam.
1 H NMR (500 MHz, DMSO-D6) δ 1.08 (s, 4.5 H) 1.14 (s, 4.5 H) 5.09 (d, *J*=7.63 Hz, 0.5 H) 5.26 (d, *J*=7.94 Hz, 0.5 H) 5.84 (d, *J*=7.79 Hz, 0.5 H) 5.91 (d, *J*=7.94 Hz, 0.5 H) 7.23 (d, *J*=7.33 Hz, 0.5 H) 7.45 (m, 0.5 H) 7.54 (m, 1.5 H) 7.59 (m, 1.5 H) 7.67 (m, 1H) 7.71 (td, *J*=7.71, 1.22 Hz, 0.5 H) 7.81 (m, 1.5 H) 7.94 (m, 3 H) 8.13 (d, *J*=8.55 Hz, 0.5 H) 8.17 (d, *J*=7.94 Hz, 0.5 H)
MS (ESI) m/z 443 ([M+NH4]+);
Anal. calcd for C₂₅H₂₂F₃NO₂: C:70.58 H:5.21 N:3.29 Found: C:70.64 H:5.35 N:3.19.

### Example 9 Part 1

### tert-Butyl (E)-2-cyano-3-(1-naphthyl)prop-2-enoate

1-naphthaldehyde (17.4 mL, 128.06 mmol), *tert*-butyl cyano acetate (21.8 mL, 153.67 mmol), piperidinium acetate (64 mmol), toluene (250 mL), and ethanol (20mL) were combined in a flask fitted with a Dean-Stark apparatus and refluxed with removal of water for 4 hrs. The reaction was cooled, taken up in isopropyl acetate, washed twice with 1 N HCl, once with saturated aqueous NaHCO₃, brine, dried with MgSO₄, filtered, and evaporated. Reaction yielded 33g of the title compound as an oil. The oil crystallized (thick needles) after standing for a prolonged period (4 months) at room temperature.
mp 51-53 °C;
1H NMR (400 MHz, DMSO-D6) δ 1.55 (s, 9 H) 7.62 (m, 3 H) 8.00 (m, 2 H) 8.10 (s, 1 H) 8.12 (s, 1 H) 8.95 (s, 1 H)
MS (APCI) m/z 280 ([M+H]+);
Anal. calcd for C₁₈H₁₇NO₂: C:77.40 H:6.13 N:5.01 Found: C:76.30 H:5.95 N:4.81.

### Example 9 Part 2

### tert-Butyl 2-cyano-3,3-di(1-naphthyl)propanoate

*Tert*-butyl (E)-2-cyano-3-(1-naphthyl)prop-2-enoate (559 mg, 2 mmol) was disolved in 15 mL dry THF and stirred under argon at room temperature while 0.25 M 1-naphthyl magnesium bromide (9.6 mL, 2.4 mmol) was added dropwise. The reaction was stirred 2 hrs., quenched with 1 N HCl, taken up in ethyl acetate, washed with NaHCO₃, brine, dried with MgSO₄, filtered, and evaporated. The crude reaction mixture was purified via crystallization from ethanol to yield 480 mg of the title compound as a crystalline solid.
mp 156-157 °C;
1H NMR (400 MHz, DMSO-D6) δ 1.06 (s, 9 H) 5.26 (d, *J*=8.06 Hz, 1 H) 6.27 (d, *J*=8.06 Hz, 1 H) 7.31 (dd, *J*=7.32, 1.22 Hz, 1 H) 7.40 (m, 2 H) 7.47 (m, 1 H) 7.61 (m, 2 H) 7.71 (ddd, *J*=8.48, 6.90, 1.46 Hz, 1 H) 7.90 (m, 5 H) 8.00 (dd, *J*=8.18, 1.10 Hz, 1 H) 8.39 (d, *J*=8.54 Hz, 1H)
MS (APCI) m/z 406 ([M-H]-);
Anal. calcd for C₂₈H₂₅NO₂: C:82.53 H:6.18 N:3.44 Found: C:81.44 H:5.93 N:3.39.

### Example 10

### Ethyl 2-cyano-3-(2-methoxyphenyl)-3-(1-naphthyl)propanoate

The title compound was prepared according to example 6 using 2-cyano-3-naphthalen-1-yl-acrylic acid ethyl ester and 2-methoxyphenyl magnesium bromide as starting materials. This provided the title compound as a low melting solid which NMR shows to be a 50:50 mixture of diastereomers
1H NMR (400 MHz, DMSO-D6) δ 0.88 (m, 3 H) 3.84 (s, 1.5 H) 3.94 (s, 1.5 H) 3.99 (m, 2 H) 5.15 (d, *J*=8.30 Hz, 0.5 H) 5.25 (d, *J*=9.27 Hz, 0.5 H) 5.84 (d, *J*=8.30 Hz, 0.5 H) 5.88 (d, *J*=9.52 Hz, 0.5 H) 6.78 (td, *J*=7.44, 0.98 Hz, 0.5 H) 6.88 (td, *J*=7.38, 0.85 Hz, 0.5 H) 6.95 (dd, *J*=7.69, 1.59 Hz, 0.5 H) 7.05 (t, *J*=7.69 Hz, 1H) 7.23 (ddd, *J*=15.68, 7.99, 1.71 Hz, 1 H) 7.35 (dd, *J*=7.81, 1.71 Hz, 0.5 H) 7.52 (m, 3 H) 7.60 (d, *J*=7.81 Hz, 0.5 H) 7.65 (d, *J*=7.08 Hz, 0.5 H) 7.83 (d, *J*=8.30 Hz, 0.5 H) 7.90 (m, 3 H) 8.11 (d, *J*=8.79 Hz, 0.5 H)
MS (EI) m/z M+. (359);
Anal. calcd for C₂₃H₂₁NO₃: C:76.86 H:5.89 N:3.90 Found: C:76.51 H:5.85 N:3.83.

### Example 11

### Ethyl (RR,SS)-2-cyano-3-(2-methoxyphenyl)-3-(1-naphthyl)propanoate

Fractional crystallization of the solid from example 10 in ethyl acetate/hexanes provided colorless needles of the title compound. mp 123-126 °C;
1H NMR (500 MHz, DMSO-D6) δ 0.89 (t, *J*=7.10 Hz, 3 H) 3.94 (s, 3 H) 4.01 (ddd, *J*=14.16, 7.06, 1.07 Hz, 2 H) 5.15 (d, *J*=8.40 Hz, 1 H) 5.85 (d, *J*=8.55 Hz, 1 H) 6.78 (td, *J*=7.52, 0.99 Hz, 1 H) 6.96 (dd, *J*=7.64, 1.68 Hz, 1 H) 7.06 (dd, *J*=8.25, 0.92 Hz, 1 H) 7.22 (ddd, *J*=7.33, 1.83, 0.61 Hz, 1 H) 7.48 (m, 2 H) 7.59 (dd, *J*=8.17, 7.41 Hz, 1 H) 7.91 (m, 4 H)
MS (ESI) m/z 377 ([M+NH4]+);
Anal. calcd for C₂₃H₂₁NO₃ . 0.15 H2O: C:76.29 H:5.93 N:3.87 Found: C:76.33 H:5.82 N:3.70.

### Example 12

### tert-Butyl 2-cyano-3-(2-isopropylphenyl)-3-(1-naphthyl)propanoate

A mixture of Cul (681 mg, 3.58mmol) in THF (10mL), cooled to 0°, was treated with a cold, 0°, solution of 2-isopropyl lithium (from 1.43g of 1-bromo-2-isopropylbenzene and nBuLi (4.48mL, 1.6M in hexane) in THF (10mL). This mixture was added to a cold solution of 2-cyano-3-naphthalen-1-yl-acrylic acid *tert*-butyl ester in THF (10mL). The reaction was stirred and allowed to warm to room temperature over an hour. The reaction was quenched with ammonium chloride and diluted with either. The aqueous layer was washed twice with ether and the organic layers were combined and washed with saturated ammonium chloride and brine. The sample was dried over Na₂SO₄ and was filtered and evaporated to a solid. Trituration of the solid with provided the title compound as a white powder.
1H NMR (500 MHz, DMSO-D6) δ 0.64 (d, *J*=6.72 Hz, 1.5 H) 1.00 (d, *J*=6.72 Hz, 1 H) 1.11 (s, 4.5 H) 1.18 (s, 4.5 H) 1.22 (d, *J*=6.72 Hz, 1.5 H) 1.27 (d, *J*=6.57 Hz, 1 H) 2.96 (m, 0.5 H) 5.00 (d, *J*=8.25 Hz, 0.5 H) 5.11 (d, *J*=7.94 Hz, 0.5 H) 5.77 (d, *J*=7.94 Hz, 0.5 H) 5.78 (d, *J*=8.09 Hz, 0.5 H) 7.12 (t, *J*=8.09 Hz, 0.5 H) 7.31 (m, 3.5 H) 7.44 (t, *J*=7.71 Hz, 0.5 H) 7.52 (m, 1 H) 7.59 (t, *J*=7.41 Hz, 0.5 H) 7.70 (m, 0.5 H) 7.75 (m, 0.5 H) 7.87 (t, *J*=8.78 Hz, 1 H) 7.95 (d, *J*=7.94 Hz, 0.5 H) 8.01 (m, 1 H) 8.22 (d, *J*=8.70 Hz, 0.5 H)
MS (ESI) m/z 417 ([M+NH4]+);
Anal. calcd for C₂₇H₂₉NO₂: C:81.17 H:7.32 N:3.51 Found: C:81.05 H:7.34 N:3.49.

### Example 13

### tert-Butyl 2-cyano-3-(2-methoxyphenyl)-3-(1-naphthyl)propanoate

*Tert*-butyl (E)-2-cyano-3-(1-naphthyl)prop-2-enoate (2.23g, 8 mmol) was dissolved in 40 mL dry THF and stirred under argon at room temperature while 1 M 2-methoxyphenyl magnesium bromide (9.6 mL, 9.6 mmol) was added dropwise. The reaction was stirred 2 hrs., quenched with 1 N HCl, taken up in ethyl acetate, washed with NaHCO₃, brine, dried with MgSO₄, filtered, and evaporated. The crude reaction mixture was purified on silica gel (20% ethyl acetate/hexanes) to yield a yellowish oil which recrystallized from ethanol to yield 1.661 g product as a crystalline polymorphic solid.
1 H NMR (400 MHz, DMSO-D6) δ 1.04 (s, 4.5 H) 1.09 (s, 4.5 H) 3.83 (s, 1.5 H) 3.95 (s, 1.5 H) 5.06 (d, *J*=8.79 Hz, 0.5 H) 5.11 (d, *J*=10.49 Hz, 0.5 H) 5.81 (d, *J*=8.79 Hz, 0.5 H) 5.86 (d, *J*=10.74 Hz, 1.5H) 6.80 (t, *J*=7.08 Hz, 0.5 H) 6.92 (t, *J*=7.44 Hz, 0.5 H) 7.03 (m, 1.5 H) 7.23 (m, 1 H) 7.50 (m, 2 H) 7.59 (m, 1 H) 7.65 (d, *J*=6.59 Hz, 0.5 H) 7.82 (d, *J*=8.05 Hz, 0.5 H) 7.92 (m, 3 H) 8.22 (d, *J*=8.54 Hz, 0.5 H)
MS (APCI) m/z 388 ([M+H]+);
Anal. calcd for C₂₅H₂₅NO₃: C:77.49 H:6.50 N:3.61 Found: C:77.40 H:6.25 N:3.57

### Example 14

### tert-Butyt 2-cyano-3-(1-naphthyl)-3-[2-(trifluoromethoxy)phenyl]propanoate

The title compound was prepared according to example 6 using 2-cyano-3-(2-trifluoromethoxy-phenyl)-acrylic acid *tert*-butyl ester as the starting material. The crude product was chromatographed on silica using 25% ethyl acetate/ hexanes. Recrystallization from methylene chloride/ hexane provided the title compound as a crystalline solid.
mp 115-118 °C
1 H NMR (500 MHz, DMSO-D6) δ 1.04 (s, 5 H) 1.16 (s, 3 H) 5.19 (d, *J*=9.32 Hz, 0.4 H) 5.25 (d, *J*=9.47 Hz, 0.6 H) 5.76 (d, *J*=9.16 Hz, 0.4 H) 5.80 (d, *J*=9.62 Hz, 0.6 H) 7.46 (m, 6.4 H) 7.65 (ddd, *J*=8.44, 6.99, 1.22 Hz, 0.6 H) 7.82 (d, *J*=7.18 Hz, 0.4 H) 7.87 (dd, *J*=5.65, 3.66 Hz, 0.6 H) 7.91 (d, *J*=8.09 Hz, 0.4 H) 7.97 (d, *J*=7.94 Hz, 1 H) 7.99 (dd, *J*=7.56, 1.91 Hz, 0.6 H) 8.04 (d, *J*=8.40 Hz, 0.4 H) 8.19 (d, *J*=8.70 Hz, 0.6 H)
MS (ESI) m/z 440 ([M-H]-);
Anal. calcd for C₂₅H₂₂F₃NO₃: C:68.02 H:5.02 N:3.17 Found: C:67.88 H:5.04 N:3.11.

### Example 15

### Ethyl 2-cyano-3-(1-naphthyl)-3-phenylpropanoate

A solution of 2-cyano-3-naphthalen-1-yl-acrylic acid ethyl ester (0.502mg,2mmol) in THF(15mL) was treated with phenyl magnesiun bromide (1.2mL,2M in THF) dropwise with stirring under nitrogen. After one hour the reaction was quenched with 1N HCl. The solution was diluted with isopropyl acetate (15mL) and the organic layer was isolated. The organic layer was washed with bicarbonate solution and brine. Chromatography over silica gel (15% MTBE/hexane) provided the title compound as an oil from which solidified on standing. NMR suggests a 6:1 ratio of diastereomers.
1H NMR (400 MHz, DMSO-D6) δ 0.89 (dt, *J*=9.70, 7.11 Hz, 3 H) 4.00 (ddd, *J*=14.28, 7.08, 1.10 Hz, 2 H) 5.33 (d, *J*=9.76 Hz, 0.8 H) 5.40 (d, *J*=9.76 Hz, 0.2 H) 5.51 (d, *J*=9.76 Hz, 0.8 H) 5.55 (d, *J*=9.76 Hz, 0.2 H) 7.25 (m, 3.2 H) 7.40 (d, *J*=7.08 Hz, 1.8 H) 7.52 (m, 2.6 H) 7.60 (t, *J*=7.81 Hz, 1 H) 7.72 (d, *J*=7.08 Hz, 0.2 H) 7.84 (d, *J*=8.79 Hz, 0.2 H) 7.90 (d, *J*=8.05 Hz, 1 H) 7.94 (m, 1.8 H) 8.18 (m, 0.8 H) 8.30 (d, *J*=7.57 Hz, 0.2 H)
MS (EI) m/z M+. (329);
Anal. calcd for C₂₂H₁₉NO₂: C:80.22 H:5.81 N:4.25 Found: C:80.03 H:5.94 N:4.21.

### Example 16

### Ethyl 2-cyano-3,3-bis(2-methoxyphenyl)propanoate

A solution of 2-cyano-3-(2-methoxy-phenyl)-acrylic acid ethyl ester (2mmol) in THF (10mL) is treated with 2-methoxyphenyl magnesium bromide (2.4mmol,1M THF) at room temperature. The reaction is stirred for 3 hours, quenched with 1 N HCl and diluted with isopropyl acetate (15mL). The aqueous layer is removed and the organic layer is washed with bicarbonate then brine and is dried over Na₂SO₄. Evaporation of the sample yields an oil which is chromatographed on silica gel (25% ethyl acetate hexane) to yield the title compound as a viscous oil.
1 H NMR (DMSO-D6): δ 0.93 (t, J=7.20 Hz, 3 H), 3.71 (s, 3 H), 3.79 (s, 3H), 4.00 (ddd, J=14.15 Hz, 7.08 Hz, 1.22 Hz, 2H), 4.98 (d, J=8.79 Hz, 1H), 5.35 (d, J=8.79 Hz, 1H), 6.85 (td, J=7.44 Hz, 0.98 Hz, 1H), 6.97 (m, 3H), 7.07 (dd, J=7.57 Hz, J=1.71 Hz, 1H), 7.24 (m, 2H), 7.45 (dd, J=7.57 Hz, 1.46 Hz, 1H)
MS (APCI) m/z [M+NH4]+ (357);
Anal. calcd for C₂₀H₂₁NO₄: C:70.78 H:6.24 N:4.13 Found: C:70.64 H:6.07 N:4.03.

### Example 17

### Ethyl 2-cyano-3-(1-naphthyl)-3-(2-nitrophenyl)propanoate

The title compound was prepared using 2-cyano-3-(2-nitro-phenyl)-acrylic acid ethyl
ester according to example 6.The title compound was a sticky viscous oil
1 H NMR (DMSO-D6): 0.92 (td, J=7.08 Hz, 3.66 Hz, 2.5 H), 1.05 (t, J=7.08 Hz, 0.5 H), 3.44 (m, 0.5H), 4.02 (m, 1.5H), 4.35 (t, J=5.13 Hz, 0.5H), 5.41 (d, J=2.20 Hz, 0.5H), 5.43 (d, J=2.93 Hz, 0.5H), 6.25 (t, J=8.18 Hz, 1H), 7.41 (m, 1.5H), 7.50 (m, 2.5H), 7.61 (m, 1.5H), 7.82.(m, 0.5 H), 7.91 (d, J=8.05 Hz, 1.5H), 7.96 (m, 1.5 H), 8.01 (dd, J=8.05 Hz, 1.22 Hz, 0.5 H), 8.05 (d, J=8.30 Hz, 0.5H), 8.10 (dd, J=8.05 Hz, 1.22 Hz, 0.5H)
MS (APCI) m/z 375 ([M+H]+);
Anal. calcd for C₂₂H₁₈N₂O₄: C:70.58 H:4.85 N:7.48 Found: C:69.19 H:4.87 N:6.97.

### Example 18

### tert-Butyl 2-cyano-3-(2,6-dimethylphenyl)-3-(1-naphthyl)propanoate

2,6 (Dimethyl)phenyl magnesium bromide (50mL , 1.0M in THF) was added dropwise to a a stirred solution of 2-cyano-3-naphthalen-1-yl-acrylic acid *tert*-butyl ester (11.6g, 41.5mmol) in THF (170mL) under N₂. The reaction was stirred overnight during which time a precipitate formed. The reaction was then treated with HCl (250ml, 1 N) and diluted with ethyl acetate (500mL). The organic layer was separated and the aqueous layer was extracted with ethyl acetate ((250mL). The organic layers were combined and were washed with saturated brine (250mL), dried over Na₂SO₄ and concentrated *in vacuo* to yield 16.1 g of a solid. Recrystallization of the crude product yielded 12.4 g of the title compound
mp 124-128 °C;
1H NMR (500 MHz, DMSO-D6) δ 1.23 (s, 9 H) 1.33 (s, 6 H) 5.10 (d, *J*=6.57 Hz, 1 H) 5.44 (d, *J*=11.76 Hz, 0.5 H) 5.58 (d, *J*=6.87 Hz, 1.5 H) 7.00 (s, 1 H) 7.07 (m, 1 H) 7.35 (m, 1H) 7.44 (m, 1 H) 7.53 (m, 0.5 H) 7.59 (t, *J*=7.79 Hz, 1 H) 7.64 (d, *J*=7.48 Hz, 0.5 H) 7.87 (t, *J*=6.95 Hz, 1 H) 7.92 (dd, *J*=11.53, 8.17 Hz, 2H)
MS (ESI) *m*/*z* 403 ([M+NH4]+); Anal. calcd for C₂₆H₂₇NO₂: C:81.01 H:7.06 N:3.63 Found: C:80.67 H:7.08 N:3.55.

### Example 19

### tert-Butyl (RR,SS)-2-cyano-3-(1-naphthyl)-3-[2-(trifluoromethyl)phenyl]propanoate

A solution of 2-(trifluoromethyl)phenyl magnesium bromide in THF was prepared (*J. Med. Chem.* 33 (1990) 1452) this material was used in the procedure described in example 18 to yield the title compound as a reddish solid. Chromatography over SiO₂ using 105 to 40% ethyl acetate/hexanes yielded the title compound as a white solid.
mp 120.5-125.0 °C; 1H NMR (500 MHz, DMSO-D6) δ 1.08 (s, 5 H) 1.14 (s, 3 H) 5.09 (d, *J*=7.79 Hz, 0.4 H) 5.26 (d, *J*=7.94 Hz, 0.6 H) 5.85 (d, *J*=7.79 Hz, 0.4 H) 5.91 (d, *J*=7.79 Hz, 0.6 H) 7.23 (d, *J*=7.18 Hz, 0.5 H) 7.45 (t, *J*=7.64 Hz, 0.5 H) 7.56 (m, 3 H) 7.69 (m, 1.5 H) 7.81 (m, 1.5 H) 7.94 (m, 3 H) 8.15 (dd, *J*=16.49, 8.25 Hz, 1 H); MS (ESI) *m*/*z* 443 ([M+NH4]+); Anal. calcd for C₂₅H₂₂F₃NO₂: C:70.58 H:5.21 N:3.29 Found: C:70.62 H:5.14

### Example 20

### tert-Butyl (RR,SS)-2-cyano-3-(2-methoxyphenyl)-2-methyl-3-(1-naphthyl)propanoate

A solution of 2-cyano-3-(2-methoxy-phenyl)-3-naphthalen-1-yl-propionic acid *tert*-butyl ester in THF (25mL) was treated at room temperature with potassium bistrimethylsilyl amide (3.64mL, 2.4mmol, 0.66M in toluene). The reaction was stirred for two hours and then methyl iodide was added (0.5mL, 8mmol). The reaction was stirred at room temperature for one hour and was quenched with 1 N HCl. The reaction was diluted with ethyl acetate and the organic layer was isolated. The organic layer was washes with saturated sodium bicarbonate solution and brine. The organic layer was dried over Na₂SO₄, filtered and was concentrated *in vacuo*. Trituration of the resulting solid with ethanol provided 700mg of the title compound as a white powder.
1H NMR (400 MHz, DMSO-D6) δ 1.02 (s, 9 H) 1.62 (s, 3 H) 4.04 (s, 3 H) 5.76 (s, 1 H) 6.83 (td, *J*=7.50, 1.10 Hz, 1 H) 7.21 (m, 3 H) 7.47 (m, 2 H) 7.60 (t, *J*=7.32 Hz, 1 H) 7.89 (m, 3 H) 8.06 (d, *J*=7.08 Hz, 1H)
MS (APCI) m/z 402 ([M+H]+);
Anal. calcd for C₂₆H₂₇NO₃: C:77.78 H:6.78 N:3.49 Found: C:77.41 H:6.84 N:3.67.

### Example 21 Part 1

### (-) Ethyl (SS)2-cyano-3-(2-methoxyphenyl)-2-methyl-3-(1-naphthyl)propanoate

A sample of ethyl (*RR,SS*)cyano-3-(2-methoxyphenyl)-2-methyl-3-(1-naphthyl)propanoate was dissolved in methanol and was resolved by chromatography on a preparative HPLC system using a Chiralcel OD (25 x 5cm) column and 8:2 methanol:water as the eluant. The title compound was the first peak to elute. Recrystallization from ethanol provided the title compound as colorless crystals.
mp 134.5-135.5 °C;
[α]_{D}²⁵ = -297.79° (1%, CHCl₃);

### Example 21 Part 2

### (+) Ethyl (R,R)-2-cyano-3-(2-methoxyphenyl)-2-methyl-3-(1-naphthyl)propanoate

The title compound was the second peak to elute in example 21 part 1. Recrystallization from ethanol provided the title compound as colorless crystals. mp 134.5-135.5 °C;
[α]_{D}²⁵ = +289.80° (1%, CHCl₃);

### Example 22

### Ethyl (RR,SS)-2-cyano-2-[(2-methoxyphenyl)(1-naphthyl)methyl]-4-pentenoate

The title compound was prepared according to example 20 using allyl bromide. Recrystallization of the crude product from methanol provided the title compound as an off white solid.
1 H NMR (500 MHz, DMSO-D6) δ 0.73 (t, *J*=7.10 Hz, 3 H) 2.43 (dd, *J*=13.74, 6.72 Hz, 1 H) 2.94 (dd, *J*=13.90, 7.94 Hz, 1 H) 3.92 (m, 2 H) 4.06 (s, 3 H) 5.18 (dd, *J*=11.07, 1.60 Hz, 1 H) 5.21 (d, *J*=3.05 Hz, 1 H) 5.71 (m, 1 H) 5.88 (s, 1 H) 6.84 (t, *J*=7.02 Hz, 1 H) 7.16 (d, *J*=8.25 Hz, 1 H) 7.25 (m, 2 H) 7.47 (m, 2 H) 7.58 (m, 1 H) 7.85 (d, *J*=8.25 Hz, 1 H) 7.88 (m, 1 H) 7.93 (m, 1 H) 8.08 (d, *J*=7.02 Hz, 1H)
MS (ESI) m/z 417 ([M+NH4]+);
Anal. calcd for C₂₆H₂₅NO₃: C:78.17H:6.31 N:3.51 Found: C:78.53 H:6.31 N:3.48.

### Example 23

### Ethyl (RR,SS)-2-benzyl-2-cyano-3-(2-methoxyphenyl)-3-(1-naphthyl)propanoate

The title compound was prepared according to example 20 using benzyl bromide. Recrystallization of the crude product from methanol provided the title compound as a white solid.
1H NMR (500 MHz, DMSO-D6) δ 0.58 (t, *J*=7.10 Hz, 3 H) 2.90 (d, *J*=13.44 Hz, 1H) 3.57 (d, *J*=13.59 Hz, 1 H) 3.79 (m, 2 H) 4.09 (s, 3H) 6.05 (s, 1H) 6.89 (t, *J*=7.86 Hz, 1 H) 7.10 (m, 2 H) 7.19 (d, *J*=7.94 Hz, 1H) 7.30 (m, 5H) 7.49 (m, 2H) 7.56 (m, 1H) 7.84 (d, *J*=8.25 Hz, 1H) 7.89 (d, *J*=9.47 Hz, 1 H) 8.00 (d, *J*=8.09 Hz, 1 H) 8.07 (d, *J*=7.18 Hz, 1H)
MS (ESI) m/z 467 ([M+NH4]+);
Anal. calcd for C₃₀H₂₇NO₃: C:80.15 H:6.05 N:3.12 Found: C:79.99 H:6.00 N:3.04.

### Example 24

### tert-Butyl (RR,SS)-2-cyano-3-(2-isopropylphenyl)-2-methyl-3-(1-naphthyl)propanoate

The title compound was prepared according to example 20 using tert-butyl 2-cyano-3-(2-isopropylphenyl)-3-(1-naphthyl)propanoate as the starting material. Recrystallization from MeOH provided the title compound.
1H NMR (500 MHz, DMSO-D6) δ 0.26 (d, *J*=6.72 Hz, 3 H) 1.19 (d, *J*=6.72 Hz, 2H) 1.21 (s, 9 H) 1.54 (s, 3 H) 2.95 (m, 1 H) 5.62 (s, 1H) 7.26 (m, 2 H) 7.33 (m, 1H) 7.44 (m, 2 H) 7.58 (m, 1H) 7.72 (t, *J*=7.18 Hz, 1H) 7.88 (d, *J*=7.79 Hz, 1H) 7.99 (dd, *J*=7.79, 4.28 Hz, 2H) 8.47 (d, *J*=8.70 Hz, 1H)
MS (ESI) m/z 370 ([M+H]+);
Anal. calcd for C₂₈H₃₁NO₂: C:81.32 H:7.56 N:3.39 Found: C:81.23 H:7.54 N:3.38

### Example 25 Part 1

### tert-Butyl (RS,SR)-2-cyano-2-methyl-3-(1-naphthyl)-3-[2-(trifluoromethyl) phenyl]propanoate

The title compound was prepared according to example 20 using *tert*-butyl 2-cyano-3-(2- trifluoromethyl)phenyl)-3-(1-naphthyl)propanoate as the starting material.
Fractional recrystallization of the solid from MeOH provided the title compound as a white solid.
mp 159-161 °C;
1H NMR (500 MHz, DMSO-D6) δ 1.14 (s, 9 H) 1.57 (s, 3 H) 5.78 (s, 1 H) 7.48 (t, *J*=7.71 Hz, 1H) 7.54 (m, 1 H) 7.57 (t, *J*=7.56 Hz, 2 H) 7.70 (ddd, *J*=8.47, 6.95, 1.22 Hz, 1H) 7.74 (d, *J*=7.79 Hz, 1 H) 7.91 (m, 2H) 7.98 (d, *J*=7.48 Hz, 1H) 8.30 (d, *J*=8.70 Hz, 1H) 8.40 (d, *J*=7.94 Hz, 1H)
MS (ESI) m/z 457 ([M+NH4]+);
Anal. calcd for C₂₆H₂₄F₃NO₂: C:71.06 H:5.50 N:3.19 Found: C:71.08 H:5.42 N:3.19.

### Example 25 Part 2

### tert-Butyl(R,R/SS)2-cyano-2-methyl-3-(1-naphthyl)-3-[2-(trifluoromethyl) phenyl]propanoate

The mother liquors from example 25 part 1 were separated on a Primesphere C18 (25x5cm) column in 1:4 water:methanol. This provided the title compound as a white solid.
mp 136-138 °C;
1H NMR (500 MHz, DMSO-D6) δ 0.94 (s, 9 H) 1.58 (s, 3H) 5.81 (s, 1 H) 7.51 (t, *J*=7.41 Hz, 1 H) 7.58 (m, 3 H) 7.79 (d, *J*=7.79 Hz, 1 H) 7.82 (d, *J*=7.63 Hz, 1H) 7.93 (dd, *J*=10.69, 8.55 Hz, 2 H) 8.08 (t, *J*=8.17 Hz, 2 H) 8.22 (d, *J*=7.94 Hz, 1H)
MS (ESI) m/z 457 ([M+NH4]+);
Anal. calcd for C₂₆H₂₄F₃NO₂: C:71.06 H:5.50 N:3.19 Found: C:71.10 H:5.48 N:3.03.

### Example 26

### tert-Butyl (RS,SR)-2-cyano-3-(2,6-dimethylphenyl)-2-methyl-3-(1-naphthyl)propanoate

The title compound was prepared according to example 20 using 2-cyano-3-(2,6-dimethyl-phenyl)-3-naphthalen-1-yl-propionic acid *tert*-butyl ester as the starting material. Recrystallization from MeOH provided the title compound as a white solid.
mp 155-156°C;
1H NMR (500 MHz, DMSO-D6) δ 1.28 (s, 9 H) 1.68 (s, 3 H) 1.74 (s, 3 H) 2.78 (s, 3 H) 5.52 (s, 1 H) 6.82 (d, *J*=7.48 Hz, 1 H) 7.11 (t, *J*=7.64 Hz, 1 H) 7.28 (d, *J*=7.33 Hz, 1 H) 7.34 (td, *J*=7.71, 1.07 Hz, 1 H) 7.44 (t, *J*=7.41 Hz, 1 H) 7.48 (d, *J*=8.70 Hz, 1H) 7.61 (t, *J*=7.79 Hz, 1 H) 7.91 (dd, *J*=11.30, 8.09 Hz, 2 H) 8.01 (d, *J*=7.33 Hz, 1H)
MS (APCI) m/z 400 ([M+H]+);
Anal. calcd for C₂₇H₂₉NO₂: C:81.17 H:7.32 N:3.51 Found: C:81.03 H:7.27 N:3.46.

### Example 27

### tert-Butyl (RR,SS)-2-benzyl-2-cyano-3-(2-methoxyphenyl)-3-(1-naphthyl)propanoate

The title compound was prepared according to example 20 using benzyl bromide. Trituration of the solid in methanol provided the title compound as a white powder.
mp 208 °C;
1H NMR (500 MHz, DMSO-D6) δ 0.85 (s, 9 H) 2.89 (d, *J*=13.44 Hz, 1H) 3.53 (d, *J*=13.59 Hz, 1H) 4.09 (s, 3H) 6.00 (s, 1 H) 6.90 (td, *J*=7.52, 0.99 Hz, 1H) 7.17 (m, 3 H) 7.29 (m, 4H) 7.36 (dd, *J*=7.79, 1.53 Hz, 1 H) 7.50 (m, 2 H) 7.59 (m, 1 H) 7.85 (d, *J*=8.25 Hz, 1 H) 7.90 (dd, *J*=8.09, 1.07 Hz, 1 H) 8.03 (d, *J*=8.40 Hz, 1 H) 8.14 (d, *J*=7.18 Hz, 1H)
MS (ESI) m/z 495 ([M+NH4]+);
Anal. calcd for C₃₂H₃₁NO₃: C:80.48 H:6.54 N:2.93 Found: C:80.48 H:6.42 N:2.93.

### Example 28

### tert-Butyl (RR, SS)-2-(3-chlorobenzyl)-2-cyano-3-(2-methoxyphenyl)-3-(1-naphthyl)propanoate

The title compound was prepared according to example 20using *tert*-butyl (*RR,SS*)-2-benzyl-2-cyano-3-(2-methoxyphenyl)-3-(1-naphthyl)propanoate and 3-chloro-benzyl bromide as the starting materials. Trituration with methanol provided the title compound as a white solid.
mp 175-179 °C;
1 H NMR (500 MHz, DMSO-D6) δ 0.87 (s, 9 H) 2.91 (d, *J*=13.59 Hz, 1 H) 3.56 (d, *J*=13.74 Hz, 1 H) 4.08 (s, 3 H) 5.99 (s, 1 H) 6.89 (t, *J*=7.56 Hz, 1 H) 7.15 (d, *J*=7.18 Hz, 1 H) 7.19 (m, 2 H) 7.29 (td, *J*=7.83, 1.30 Hz, 1 H) 7.36 (m, 3 H) 7.50 (m, 2 H) 7.59 (t, *J*=7.79 Hz, 1 H) 7.86 (d, *J*=8.25 Hz, 1 H) 7.90 (dd, *J*=7.86, 1.15 Hz, 1 H) 8.01 (d, *J*=8.25 Hz, 1 H) 8.12 (d, *J*=7.18 Hz, 1 H)
MS (APCI) m/z 529 ([M+NH4]+);
Anal. calcd for C₃₂H₃₀ClNO₃: C:75.06 H:5.91 N:2.74 Found: C:74.84 H:5.81 N:2.72.

### Example 29

### tert-Butyl (RR,SS)-2-(2-bromobenzyl)-2-cyano-3-(2-methoxyphenyl)-3-(1-naphthyl)propanoate

The title compound was prepared according to example 20using *tert*-butyl (*RR,SS*)-2-benzyl-2-cyano-3-(2-methoxyphenyl)-3-(1-naphthyl)propanoate and 2-bromo-benzyl bromide as the starting materials. Chromatography on silica gel with 25% ethyl acetate/hexanes as the eluant provided the title compound as a white solid.
1H NMR (500 MHz, DMSO-D6) δ 0.89 (s, 9 H) 3.36 (d, *J*=14.35 Hz, 1 H) 3.75 (d, *J*=14.51 Hz, 1 H) 4.11 (s, 3 H) 6.01 (s, 1 H) 6.90 (t, *J*=7.33 Hz, 1 H) 7.32 (m, 6 H) 7.54 (m, 4 H) 7.86 (d, *J*=8.09 Hz, 1 H) 7.90 (d, *J*=7.79 Hz, 1 H) 8.06 (d, *J*=8.55 Hz, 1 H) 8.17 (d, *J*=7.02 Hz, 1 H)
MS (ESI) m/z 575 ([M+NH4]+);
Anal. calcd for C₃₂H₃₀BrNO₃: C:69.07 H:5.43 N:2.52 Found: C:69.16 H:5.42 N:2.50.

### Example 30

### tert-Butyl (RR,SS)-2-(2-chlorobenzyl)-2-cyano-3-(2-methoxyphenyl)-3-(1-naphthyl)propanoate

The title compound was prepared according to example 20using *tert*-butyl (*RR,SS*)-2-benzyl-2-cyano-3-(2-methoxyphenyl)-3-(1-naphthyl)propanoate and 2-chlorobenzyl bromide as the starting materials. Trituration with methanol provided the title compound as a white solid.
1H NMR (500 MHz, DMSO-D6) δ 0.89 (s, 9 H) 3.32 (d, *J*=13.29 Hz, 1 H) 3.71 (d, *J*=14.35 Hz, 1 H) 4.10 (s, 3 H) 6.01 (s, 1 H) 6.90 (td, *J*=7.56, 1.07 Hz, 1 H) 7.20 (dd, *J*=8.32, 0.84 Hz, 1 H) 7.30 (m, 4 H) 7.42 (m, 2 H) 7.51 (m, 2 H) 7.59 (m, 1 H) 7.86 (d, *J*=8.25 Hz, 1 H) 7.90 (dd, *J*=8.02, 1.30 Hz, 1 H) 8.06 (d, *J*=8.40 Hz, 1 H) 8.16 (d, *J*=7.02 Hz, 1 H)
MS (ESI) m/z 529 ([M+NH4]+);
Anal. calcd for C₃₂H₃₀ClNO₃ . 0.15 H2O: C:74.67 H:5.93 N:2.72 Found: C:74.63 H:5.87 N:2.66.

### Example 31

### tert-Butyl (RR,SS)-2-cyano-2-(2,6-dichlorobenzyl)-3-(2-methoxyphenyl)-3-(1-naphthyl)propanoate

The title compound was prepared according to example 20using *tert*-butyl (*RR*,*SS*)-2-benzyl-2-cyano-3-(2-methoxyphenyl)-3-(1-naphthyl)propanoate and 2,6-dichlorobenzyl bromide as the starting materials. Trituration with methanol provided the title compound as a white solid.
1H NMR (500 MHz, DMSO-D6) δ 0.80 (s, 9 H) 3.61 (d, *J*=14.35 Hz, 1 H) 3.83 (d, *J*=14.35 Hz, 1 H) 4.11 (s, 3H) 6.05 (s, 1 H) 6.89 (td, *J*=7.56, 1.07 Hz, 1 H) 7.18 (dd, *J*=8.40, 0.92 Hz, 1 H) 7.30 (m, 2 H) 7.42 (m, 2 H) 7.53 (m, 4 H) 7.86 (d, *J*=8.25 Hz, 1 H) 7.90 (dd, *J*=8.09, 1.07 Hz, 1H) 8.17 (t, *J*=7.86 Hz, 2 H)
MS (ESI) m/z 562 ([M+NH4]+);
Anal. calcd for C₃₂H₂₉Cl₂NO₃. 0.30 H₂O: C:69.64 H:5.41 N:2.54 Found: C:69.57 H:5.44 N:2.44.

### Example 32

### Ethyl (RR,SS)-2-cyano-3-(2,4-dimethoxyphenyl)-2-methyl-3-(1-naphthyl)propanoate

The title compound was prepared according to example 20 using ethyl 2-cyano-3-(2,4-dimethoxyphenyl)-3-(1-naphthyl)propanoate. Chromatography on silica gel using 25% ethyl acetate/hexanes as eluant provided the title compound as a white powder.
1H NMR (500 MHz, DMSO-D6) δ 0.75 (t, *J*=7.02 Hz, 3 H) 1.64 (s, 3 H) 3.70 (s, 3 H) 3.97 (m, 2 H) 4.04 (s, 3 H) 5.68 (s, 1 H) 6.42 (dd, *J*=8.70, 2.44 Hz, 1 H) 6.69 (d, *J*=2.44 Hz, 1 H) 7.03 (d, *J*=8.70 Hz, 1 H) 7.46 (dq, *J*=9.62, 0.92 Hz, 2 H) 7.56 (t, *J*=7.48 Hz, 1 H) 7.83 (d, *J*=8.25 Hz, 1 H) 7.87 (m, 2 H) 8.00 (d, *J*=7.18 Hz, 1 H)
MS (ESI) m/z 404 ([M+H]+);
Anal. calcd for C₂₅H₂₅NO₄: C:74.42 H:6.25 N:3.47 Found: C:73.77 H:6.32 N:3.31.

### Example 33

### tert-Butyl (RS,SR)-2-cyano-2-methyl-3-(1-naphthyl)-3-[2-(trifluoromethoxy)phenyl]propanoate

The title compound was prepared according to example 20 using *tert*-butyl 2-cyano-3-(1-naphthyl)-3-[2-(trifluoromethoxy)phenyl]propanoate as the starting material. Recrystallization from ethanol provided the title compound as a crystalline solid.
mp 150-153 °C;
1H NMR (500 MHz, DMSO-D6) δ 1.25 (s, 9 H) 1.48 (s, 3 H) 5.70 (s, 1 H) 7.29 (d, *J*=8.40 Hz, 1 H) 7.46 (ddd, *J*=8.09, 7.33, 1.07 Hz, 1 H) 7.49 (m, 2 H) 7.58 (td, *J*=7.25, 5.65 Hz, 2 H) 7.69 (t, *J*=7.41 Hz, 1H) 7.91 (dd, *J*=6.41, 2.90 Hz, 1 H) 7.99 (d, *J*=8.09 Hz, 1 H) 8.18 (dd, *J*=7.86, 0.99 Hz, 1 H) 8.36 (d, *J*=8.70 Hz, 1 H)
MS m/z 00-E1394P;
Anal. calcd for C₂₆H₂₄F₃NO₃: C:68.56 H:5.31 N:3.08 Found: C:68.36 H:5.24 N:2.96.

### Example 34

### Ethyl 2-cyano-2-methyl-3,3-di(1-naphthyl)propanoate

A solution of 2-cyano-3,3-di-naphthalen-1-yl-propionic acid ethyl ester was dissolved in THF (3mL) and treated with sodium bis-trimethylsilyl amide (39µl, 1M in THF) at room temperature. After 5 minutes methyl iodide was added (9µl) and the reaction was stirred for 4 hours. The reaction was quenched with 1 N HCl (3ml) and was diluted with isopropyl acetate (3mL). The organic layer was washed with bicarbonate then brine; dried over Na₂SO₄, filtered and evaporated to yield the title compound as a crystalline material.
mp 186-188 °C;
1 H NMR (400 MHz, DMSO-D6) δ 0.81 (t, *J*=7.08 Hz, 3 H) 1.66 (s, 3 H) 4.01 (m, 2 H) 6.19 (s, 1 H) 7.32 (td, *J*=7.69, 1.22 Hz, 1 H) 7.43 (q, *J*=7.57 Hz, 2 H) 7.57 (d, *J*=7.32 Hz, 1 H) 7.62 (td, *J*=7.69, 3.90 Hz, 2 H) 7.77 (td, *J*=7.69, 0.98 Hz, 1 H) 7.89 (m, 4 H) 8.01 (d, *J*=7.32 Hz, 1 H) 8.10 (d, *J*=7.08 Hz, 1 H) 8.76 (d, *J*=8.54 Hz, 1 H)
MS (EI) m/z M+. (393);
Anal. calcd for C₂₇H₂₃NO₂: C:82.42 H:5.89 N:3.56 Found: C:82.20 H:5.84 N:3.52.

### Example 35

### Ethyl 2-cyano-3-(3-methoxyphenyl)-3-(1-naphthyl)propanoate

The title compound was prepared according to example 15, using 3-methoxyphenyl magnesium bromide. The sample is a viscous oil.
1 H NMR (400 MHz, DMSO-D6) δ 0.85 (t, *J*=7.08 Hz, 1.5 H) 0.90 (t, *J*=7.20 Hz, 1.5 H) 2.04 (s, 1 H) 3.66 (s, 1.5 H) 3.67 (s, 1.5 H) 3.97 (m, 2 H) 5.30 (d, *J*=9.52 Hz, 0.5 H) 5.38 (d, *J*=10.01 Hz, 0.5 H) 5.46 (d, *J*=9.76 Hz, 0.5 H) 5.50 (d, *J*=9.76 Hz, 0.5 H) 6.76 (m, 1 H) 6.89 (d, *J*=8.30 Hz, 0.5 H) 6.97 (t, *J*=1.95 Hz, 0.5 H) 7.10 (m, 1 H) 7.15 (t, *J*=7.93 Hz, 0.5 H) 7.20 (t, *J*=7.93 Hz, 0.5 H) 7.49 (m, 1.5 H) 7.57 (t, *J*=7.69 Hz, 0.5 H) 7.70 (d, *J*=7.08 Hz, 0.5 H) 7.81 (d, *J*=8.05 Hz, 0.5 H) 7.90 (m, 2 H) 8.16 (d, *J*=9.52 Hz, 0.5 H) 8.30 (d, *J*=8.05 Hz, 0.5 H)
MS (EI) m/z M+. (359);
Anal. calcd for C₂₃H₂₁NO₃: C:76.86 H:5.89 N:3.90 Found: C:75.45 H:5.83 N:3.58

### Example 36

### Ethyl 2-cyano-3-(4-methoxyphenyl)-3-(1-naphthyl)propanoate

The title compound was prepared according to example 15, using 4-methoxyphenyl magnesium bromide. The sample is a viscous oil
1H NMR (400 MHz, DMSO-D6) δ 0.91 (dt, *J*=13.97, 7.05 Hz, 3 H) 3.66 (s, 1.5 H) 3.69 (s, 1.5 H) 4.01 (m, 2 H) 5.26 (d, *J*=9.76 Hz, 0.5 H) 5.33 (d, J=9.52 Hz, 0.5 H) 5.45 (d, *J*=9.76 Hz, 0.5 H) 5.49 (d, *J*=9.76 Hz, 0.5 H) 6.69 (m, 0.5 H) 6.84 (ddd, *J*=14.52, 6.71, 1.95 Hz, 2 H) 7.30 (d, *J*=8.79 Hz, 1 H) 7.48 (m, 3 H) 7.58 (t, *J*=7.57 Hz, 0.5 H) 7.71 (d, *J*=7.57 Hz, 0.5 H) 7.83 (d, *J*=8.30 Hz, 0.5 H) 7.91 (m, 2 H) 8.14 (dd, *J*=5.74, 4.03 Hz, 0.5 H) 8.26 (d, *J*=8.30 Hz, 0.5 H)
MS (EI) m/z M+. (359);
Anal. calcd for C₂₃H₂₁NO₃: C:76.86 H:5.89 N:3.90 Found: C:75.32 H:5.89 N:3.53.

### Example 37

### Ethyl 2-cyano-3-(4-methylphenyl)-3-(1-naphthyl)propanoate

The title compound was prepared according to example 15, using 4-methylphenyl magnesium bromide. The sample is a viscous oil.
1 H NMR (400 MHz, DMSO-D6) δ 0.90 (dt, *J*=12.45, 7.08 Hz, 3 H) 2.06 (s, 3 H) 2.19 (s, 1.5 H) 2.22 (s, 1.5 H) 4.00 (ddd, *J*=13.85, 7.02, 4.03 Hz, 2 H) 5.28 (d, *J*=9.52 Hz, 0.5 H) 5.35 (d, *J*=9.52 Hz, 0.5 H) 5.46 (d, *J*=9.76 Hz, 0.5 H) 5.50 (d, *J*=9.52 Hz, 0.5 H) 7.09 (dd, *J*=15.13, 7.81 Hz, 2 H) 7.26 (d, *J*=8.30 Hz, 1 H) 7.41 (d, *J*=8.30 Hz, 1 H) 7.52 (m, 2.5 H) 7.70 (d, *J*=6.59 Hz, 0.5 H) 7.83 (d, *J*=8.05 Hz, 0.5 H) 7.91 (m, 1 H) 8.14 (dd, *J*=5.13, 4.39 Hz, 0.5 H) 8.26 (d, *J*=8.54 Hz, 0.5 H)
MS (EI) m/z M+. (343);
Anal. calcd for C₂₃H₂₁NO₂: C:80.44 H:6.16 N:4.08 Found: C:78.76 H:6.32 N:3.81.

### Example 38

### Ethyl 2-cyano-3-(2-methylphenyl)-3-(1-naphthyl)propanoate

The title compound was prepared according to example 15, using 2-methylphenyl magnesium bromide. The sample is a viscous oil.
1 H NMR (400 MHz, DMSO-D6) δ 0.81 (t, *J*=7.08 Hz, 1.5 H) 0.92 (t, *J*=7.08 Hz, 1.5 H) 2.13 (s, 3 H) 3.88 (m, 1 H) 4.05 (q, *J*=7.08 Hz, 1 H) 5.22 (t, *J*=8.18 Hz, 1 H) 5.62 (d, *J*=7.57 Hz, 0.5 H) 5.67 (d, *J*=8.79 Hz, 0.5 H) 7.20 (m, 3.5 H) 7.48 (m, 1.5 H) 7.57 (m, 1H) 7.65 (m, 1.5 H) 7.77 (d, *J*=6.83 Hz, 0.5 H) 7.89 (m, 2 H) 7.97 (dd, *J*=8.05, 0.98 Hz, 0.5 H) 8.22 (d, *J*=8.54 Hz, 0.5 H)
MS (APCI) m/z [M+H]+ (344);
Anal. calcd for C₂₃H₂₁NO₂: C:80.44 H:6.16 N:4.08 Found: C:78.56 H:6.10 N:3.91.

### Example 39

### Ethyl 2-cyano-3-(1-naphthyl)-3-(2-naphthyl)propanoate

The title compound was prepared according to example 15, using 2-naphthyl magnesium bromide. The sample is a viscous oil.
1H NMR (400 MHz, DMSO-D6) δ 0.82 (t, *J*=7.08 Hz, 1.5 H) 0.89 (t, *J*=7.08 Hz, 1.5 H) 3.99 (m, 2 H) 5.46 (d, *J*=9.76 Hz, 0.5 H) 5.51 (d, *J*=9.76 Hz, 0.5 H) 5.70 (d, *J*=9.76 Hz, 0.5 H) 5.74 (d, *J*=9.76 Hz, 0.5 H) 7.51 (m, 5 H) 7.64 (m, 1 H) 7.83 (m, 4 H) 7.93 (m, 2 H) 8.02 (d, *J*=7.08 Hz, 0.5 H) 8.15 (d, *J*=1.46 Hz, 0.5 H) 8.25 (m, 0.5 H) 8.38 (d, *J*=8.54 Hz, 0.5 H)
MS (APCl) m/z 380 ([M+H]+);
Anal. calcd for C₂₆H₂₁NO₂: C:82.30 H:5.58 N:3.69 Found: C:81.94 H:5.55 N:3.43.

### Example 40

### Ethyl 2-cyano-3-(4-fluoro-1-naphthyl)-3-(1-naphthyl)propanoate

The title compound was prepared according to example 15, using 4-fluoro-(1-naphthyl) magnesium bromide. The sample is a white foam.
1 H NMR (400 MHz, DMSO-D6) δ 0.83 (m, 3 H) 3.93 (m, *J*=17.51, 14.15, 7.14, 3.66 Hz, 2 H) 5.37 (d, *J*=2.20 Hz, 0.5 H) 5.39 (d, *J*=2.20 Hz, 0.5 H) 6.30 (d, *J*=7.81 Hz, 1 H) 7.25 (d, *J*=8.05 Hz, 1 H) 7.31 (dd, *J*=7.32, 0.98 Hz, 0.5 H) 7.41 (m, 1 H) 7.47 (m, 1 H) 7.53 (ddd, *J*=8.48, 6.89, 1.46 Hz, 0.5 H) 7.60 (m, 1.5 H) 7.67 (m, 0.5 H) 7.73 (d, *J*=7.32 Hz, 0.5 H) 7.83 (m, 2.5 H) 7.97 (m, 2 H) 8.09 (dd, *J*=8.30, 0.98 Hz, 0.5 H) 8.13 (dd, *J*=8.18, 1.10 Hz, 0.5 H) 8.35 (d, *J*=8.54 Hz, 0.5 H) 8.46 (d, *J*=8.79 Hz, 0.5 H)
MS (APCI) m/z 398 ([M+H]+);
Anal. calcd for C₂₆H₂₀FNO₂: C:78.57 H:5.07 N:3.52 Found: C:77.65 H:4.97 N:3.43.

### Example 41

### Ethyl 2-cyano-3-[4-(methylthio)phenyl]-3-(1-naphthyl)propanoate

The title compound was prepared according to example 15, using 4-(thiomethyl)phenyl magnesium bromide. The sample is a white foam
1 H NMR (400 MHz, DMSO-D6) δ 0.91 (dt, *J*=14.34, 7.11 Hz, 3 H) 2.39 (d, *J*=9.76. Hz, 3 H) 4.01 (m, 2 H) 5.31 (d, *J*=9.52 Hz, 0.5 H) 5.39 (d, *J*=9.52 Hz, 0.5 H) 5.49 (d, *J*=9.52 Hz, 0.5 H) 5.52 (d, *J*=9.76 Hz, 0.5 H) 7.17 (m, 2 H) 7.33 (d, *J*=8.30 Hz, 1 H) 7.50 (m, 3.5 H) 7.59 (dd, *J*=8.30, 7.32 Hz, 0.5 H) 7.72 (d, *J*=6.59 Hz, 0.5 H) 7.84 (d, *J*=8.30 Hz, 0.5 H) 7.92 (m, 2 H) 8.15 (m, 0.5 H) 8.27 (d, *J*=8.54 Hz, 0.5 H)
MS (EI) m/z M+. (375);
Anal. calcd for C₂₃H₂₁NO₂S: C:73.57 H:5.64 N:3.73 Found: C:73.45 H:5.44 N:3.71.

### Example 42

### Ethyl 3-[1,1'-biphenyl]-4-yl-2-cyano-3-(1-naphthyl)propanoate

The title compound was prepared according to example 15, using 4-biphenyl magnesium bromide. The sample is a white foam
1 H NMR (400 MHz, DMSO-D6) δ 0.90 (q, *J*=7.08 Hz, 3 H) 4.01 (m, 2 H) 5.39 (d, *J*=9.76 Hz, 0.5 H) 5.46 (d, *J*=9.76 Hz, 0.5 H) 5.58 (d, *J*=9.76 Hz, 0.5 H) 5.62 (d, *J*=10.01 Hz, 0.5 H) 7.32 (m, 1 H) 7.42 (m, 2 H) 7.57 (m, 9 H) 7.78 (d, *J*=6.59 Hz, 0.5 H) 7.85 (d, *J*=8.30 Hz, 0.5 H) 7.94 (m, 2 H) 8.23 (m, 0.5 H) 8.35 (d, *J*=8.30 Hz, 0.5 H)
MS (APCI) m/z 404 ([M-H]-);
Anal. calcd for C₂₈H₂₃NO₂: C:82.94 H:5.72 N:3.45 Found: C:82.84 H:5.71 N:3.38.

### Example 43

### Ethyl 3-[1,1'-biphenyl]-2-yl-2-cyano-3-(1-naphthyl)propanoate

The title compound was prepared according to example 15, using 2-biphenyl magnesium bromide. The sample is a white foam
1H NMR (400 MHz, DMSO-D6) δ 0.73 (t, *J*=7.08 Hz, 1.5 H) 0.90 (t, *J*=7.08 Hz, 1.5 H) 3.82 (m, 1 H) 4.02 (q, *J*=7.08 Hz, 1 H) 5.23 (d, *J*=8.30 Hz, 0.5 H) 5.31 (d, *J*=9.52 Hz, 0.5 H) 5.55 (d, *J*=8.30 Hz, 0.5 H) 5.66 (d, *J*=9.52 Hz, 0.5 H) 7.03 (d, *J*=8.79 Hz, 1.5 H) 7.18 (m, 2.5 H) 7.40 (m, 7.5 H) 7.62 (m, 1 H) 7.82 (m, 2 H) 8.05 (d, *J*=7.57 Hz, 0.5 H)
MS (APCI) m/z 404 ([M-H]-);
Anal. calcd for C₂₈H₂₃NO₂: C:82.94 H:5.72 N:3.45 Found: C:82.40 H:5.94 N:3.39.

### Example 44

### Ethyl 3-(4-chlorophenyl)-2-cyano-3-(1-naphthyl)propanoate

The title compound was prepared according to example 15, using 4-chlorophenyl magnesium bromide. The sample is a sticky oil.
1 H NMR (400 MHz, DMSO-D6) δ 0.89 (dt, *J*=16.60, 7.08 Hz, 3 H) 2.42 (s, 1.5 H) 2.63 (s, 1.5 H) 4.01 (m, 2 H) 5.12 (d, *J*=7.08 Hz, 0.5 H) 5.26 (d, *J*=8.79 Hz, 0.5 H) 5.83 (d, *J*=7.32 Hz, 0.5 H) 5.92 (d, *J*=8.79 Hz, 0.5 H) 7.02 (m, 1H) 7.28 (m, 1.5) 7.39 (t, *J*=8.30 Hz, 1 H) 7.56 (m, 3 H) 7.88 (m, 3 H) 8.24 (d, *J*=8.54 Hz, 0.5 H)
MS (APCI) m/z [M+H]+ (364);
Anal. calcd for C₂₂H₁₈ClNO₂: C:72.63 H:4.99 N:3.85 Found: C:71.56 H:4.95 N:3.65.

### Example 45

### Ethyl 2-cyano-3-[2-(methylthio)phenyl]-3-(1-naphthyl)propanoate

The title compound was prepared according to example 15 using 2-(thiomethyl)phenyl magnesium bromide. The sample is a sticky white foam
1H NMR (400 MHz, DMSO-D6) δ 0.91 (dt, *J*=16.23, 7.14 Hz, 3 H) 4.01 (m, 2 H) 5.36 (d, *J*=9.52 Hz, 0.5 H) 5.43 (d, *J*=9.76 Hz, 0.5 H) 5.56 (d, *J*=9.52 Hz, 0.5 H) 5.60 (d, *J*=10.01 Hz, 0.5 H) 7.39 (m, 3 H) 7.55 (m, 4 H) 7.72 (d, *J*=6.83 Hz, 0.5 H) 7.85 (d, *J*=8.30 Hz, 0.5 H) 7.92 (m, 2 H) 8.17 (m, 0.5 H) 8.29 (d, *J*=8.54 Hz, 0.5 H)
MS (APCI) m/z [M+H]+ (376);
Anal. calcd for C₂₃H₂₁NO₂S: C:73.57 H:5.64 N:3.73 Found: C:73.58 H:5.88 N:3.58.

### Example 46

### Ethyl-(RR,SS)- 2-cyano-3-(2-methoxyphenyl)-2-methyl-3-(1-naphthyl)propanoate

A solution of 2-cyano-3-(2-methoxy-phenyl)-3-naphthalen-1-yl-propionic acid ethyl ester (205mg, 0.57mmol) in THF (10mL) is cooled in a dry ice ethanol bath. A solution of KHMDS (1.04ml, 0.68mmol, 0.66M toluene) is added dropwise. The bath is removed and the reaction is warmed to room temperature over an hour. To this is added methyl iodide (162mg, 1.14mmol) and the reaction is stirred at room temperature for 30 minutes. The reaction is quenched with 1NHCl and is diluted with ethyl acetate. The organic layer is isolated and is washed with saturated bicarbonate solution then brine. The organic layer is dried with Na₂SO₄, is filtered and evaporated to a solid. Chromatography over silica gel (20%ethyl acetate/hexane) yields the title compound as a solid. Recrystallization from MeOH yields the title compound (80mg) as white crystals.
mp 134.5-135.5 °C;
1H NMR (400 MHz, DMSO-D6) δ 0.75 (t, *J*=7.08 Hz, 3 H) 1.65 (s, 3 H) 3.98 (m, *J*=17.79, 10.65, 7.08, 3.78 Hz, 2 H) 4.05 (s, 3 H) 5.81 (s, 1 H) 6.83 (td, *J*=7.50, 1.10 Hz, 1 H) 7.16 (dt, *J*=7.87, 1.56 Hz, 1 H) 7.26 (td, *J*=7.81, 1.71 Hz, 1 H) 7.46 (m, 1 H) 7.58 (dd, *J*=8.18, 7.44 Hz, 1H) 7.85 (d, *J*=8.54 Hz, 1 H) 7.89 (m, 1 H) 8.03 (d, *J*=7.08 Hz, 1 H)
MS (APCI) m/z [M+H]+ (374);
Anal. calcd for C₂₄H₂₃NO₃: C:77.19 H:6.21 N:3.75 Found: C:77.05 H:6.24 N:3.74.

### Example 47

### Ethyl (RR,SS)-2-cyano-2-methyl-3-[2-(methylthio)phenyl]-3-(1-naphthyl)propanoate

The title compound is prepared from 2-cyano-3-(2-methylsulfanyl-phenyl)-3-naphthalen-1-yl-propionic acid ethyl ester according to example 46. The title compound is obtained as white crystals from MeOH.
mp 154-156 °C;
1H NMR (400 MHz, DMSO-D6) δ 0.75 (t, *J*=7.08 Hz, 3 H) 1.71 (s, 3 H) 2.70 (s, 3 H) 3.96 (m, 2 H) 5.81 (s, 1 H) 7.11 (td, *J*=7.69, 1.22 Hz, 1 H) 7.29 (td, *J*=7.57, 1.46 Hz, 1 H) 7.34 (dd, *J*=7.93, 1.34 Hz, 1 H) 7.48 (dt, *J*=6.53, 2.96 Hz, 2 H) 7.53 (dd, *J*=8.05, 0.98 Hz, 1 H) 7.60 (t, *J*=7.57 Hz, 1 H) 7.89 (m, 2 H) 8.05 (d, *J*=7.08 Hz, 1 H) 8.15 (d, *J*=9.52 Hz, 1 H)
MS (APCI) m/z [M+H]⁺ (390);
Anal. calcd for C₂₄H₂₃NO₂S: C:74.01 H:5.95 N:3.60 Found: C:73.97 H:5.91 N:3.55.

### Example 48

### (RR,SS)-2-Cyano-3-(2-methoxyphenyl)-2-methyl-3-(1-naphthyl)propanoic acid

Ethyl-(RR,SS)- 2-cyano-3-(2-methoxyphenyl)-2-methyl-3-(1-naphthyl)propanoate (12.42g, 33.33 mmol) was dissolved in 100 mL THF to which was added 2.5 M NaOH (18 mL). Reaction mixture heated to reflux for 8 hrs, allowed to cool and stir overnight. The reaction mixture was acidified with 1 N HCl, taken up in ethyl acetate, washed with brine, dried with MgSO₄, filtered, evaporated, and recrystallized from ethanol to tyield 11.44 g the title compound as a crystalline solid.
mp 229 °C (dec);
1 H NMR (400 MHz, DMSO-D6) δ 1.62 (s, 2 H) 4.05 (s, 2 H) 5.77 (s, 1H) 6.80 (td, *J*=7.50, 1.10 Hz, 1 H) 7.11 (dd, *J*=7.69, 1.59 Hz, 1H) 7.15 (dd, *J*=8.30, 0.98 Hz, 1 H) 7.23 (td, *J*=7.81, 1.71 Hz, 1 H) 7.44 (m, 1 H) 7.59 (m, 1 H) 7.84 (d, *J*=8.30 Hz, 1 H) 7.89 (m, 1 H) 8.10 (d, *J*=7.32 Hz, 1 H)
MS (APCI) m/z 344 ([M-H]-);
Anal. calcd for C₂₂H₁₉NO₃ • 0.35 H₂O: C:75.13 H:5.65 N:3.98 Found: C:75.16 H:5.61 N:3.76.

### Example 49

### (RR,SS)-2-Cyano-3-(2-isopropylphenyl)-2-methyl-3-(1-naphthyl)propanoic acid

A sample of *tert*-butyl (*RR,SS*)-2-cyano-3-(2-isopropylphenyl)-2-methyl-3-(1-naphthyl)propanoate (3.9g, 9.4mmol) was dissolved in CH₂Cl₂ (40mL). The sample was treated with trifluoroacetic acid (40mL) and was stirred for 2 hours. The mixture was evaporated to a solid. Recrystallization from ethyl acetate/hexanes provided the title compound as white powder.
mp 214 °C(dec);
1H NMR (500 MHz, DMSO-D6) δ 0.33 (d, *J*=6.72 Hz, 3 H) 1.17 (d, *J*=6.87 Hz, 3 H) 1.52 (s, 3 H) 2.97 (m, 1 H) 5.68 (s, 1 H) 7.25 (m, 2 H) 7.32 (td, *J*=7.22, 2.21 Hz, 1 H) 7.41 (dd, *J*=7.33, 1.22 Hz, 1 H) 7.47 (t, *J*=7.71 Hz, 1 H) 7.58 (m, 1 H) 7.70 (td, *J*=7.71, 1.07 Hz, 1 H) 7.87 (d, *J*=7.79 Hz, 1 H) 7.99 (dd, *J*=8.17, 0.69 Hz, 1 H) 8.05 (d, *J*=7.63 Hz, 1 H) 8.46 (d, *J*=8.70 Hz, 1 H) 14.01 (bs, 1H)
MS (ESI) m/z 375 ([M+NH4]+);
Anal. calcd for C₂₄H₂₃NO₂: C:80.64 H:6.49 N:3.92 Found: C:80.75 H:6.54 N:3.87.

### Example 50

### (RS,SR)-2-Cyano-2-methyl-3-(1-naphthyl)-3-[2-(trifluoromethyl)phenyl]propanoic acid

The title compound was prepared according to example 49 using tert-butyl (RS,SR)-2-cyano-2-methyl-3-(1-naphthyl)-3-[2-(trifluoromethyl) phenyl] propanoate as the starting material. Recrystallization from ethyl acetate/hexane provided the title compound as a white solid.
mp 220 °C (dec); 1 H NMR (500 MHz, DMSO-D6) δ 1.54 (s, 3 H) 5.83 (s, 1 H) 7.52 (m, 4 H) 7.69 (m, 2 H) 7.90 (m, 2 H) 7.97 (d, *J*=7.94 Hz, 1 H) 8.32 (d, *J*=8.70 Hz, 1 H) 8.48 (d, *J*=8.09 Hz, 1 H) 14.08 (bs, 1 H)
MS (ESI) m/z 401 ([M+NH4]+);
Anal. calcd for C₂₂H₁₆F₃NO₂: C:68.93 H:4.21 N:3.65 Found: C:68.93 H:4.20 N:3.61.

### Example 51

### (RR,SS)-2-Benzyl-2-cyano-3-(2-methoxyphenyl)-3-(1-naphthyl)propanoic acid

The title compound was prepared according to example 49 using *tert*-butyl (*RR,SS*)-2-benzyl-2-cyano-3-(2-methoxyphenyl)-3-(1-naphthyl)propanoate as the starting material. Trituration with heptane provided the title compound as a white solid.
mp 230-233 °C;
1H NMR (500 MHz, DMSO-D6) δ 2.81 (d, *J*=13.29 Hz, 1 H) 3.55 (d, *J*=13.44 Hz, 1 H) 4.08 (s, 3 H) 6.01 (s, 1 H) 6.87 (td, *J*=7.52, 0.69 Hz, 1 H) 7.14 (dd, *J*=7.18, 2.29 Hz, 2 H) 7.18 (d, *J*=8.25 Hz, 1 H) 7.29 (m, 5 H) 7.47 (m, 2 H) 7.57 (m, 1 H) 7.84 (d, *J*=8.25 Hz, 1 H) 7.88 (dd, *J*=7.18, 2.75 Hz, 1 H) 8.01 (dd, *J*=7.48, 1.68 Hz, 1 H) 8.18
(d, *J*=7.18 Hz, 1 H)
MS (ESI) m/z 422 ([M+H]+);
Anal. calcd for C₂₈H₂₃NO₃ . 0.30 H2O: C:78.78 H:5.57 N:3.28 Found: C:78.84 H:5.50 N:3.28.

### Example 52 Part 1

### tert-Butyl (R,R)-2-cyano-3-(2-isopropylphenyl)-2-methyl-3-(1-naphthyl)propanoate

A sample of tert-butyl (*RR,SS*)-2-cyano-3-(2-isopropylphenyl)-2-methyl-3-(1-naphthyl)propanoate was dissolved in methanol and was resolved by chromatography on a preparative HPLC system using a Chiralcel OD (25 x 5cm) column and 8:2 methanol:water as the eluant. The title compound was the first peak to elute. Recrystallization from ethanol provided the title compound as colorless crystals
[α]_{D}²⁵ = -439.8° (1 %, CHCl₃);

### Example 52 Part 2

### tert-butyl (S,S)-2-cyano-3-(2-isopropylphenyl)-2-methyl-3-(1-naphthyl)propanoate

The title compound is the second peak to elute in example 51 part 1. Recrystallization from methanol provided the title compound as colorless needles. [α]_{D}²⁵ = +434.2° (1 %, CHCl₃);

### Example 53 Part 1

### tert-butyl (S,S)-2-cyano-3-(2-methoxyphenyl)-2-methyl-3-(1-naphthyl)propanoate

A sample of *tert*-butyl (*RR,SS*)-2-cyano-3-(2-methoxyphenyl)-2-methyl-3-(1-naphthyl)propanoate was dissolved in methanol and was resolved by chromatography on a preparative HPLC system using a Chiralcel OD (25 x 5cm) column and 8:2 methanol:water as the eluant. The title compound was the first peak to elute. Recrystallization from ethanol provided the title compound as colorless crystals
[α]_{D}²⁵ = -283.8° (0.5%, CHCl₃);
1H NMR (500 MHz, DMSO-D6) δ 1.03 (s, 9 H) 1.63 (s, 3 H) 4.06 (s, 3 H) 5.78 (s, 1 H) 6.85 (td, *J*=7.48, 1.07 Hz, 1 H) 7.17 (d, *J*=8.25 Hz, 1 H) 7.22 (dd, *J*=7.71, 1.60 Hz, 1 H) 7.26 (ddd, J=8.63, 6.95, 1.68 Hz, 1 H) 7.48 (m, 2 H) 7.62 (t, J=7.48 Hz, 1 H) 7.87 (d, J=8.25 Hz, 1 H) 7.92 (m, 2 H) 8.08 (d, J=7.18 Hz, 1 H)
Anal. calcd for C₂₆H₂₇NO₃: C:77.78 H:6.78 N:3.49 Found: C:76.93 H:6.86 N:3.47.

### Example 53 Part 2

### tert-butyl (R,R)-2-cyano-3-(2-methoxyphenyl)-2-methyl-3-(1-naphthyl)propanoate

The title compound is the second peak to elute in example 53 part 1. Recrystallization from methanol provided the title compound as colorless needles. [α]_{D}²⁵ = +272.0° (0. 5%, CHCl₃);
1 H NMR (500 MHz, DMSO-D6) δ 1.03 (s, 9 H) 1.63 (s, 3 H) 4.06 (s, 3 H) 5.78 (s, 1 H) 6.85 (td, *J*=7.48, 1.07 Hz, 1 H) 7.17 (d, *J*=8.25 Hz, 1 H) 7.22 (dd, *J*=7.71, 1.60 Hz, 1 H) 7.26 (ddd, *J*=8.63, 6.95, 1.68 Hz, 1 H) 7.48 (m, 2 H) 7.62 (t, *J*=7.48 Hz, 1 H) 7.87 (d, *J*=8.25 Hz, 1 H) 7.92 (m, 2 H) 8.08 (d, *J*=7.18 Hz, 1 H)
Anal. calcd for C₂₆H₂₇NO₃: C:77.78 H:6.78 N:3.49 Found: C:77.31 H:6.86 N:3.48.

### Example 54

### (RR,SS)-3-(2-methoxyphenyl)-2-methyl-3-(1-naphthyl)-2-(piperazin-1-ylcarbonyl)propanenitrile

(*RR,SS*)-2-cyano-3-(2-methoxyphenyl)-2-methyl-3-(1-naphthyl)propanoic acid(10 g, 28.95 mmol) was dissolved in 250 mL THF. A catalytic amount of DMF was added to the reaction mixture. Oxalyl chloride (3.03 mL, 34.72 mmol) was added slowly. The reaction mixture was stirred until no more evolution of gas was observed. The reaction mixture was rapidly heated to reflux, then cooled and evaporated. 50 mL toluene was added and the reaction mixture was evaporated to dryness. The acid chloride was dissolved in 500 mL CH₂Cl₂ and placed under nitrogen. In a separate flask piperazine (3g, 34.74 mmol) was dissolved in 200 mL CH₂Cl₂ under nitrogen. To this stirred solution was added a catalytic amount of DMAP, and triethylamine (14.12 mL, 101.33 mmol). The acid chloride solution was added slowly to this stirred mixture and allowed to react overnight after which water was added to the reaction mixture. The organic layer was separated and washed with brine, dried over MgSO₄, filtered, evaporated to dryness, and purified on silica gel (5% methanol/methylene chloride + 0.5% Et₃N) to yield 7.26 g of the title compound as a white foam.
1H NMR 500 MHz (DMSO-D6): δ 7.95 (d, 1 H, J=7.18 Hz), 7.85 (m, 2H), 7.78 (d, 1H, J=8.25 Hz), 7.52 (t, 1H, J=7.48 Hz), 7.41 (m, 2H), 7.21 (td, 1H, J=7.71 Hz, 1.53 Hz), 7.12 (d, 1H, J=8.25 Hz), 7.08 (dd, 1H, J=7.79 Hz, 1.53 Hz), 6.77 (t, 1H, J=7.48 Hz), 5.99 (s, 1 H), 4.00 (s, 3H), 3.44 (bs, 4H), 2.62 (bs, 2H), 2.55 (bs, 2H), 1.59 (s, 3H)
MS (ESI) m/z 414 ([M+H]+);
Anal. calcd for C₂₆H₂₇N₃O₂ · 0.20 H₂O: C:74.95 H:6.95 N:9.80 Found: C:74.73 H:6.72 N:9.68

### Example 55

### (RR.SS)(3-[4-(3-chloro-2-methylphenyl)piperazin-1-yl]-2-[(2-methoxyphenyl)(1-naphthyl)methyl]-2-methyl-3-oxopropanenitrile)

A mixture of 2-[(2-methoxy-phenyl)-naphthalen-1-yl-methyl]-2-methyl-3-oxo-3-piperazin-1-yl-propionitrile (442mg, 1.05mmol) , 1-chloro-3-iodo-2-methyl-benzene (258mg, 1.02mmol), NaOtBu (140mg, 1,45mmol), Pd₂(dba)₃ (27mg, 0.029mmol), racemic BINAP (39mg, 0.06mmol) in toluene (5mL) in a Carius tube was degassed with argon. The tube was sealed and placed in a preheated oil bath (80°) for 21 hours. The sample was cooled, diluted with isopropyl acetate (50mL), washed with brine then water, was dried over Na₂SO₄, filtered and concentrated to an oil. Chromatography on silica gel using 15% isopropyl acetate /hexane provided a foam which was recrystallized from ethyl acetate/hexane to provide 220mg of the title compound as an amorphous white powder.
¹H NMR 500MHz(DMSO-D6): δ 7.99 (d, 1H, J = 7.18Hz), 7.88 (m, 2H), 7.80 (d, 1H, J = 8.24Hz), 7.55 (t, 1H, J = 7.63Hz), 7.42 (m,.2H), 7.23 (dt, 1 H, J = 8.56, 1.53Hz), &.14 (m, 4H), 7.12 (brs, 1H), 6.80 (t, 1h, J = 7.48Hz), 4.01 (s, 3H), 2.26 (s,3H), 1.64, (s,3H); MS (ESI) m/z 538 ([M+H]+); Anal. calcd for C₃₃H₃₂ClN₃O₂: C:73.66 H:5.99 N:7.81 Found: C:73.98 H:6.33 N:7.46.

### Example 56 Part 1

### (RR,S,S)-3-(2-methoxyphenyl)-2-methyl-2-{[4-(2-methylphenyl)piperazin-1-yl]carbonyl}3-(1-naphthyl)propanenitrile

A solution of (*R,R*/*S,S*)2-cyano-3-(2-methoxy-phenyl)-2-methyl-3-naphthalen-1-yl-propionic acid (1.2g, 347mmol) in THF (40mL) is treated with DMF (2 drops). Oxalyl chloride (0.4mL, 4.60mmol) is added dropwise in order to control gas evolution; when the gas evolution stopped the solution is heated to reflux for 5 minutes. The solution is cooled, the THF is evaporated *in vacuo* and the solid is dissolved in dry toluene (15mL) and evaporated to a solid. This procedure is repeated twice. The acid chloride is dissolved in dichloromethane (60mL). 1-o-tolyl-piperazine hydrochloride (830mg, 3.91 mmol) and a crystal of DMAP are added to the solution of acid. This is followed by the dropwise addition of TEA (1.1 mL, 7.84mmol). The reaction is stirred overnight. The reaction mixture is diluted with dichloromethane (50mL), washed with aqueous HCl (10mL 0.5N) then saturated NaHCO₃ (10mL) and brine (10mL). The sample is dried over NaSO₄, filtered and concentrated *in vacuo.* This provided 1.7g a tan solid.
MS (ESI) m/z 504 ([M+H]+);
Anal. calcd for C₃₃H₃₃N₃O₂: C:78.70 H:6.60 N:8.34 Found: C:78.51 H:6.85 N:7.91.

### Example 56 Part 2

### (SS)-3-(2-methoxyphenyl)-2-methyl-2-{[4-(2-methylphenyl)-1-piperazinyl]carbonyl}-3-(1-naphthyl)propanenitrile

Preparative chiral HPLC (Chiracel OD, 25x2cm) in 5%methanol/water of (*RR,S,S*)-3-(2-methoxyphenyl)-2-methyl-2-{[4-(2-methylphenyl)piperazin-1-yl]carbonyl}-3-(1-naphthyl)propanenitrile, prepared in example 56 part 1, provided 570 mg of title compound as the first peak to elute.Recrystallization from ethanol /hexane provided off white crystals.
mp 175-177 °C;
[α]_{D}²⁵ = -188.7° (1%, CHCl₃);
¹H NMR 500MHz(DMSO-D6): δ 7.99 (d, 1H, J = 7.17Hz), 87.89 (d, 1H, J = 9.78), 7.87 (d, 1H, J = 10.38Hz), 7.80 (d, 1H, J = 8.10Hz), 7.55 (t, 1 H, J = 7.48Hz), 7.42 (m,2H), 7.23 (t, 1 H, J = 7.63Hz), 7.14 (m, 4H), 6.95 (t, 1 H, J = 7.18Hz), 6.48 (brs, 1 H), 6.79 (t, 1H, J = 7.48Hz), 6.02 (s, 1 H), 4.01 (s,3H), 3.86 (brs, 3H), 2.68 (brs,3H), 2.22 (s,3H), 1.65 (s,3H)
MS (ESI) m/z: 504 ([M+H]+)
Anal. calcd for C₃₃H₃₃N₃O₂: C:78.70 H:6.60 N:8.34 Found: C:78.33 H:6.63 N:8.28.

### Example 56 Part 3

### (R,R)-3-(2-methoxyphenyl)-2-methyl-2-{[4-(2-methylphenyl)piperazin-1-yl]carbonyl}-3-(1-naphthyl)propanenitrile

The second peak from the preparative HPLC described in example 56 part 2 provided 510mg of the title compound. Recrystallization from ethanol/hexanes yielded a white powder.
mp 173-176 °C;
[α]_{D}²⁵ = +184.1° (1%, CHCl₃);
Anal. calcd for C₃₃H₃₃N₃O₂: C:78.70 H:6.60 N:8.34 Found: C:77.93 H:6.63 N:8.13.

### Example 57

### (S,S)-3-(2-methoxyphenyl)-2-methyl-2-{[4-(3-methylphenyl)piperazin-1-yl]carbonyl}-3-(1-naphthyl)propanenitrile

The title compound was prepared according to Example 2B using racemic (*RR,SS*)(3-(2-methoxyphenyl)-2-methyl-2-{[4-(3-methylphenyl)piperazin-1-yl]carbonyl}-3-(1-naphthyl)propanenitrile as the starting material. Recrystallization from ethanol/ether yielded white crystals.
mp 104-105 °C;
[α]_{D}²⁵ = -175.00° (1%, CHCl₃);
HRMS Calc'd for 1.00 C₃₃H₃₃N3O₂; Theory 503.644; Found: 503.257277

### Example 58

### (S)-3-[4-(3,5-dimethoxyphenyl)piperazin-1-yl]-2-[(S)-(2-methoxyphenyl)(1-naphthyl)methyl]-2-methyl-3-oxopropanenitrile

A solution of (*S,S*)2-cyano-3-(2-methoxy-phenyl)-2-methyl-3-naphthalen-1-yl-propionic acid (0.45g, 1.30 mmol) in THF (150mL) is treated with DMF (2 drops). Oxalyl chloride (0.16mL, 1.84 mmol) is added dropwise in order to control gas evolution; when the gas evolution stopped the solution is heated to reflux for 5 minutes. The solution is cooled, the THF is evaporated *in vacuo* and the solid is dissolved in dry toluene (15mL) and evaporated to a solid. This procedure is repeated twice. The acid chloride is dissolved in dichloromethane (10mL) this is added to a solution of 1-(3,5-dimethoxy-phenyl)-piperazine (305mg, 1.36 mmol) and a crystal of DMAP in dichloromethane (15mL). This is followed by the dropwise addition of TEA (0.6mL, 4.27 mmol). The reaction is stirred overnight. The reaction mixture is diluted with dichloromethane (50mL), washed with aqueous HCl (10mL 0.5N) then saturated NaHCO₃ (10mL) and brine (10mL). The sample is dried over NaSO₄, filtered and concentrated *in vacuo.* Chromatography on silica gel using 30% ethyl acetate/hexanes provided 510mg the title compound as a white solid. Recrystallization from ethyl acetate/hexanes yielded colorless needles.
mp 186-188 °C;
[α]_{D}²⁵ = -153.91° (1%, CHCl₃);
¹H NMR 500MHz (DMSO-D6): δ 7.96 (d,1H, J = 7.33Hz), 7,86 (m, 2H), 7.78 (d,1H, J = 8.24Hz), 7.53 (t, 1H, J = 7.94Hz), 7.22 (t, 1H, J= 7.48Hz), 7.12 (m, 2H), 6.79 (t,1H, J = 7.49Hz), 6.01 (m, 4H), 4.01 (s,3H), 3.68 (s,6H), 3.05 (brs,4H), 1.63 (s,3H)
(ESI) *m*/*z* 550 ([M+H]+);
Anal. calcd for C₃₄H₃₅N₃O₄: C:74.29 H:6.42 N:7.64 Found: C:74.10 H:6.35 N:7.87.

### Example 59

### (S)3-(4-Indan-4-yl-piperazin-1-yl)-2-[(S) (2-methoxy-phenyl)-naphthalen-1-yl-methyl]-2-methyl-3-oxo-propionitrile

The title compound was prepared in 77% yield according to Example 58 using 1-indan-4-yl-piperazine hydrochloride. Recrystallization from ethanol/ether yielded white crystals.
mp 188-190 °C;
[α]_{D}²⁵ = -180.0° (1%,CHCl₃); ¹H NMR 500MHz (DMSO-D6): δ 7.98 (d, 1H, J = 7.07Hz), 7.87 (m, 2H), 7.79(d, 1H, J = 8.17Hz), 7.54 (t, 1H, J = 7.78Hz), 7.43 (m, 2H), 7.23 (t, 1H, J = 7.38Hz), 7.13 (m, 2H), 7.03 (t, 1H, J = 7.63Hz), 6.88 (d,1H, J = 7.33Hz), 6.79 (t,1H, J = 7.63Hz), 6.57 (brs,1H), 6.02 (s,1H), 4.01 (s, 3H), 2.79 (t, 2H, J = 7.18Hz), 2.74 (t, 2H, J = 7.03Hz), 1.94 (qiun, 2H, J = 7.18 Hz), 1.64 (s, 3H); MS (ESI) *m*/*z* 530 ([M+H]+); Anal. calcd for C₃₅H₃₅N₃O₂: C:79.37 H:6.66 N:7.93 Found: C:78.82 H:6.76 N:7.81

### Example 60

### (S,S)-3-(2-methoxyphenyl)-2-methyl-3-(1-naphthyl)-2-{[4-(1-naphthyl)piperazin-1-yl]carbonyl}propanenitrile

The title compound was prepared as a white powder, according to Example 58, using 1-naphth-4-yl-piperazine hydrochloride.
[α]_{D}²⁵ = -156.06° (1%, CHCl₃);
¹H NMR 500MHz (DMSO-D6): δ 8.13 (d,1H, J = 8.86Hz), 8.03 (d, 1H, J = 7.33Hz), 7.89 (m, 2H), 7.81 d,1H, J = 8.25Hz), 7.61 (d, 1H, J = 8.09Hz), 7.57 (t,1H, J = 8.09Hz), 7.49 (quin,2H, J = 3.82Hz), 7.43 (quin, 2H, J = 3.82Hz), 7.42 D(t, 1H, J = 8.25Hz), 7.23 (t, 1H, J = 7.33Hz), 7.14 (s,1H), 7.13 (d, 1H, J = 4.29Hz), 6.98 (brs,2H), 6.80 (t, 1H, J = 7.78Hz), 4.02 (s, 3H), 2.88 (brs, 2H), 1.67 (s, 3H)
MS (ESI) m/z 540 ([M+H]+);
Anal. calcd for C₃₆H₃₃N₃O₂: C:80.12 H:6.16 N:7.79 Found: C:79.37 H:6.42 N:7.25.

### Example 61

### (S)-3-[4-(3,4-dimethylphenyl)piperazin-1-yl]-2-[(S)-(2-methoxyphenyl)(1-naphthyl)methyl]-2-methyl-3-oxopropanenitrile

The title compound was prepared in 25% yield according to Example 58 using 1-indan-4-yl-piperazine hydrochloride. Recrystallization from ethyl acetate/hexane yielded a white solid.
mp 181-183 °C;
[α]_{D}²⁵ = -180.07° (1%, CHCl₃); H NMR 500MHz, (DMSO-D6): δ 7.97 (d,1H, J = 6.99 Hz), 7.86 (m, 2H), 7.78 (d, 1 H, J = 7.97Hz), 7.52 (t, 1H, J = 7.39Hz), 7.42 (m, 2H),7.23 (dt, 1H, J = 8.36, 1.60Hz), 7.12 (m,2H), 6.95 (d,1H, J = 8.36Hz), 6.79 (t, 1H, J = 7.57Hz), 6.70 (s,1H), 6.61 (d, 1H, J = 7.38Hz), 6.01 (s,1H), 4.01 (s, 3H), 3.67 (brs, 3H), 2.97 (brs, 3H), 2.14 (s,3H), 2.10 (s,3H), 1.63 (s,3H)
MS (ESI) *m*/*z* 518 ([M+H]+);
Anal. calcd for C₃₄H₃₅N₃O₂: C:78.89 H:6.81 N:8.12 Found: C:77.36 H:6.59 N:7.85.

### Example 62

### (RR,SS) 3-[4-(H-indol-4-yl)piperazin-1-yl]-2-[(2-methoxyphenyl)(1-naphthyl)methyl]-2-methyl-3-oxopropanenitrile

The title compound was prepared in 74% yield according to Example 58 using 4-piperazin-1-yl-1H-indole hydrochloride and (*RR*,*SS*)2-cyano-3-(2-methoxy-phenyl)-2-methyl-3-naphthalen-1-yl-propionic acid. Recrystallization from ethyl acetate/hexane yielded pink crystals.
mp 225-226 °C;
¹H NMR 500MHz (DMSO-D6): δ 11.03 (s,1H), 7.98 (d, 1Hj = 2.08Hz), 7.85 (m,2H), 7.77 (d, 1H, J = 8.17Hz), 7.53 (t,1H, J = 7.45Hz), 7,42 (m,2H), 7.21 (m 2H), 7.11 (t, 2H, J = 8.42Hz), 7.00 (t, 1 H, J = 8.06Hz), 6.78 (t,1H, J = 7.57Hz), 6.38 (s,1H), 6.34 (m,1H), 6.02 (s,1H), 3.73 (brm,3H), 2.97(brm,3HO, 1.64 (s,3H)
MS (ESI) *m*/*z* 529 ([M+H]+);
Anal. calcd for C₃₄H₃₂N₄O₂: C:77.25 H:6.10 N:10.60 Found: C:76.86 H:6.13 N:10.43.

### Example 63

### (S)-3-[4-(3-chlorophenyl)piperazin-1-yl]-2-[(S)-(2-methoxyphenyl)(1-naphthyl)methyl]-2-methyl-3-oxopropanenitrile

The title compound was prepared in 72% yield according to Example 58 using 1-(3-chlorophenyl)-piperazine hydrochloride. Recrystallization from ethyl acetate/hexane yielded white crystals.
mp 174-176 °C;
[α]_{D}²⁵ = -146.5° (1% , CHCl₃);
¹H NMR 500MHz (DMSO-D6): δ 7.97 (d, 1 H, J = 7.33Hz), 7.86 (m, 2H), 7.78 (d, 1H J = 8.24Hz), 7.52 (t, 1H, J = 7.63Hz), 7.41 (m, 2H), 7.23 (m, 2H), 7.13 (d,1H, J = 9.47Hz), 7.11 (dd, 1H, J = 6.11,1.68 Hz), 6.91 (s,1H),6.86 (dd,1H J = 8.40, 1.83Hz), 6.80 (d, 1H, J = 7.64Hz, 6.78 (dt,1H, J = 7.33, 1.06 Hz), 6.01 (s,1H), 4.01 (s, 3H), 3.75 (brm,3H), 3.12 (brm,3H), 1.63 (s,3H)
MS (ESI) m/z 524 ([M+H]+);
Anal. calcd for C₃₂H₃₀ClN₃O₂: C:73.34 H:5.77 N:8.02 Found: C:73.71 H:6.23 N:7.51.

### Example 64

### (S)-3-[4-(2,3-dimethylphenyl)piperazin-1-yl]-2-[(S)-(2-methoxyphenyl)(1-naphthyl)methyl]-2-methyl-3-oxopropanenitrile

The title compound was prepared in 67% yield according to Example 58 using 1-(2,3-dimethylphenyl)-piperazine hydrochloride. Recrystallization from ethyl acetate/hexane yielded white crystals.
mp 194-195 °C;
[α]_{D}²⁵ = -180.00° (1%, CHCl₃);
¹H NMR 500MHz (DMSO-D6): δ 7.99 (d,1H, J = 7.33Hz), 7.88 (m,1H), 7.81 (d,1H, J = 8.09Hz), 7.56 (t, 1H, J = 7.63Hz), 7.43 (quin,2H, J = 1.83Hz), 7.23 (t,1H, J = 6.88Hz), 7.13 (d,2H, J = 8.09Hz), 7.01 (t,1H, J = 7.64Hz), 6.87 (d,1H, J = 7.48Hz), 6.80 (t,1H, J = 7.63Hz), 6.71 (brs,1H), 6.03 (s,3H), 4.01 (s,3H), 3.65 (brm,3H), 2.65 (brm,3H), 2.19 (s,3H), 2.14 (s,3H), 1.65 (s,3H),
MS (ESI) *m*/*z* 518 ([M+H]+);
Anal. calcd for C₃₄H₃₅N₃O₂ : C:78.01 H:6.76 N:8.12 Found: C:78.48 H:6.88 N:8.00.

### Example 65

### (S)-3-[4-(4-chlorophenyl)piperazin-1-yl]-2-[(S)-(2-methoxyphenyl)(1-naphthyl)methyl]-2-methyl-3-oxopropanenitrile

The title compound was prepared in 35% yield according to Example 58 using 1-(4-chloro-phenyl)-piperazine dihydrochloride. Recrystallization from ethyl acetate/hexane yielded white crystals.
mp 178-180 °C;
[α]_{D}²⁵ = -155.6° (1%, CHCl₃);
¹H NMR 500MHz (DMSO-D6): δ 7.98 (d,1H, J = 7.33Hz),7.87 (m,2H), 7.98 (d,1H, J = 8.25Hz), 7.53 (t,1H, J = 7.63Hz), 7.43 (d,1H, J = 9.32Hz), 7.43 (quin,1H, J = 4.54Hz), 7.24 (m,3H), 7.14 (t,2H, J = 8.25Hz), 6.80 (t,1H, J = 7.48Hz), 6.01 (s,1H), 4.01 (s,3H), 3.65 (brs,3H), 3.09 (brm,4H), 1.64 (s,3H)
MS (ESI) *m*/*z* 524 ([M+H]+);
Anal. calcd for C₃₂H₃₀ClN₃O₂: C:73.34 H:5.77 N:8.02 Found: C:72.8 H:5.97 N:7.67

### Example 66

### (S)-3-[4-(1H-Indol-4-yl)-piperazin-1-yl]-2-[(S)-(2-methoxy-phenyl)-naphthalen-1-yl-methyl]-2-methyl-3-oxo-propionitrile

The title compound was prepared in 67% yield according to Example 58 using of (*S,S*)2-cyano-3-(2-methoxy-phenyl)-2-methyl-3-naphthalen-1-yl-propionic acid Recrystallization from ethyl acetate/hexane yielded white crystals.
mp 174-176 °C;
[α]_{D}²⁵ = -191.9° (1%, CHCl₃);
MS (ESI) *m*/*z* 529 ([M+H]+);
Anal. calcd for C₃₄H₃₂N₄O₂: C:77.25 H:6.10 N:10.60 Found: C:75.4 H:6.31 N:10.16

### Example 67

### (S,S)-3-(2-methoxyphenyl)-2-methyl-3-(1-naphthyl)-2-({4-[3-(trifluoromethyl)phenyl] piperidin-1-yl}carbonyl)propanenitrile

The title compound was prepared in 75% yield according to Example 58 using 4-(3-trifluoromethyl-phenyl)-piperidine. Recrystallization from ethyl acetate/hexane yielded white crystals
mp 123-127 °C;
[α]_{D}²⁵ = -144.4° (1%, CHCl₃);
¹H NMR 500MHz (DMSO-D6): δ 8.02 (d,1 H, J = 7.03Hz), 7.88 (m,2H), 7.82 (d,1H, J = 8.25Hz), 7.56 (t,1H, J = 7.79Hz),7.51 (brm,2H), 7.42 (d,1H, J = 7.48Hz), 7.42 (quin,1H, J = 3.51 Hz), 7.23 (t,1H, J = 7.03Hz), 7.14 (m,2H), 6.80 (t,1H, J = 7.48Hz), 6.02 (s,1H),4.37 (brm, 2H), 4.01 (s, 3H), 2.88 (brm, 2H), 1.66 (s, 3H)
MS (ESI) *m*/*z* 557 ([M+H]+);
Anal. calcd for C₃₄H₃₁F₃N₂O₂: C:73.35 H:5.61 N:5.03 Found: C:73.99 H:6.00 N:4.72

### Example 68

### (S)-3-[4-(4-Chloro-phenyl)3,6-dihydro-2H-pyridin-1-yl]-2-[(S)-(2-methoxy-phenyl)-naphthalen-1-yl-methyl]-2-methyl-3-oxo-propionitrile

The title compound was prepared in 75% yield according to Example 58 using 4-(4-chloro-phenyl)-1,2,3,6-tetrahydro-pyridine.. Recrystallization from ethyl acetate/hexane yielded white crystals.
mp 118-122°C;
[α]_{D}²⁵ 5 = -129.1° (1%, CHCl₃);
¹H NMR 500MHz (DMSO-D6): δ 7.94 (d ,1H, J = 7.17Hz), 7.92 (d ,1H, J = 8.70Hz), 7.79 (brm,1H), 7.76 (d ,1H, J = 8.25Hz), 7.49 (t ,1H, J = 7.64Hz), 7.39 (m,6H), 7.23 (t ,1H, J = 7.18Hz),7.18 (brd,1H, J = 6.24Hz), 7.12 (d ,1H, J = 8.40Hz), 6.82 (d ,1H, J = 7.48Hz), 6.11 (brs,1H), 6.03 (s,1H3.99 (s,1H), 1.64 (s,3H)
MS (ESI) *m*/*z* 521 ([M+H]+);
Anal. calcd for C₃₃H₂₉ClN₂O₂: C:76.07 H:5.61 N:5.38 Found: C:75.51 H:5.63 N:5.19

### Example 69

### (S,S)-3-(2-Methoxy-phenyl)-2-methyl-3-naphthalen-1-yl-2-[4-(3-trifluoromethylphenyl)-3,6-dihydro-2H-pyridine-1-carbonyl]-propionitrile

The title compound was prepared in 49% yield, as a white solid, according to Example 58 using 4-(3-trifluoromethylphenyl)-1,2,3,6-tetrahydro-pyridine hydrochloride.
[α]_{D}²⁵ = -123.5° (1 % , CHCl₃)
¹H NMR 500MHz (DMSO-D6): δ 7.97 (d, 1H, J = 3.30Hz), 7.91 (d, 1 H, J = 8.70Hz), 7.76 (d, 1H, J = 3.30Hz),7.76 (brs,1H), 7.75 (d, 1H, J = 8.24Hz),7.65 (brs,1H), 7.62 (d, 1 H, J = 7.63Hz), 7.58 (d, 1H, J = 7.60Hz), 7.50 (t, 1H, J = 7.64Hz), 7.42 (t, 1H, J = 7.48Hz), 7.38 (brm,1H), 7.24 (t, 1 H, J = 7.18Hz), 7.22, (brm, 1H), 7.12 (d, 1H, J =8.25 Hz), 6.82 (t,1H, J = 7.64Hz), 6.21 (brs,1H), 6.03 (s,1H), 4.01 s,3H), 1.65 (s, 3H)
MS (ESI) *m*/*z* 555 ([M+H]+)
Anal. calcd for C₃₄H₂₉F₃N₂O₂: C:73.63 H:5.27 N:5.05 Found: C:73.45 H:5.51 N:5.06.

### Example 70

### (SS)2-[4-(4-Chloro-phenyl)-piperidine-1-carbonyl]-3-(2-methoxy-phenyl)-2-methyl-3-naphthalen-1-yl-propionitrile

The title compound was prepared in 97% yield, as a white solid, according to Example 58 using 4-(4-chlorophenyl)piperidine hydrochloride
mp 156-158 °C;
[α]_{D}²⁵ = -174.89°, (1%, CHCl₃);
¹H NMR (DMSO-D6): δ 8.01 (d,1H, J = 6.87Hz), 7.88 (t,2H, J = 10.09Hz), 7.83 (d,1H, J = 8.25Hz), 7.56 (t,1H, J = 7.63Hz), 7.42 (quin,2H, J = 9.05Hz), 7.30 (brm,1H). 7.23 (t,1H, J = 7.18Hz), 7.13 (d,2H, J = 8.24Hz), 6.80 (t,1H, J = 7.38Hz), 6.01 (s,1H), 4.01 (s,3H),1.64 (s,3H)
MS (ESI) *m*/*z* 523 ([M+H]+);
Anal. calcd for C₃₃H₃₁ClN₂O₂: C:75.78 H:5.97 N:5.36 Found: C:75.58 H:5.99 :N: 5.07

### Example 71

### (RR,SS)-3-(2-methoxyphenyl)-2-methyl-3-(1-naphthyl)-2-({4-[3-(trifluoromethyl) phenyl]piperidin-1-yl}carbonyl)propanenitrile

The title compound was prepared in 35% yield according to Example 56 using racemic 4-(3-trifluoromethyphenyl)piperidine hydrochloride.
MS (ESI) m/z 595 ([M+K]+);
Anal. calcd for C₃₄H₃₁F₃N₂O₂: C:73.37 H:5.61 N:5.03 Found: C:73.31 H:5.88 N:4.78.

### Example 72

### (RR,SS)-3-(2-methoxyphenyl)-2-methyl-3-(1-naphthyl)-2-(piperidin-1-ylcarbonyl)propanenitrile

(*RR,SS*)-2-cyano-3-(2-methoxyphenyl)-2-methyl-3-(1-naphthyl)propanoic acid(1 g, 2.89 mmol) was dissolved in 50 mL THF. A catalytic amount of DMF was added to the reaction mixture. Oxalyl chloride (0.303 mL, 3.47 mmol) was added slowly. The reaction mixture was stirred until no more evolution of gas was observed. The reaction mixture was rapidly heated to reflux, then cooled and evaporated. 50 mL toluene was added and the reaction mixture was evaporated to dryness. The acid chloride was dissolved in 10 mL CH₂Cl₂ and placed under nitrogen. In a separate flask piperidine (0.49 mL, 4.91 mmol) was dissolved in 50 mL CH₂Cl₂ under nitrogen. To this stirred solution was added a catalytic amount of DMAP, and triethylamine (0.69 mL, 4.91 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and recrystallized from ethanol to yield 851 mg of the title compound as a crystalline solid.
mp 194-196 °C;
1H NMR 400 MHz (DMSO-D6): δ 7.94 (d, 1H, J=7.32 Hz), 7.85 (m, 2H), 7.78 (d, 1H, J=8.30 Hz), 7.52 (t, 1 H, J=7.32 Hz), 7.40 (m, 2H), 7.21 (td, 1 H, J=7.75 Hz, 1.71 Hz), 7.12 (dd, 1 H J=8.30 Hz, 0.98 Hz), 7.06 (dd, 1H, J=7.81 Hz, 1.71 Hz), 6.77 (td, 1H, J=7.57 Hz, 0.98 Hz), 5.99 (s, 1 H) 4.01 (s, 3H), 3.49 (bs, 4H), 1.58 (s, 1 H), 1.41 (bs, 6H)
MS (APCI) m/z 413 ([M+H]+);
Anal. calcd for C₂₇H₂₈N₂O₂: C:78.61 H:6.84 N:6.79 Found: C:78.32 H:6.86 N:6.69.

### Example 73

### (RR,SS)-2-cyano-3-(2-methoxyphenyl)-2-methyl-3-(1-naphthyl)propanamide

(*RR,SS*)-2-cyano-3-(2-methoxyphenyl)-2-methyl-3-(1-naphthyl)propanoic acid(345 mg, 1 mmol) was dissolved in 10 mL THF. A catalytic amount of DMF was added to the reaction mixture. Thionyl chloride (131 mg, 1.1 mmol) was added slowly. The reaction mixture was stirred 2 hours. The reaction mixture was rapidly heated to reflux, then cooled and evaporated. 10 mL toluene was added and the reaction mixture was evaporated to dryness. The acid chloride was dissolved in 5 mL CH₂Cl₂ and placed under nitrogen. In a separate flask, methanolic ammonia (2M 1.5 mL, 3 mmol) was dissolved in 10 mL CH₂Cl₂ under nitrogen. To this stirred solution was added a catalytic amount of DMAP, and triethylamine (0.35 mL, 2.5 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and purified on silica gel (30% ethyl acetate/hexanes) to yield 66 mg of the title compound.
1 H NMR 500 MHz (DMSO-D6): δ 8.07 (d, 1 H, J=7.18 Hz), 7.94 (m, 1 H), 7.85 (m, 1H), 7.79 (m, 2H), 7.53 (t, 1H, J=7.79 Hz), 7.41 (m, 2H), 7.35 (s, 1H), 7.20 (td, 1 H, J=7.55 Hz, 1.53 Hz), 7.11 (d, 1H, J=7.94 Hz), 7.08 (dd, 1H, J=7.78 Hz, 1.53 Hz), 6.76 (t, 1H, J=7.17 Hz), .85 (s, 1 H), 4.03 (s, 3H), 1.57 (s, 3H).
MS (ESI) m/z 362 ([M+NH4]+);
Anal. calcd for C₂₂H₂₀N₂O₂: C:76.72 H:5.85 N:8.13 Found: C:76.68 H:5.83 N:8.13.

### Example 74

### (RR,SS)-2-cyano-N-ethyl-3-(2-methoxyphenyl)-2-methyl-3-(1-naphthyl)propanamide

(*RR,SS*)-2-cyano-3-(2-methoxyphenyl)-2-methyl-3-(1-naphthyl)propanoic
acid(345 mg, 1 mmol) was dissolved in 10 mL THF. A catalytic amount of DMF was added to the reaction mixture. Thionyl chloride (131 mg, 1.1 mmol) was added slowly. The reaction mixture was stirred 2 hours. The reaction mixture was rapidly heated to reflux, then cooled and evaporated. 10 mL toluene was added and the reaction mixture was evaporated to dryness. The acid chloride was dissolved in CH₂Cl₂ (5 mL) and placed under nitrogen, In a separate flask, ethyl amine (135 mg, 3 mmol) was dissolved in 10 mL CH₂Cl₂ under nitrogen. To this stirred solution was added a catalytic amount of DMAP, and triethylamine (0.35 mL, 2.5 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and purified on silica gel (30% ethyl acetate/hexanes) to yield 89 mg of the title compound.
¹H NMR 500 MHz (DMSO-D6): δ 8.27 (t, 1H, J=5.65 Hz), 8.01 (d, 1H, J=7.33 Hz), 7.94 (m, 1 H), 7.84 (m, 1 H), 7.79 (d, 1 H, J=8.25 Hz), 7.51 (t, 1H, J=7.64 Hz), 7.41 (m, 2H), 7.21 (td, 1H, J=7.79 Hz, 1.60 Hz), 7.13 (m, 2H), 7.78 (td, 1 H, 7.48 Hz, 0.91 Hz), 5.86 (s, 1H), 4.02 (s, 3H), 2.89 (q; 1H, J=7.02 Hz), 2.87 (q, 1H, J=7.02 Hz), 1.55 (s, 3H), 0.58 (t, 3H, J=7.18 Hz)
MS (ESI) m/z 373 ([M+H]+);
Anal. calcd for C₂₄H₂₄N₂O₂: C:77.39 H:6.49 N:7.52 Found: C:77.37 H:6.65 N:7.59.

### Example 75

### (RR,SS)-N-(tert-butyl)-2-cyano-3-(2-methoxyphenyl)-2-methyl-3-(1-naphthyl)propanamide

(*RR,SS*)-2-cyano-3-(2-methoxyphenyl)-2-methyl-3-(1-naphthyl)propanoic acid (345 mg, 1 mmol) was dissolved in 10 mL THF. A catalytic amount of DMF was added to the reaction mixture. Thionyl chloride (131 mg, 1.1 mmol) was added slowly. The reaction mixture was stirred 2 hours. The reaction mixture was rapidly heated to reflux, then cooled and evaporated. 10 mL toluene was added and the reaction mixture was evaporated to dryness. The acid chloride was dissolved in 5 mL CH₂Cl₂ and placed under nitrogen. In a separate flask, *tert*-butyl amine (219 mg, 3 mmol) was dissolved in 10 mL CH₂Cl₂ under nitrogen. To this stirred solution was added a catalytic amount of DMAP, and triethylamine (0.35 mL, 2.5 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and purified on silica gel (30% ethyl acetate/hexanes) to yield 135 mg of the title compound.
¹H NMR 500 MHz (DMSO-D6): δ 8.02 (d, 1H, J=7.18 Hz), 7.95 (m, 1 H), 7.84 (m, 1H), 7.78 (d, 1H, J=8.25 Hz), 7.52 (t, 1H, J=7.94 Hz), 7.41 (m, 2H), 7.22 (s, 1H), 7.20 (dd, 1H, J=7.48 Hz, 1.07 Hz), 7.15 (dd, 1H, J=7.79 Hz, 1.68 Hz), 7.11 (d, 1H, J=7.64 Hz), 6.78 (td, 1H, J=7.48 Hz, 0.92 Hz), 5.86 (s, 1H), 4.02 (s, 3H), 1.59 (s, 3H), 0.94 (s, 9H)
MS (ESI) m/z 399 ([M-H]-);
Anal. calcd for C₂₆H₂₈N₂O₂: C:77.97 H:7.05 N:6.99 Found: C:77.80 H:7.19 N:6.84.

### Example 76

### (RR,SS)-2-cyano-3-(2-methoxyphenyl)-N,N,2-trimethyl-3-(1-naphthyl)propanamide

(*RR,SS*)-2-cyano-3-(2-methoxyphenyl)-2-methyl-3-(1-naphthyl)propanoic acid(345 mg, 1 mmol) was dissolved in 10 mL THF. A catalytic amount of DMF was added to the reaction mixture. Thionyl chloride (131 mg, 1.1 mmol) was added slowly. The reaction mixture was stirred 2 hours. The reaction mixture was rapidly heated to reflux, then cooled and evaporated. 10 mL toluene was added and the reaction mixture was evaporated to dryness. The acid chloride was dissolved in 5 mL CH₂Cl₂ and placed under nitrogen. In a separate flask, dimethyl amine (135 mg, 3 mmol) was dissolved in 10 mL CH₂Cl₂ under nitrogen. To this stirred solution was added a catalytic amount of DMAP, and triethylamine (0.35 mL, 2.5 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and purified on silica gel (30% ethyl acetate/hexanes) to yield 129 mg of the title compound.
¹H NMR 500 MHz (DMSO-D6): δ 7.91 (d, 1 H, J=7.18 Hz), 7.86 (t, 1H, J=6.41 Hz), 7.85 (t, 1H, J=7.18 Hz), 7.78 (d, 1H, J=8.25 Hz), 7.52 (t, 1H, J=7.64 Hz), 7.41 (m, 2H), 7.21 (td, 1H, J=7.79 Hz, 1.53 Hz), 7.12 (d, 1H, J=7.64 Hz), 7.08 (dd, 1H, J=6.26 Hz, 1.53 Hz), 6.77 (td, 1H, J=7.48 Hz, 0.84 Hz), 6.01 (s, 1H), 4.01 (s, 3H) 2.95 (bs, 6H), 1.60 (s, 3H)
MS (ESI) *m*/*z* 373 ([M+H]+);
Anal. calcd for C₂₄H₂₄N₂O₂: C:77.39 H:6.49 N:7.52 Found: C:77.00 H:6.65 N:7.46.

### Example 77

### (RR,SS)-2-cyano-N-methoxy-3-(2-methoxyphenyl)-N,2-dimethyl-3-(1-naphthyl)propanamide

(*RR,SS*)-2-cyano-3-(2-methoxyphenyl)-2-methyl-3-(1-naphthyl)propanoic acid(1 g, 2.89 mmol) was dissolved in 50 mL THF. A catalytic amount of DMF was added to the reaction mixture. Oxalyl chloride (0.303 mL, 3.47 mmol) was added slowly. The reaction mixture was stirred until no more evolution of gas was observed. The reaction mixture was rapidly heated to reflux, then cooled and evaporated. 50 mL toluene was added and the reaction mixture was evaporated to dryness. The acid chloride was dissolved in 10 mL CH₂Cl₂ and placed under nitrogen. In a separate flask N-O-dimethyl-hydroxyl amine hydrochloride (424 mg, 4.34 mmol) was dissolved in 50 mL CH₂Cl₂ under nitrogen. To this stirred solution was added a catalytic amount of DMAP, and triethylamine (0.69 mL, 4.91 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and recrystallized from methanol to yield 644 mg of the title compound as a crystalline solid.
mp 151-153 °C;
¹H NMR 500 MHz (DMSO-D6): δ 7.79 (d, 1H, J=7.18 Hz), 7.85 (m, 2H), 7.79 (d, 1 H, J=8.25 Hz), 7.54 (t, 1H, J=7.79 Hz), 7.41 (m, 2H), 7.21 (td, 1H, J=8.86 Hz, 1.68 Hz), 7.13 (d, 1H, J=8.25 Hz), 7.06 (dd, 1H, J=7.79 Hz, 1.37 Hz), 6.76 (t, 1H, J=7.48 Hz), 6.01 (s, 1 H), 4.03 (s, 3H), 3.82 (s, 3H), 3.00 (s, 3H), 1.59 (s, 3H)
MS (ESI) m/z 389 ([M+H]+);
MS (ESI) m/z 406 ([M+NH4]+);
Anal. calcd for C₂₄H₂₄N₂O₃: C:74.21 H:6.23 N:7.21 Found: C:73.82 H:6.20 N:7.02.

### Example 78

### (RR,SS)-2-benzyl-3-[4-(3,5-dichloro-4-pyridinyl)-1-piperazinyl]-2-[(2-methoxyphenyl)(1-naphthyl)methyl]-3-oxopropanenitrile

(*RR,SS*)-2-benzyl-2-cyano-3-(2-methoxyphenyl)-3-(1-naphthyl)propanoic acid (250 mg, mmol) was dissolved in 10 mL THF. A catalytic amount of DMF was added to the reaction mixture. Oxalyl chloride (0.062 mL, 0.712 mmol) was added slowly. The reaction mixture was stirred until no more evolution of gas was observed. The reaction mixture was rapidly heated to reflux, then cooled and evaporated. 20 mL toluene was added and the reaction mixture was evaporated to dryness. The acid chloride was dissolved in 5 mL CH₂Cl₂ and placed under nitrogen. In a separate flask 1-(3,5-dichloropyridin-4-yl)piperazine (207 mg, 0.890 mmol) was dissolved in 15 mL CH₂Cl₂ under nitrogen. To this stirred solution was added a catalytic amount of DMAP, and triethylamine (0.250 mL, 1.78 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and purified on silica gel (20% ethyl acetate/Hexanes +1% NH₄OH) to yield 140 mg of the title compound as a white foam.
¹H NMR (DMSO-D6): δ 8.42 (s, 1H), 8.02 (d, 1H, J=6.37 Hz), 7.95 (d, 1H, J=4.41 Hz), 7.87 (d, 1 H, J=5.43 Hz), 7.82 (d, 1H, 8.19 Hz), 7.57 (t, 1 H, J=7.63 Hz), 7.47 (m, 2H), 7.35 (m, 3H), 7.28 (m, 3H), 7.19 (m, 3H), 6.86 (t, 1 H, J=7.54 Hz), 6.36 (s, 1 H), 4.07 (s, 3H)
MS (ESI) m/z 635 ([M+H]+);
Anal. calcd for C₃₇H₃₂Cl₂N₄O₂ • 0.20 C₆H₁₄: C:70.28 H:5.37 N:8.58 Found: C:70.03 H:5.04 N:8.26.

### Example 79

### (R,S)-3-[4-(2,3-dimethylphenyl)piperazin-1-yl]-2-[(R,S)-(2-methoxyphenyl)(1-naphthyl)methyl]-2-methyl-3-oxopropanenitrile

(*RR,SS*)*-*2-cyano-3-(2-methoxyphenyl)-2-methyl-3-(1-naphthyl)propanoic acid(5 g, 14.47 mmol) was dissolved in 50 mL THF. A catalytic amount of DMF was added to the reaction mixture. Oxalyl chloride (1.52 mL, 17.36 mmol) was added slowly. The reaction mixture was stirred until no more evolution of gas was observed. The reaction mixture was rapidly heated to reflux, then cooled and evaporated. 50 mL toluene was added and the reaction mixture was evaporated to dryness. The acid chloride was dissolved in 50 mL CH₂Cl₂ and placed under nitrogen. In a separate flask 1-(2,3-Dimethyl-phenyl)-piperazine hydrochloride (5.58 g, 24.60 mmol) was dissolved in 100 mL CH₂Cl₂ under nitrogen. To this stirred solution was added a catalytic amount of DMAP, and triethylamine (6.87 mL, 49.20 mmol). The acid chloride in CH₂Cl₂ was added rapidly to this stirred mixture and allowed to react for 2 hours after which water was added to the reaction mixture. The organic layer was separated and washed with brine, dried over MgSO₄, filtered, evaporated to dryness, and recrystallized from ethyl acetate/hexanes to yield 2.37 g of the title compound as a crystalline solid.
mp 219°C;
1 H NMR 500 MHz (DMSO-D6): δ 8.00 (d, 1H, J=7.18 Hz), 7.88 (m, 2H), 7.80 (d, 1 H, J=8.25Hz), 7.55 (t, 1H, J=7.64 Hz), 7.42 (m, 2H), 7.23 (td, 1 H, J=8.70 Hz, 1.68 Hz), 7.13 (dd, 2H, J=7.94 Hz, 1.68 Hz), 7.00 (t, 1H, J=7.79 Hz), 6.80 (t, 1H, J=7.94 Hz), 6.70 (bs, 1 H), 6.02 (s, 1 H), 4.01 (s, 3H), 3.70 (bs, 4H), 2.62 (bs, 4H), 2.18 (s, 3H), 2.13 (s, 3H), 1.65 (s, 3H)
MS (ESI) m/z 518 ([M+H]+);
Anal. calcd for C₃₄H₃₅N₃O₂: C:78.89 H:6.81 N:8.12 Found: C:78.76 H:6.93 N:7.94.

### Example 80

### (R,S)-3-[4-(3-isopropylphenyl)piperazin-1-yl]-2-[(R,S)-(2-methoxyphenyl)(1-naphthyl)methyl]-2-methyl-3-oxopropanenitrile

(*RR,SS*)-2-cyano-3-(2-methoxyphenyl)-2-methyl-3-(1-naphthyl)propanoic acid(0.25 g, 0.72 mmol) was dissolved in 10 mL THF. A catalytic amount of DMF was added to the reaction mixture. Oxalyl chloride (0.075 mL, 0.87 mmol) was added slowly. The reaction mixture was stirred until no more evolution of gas was observed. The reaction mixture was rapidly heated to reflux, then cooled and evaporated. 20 mL toluene was added and the reaction mixture was evaporated to dryness. The acid chloride was dissolved in 10 mL CH₂Cl₂ and placed under nitrogen. In a separate flask 1-(3-isopropyl-phenyl)-piperazine (178 mg, 0.087 mmol) was dissolved in 10 mL CH₂Cl₂ under nitrogen. To this stirred solution was added a catalytic amount of DMAP, and triethylamine (0.35 mL, 2.53 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, and purified on silica gel (35% ethyl acetate/hexanes) to yield 150 mg of the title compound as a white powder.
¹H NMR 500 MHz (DMSO-D6): δ 7.97 (d, 1H, J=7.18 Hz), 7.88 (m, 1H), 7.84 (m, 1H), 7.78 (d, 1H, J=8.09 Hz), 7.52 (t, 1H, J=7.64 Hz), 7.42 (m, 2H), 7.23 (td, 1H, J=7.79 Hz, 1.53 Hz), 7.12 (m, 3H), 6.79 (t, 1H, J=7.48 Hz), 6.69 (d, 2H, J=7.64 Hz), 6.01 (s, 1 H), 4.01 (s, 3H), 3.68 (bs, 4H), 3.03 (bs, 4H), 2.79 (sep, 1H, J=6.87 Hz), 1.64 (s, 3H), 1.17 (s, 3H), 1.15 (s, 3H)
MS (ESI) m/z 532 ([M+H]+);
Anal. calcd for C₃₅H₃₇N₃O₂ • 0.10 H₂O • 0.15 C₆H₁₄: C:78.91 H:7.25 N:7.69 Found: C:78.91 H:7.49 N:7.48.

### Example 81

### (RR,SS)-3-[4-(3,5-dichloropyridin-4-yl)piperazin-1-yl]-2-[(2-methoxyphenyl)(1-naphthyl)methyl]-2-methyl-3-oxopropanenitrile

(*RR*,*SS*)-2-cyano-3-(2-methoxyphenyl)-2-methyl-3-(1-naphthyl)propanoic acid(1.5 g, 4.34 mmol) was dissolved in 50 mL THF. A catalytic amount of DMF was added to the reaction mixture. Oxalyl chloride (0.46 mL, 5.21 mmol) was added slowly. The reaction mixture was stirred until no more evolution of gas was observed. The reaction mixture was rapidly heated to reflux, then cooled and evaporated. 50 mL toluene was added and the reaction mixture was evaporated to dryness. The acid chloride was dissolved in 20 mL CH₂Cl₂ and placed under nitrogen. In a separate flask 2,6-dichloro-4-pyridyl-piperazine (1.51 g, 6.51 mmol) was dissolved in 20 mL CH₂Cl₂ under nitrogen. To this stirred solution was added a catalytic amount of DMAP, and triethylamine (1.81 mL, 13.02 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, and recrystallized from ethanol to yield 1.92 g of the title compound as a crystalline solid.
mp 242-243 °C;
¹H NMR 500 MHz (DMSO-D6): δ 8.44 (s, 2H), 8.00 (d, 1 H, J=33 Hz), 7.87 (m, 2H), 7.80 (d, 1H, J=8.25 Hz), 7.55 (t, 1H, J=7.64 Hz), 7.42 (m, 2H), 7.23 (td, 1H, J=7.79 Hz, 1.53 Hz), 7.14 (m, 2H), 6.80 (td, 1H, J=7.48, 0.92 Hz), 6.02 (s, 1 H), 4.01 (s, 3H). 3.65 (bs, 4H), 3.27 (bs, 4H), 1.66 (s, 3H)
MS (ESI) m/z 559 ([M+H]+);
Anal. calcd for C₃₁H₂₈Cl₂N₄O₂: C:66.55 H:5.04 N:10.01 Found: C:66.29 H:4.79 N:9.76.

### Example 82

### (2SS)-3-[4-(3-chloro-2-methylphenyl)piperazin-1-yl]-2-[(2-methoxyphenyl)(1-naphthyl)methyl]-2-methyl-3-oxopropanenitrile

(S*,*S)-2-cyano-3-(2-methoxyphenyl)-2-methyl-3-(1-naphthyl)propanoic acid(12 g, 34.74 mmol) was dissolved in 500 mL THF. A catalytic amount of DMF was added to the reaction mixture. Oxalyl chloride (3.64 mL, 41.69 mmol) was added slowly. The reaction mixture was stirred until no more evolution of gas was observed. The reaction mixture was rapidly heated to reflux, then cooled and evaporated. 250 mL toluene was added and the reaction mixture was evaporated to dryness. The acid chloride was dissolved in 250 mL CH₂Cl₂ and placed under nitrogen. In a separate flask 1-(3-chloro-2-methylphenyl)piperazine hydrochloride (10.67 g, 41.69 mmol) was dissolved in 250 mL CH₂Cl₂ under nitrogen. To this stirred solution was added a catalytic amount of DMAP, and triethylamine (21.80 mL, 156.33 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, purified on silica gel (20% hexanes/CH₂Cl₂), the purified fractions were evaporated and recrystallized from ethyl acetate/hexanes to yield 12.58 g of the title compound as a crystalline solid.
mp 150-153 °C;
[α]_{D}²⁵ = -167.9° (1% , CHCl3);
1 H NMR 500 MHz (DMSO-D6): δ 8.00 (d, 1H, J=7.33 Hz), 7.88 (m, 2H), 7.86 (d(1H, J=8.25 Hz), 7.55 (t, 1H, J=7.64 Hz), 7.43 (m, 2H), 7.23 (td, 1H, J=7.79 Hz, 1.37 Hz), 7.14 (m, 3H), 6.86 (bs, 1H), 6.80 (t, 1H, J=7.48 Hz), 6.02 (s, 1H), 4.01 (s, 3H), 3.69 (bs, 4H), 2.70 (bs, 4H), 2.27 (s, 3H), 1.65 (s, 3H)
MS (ESI) m/z 538 ([M+H]+);
Anal. calcd for C₃₃H₃₂ClN₃O₂: C:73.66 H:5.99 N:7.81 Found: C:73.33 H:6.04 N:7.72.

### Example 83

### (S)-3-[4-(2-fluorophenyl)piperazin-1-yl]-2-[(S)-(2-methoxyphenyl)(1-naphthyl)methyl]-2-methyl-3-oxopropanenitrile

(S,S)-2-cyano-3-(2-methoxyphenyl)-2-methyl-3-(1-naphthyl)propanoic acid(450 mg, 1.30 mmol) was dissolved in 20 mL THF. A catalytic amount of DMF was added to the reaction mixture. Oxalyl chloride (0.160 mL, 1.82 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(2-flouro- phenyl)piperazine hydrochloride (247 mg, 1.37 mmol) was dissolved in 15 mL CH₂Cl₂ under nitrogen. To this stirred solution was added a catalytic amount of DMAP, and triethylamine (0.82 mL, 5.86 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, and recrystallized from methanol to yield 480 mg of the title compound as a crystalline solid.
mp 145-148 °C;
[α]_{D}²⁵ = -172.15° (1% , CHCl3);
¹H NMR 500 MHz (DMSO-D6): δ 7.98 (d,1H, J=7.33 Hz), 7.87 (m, 2H), 7.79 (d, 1H, J=8.25 Hz), 7.54 (t, 1H, J=7.94 Hz), 7.42 (m, 2H), 7.23 (td, 1H, J=8.86 Hz, 1.53 Hz), 7.12 (m, 4H), 6.98 (m, 1H), 6.93 (bs, 1H), 6.79 (t, 1H, J=7.33 Hz), 6.01 (s, 1H), 4.01 (s, 3H), 3.69 (bs, 4H), 2.88 (bs, 4H), 1.64 (s, 3H)
MS (ESI) m/z 508 ([M+H]+);
Anal. calcd for C₃₂H₃₀FN₃O₂ • 0.25 H₂O: C:75.05 H:6.00 N:8.21 Found: C:75.02 H:6.07 N:8.23.

### Example 84

### (S)-3-[4-(2-chlorophenyl)piperazin-1-yl]-2-[(S)-(2-methoxyphenyl)(1-naphthyl) methyl]-2-methyl-3-oxopropanenitrile

(S,S)-2-cyano-3-(2-methoxyphenyl)-2-methyl-3-(1-naphthyl)propanoic acid(450 mg, 1.30 mmol) was dissolved in 20 mL THF. A catalytic amount of DMF was added to the reaction mixture. Oxalyl chloride (0.160 mL, 1.82 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(2-chloro- phenyl)piperazine hydrochloride (269 mg, 1.37 mmol) was dissolved in 15 mL CH₂Cl₂ under nitrogen. To this stirred solution was added a catalytic amount of DMAP, and triethylamine (0.82 mL, 5.86 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, and recrystallized from methanol to yield 430 mg of the title compound as a crystalline solid.
mp 145-147 °C;
[α]_{D}²⁵ = -168.03° (1% solution, CHCl₃);
1H NMR 500 MHz (DMSO-D6): δ 7.98 (d,1H, J=7.18 Hz), 7.87 (m, 2H), 7.79 (d, 1H, J=8.25 Hz), 7.54 (t, 1H, J=7.94 Hz), 7.42 (m, 3H), 7.28 (t, 1H, J=7.33 Hz), 7.23 (t, 1H, J=7.18 Hz), 7.12 (m, 2H), 6.80 (t, 1H, J=7.64 Hz), 6.01 (s, 1H), 4.01 (s, 3H), 3.69 (bs, 4H), 2.88 (bs, 4H), 1.64 (s, 3H)
MS (ESI) m/z 524 ([M+H]+);
Anal. calcd for C₃₂H₃₀ClN₃O₂ •0.20 H₂O: C:72.84 H:5.81 N:7.96 Found: C:72.79 H:5.88 N:7.92.

### Example 85

### (S,S)-3-(2-methoxyphenyl)-2-{[4-(3-methoxyphenyl)piperazin-1-yl]carbonyl}-2-methyl-3-(1-naphthyl)propanenitrile

(S,S)-2-cyano-3-(2-methoxyphenyl)-2-methyl-3-(1-naphthyl)propanoic acid(450 mg, 1.30 mmol) was dissolved in 20 mL THF. A catalytic amount of DMF was added to the reaction mixture. Oxalyl chloride (0.160 mL, 1.82 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(3-methoxy- phenyl)piperazine dihydrochloride (593 mg, 1.37 mmol) was dissolved in 15 mL CH₂Cl₂ under nitrogen. To this stirred solution was added a catalytic amount of DMAP, and triethylamine (0.82 mL, 5.86 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness, and recrystallized from ethanol/diethyl ether to yield 530 mg of the title compound as a crystalline solid.
mp 147-148 °C;
[α]_{D}²⁵ = -160.87° (1 % , CHCl3);
1 H NMR 500 MHz (DMSO-D6): δ 7.97 (d, 1H, J=7.33 Hz), 7.87 (m, 1 H), 7.84 (m, 1 H), 7.78 (d, 1H, J=8.25 Hz), 7.52 (t, 1 H, J=7.79 Hz), 7.41 (m, 2H), 7.23 (td, 1 H, J=7.79 Hz, 1.53 Hz), 7.12 (m, 3H), 6.79 (t, 1 H, J=7.33 Hz), 6.48 (d, 1H, J=8.40 Hz), 6.42 (s, 1 H), 6.38 (dd, 1H, J=8.09 Hz, 1.83 Hz), 6.01 (s, 1 H), 4.01 (s, 3H), 3.70 (s, 3H), 3.67 (bs, 4H), 3.08 (bs, 4H), 1.63 (s, 3H)
MS (ESI) m/z 520 ([M+H]+);
Anal. calcd for C₃₃H₃₃N₃O₃: C:76.28 H:6.40 N:8.09 Found: C:76.06 H:6.39 N:8.06.

### Example 86

### (R,S)-3-[4-(3-chloropyridin-4-yl)piperazin-1-yl]-2-[(R,S)-(2-methoxyphenyl)(1-naphthyl)methyl]-2-methyl-3-oxopropanenitrile

(R,S)-3-[4-(3,5-dichloropyridin-4-yl)piperazin-1-yl]-2-[(R,S)-(2-methoxyphenyl)(1-naphthyl)methyl]-2-methyl-3-oxopropanenitrile (600 mg), 10% palladium on carbon (50 mg), triethyl amine (1 mL) were dissolved in THF (20 mL), evacuated and placed under one atmosphere of hydrogen overnight. The reaction mixture was filtered and evaporated to dryness. Purification of silica gel (50% ethyl acetate/CH₂Cl₂) afforded 167 mg of the title compound as an off white solid
¹H NMR 400 MHz (DMSO-D6): δ 8.41 (s, 1 H), 8.32 (d, 1H, J=5.44 Hz), 7.97 (d, 1H, J=7.19 Hz), 7.87 (m, 2H), 7.79 (d, 1H, 8.16 Hz), 7.54 (t, 1 H, J=7.87 Hz), 7.42 (m, 2H), 7.23 (m, 1H), 7.12 (m, 2H), 7.00 (s, 1H), 6.80 (t, 1 H, J=6.80 Hz), 6.01 (s, 1 H), 4.01 (s, 3H), 3.71 (bs, 4H), 3.25 (bs, 4H), 1.64 (s, 3H); MS (ESI) m/z 525 ([M+H]+);
Anal. calcd for C₃₁H₂₉ClN₄O₂ • 0.30 C₆H₁₄ •CH₂Cl₂: C:70.22 H:5.99 N:9.94 Found: C:70.31 H:5.86 N:9.81.

### Example 87

### (S)-3-[4-(2,3-dichlorophenyl)piperazin-1-yl]-2-[(S)-(2-methoxyphenyl)(1-naphthyl)methyl]-2-methyl-3-oxopropanenitrile

(S,S)-2-cyano-3-(2-methoxyphenyl)-2-methyl-3-(1-naphthyl)propanoic acid (450 mg, 1.30 mmol) was dissolved in 20 mL THF. A catalytic amount of DMF was added to the reaction mixture. Oxalyl chloride (0.160 mL, 1.82 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(2,3-dichloro- phenyl)piperazine hydrochloride (367 mg, 1.37 mmol) was dissolved in 15 mL CH₂Cl₂ under nitrogen. To this stirred solution was added a catalytic amount of DMAP, and triethylamine (0.82 mL, 5.86 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness. Purification of silica gel (CH₂Cl₂) afforded 462 mg of the title compound as a powder.
[α]_{D}²⁵ = -159.1°(1%, CHCl3);
¹H NMR 500 MHz (DMSO-D6): δ 799 (d, 1H, J=7.33 Hz), 7.87 (m, 2H), 7.79 (d, 1 H, 8.25 Hz), 7.55 (t, 1 H, J=7.79 Hz), 7.42 (m, 2H), 7.31 (m, 2H), 7.23 (m, 1H), 7.3 (d, 2H, J=8.09 Hz), 7.02 (bs, 1 H), 6.80 (t, 1H, J=7.48 Hz), 6.01 (s, 1 H), 4.01 (s, 3H), 3.67 (bs, 4H), 2.85 (bs, 4H), 1.64 (s, 3H)
MS (ESI) m/z 558 ([M+H]+);
Anal. calcd for C₃₂H₂₉Cl₂N₃O₂ • 0.25 C₆H₁₄: C:69.37 H:5.65 N:7.24 Found: C:69.61 H:5.53 N:7.24.

### Example 88

### (2S)-3-[4-[4-chloro-3-(trifluoromethyl)phenyl]-3,6-dihydropyridin-1(2H)-yl]-2-[(S)-(2-methoxyphenyl)(1-naphthyl)methyl]-2-methyl-3-oxopropanenitrile

(S,S)-2-cyano-3-(2-methoxyphenyl)-2-methyl-3-(1-naphthyl)propanoic acid(500 mg, 1.45 mmol) was dissolved in 20 mL THF. A catalytic amount of DMF was added to the reaction mixture. Oxalyl chloride (0.152 mL, 1.73 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(4-chloro-3-triflouromethyl phenyl)-3,6-dihydropyridine hydrochloride (520 mg, 1.73 mmol) was dissolved in 15 mL CH₂Cl₂ under nitrogen. To this stirred solution was added a catalytic amount of DMAP, and triethylamine (0.91 mL, 6.51 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness. Purification of silica gel (20% hexanes CH₂Cl₂) afforded 406 mg of the title compound.
¹H NMR 500 MHz (DMSO-D6): δ 7.95 (d, 1H, J=7.18 Hz), 7.91 (d, 1 H, J=8.70 Hz), 7.71 (m), 7.49 (t, 1H, J=7.79 Hz), 7.42 (m, 1 H), 7.37 (bs, 1 H), 7.24 (m, 1 H), 7.22 (bs, 1 H), 7.12 (d, 1H, J=8.25 Hz), 6.83 (t, 1H, J=7.48 Hz), 6.22 (bs, 1H), 6.02 (s, 1H), 3.98 (s, 3H), 3.60 (2H,bs), 1.65 (s, 3H)
MS (ESI) m/z 587 ([M-H]-);
Anal. calcd for C₃₄H₂₈ClF₃N₂O₂: C:69.33 H:4.79 N:4.76 Found: C:69.06 H:4.87 N:4.65.

### Example 89

### (2S)-3-{4-[4-chloro-3-(trifluoromethyl)phenyl]piperidin-1-yl}-2-[(S)-(2-methoxyphenyl)(1-naphthyl)methyl]-2-methyl-3-oxopropanenitrile

(S,S)-2-cyano-3-(2-methoxyphenyl)-2-methyl-3-(1-naphthyl)propanoic acid(500 mg, 1.45 mmol) was dissolved in 20 mL THF. A catalytic amount of DMF was added to the reaction mixture. Oxalyl chloride (0.152 mL, 1.73 mmol) was added slowly. The reaction mixture was stirred until no more evolution of gas was observed. The reaction mixture was rapidly heated to reflux, then cooled and evaporated. 25 mL toluene was added and the reaction mixture was evaporated to dryness. The acid chloride was dissolved in 10 mL CH₂Cl₂ and placed under nitrogen. In a separate flask 1-(4-chloro-3-triflouromethyl phenyl)-pyridine hydrochloride (520 mg, 1.73 mmol) was dissolved in 15 mL CH₂Cl₂ under nitrogen. To this stirred solution was added a catalytic amount of DMAP, and triethylamine (0.91 mL, 6.51 mmol). The acid chloride in CH₂Cl₂ was added rapidly to this stirred mixture and allowed to react overnight after which water was added to the reaction mixture. The organic layer was separated and washed with brine, dried over MgSO₄, filtered, evaporated to dryness. Purification of silica gel (20% hexanes/CH₂Cl₂) afforded 510 mg of the title compound.
¹H NMR 500 MHz (DMSO-D6): δ 8.02 (d, 1H, J=7.02 Hz), 7.88 (dd, 2H, J=6.18 Hz, 2.52 Hz), 7.81 (d, 1 H, J=8.40 Hz), 7.61 (bs, 2H), 7.56 (t, 1H, J=7.79 Hz), 7.42 (m, 2H), 7.23 (td (1H, J=6.87 Hz, 1.37 Hz), 7.14 (m, 2H), 6.80 (t, 1H, J=7.48 Hz), 6.02 (s, 1 H), 4.35 (bs, 1H), 4.01 (s, 3H), 2.89 (bs, 4H), 1.75 (bs, 4H), 1.65 (s, 3H)
MS (ESI) m/z 649 ([M+CH3COO]-);
Anal. calcd for C₃₄H₃₀ClF₃N₂O₂: C:69.09 H:5.12 N:4.74 Found: C:69.21 H:5.13 N:4.62.

### Example 90

### 2-cyano-3,3-di(1-naphthyl)propanamide

Ethyl 2-cyano-3,3-di(1-naphthyl)propanoate (250 mg, 0.66 mmol) was disolved in THF (2 mL) to which concentrated ammonium hydroxide (5 mL) was added. The reaction mixture was allowed to stir overnight, then taken up in ethyl acetate, washed with saturated NaHCO₃, Brine, dried with MgSO₄, evaporated, and purified on silica gel to yield 38 mg of the title compound.
¹H NMR 400 MHz (DMSO-D6): δ 8.27 (m, 2H), 7.93 (m, 3H), 7.81 (m, 2H), 7.65 (d, 1H, J=7.65 Hz), 7.50 (m, 8H), 6.35 (d, 1H, J=9.76 Hz), 4.90m (d, 1H, J=9.76 Hz). MS (APCI) m/z 368 ([M+NH4]+);
Anal. calcd for C₂₄H₁₈N₂O • 0.5 H₂O • 0.25 C₆H₁₄: C:80.39 H:5.95 N:7.35 Found: C:80.27 H:5.69 N:7.11.

### Example 91

### (RR/SS)-3-(2-Methoxy-phenyl)-2-methyl-3-naphthalen-1-yl-2-(4-oxy-4-o-tolyl-piperazine-1-carbonyl)-propionitrile

A solution of (*RR*,*SS*)-3-(2-methoxyphenyl)-2-methyl-2-{[4-(2-methylphenyl) piperazin-1-yl]carbonyl}-3-(1-naphthyl)propanenitrile (256mg, 0.50mmol) in dichloromethane (5mL) is treated with 3- chloroperoxybenzoic acid (86mg, 0.5mmol) at reflux for 53 hours (approximately 35% conversion by LC/MS). The sample is cooled and quenched with saturated aqueous sodium bicarbonate. The organic layer is washed with water dried over Na₂SO₄, Filtered and concentrated *in vacuo.* Chromatography over silica using 5% methanol in dichloromethane yielded 80mg of the title compound. Recrystallization from hexane/ethyl acetate provided the title compound as a white crystal.
mp 178-180 °C dec;
¹H NMR 500MHz (CDCl₃): δ 8.01 (d, 1H, J = 7.17Hz),7.88 (m, 2H), 7.81 (d, 1 H, J = 8.24Hz), 7.56 (t, 1H, J = 7.63Hz), 7.44 (quin, 2H, J = 4.12Hz), 7.28 (brm, 2H), 7.81 (t, 2H, J = 7.18Hz), 7.14 (d, 1H, J = 8.24Hz), 7.13 (t,1H, J = 4.59Hz), 6.03 (s,1H), 7.81 (d, 1 H, J = 8.24Hz), 4.02 (s, 3H), 3.63 (brs, 3H), 3.23 (brs, 3H), 2.72 (brs, 3H) 3.63 (brs, 3H), 1.68. (s, 3H),
MS (ESI) *m*/*z* 520 ([M+H]+);
Anal. calcd for C₃₃H₃₃N₃O₃: C:76.28 H:6.40 N:8.09 Found: C:75.19 H:6.53 N:7.86.

### Example 92

### (RR,SS)-3-(2-methoxyphenyl)-2-methyl-3-(1-naphthyl)-2-({4-[3-(trifluoromethoxy)phenyl]piperazin-1-yl}carbonyl)propanenitrile

(*RR*,*SS*)-3-(2-methoxyphenyl)-2-methyl-3-(1-naphthyl)-2-(piperazin-1-ylcarbonyl)propanenitrile (377 mg, 0.913 mmol), *meta*-trifluoromethoxy bromobenzene (200 mg, 0.830 mmol), sodium *tert*-butoxide (111 mg, 1.16 mmol), tris (dibenzylideneacetone) dipalladium (0) (22.8 mg, 0.025 mmol), racemic BINAP (31 mg, 0.05 mmol), and toluene (3 mL) were combined in a Carius tube, vacuum degassed, placed under an argon atmosphere, sealed and heated to 80°C overnight. The reaction was cooled, taken up in diethyl ether, filtered, washed with water, saturated aqueous sodium bicarbonate, brine, dried over MgSO₄, filtered, evaporated, and purified on slica gel (10% hexanes/CH₂Cl₂) to yield 255 mg of the title compound as a white powder.
¹H NMR 500 MHz (DMSO-D6): δ 7.97 (d, 1H, J=7.18 Hz), 7.88 (m, 1 H), 7.84 (m, 1H), 7.78 (d, 1H, J=8.25 Hz), 7.52 (t, 1 H, J=7.48 Hz), 7.41 (m, 2H), 7.30 (t, 1 H, J=8.25 Hz), 7.23 (td, 1H, J=7.79 Hz, 1.68 Hz), 7.12 (m, 2H), 6.91 (dd, 1H, J=8.40 Hz, 1.68 Hz), 6.79 (m, 2H), 6.72 (d, 1H, J=8.09 Hz), 6.01 (s, 1H), 4.01 (s, 3H), 3.68 (bs, 4H0 3.16 (bs, 4H) 1.63 (s, 3H)
MS (ESI) m/z 574 ([M+H]+);
Anal. calcd for C₃₃H₃₀F₃N₃O₃ •0.35 C₆H₁₄: C:69.82 H:5.83 N:6.96 Found: C:69.81 H:5.91 N:6.74.

### Example 93

### (R,S)-3-[4-(2,3-difluorophenyl)piperazin-1-yl]-2-[(R,S)-(2-methoxyphenyl)(1-naphthyl)methyl]-2-methyl-3-oxopropanenitrile

(*RR*,*SS*)-3-(2-methoxyphenyl)-2-methyl-3-(1-naphthyl)-2-(piperazin-1-ylcarbonyl)propanenitrile (471 mg, 1.14 mmol), 2,3-difluorobromobenzene (200 mg, 1.04 mmol), sodium *tert*-butoxide (139 mg, 1.45 mmol), tris (dibenzylideneacetone) dipalladium (0) (28 mg, 0.031 mmol), racemic BINAP (39 mg, 0.062 mmol), and toluene (3 mL) were combined in a Carius tube, vacuum degassed, placed under an argon atmosphere, sealed and heated to 80°C overnight. The reaction was cooled, taken up in diethyl ether, filtered, washed with water, saturated aqueous sodium bicarbonate, brine, dried over MgSO₄, filtered, evaporated, and purified on slica gel (10% hexanes/CH₂Cl₂) to yield 280 mg of the title compound as an off-white crystal solid.
mp 198-200 °C;
¹H NMR 500 MHz (DMSO-D6): δ 7.98 (d, 1H, J=7.33 Hz), 7.86 (m, 2H), 7.79 (d, 1H, J=8.25 Hz), 7.54 (t, 1H, J=7.64 Hz), 7.42 (m, 2H), 7.23 (t, 1 H, J=7.64 Hz), 7.12 (m, 2H), 7.08 (m, 1 H), 7.00 (q, 1H, J=9.01 Hz), 6.79 (m, 2H), 6.01 (s, 1H), 4.01 (s, 3H), 3.73 (bs, 4H), 2.96 (bs, 4H), 1.64 (s, 3H)
MS (ESI) m/z 526 ([M+H]+);
Anal. calcd for C₃₂H₂₉F₂N₃O₂ • 0.20 H₂O: C:72.63 H:5.60 N:7.94 Found: C:72.65 H:5.58 N:7.79.

### Example 94

### (R,S)-3-[4-(3-fluorophenyl)piperazin-1-yl]-2-[(R,S)-(2-methoxyphenyl)(1-naphthyl)methyl]-2-methyl-3-oxopropanenitrile

(*RR,SS*)-3-(2-methoxyphenyl)-2-methyl-3-(1-naphthyl)-2-(piperazin-1-ylcarbonyl)propanenitrile (519 mg, 1.25 mmol), 3-fluorobromobenzene (200 mg, 1.14 mmol), sodium *tert*-butoxide (154 mg, 1.60 mmol), tris (dibenzylideneacetone) dipalladium (0) (31 mg, 0.034 mmol), racemic BINAP (42 mg, 0.068 mmol), and toluene (3 mL) were combined in a Carius tube, vacuum degassed, placed under an argon atmosphere, sealed and heated to 80°C overnight. The reaction was cooled, taken up in diethyl ether, filtered, washed with water, saturated aqueous sodium bicarbonate, brine, dried over MgSO₄, filtered, evaporated, and purified on slica gel (10% hexanes/CH₂Cl₂) to yield 295 mg of the title compound as a white powder.
¹H NMR 500 MHz (DMSO-D6): δ 7.96 (d, 1H, J=7.18 Hz), 7.88 (m, 1H), 7.83 (m, 1 H), 7.52 (t, 1H, J=7.48 Hz), 7.41 (m, 2H), 7.21 (m, 2H), 7.12 (m, 2H)< 6.79 (td, 1H, J=7.48 Hz, 0.84 Hz), 6.72 (m, 2H), 6.56 (td, 1H, J=8.40 Hz, 1.68 Hz), 6.01 (s, 1H), 4.01 (s, 3H), 3.68 (bs, 4H), 3.14 (bs, 4H), 1.63 (s, 3H)
MS (ESI) m/z 508 ([M+H]+);
Anal. calcd for C₃₂H₃₀FN₃O₂ • 0.20 H₂O: C:75.18 H:5.99 N:8.22 Found: C:75.15 H:5.95 N:8.02.

### Example 95

### (RR,SS)-3-(2-methoxyphenyl)-2-methyl-3-(1-naphthyl)-2-[(4-pyridin-3-ylpiperazin-1-yl)carbonyl]propanenitrile

(*RR,SS*)-3-(2-methoxyphenyl)-2-methyl-3-(1-naphthyl)-2-(piperazin-1-ylcarbonyl)propanenitrile (576 mg, 1.39 mmol), 3-bromopyridine (200 mg, 1.27 mmol), sodium *tert*-butoxide (170 mg, 1.77 mmol), tris (dibenzylideneacetone) dipalladium (0) (35 mg, 0.038 mmol), racemic BINAP (47 mg, 0.076 mmol), and toluene (3 mL) were combined in a Carius tube, vacuum degassed, placed under an argon atmosphere, sealed and heated to 80°C overnight. The reaction was cooled, taken up in diethyl ether, filtered, washed with water, saturated aqueous sodium bicarbonate, brine, dried over MgSO₄, filtered, evaporated, and purified on slica gel (5% methanol/*iso*-propyl acetate) to yield 234 mg of the title compound which was recrystallized from ethanol to yield 158 mg of the title compound as a crystal solid.
mp 186-188 °C;
¹H NMR 500 MHz (DMSO-D6): δ 8.26 (d, 1H, J=2.75 Hz), 8.01 (dd, 1H, J=4.43 Hz, 1.07 Hz), 7.97 (d, 1 H, J=7.18 Hz), 7.88 (m, 1H), 7.78 (d, 1 H, J=8.25 z), 7.53 (t, 1 H, 7.64Hz), 7.42 (m, 2H), 7.28 (dd, 1H, J=8.40 Hz, 1.53 Hz), 7.22 (m, 2H), 7.12 (t, 2H, J=7.79 Hz), 6.79 (t, 1H, J=7.79 Hz), 6.01 (s, 1H), 4.01 (s, 3H), 3.62 (bs, 4H), 3.17 (bs, 4H), 1.64 (s, 3H)
MS (ESI) m/z 491 ([M+H]+);
Anal. calcd for C₃₁H₃₀N₄O₂: C:75.89 H:6.16 N:11.42 Found: C:75.70 H:6.22 N:11.32.

### Example 96

### (RR,SS)-3-[4-(2,3-dichlorophenyl)piperazin-1-yl]-2-[(2-methoxyphenyl)(1-naphthyl)methyl]-2-methyl-3-oxopropanenitrile

(*RR*,*SS*)-3-(2-methoxyphenyl)-2-methyl-3-(1-naphthyl)-2-(piperazin-1-ylcarbonyl)propanenitrile (403 mg, 0.97 mmol), 2,3-dichlorobromobenzene (200 mg, 0.89 mmol), sodium *tert*-butoxide (119 mg, 1.24 mmol), tris (dibenzylideneacetone) dipalladium (0) (24 mg, 0.027 mmol), racemic BINAP (33 mg, 0.053 mmol), and toluene (3 mL) were combined in a Carius tube, vacuum degassed, placed under an argon atmosphere, sealed and heated to 80°C overnight. The reaction was cooled, taken up in diethyl ether, filtered, washed with water, saturated aqueous sodium bicarbonate, brine, dried over MgSO₄, filtered, evaporated, and purified on slica gel (10% hexanes/CH₂Cl₂) and recrystallized from ethanol to yield 228 mg of the title compound as a crystal solid.
mp 220-222 °C;
1H NMR 500 MHz (DMSO-D6): δ 7.99 (d, 1H, J=7.33 Hz), 7.87 (m, 2H), 7.80 (d, 1H, J=8.25 Hz), 7.55 (t, 1H, J=7.64 Hz), 7.43 (m, 2H), 7.31 (m, 2H), 7.23 (td, 1H, J=8.55 Hz, 1.53 Hz), 7.14 (d, 2H, J=7.94 Hz), 7.02 (bs, 1H), 6.80 (td, 1H, J=7.56 Hz, 0.92 Hz) 6.02 (s, 1 H), 4.02 (s, 3H), 3.63 (bs, 4H), 2.85 (bs, 4H), 1.65 (s, 3H)
MS (ESI) m/z 558 ([M+H]+);
Anal. calcd for C₃₂H₂₉Cl₂N₃O₂: C:68.82 H:5.23 N:7.52 Found: C:68.59 H:5.19 N:7.34.

### General Procedure Arylpiperazine Amides

A 0.5M solution of (*SS*)2-cyano-3-(2-methoxy-phenyl)-2-methyl-3-naphthalen-1-yl-propionyl chloride in acetonitrile was prepared from the parent acid using oxalyl chloride and DMF in methylene chloride. A 0.5M solution of amine in acetonitrile was prepared, and a 1.5M solution of triethyl amine in acetonitrile was prepared.

450 µL of amine solution, and 150µL each of acid chloride solution and triethyl amine solution were placed in a vial, capped and shaken at room temperature overnight. The solvent was removed *in vacuo* and the sample was redissolved in dichloromethane (2mL) and phosphate buffer (2mL, 1.0M,pH 7.0). The sample is vortexed for one minute and then centrifuged until two layers form. The dichloromethane layer was removed and placed in a clean vial. The aqueous layer was extracted with additional dichloromethane (2mL) and the organic layers combined. The organic layer was extracted with aqueous 10% NaHCO₃ (2mL) and water (2mL). In each case the mixture was vortexed for one minute, centrifuged, to afford two layers, and the aqueous layer was removed. The organic layer was removed *in vacuo* the residue was diluted up to 800 µL in DMSO. This solution was used in the test procedure directly.

### Example 97

### (RR,SS)-3-(2-methoxyphenyl)-2-methyl-2-{[4-(3-methylphenyl)piperazin-1-yl]carbonyl}-3-(1-naphthyl)propanenitrile

HRMS Calc'd for 1.00 C33H33N3O2; Theory 503.644; Found: 503.257277

### Example 98

### (RR,SS)-3-(2-methoxyphenyl)-2-methyl-3-(1-naphthyl)-2-(pyrrolidin-1-ylcarbonyl)propanenitrile

HRMS Calc'd for C26H26N2O2; Theory: 398.5047; Found: 398.199428

### Example 99

### (RR,SS)-3-(2-methoxyphenyl)-2-methyl-2-(morpholin-4-ylcarbonyl)-3-(1-naphthyl)propanenitrile

HRMS Calc'd for 1.00 C26H26N2O3; Theory : 414.5041; Found: 414.194343

### Example 100

### (RR,SS)-3-[4-(2-hydroxyethyl)piperazin-1-yl]-2-[(2-methoxyphenyl)(1-naphthyl)methyl]-2-methyl-3-oxopropanenitrile

HRMS Calc'd for 1.00 C28H31 N303; Theory : 457.5726; Found: 457.236542

### Example 101

### (RR,SS)-3-(2,6-dimethylmorpholin-4-yl)-2-[(2-methoxyphenyl)(1-naphthyl)methyl]-2-methyl-3-oxopropanenitrile

HRMS Calc'd for 1.00 C28H30N2O3; Theory 442.5579; Found: 442.225643

### Example 102

### (RR,SS)-2-cyano-N,N-diethyl-3-(2-methoxyphenyl)-2-methyl-3-(1-naphthyl)propanamide

HRMS Calc'd for 1.00 C26H28N2O2; Theory 400.5206; Found: 400.215078

### Example 103

### (RR,SS)-2-cyano-N-[2-hydroxy-1-(hydroxymethyl)ethyl]-3-(2-methoxyphenyl)-2-methyl-3-(1-naphthyl)propanamide

HRMS Calc'd for 1.00 C25H26N2O4; Theory 418.4925; Found: 418.189258

### Example 104

### (RR,SS)-3-azetidin-1-yl-2-[(2-methoxyphenyl)(1-naphthyl)methyl]-2-methyl-3-oxopropanenitrile

HRMS Calc'd for 1.00 C25H24N2O2; Theory 384.4778; Found: 384.183778

### Example 105

(*RR*,*SS*)-2-cyano-N,N-diisopropyl-3-(2-methoxyphenyl)-2-methyl-3-(1-naphthyl)propanamide

HRMS Calc'd for 1.00 C28H32N2O2; Theory 428.5744; Found: 428.246378

### Example 106

### (RR,SS)-3-(2-methoxyphenyl)-2-methyl-3-(1-naphthyl)-2-[(3,3,5-trimethylazepan-1-yl)carbonyl]propanenitrile

HRMS Calc'd for 1.00 C31H36N2O2; Theory 468.6391; Found: 468.27767878

### Example 107

### (RR,SS)-3-(2,3-dihydro-H-indol-1-yl)-2-[(2-methoxyphenyl)(1-naphthyl)methyl]-2-methyl-3-oxopropanenitrile

HRMS Calc'd for 1.00 C30H26N2O2; Theory 446.5487; Found: 446.199428

### Example 108

### (RR,SS)-3-(2-methoxyphenyl)-2-methyl-3-(1-naphthyl)-2-(thiomorpholin-4-ylcarbonyl)propanenitrile

HRMS Calc'd for 1.00 C26H26N2O2S; Theory 430.5707; Found: 430.171498

### Example 109

### (RR,SS)-3-azepan-1-yl-2-[(2-methoxyphenyl)(1-naphthyl)methyl]-2-methyl-3-oxopropanenitrile

HRMS Calc'd for 1.00 C28H30N2O2; Theory 426.5585; Found: 426.230728

### Example 110

### (RR,SS)-2-cyano-N-cyclohexyl-3-(2-methoxyphenyl)-N,2-dimethyl-3-(1-naphthyl)propanamide

HRMS Calc'd for 1.00 C29H32N2O2; Theory 440.5854; Found: 440.246378

### Example 111

### (RR,SS)-2-cyano-3-(2-methoxyphenyl)-N,2-dimethyl-3-(1-naphthyl)propanamide HRMS Calc'd for 1.00 C23H22N2O2; Theory 358.44; Found: 358.168128

### Example 112

### (RR,SS)-3-(4-benzylpiperazin-1-yl)-2-[(2-methoxyphenyl)(1-naphthyl)methyl]-2-methyl-3-oxopropanenitrile

HRMS Calc'd for 1.00 C33H33N3O2; Theory 503.644; Found: 503.257277

### Example 113

### (RR,SS)-3-(3,4-dihydroisoquinolin-2(1H)-yl)-2-[(2-methoxyphenyl)(1-naphthyl)methyl]-2-methyl-3-oxopropanenitrile

HRMS Calc'd for 1.00 C31H28N2O2; Theory 460.5756; Found: 460.215078

### Example 114

### (RR,SS)-3-(2-methoxyphenyl)-2-methyl-2-{[4-(4-methylphenyl)piperazin-1-yl]carbonyl}-3-(1-naphthyl)propanenitrile

HRMS Calc'd for 1.00 C33H33N3O2; Theory 503.644; Found: 503.257277

### Example 115

### (RR,SS)-N,N-dibenzyl-2-cyano-3-(2-methoxyphenyl)-2-methyl-3-(1-naphthyl)propanamide

HRMS Calc'd for 1.00 C36H32N2O2; Theory 524.6624; Found: 524.246378

### Example 116

### (RR,SS)-3-azocan-1-yl-2-[(2-methoxyphenyl)(1-naphthyl)methyl]-2-methyl-3-oxopropanenitrile

HRMS Calc'd for 1.00 C36H32N2O2; Theory 440.5854; Found: 440.246378

### General Procedure "Acylpiperazine" Amides

A 0.25M stock solution of (RR,SS) 3-(2-methoxy-phenyl)-2-methyl-3-naphthalen-1-yl-2-(piperazine-1-carbonyl)-propionitrile in THF and a 0.25M stock solution of acylating agent (isocyanate, acid chloride, isothiocyanate, chloroformate or acid anhydride) in THF were prepared. A 0.25M stock solution of triethyl amine in THF was prepared.

200 µL (50 µmol, 1 eq) of the acyl piperazine solution was treated with 200 µL (50 µmol, 1 eq) of the acylating agent solution in a vial. In those cases where the acylating agent is an acid chloride, acid anhyridride or chloroformate 220 µL (55 µmol, 1.1 eq) of the TEA solution was also added to the vial. The solution was shaken at room temperature for six hours. The sample was evaporated in vacuo and the residue was diluted up to 800 µL in DMSO. This solution was used in the test procedure directly.

### Example 117

### 4-chlorophenyl 4-[(RR,SS)-2-cyano-3-(2-methoxyphenyl)-2-methyl-3-(1-naphthyl)propanoyl]piperazine-1-carboxylate

HRMS Calc'd for C33H30CIN3O4; Theory 568.0717; Found: 567.192484

### Example 118

### 2-nitrophenyl 4-[(RR,SS)-2-cyano-3-(2-methoxyphenyl)-2-methyl-3-(1-naphthyl)propanoyl]piperazine-1-carboxylate

HRMS Calc'd for C33H30N4O6; Theory 578.6246; Found: 578.216536

### Example 119

### 4-(methoxycarbonyl)phenyl 4-[(RR,SS)-2-cyano-3-(2-methoxyphenyl)-2-methyl-3-(1-naphthyl)propanoyl]piperazine-1-carboxylate

HRMS Calc'd for C35H33N3O6; Theory 578.6246; Found: 591.236937

### Example 120

### 4-methylphenyl 4-[(RR,SS)-2-cyano-3-(2-methoxyphenyl)-2-methyl-3-(1-naphthyl)propanoyl]piperazine-1-carboxylate

HRMS Calc'd for C34H33N3O4; Theory 547.6538; Found: 547.247107

### Example 121

### 4-[(RR,SS)-2-cyano-3-(2-methoxyphenyl)-2-methyl-3-(1-naphthyl)propanoyl]-N-(2-methylphenyl)piperazine-1-carboxamide

HRMS Calc'd for C34H34N4O3; Theory 546.6691; Found: 5462637

### Example 122

### 4-[(RR,SS)-2-cyano-3-(2-methoxyphenyl)-2-methyl-3-(1-naphthyl)propanoyl]-N-[2-(trifluoromethyl)phenyl]piperazine-1-carboxamide

HRMS Calc'd for C34H31 F3N4O3; Theory 600.6405; Found: 600.234825

### Example 123

### 4-[(RR,SS)-2-cyano-3-(2-methoxyphenyl)-2-methyl-3-(1-naphthyl)propanoyl]-N-(3-methoxyphenyl)piperazine-1-carboxamide

HRMS Calc'd for C34H34N4O4; Theory 562.6685; Found: 562.258006

### Example 124

### 4-[(RR,SS)-2-cyano-3-(2-methoxyphenyl)-2-methyl-3-(1-naphthyl)propanoyl]-N-(4-ethoxyphenyl)piperazine-1-carboxamide

HRMS Calc'd for C35H36N4O4; Theory 576.6954; Found: 576.273656

### Example 125

### N-(2-bromophenyl)-4-[(RR,SS)-2-cyano-3-(2-methoxyphenyl)-2-methyl-3-(1-naphthyl)propanoyl]piperazine-1-carboxamide

HRMS Calc'd for C₃₃H₃₁BrN₄O₃; Theory 611.5383; Found: 610.157952

### Example 126

### 4-[(RR,SS)-2-cyano-3-(2-methoxyphenyl)-2-methyl-3-(1-naphthyl)propanoyl]-N-(4-methylphenyl)piperazine-1-carboxamide

HRMS Calc'd for C₃₄H₃₄N₄O₃; Theory 546.6691; Found: 546.263091

### Example 127

### phenyl 4-[(RR,SS)-2-cyano-3-(2-methoxyphenyl)-2-methyl-3-(1-naphthyl)propanoyl]piperazine-1-carboxylate

HRMS Calc'd for C₃₃H₃₁N₃O₄; Theory 533.627; Found: 533.231457

### Example 128

### 4-fluorophenyl 4-[(RR,SS)-2-cyano-3-(2-methoxyphenyl)-2-methyl-3-(1-naphthyl)propanoyl]piperazine-1-carboxylate

HRMS Calc'd for C₃₃H₃₀FN₃O₃; Theory 551.6174; Found: 551.222035

### Example 129

### (RR,SS)-3-[4-(4-bromobenzoyl)piperazin-1-yl]-2-[(2-methoxyphenyl)(1-naphthyl)methyl]-2-methyl-3-oxopropanenitrile

HRMS Calc'd for C₃₃H₃₀BrN₃O₃; Theory 596.5236; Found: 595.14705

### Example 130

### N-(4-chlorophenyl)-4-[(RR,SS)-2-cyano-3-(2-methoxyphenyl)-2-methyl-3-(1-naphthyl)propanoyl]piperazine-1-carboxamide

HRMS Calc'd for C33H31ClN4O3; Theory 567.087; Found: 566.208468.

### Example 131

### (2S)-3-[4-(3-bromophenyl)piperazin-1-yl]-2-[(S)-(2-methoxyphenyl)(1-naphthyl)methyl]-2-methyl-3-oxopropanenitrile

The title compound (256 mg) was prepared according to Example 58 using 3-bromophenyl piperazine hydrochloride. Recrystallization from ethyl acetate/hexanes yielded white crystals.
mp 180-182 °C; [α]_{D}²⁵ = -130.90° (c = 1% solution, CHCl₃)
Calcd for C₃₂H₃₀BrN₃O₂: C, 67.61; H, 5.32; N, 7.39. Found: C, 67.84; H, 5.75; N, 6.76.

### Example 132

### (2S,3S)-3-(2-methoxyphenyl)-2-methyl-3-(1-naphthyl)-2-{[4-(2-oxo-2,3-dihydro-1H-indol-1-yl)piperidin-1-yl]carbonyl}propanenitrile

The title compound was prepared in according to Example 58 using 2-oxo-2,3-dihydro-1H-indol-1-yl)piperidine. Recrystallization from methanol/water yielded 194 mg pink crystals.
mp 227-230°C; [α]^{D25} = -211.20° (c = 1% solution, CHCl₃); MS (ES) m/z 542.2
Anal. Calcd for C35H33N3O3: C, 77.32; H, 6.12; N, 7.73. Found: C, 77.03; H, 5.82; N, 7.42.

### Example 133

### (2S,3S)-3-(2-methoxyphenyl)-2-methyl-3-(1-naphthyl)-2-[(4-pyridin-4-ylpiperazin-1-yl)carbonyl]propanenitrile

The title compound was prepared in according to Example 58 using 4-pyridinyl piperazine. Recrystallization from methanol/water yielded 175 mg white crystals.
mp 142-144 °C; [α]_{D}²⁵ = -129.42° (c = 79% solution, CHCl₃); MS (ES) m/z 491.15; Anal. Calcd for C31H30N4O2: C, 75.89; H, 6.16; N, 11.42. Found: C, 73.40; H, 6.43; N, 10.68

### Example 134

### (2S,3S)-3-(2-methoxyphenyl)-2-methyl-3-(1-naphthyl)-2-{[4-(2-oxo-3,4-dihydroquinolin-1 (2H)-yl)piperidin-1-yl]carbonyl}propanenitrile

The title compound was prepared in according to Example 58 using [4-(2-oxo-3,4-dihydroquinolin-1(2H)-yl)piperidine. Recrystallization from CH₂Cl₂/hexane yielded 267 mg fine crystals.
mp 265-267 °C; [α]D25 = -203.0° (c = 1% SOLUTION, CHCl₃); MS (ES) m/z 558.2;MS (ES) m/z 575.2; MS (ES) m/z 580.2; Anal. Calcd for C₃₆H₃₅N₃O₃: C, 77.53; H, 6.33; N, 7.53. Found: C, 77.29; H, 6.51; N, 7.46.

### Example 135

### (2S)-3-[4-(4-bromophenyl)-3,6-dihydropyridin-1(2H)-yl]-2-[(S)-(2-methoxyphenyl)(1-naphthyl)methyl]-2-methyl-3-oxopropanenitrile

The title compound was prepared according to Example 58 using 4-(4-bromophenyl)-1,2,3,6-tetrahydropyridine. Crystallization of the solid from ethyl acetate/hexanes provided 695 mg of the title compound as off white rosettes.
mp 136-139 °C;
[α]_{D}²⁵ = -120.36° (c = 1% solution, CHCl₃);
MS (ESI) m/z 565 ([M+H]+);

### Example 136

### (2S,3S)-3-(2-methoxyphenyl)-2-methyl-3-(1-naphthyl)-2-({4-[3-(trifluoromethyl) phenyl] piperazin-1-yl}carbonyl)propanenitrile

The title compound was prepared in 76% yield according to Example 58 using 1-(3-trifluoromethyl-phenyl)-piperazine hydrochloride. Recrystallization from ethyl acetate/hexanes provided a white solid.
mp 158-162 °C;
[α]_{D}²⁵ = -136.07° (c = 59% SOLUTION, CHCl₃);
MS (ESI) m/z 558 ([M+H]+);
Anal. Calcd for C₃₃H₃₀F₃N₃O₂: C, 71.08; H, 5.42; N, 7.54. Found: C, 71.60; H, 5.76; N, 7.22.

### Example 137

### (2S)-3-[4-(4-fluorophenyl)-3,6-dihydropyridin-1(2H)-yl]-2-[(S)-(2-methoxyphenyl)(1-naphthyl)methyl]-2-methyl-3-oxopropanenitrile

The title compound was prepared in 86% yield according to Example 58 using 4-(4-fluoro-phenyl)-1,2,3,6-tetrahydro-pyridine hydrochloride. Recrystallization from ethyl acetate/hexanes provided an off white solid.
[α]D25 = -137.12° (c = .57% solution, CHCl₃);.
MS (ESI) m/z 505 ([M+H]+);
Anal. Calcd for C33H29FN2O2: C, 78.55; H, 5.79; N, 5.55. Found: C, 77.84; H, 5.75; N, 5.31.

### Example 138

### (2S,3S)-3-(2-methoxyphenyl)-2-{[4-(2-methoxyphenyl)piperidin-1-yl]carbonyl}-2-methyl-3-(1-naphthyl)propanenitrile

The title compound was prepared in 84% yield according to Example 58 using 4-(2-methoxy-phenyl) piperidine. Recrystallization from acetone provided the title compound 78% yield as rosettes.
mp 135-140 °C;
[α]_{D}²⁵ = -189.58° (c = 1 % solution, CHCl₃);
MS (ESI) m/z 519 ([M+H]+);
Anal. Calcd for C34H34N2O3: C, 78.74; H, 6.61; N, 5.40. Found: C, 77.71; H, 6.89; N, 5.09.

### Example 139

### (2S,3S)-3-(2-methoxyphenyl)-2-methyl-3-(1-naphthyl)-2-({4-[3-(trifluoromethyl) cyclohexyl]piperidin-1-yl}carbonyl)propanenitrile

The title compound was isolated by chromatographic separation of a 35g sample of (S,S)-3-(2-methoxyphenyl)-2-methyl-3-(1-naphthyl)-2-({4-[3-(trifluoromethyl)phenyl] piperidin-1-yl}carbonyl)propanenitrile (Example 67). Chromatography over silica gel using 25% CH2Cl2/hexanes provided 6g of mixed fractions enriched in the title compound. Further purification over silica gel using 40% methyl t-butyl ether/hexanes provided 200mg of the title compound.
mp 160-165 °C;
[α]_{D}²⁵ = -124.68° (c = 1% SOLUTION, CHCl₃);
MS (ES) m/z 563.2 ([M+H]+);
MS (ES) m/z 580.3 ([M+NH4]+);
Anal. Calcd for C34H37F3N2O2: C, 72.58; H, 6.63; N, 4.98. Found: C, 72.50; H, 6.73; N, 4.75.

### Example 140

### (2S)-3-{4-hydroxy-4-[3-(trifluoromethyl)phenyl]piperidin-1-yl}-2-[(S)-(2-methoxyphenyl)(1-naphthyl)methyl]-2-methyl-3-oxopropanenitrile

Chromatographic separation of the mother liquors from Example 67 over silica gel using 30% methyl t-butyl ether/hexane provided 4.5 g of mixed fractions. A 2.5 g sample of this material was further purified over silica gel using 40% ethyl acetate/hexanes. This provided 180 mg (95% pure by HPLC) of the title compound as a white solid.
[α]_{D}²⁵ = -142.94° (c = 5.98 mg/0.7mL, CHCl₃);
MS (ESI) m/z 573 ([M+H]+);

### Example 141

### Methyl (2S*,3S*)-2-cyano-2-(methoxymethyl)-3-(2-methoxyphenyl)-3-(1-naphthyl) propanoate

To a suspension of NaH (600 mg, 15 mmol, 60% dispersion in mineral oil) in dry DMF (75 mL) cooled to 0 °C was added, in one portion, methyl 2-cyano-3-(2-methoxyphenyl)-3-(1-naphthyl)propanoate (5.18 g, 15.0 mmol). The reaction mixture was stirred for 1 h at 0 °C after which chloromethyl methyl ether (12.08 g, 11.4 mL, 150 mmol) was added. The reaction mixture was allowed to stir for 45 minutes at 0 °C after which the cooling bath was removed. The reaction was allowed to stir overnight at room temperature then it was poured into water and extracted with ethyl acetate (3x 100 mL). The combined organic layers were washed with water then dried over Na₂SO₄. Removal of solvent *in vacuo* yielded an oil that was flash chromatographed on SiO₂ (15% ethyl acetate in hexanes to 20% ethyl acetate in hexanes). Two components were isolated: component 1 (2.88 g) and component 2 (490 mg). Recrystallization of the major component from ethyl acetate/hexanes/dichloromethane yielded the title compound as white crystals (2.53 g).
mp 148.3-151 °C;
¹H NMR 500 MHz (DMSO-D₆) δ 3.31 (s, 3 H); 3.49 (d, J = 9.16 Hz, 1 H); 3.51 (s, 3 H); 4.09 (s, 3 H); 4.14 (d, J = 9.16 Hz, 1 H); 5.83 (s, 1 H); 6.84 (t, J = 7.10 Hz, 1H); 7.17 (t, J = 8.39 Hz, 2 H); 7.25-7.29 (m, 1 H); 7.47-7.49 (m, 2 H); 7.60 (t, J = 7.79 Hz, 1 H); 7.84-7.91 (m, 3 H); 8.08 (d, J = 3.59 Hz, 1H);
MS (ESI) m/z 390 ([M+H]⁺);
MS (ESI) m/z 407 ([M+NH₄]⁺);
Anal. calcd for C₂₄H₂₃NO₄: C:74.02 H:5.95 N:3.60 Found: C:74.51 H:6.33 N:3.41

### Example 142

### Methyl (2S*,3S*)-2-cyano-3-(2-methoxyphenyl)-3-(1-naphthyl)-2-(pyridin-4-ylmethyl)propanoate

The title compound was prepared in 31% yield according to Example 141 using 4-picolyl chloride hydrochloride (1.23 g). During the workup, it was noted that some solid would not dissolve in either the aqueous layer or the organic layer. This material was filtered off and washed with water to yield the title compound as a white solid (1.02 g).
mp 228-230 °C dec.;
¹H NMR 500 MHz (DMSO-d₆) δ2.95 (d, J = 13.27 Hz, 1 H); 3.37 (s, 3 H); 3.61 (d, J = 13.28 Hz, 1H); 4.10 (s, 3 H); 6.07 (s, 1H); 6.90 (t, J = 7.55 Hz, 1H); 7.13 (d, J = 5.95 Hz, 2 H); 7.21 (d, J = 7.94 Hz, 1 H); 7.27 (dd, J = 1.53 Hz, 7.79 Hz, 1 H); 7.32 (td, J = 1.37 Hz, 7.82 Hz, 1 H); 7.48-7.53 (m, 2 H); 7.58 (t, J = 7.79 Hz, 1 H); 7.87 (d, J = 8.24 Hz, 1 H); 7.91-7.92 (m, 1H); 7.98 (d, J = 8.69 Hz, 1 H); 8.03 (d, J = 7.33 Hz, 1 H); 8.53 (d, J = 5.95 Hz, 2 H); MS (ES) m/z 437.13 ([M+H]⁺); Anal. calcd for C₂₈H₂₄N₂O₃·0.50 H₂O: C:75.49 H:5.66 N:6.29 Found: C:75.52 H:5.34 N:6.19.

### Example 143

### Methyl (2S*,3S*)-2-cyano-3-(2-methoxyphenyl)-3-(1-naphthyl)-2-(pyridin-2-ylmethyl) propanoate

To a suspension of NaH (80 mg, 2 mmol, of a 60% dispersion in mineral oil) in dry DMF (5 mL) cooled to 0 °C was added, in one portion, methyl 2-cyano-3-(2-methoxyphenyl)-3-(1-naphthyl)propanoate (345 mg, 1.0 mmol). The reaction was allowed to stir at 0 °C for one hour after which 2-picolyl chloride hydrochloride (164 mg, 1.0 mmol) was added to the reaction. The reaction was heated 75 °C for one hour after which the cooled reaction mixture was worked up as in Example 141. The crude material was flash chromatographed on SiO₂ (25% ethyl acetate in hexanes) to yield the title compound as a white powder (180 mg, 41%).
mp 194.4-196.3 °C; ¹H NMR 500 MHz (DMSO-d₆) *δ* 3.06 (d, J = 13.88 Hz, 1 H); 3.41 (s, 3 H); 3.77 (d, J = 13.89 Hz, 1H); 4.11 (s, 3 H); 6.05 (s, 1H); 6.89 (t, J = 7.48 Hz, 1H); 7.20 (dd, J = 7.93 Hz, 14.19 Hz, 2 H); 7.31-7.33 (m, 3 H); 7.48-7.53 (m, 2 H); 7.58 (t, J = 7.78 Hz, 1H); 7.74 (td, J = 1.78 Hz, 7.71 Hz, 1H); 7.86 (d, J = 8.24 Hz, 1 H); 7.90-7.92 (m, 1 H); 8.00 (d, J = 8.24 Hz, 1H); 8.10 (d, J = 7.17 Hz, 1 H); 8.50 (dt, J = 0.84 Hz, 4.73 Hz, 1H);
MS (ES) m/z 437.13 ([M+H]⁺);
Anal. calcd for C₂₈H₂₄N₂O₃: C:77.04 H:5.54 N:6.42 Found: C:76.78 H:5.52 N:6.30

### Example 144

### Methyl (2S*,3S*)-2-cyano-2-[(2-methoxyethoxy)methyl]-3-(2-methoxyphenyl)-3-(1-naphthyl)propanoate

The title compound was prepared according to Example 141 using 2-methoxyethoxymethyl chloride (9.34 g, 8.56 mL, 75.0 mmol) The crude material was flash chromatographed on SiO₂ twice (20% ethyl acetate in hexanes) then once with 10% ethyl acetate in hexanes to yield the title compound of a white to colorless oily foam (560 mg,b).
¹H NMR 500 MHz (DMSO-d₆) δ 3.17 (s, 3 H); 3.36 (t, J = 4.52 Hz, 2 H); 3.46 (s, 3 H); 3.48-3.59 (m, 3 H); 4.02 (s, 3 H); 4.20 (d, J = 9.51 Hz, 1 H); 5.77 (s, 1H); 6.76-6.80 (m, 1H); 7.10-7.13 (m, 2 H); 7.19-7.24 (m, 1 H); 7.40-7.44 (m, 2 H); 7.55 (t, J = 7.75 Hz, 1 H); 7.77-7.87 (m, 3 H); 8.02 (d, J = 7.08 Hz, 1 H); MS (ES) m/z 434.2 ([M+H]⁺); MS (ES) m/z 451.1 ([M+NH₄]⁺); MS (ES) m/z 884.4 ([2M+NH₄]⁺); Anal. calcd for C₂₆H₂₇NO₅: C:72.04 H:6.28 N:3.23 Found: C:71.65 H:6.54 N:3.03.

### Example 145

### Methyl (2S*,3S*)-2-cyano-3-(2-methoxyphenyl)-3-(1-naphthyl)-2-(pyridin-3-ylmethyl)propanoate

To a suspension of NaH (360 mg, 15 mmol, dry) in dry DMF (38 mL) cooled to 0 °C was added, in one portion, methyl 2-cyano-3-(2-methoxyphenyl)-3-(1-naphthyl)propanoate (2.59 g, 7.5 mmol). The reaction mixture was allowed to stir for one-half hour at 0 °C after which 3-picolyl chloride hydrochloride (1.23 g, 7.5 mmol) was added. The reaction was heated at 78 °C for 3.75 hours. Then the cooled reaction mixture was worked up as in Example 141. The crude material was flash chromatographed on SiO₂ (25% ethyl acetate in hexanes) to give a yellow solid The solid was recrystallized from ethyl acetate/methanol to yield the title compound (400 mg, 12%) as beige crystals.
mp 160-164.4 °C;
¹H NMR 500 MHz (DMSO-d₆) *δ* 2.93 (d, J = 13.67 Hz, 1 H); 3.30 (s, 3 H); 3.59 (d, J = 13.79 Hz, 1 H); 4.06 (s, 3 H); 6.03 (s, 1 H); 6.85 (td, J = 0.98 Hz, 7.50 Hz, 1 H); 7.16 (dd, J = 0.98 Hz, 8.30 Hz, 1 H); 7.20-34 (m, 3 H); 7.42-7.55 (m, 4 H); 7.82 (d, J = 8.30 Hz, 1 H); 7.85-7.87 (m, 1 H); 7.93-7.95 (m, 1 H); 7.97 (d, J = 7.19 Hz, 1 H); 8.25 (d, J = 1.70 Hz, 1 H); 8.47 (dd, J = 1.59 Hz, 4.76 Hz, 1 H);
MS (ES) m/z 437.15 ([M+H]⁺);
Anal. calcd for C₂₈H₂₄N₂O₃: C:77.04 H:5.54 N:6.42 Found: C:76.64 H:5.59 N:6.25.

### Example 146

### Methyl (2S*,3S*)-2-[(benzyloxy)methyl]-2-cyano-3-(2-methoxyphenyl)-3-(1-naphthyl)propanoate

To a suspension of NaH (200 mg, 5 mmol, 60% dispersion in mineral oil) in dry DMF (5 mL), cooled to 0 °C, was added, in one portion, methyl 2-cyano-3-(2-methoxyphenyl)-3-(1-naphthyl)propanoate (1.73 g, 5.0 mmol). The reaction mixture was allowed to stir for one hour at 0 °C, then benzyloxymethyl chloride (783 mg, 1.16 mL, 5.0 mmol; 75% purity) was added, in one portion, to the reaction mixture. The reaction mixture was then heated at 95 °C for 2 hrs. Then the reaction mixture was cooled and poured into water and extracted with ethyl acetate (2x 50 mL). The organic layer was washed with water, dried over Na₂SO₄, and filtered. Removal of the solvent *in vacuo* gave 2.8 g of a yellow oil. The oil was flash chromatographed on SiO₂ using a mixture of ethyl acetate/hexanes to yield 1.17 g, 50%, of the title compound. Recrystallization of this material from ethyl acetate/hexanes yielded the title compound as white crystals.
mp 132.9-134.4 °C;
¹H NMR 500 MHz (DMSO-d₆) δ 3.51 (s, 3 H); 3.56 (d, J = 9.00 Hz, 1 H); 3.99 (s, 3 H); 4.17 (d, J = 9.15 Hz, 1 H); 4.52 (d, J = 12.06 Hz, 1 H); 4.60 (d, J = 12.06 Hz, 1 H); 5.79 (s, 1 H); 6.82 (t, J = 7.48 Hz, 1 H); 7.11-7.15 (m, 2 H); 7.23-7.39 (m, 6 H); 7.43-7.48 (m, 2 H); 7.61 (t, J = 7.78 Hz, 1H); 7.79-7.81 (m, 1 H); 7.87-7.91 (m, 2 H); 8.07 (d, J = 7.32 Hz, 1 H); MS (ES) m/z 466.2 ([M+H]⁺); MS (ES) m/z 483.2 ([M+NH₄]⁺); MS (ES) m/z 488.2 ([M+Na]⁺); MS (ES) m/z 948.4 ([2M+NH₄]⁺); Anal. calcd for C₃₀H₂₇NO₄: C:77.40 H:5.85 N:3.01 Found: C:77.26 H:6.02 N:2.92.

### Example 147

### Methyl (2S*)-2-cyano-2-[(S*)-(2-methoxyphenyl)(1-naphthyl)methyl]-4-morpholin-4-ylbutanoate

To a suspension of NaH (400 mg, 10 mmol, 60% dispersion in mineral oil) in dry DMF (25 mL), cooled to 0 °C, was added methyl 2-cyano-3-(2-methoxyphenyl)-3-(1-naphthyl)propanoate (1.725 g, 5.0 mmol), in one portion. The reaction mixture was allowed to stir for one hour at 0 °C then 4-(2-chloroethyl)morpholine hydrochloride (930 mg, 5 mmol) was added, in one portion. The reaction mixture was heated at 100 °C for 4h then the mixture was allowed to stir at room temperature for 60 hours. The reaction mixture was heated at 100 °C for another 3 hours then cooled. The mixture was poured into water and extracted with ethyl acetate (3x 75 mL). The combined organic layers were washed with water, dried over Na₂SO₄, and filtered. The solvent was removed *in vacuo* to yield a beige foam. The foam was dissolved in MeOH and dry HCl gas was passed through the solution. The solvent was removed *in vacuo* to give a light yellow solid. This solid was recrystallized from ethyl acetate/methanol to yield the title compound as light yellow cubic crystals (535 mg). The mother liquor was concentrated and the residue was recrystallized to yield additional title compound (417 mg).
¹H NMR 500 MHz (DMSO-d₆) δ 2.29 (t, J = 11.07 Hz, 1 H); 2.75 (t, J = 11.45 Hz, 1 H); 3.03-3.15 (m, 3 H); 3.17 (s, 3 H); 3.23-3.40, (m, 3 H); 3.76 (quartet, 13.12 Hz, 2 H); 3.81-3.99 (m, 2 H); 4.10 (s, 3 H); 5.90 (s, 1 H); 6.86 (t, J = 7.55 Hz, 1 H); 7.19 (t, J = 9.23 Hz, 2 H); 7.27-7.30 (m, 1 H); 7.48-7.51 (m, 2 H); 7.61 (t, J = 7.71 Hz, 1H); 7.88-7.92 (m, 3 H); 8.02 (d, J = 7.17 Hz, 1 H); 11.20 (d, J = 1.22 Hz, 1 H);
MS (ES) m/z 459.2 ([M+H]⁺);
Anal. calcd for C₂₈H₃₀N₂O₄·HCl: C:67.94 H:6.31 N:5.66 Found: C:65.80 H:6.78 N:5.35.

### Example 148

### Methyl (2S*)-2-cyano-4-(diethylamino)-2-[(S*)-(2-methoxyphenyl)(1-naphthyl)methyl]butanoate

To a suspension of NaH (400 mg, 10 mmol, 60% dispersion in mineral oil) in dry DMF (25 mL) cooled to 0 °C was added, in one portion, methyl 2-cyano-3-(2-methoxyphenyl)-3-(1-naphthyl)propanoate (1.725 g, 5.0 mmol). The reaction mixture was allowed to stir for one hour at 0 °C after which 2-(diethylamino)ethyl bromide hydrobromide (1.31 g, 5 mmol) was added in one portion. The reaction was allowed to stir at 100 °C for 4 hours after which it was cooled to room temperature and stirred overnight. The reaction was then worked up as in Example 141 to give an oil. This oil was dissolved in MeOH and dry HCl gas was passed through the solution. The solvent was removed *in vacuo* to yield a solid that was recrystallized from diethyl ether/ethyl acetate/methanol to yield 217 mg of white crystals. The mother liquor was recrystallized to obtain additional product. This yielded in the title compound as white crystals (784 mg, 32.5% total yield).
mp 174 °C dec.;
¹H NMR 500 MHz (DMSO-d₆) δ 1.16 (t, J = 7.02 Hz, 6 H); 2.24 (td, J = 5.21 Hz, 13.44 Hz, 1 H); 2.71 (td, J = 5.83 Hz, 12.66 Hz, 1 H); 3.00-3.07 (m, 1 H); 3.07-3.10 (m, 5 H); 3.50 (s, 3 H); 4.09 (s, 3 H); 5.90 (s, 1 H); 6.87 (t, J = 7.48 Hz, 1H); 7.19-7.24 (m, 2 H); 7.30 (t, J = 7.86 Hz, 1 H); 7.48-7.50 (m, 2 H); 7.61 (t, J = 7.78 Hz, 1 H); 7.88-7.93 (m, 3 H); 8.02 (d, J = 7.31 Hz, 1 H); 10.07 (d, J = 1.83 Hz, 1 H); MS (ES) m/z 445.2 ([M+H]⁺); Anal. calcd for C₂₈H₃₂N₂O₃·HCl: C:69.91 H:6.91 N:5.82 Found: C:69.33 H:6.91 N:5.70.

### Example 149

### tert-Butyl (2S*,3S*)-2-cyano-2-(methoxymethyl)-3-(2-methoxyphenyl)-3-(1-naphthyl)propanoate

The title compound was prepared in 43% yield according to Example 141 using tert-butyl 2-cyano-3-(2-methoxyphenyl)-3-(1-naphthyl)propanoate (1.94 g, 5.0 mmol), 2-methoxyethoxymethyl chloride (4.02 g, 3.8 mL, 50.0 mmol) and NaH (200 mg, 5.0 mmol, 60% dispersion in mineral oil). Upon concentrating the ethyl acetate used in the workup, some of the title compound crystallized from solution. These crystals were filtered, washed with cold ethyl acetate, and recrystallized from ethyl acetate to yield the title compound as white crystals (789 mg). The mother liquor recrystallized from ethyl acetate/hexanes twice to give additional title compound (148 mg) for a total yield of 927 mg.
mp 163-165.3 °C; ¹H NMR 500 MHz (DMSO-d₆) δ 0.95 (s, 9 H); 3.27 (s, 3 H); 3.44 (d, J = 9.27 Hz, 1 H); 4.01-4.03 (m, 4 H); 5.73 (s, 1 H); 6.81 (td, J = 1.06 Hz, 7.56 Hz, 1 H); 7.10 (m, 1 H); 7.19-7.23 (m, 2 H); 7.40-7.46 (m, 2 H); 7.55-7.59 (m, 1 H); 7.812-7.87 (m, 3 H); 8.09 (d, J = 7.19 Hz, 1 H); MS (ES) m/z 432.2 ([M+H]⁺); MS (ES) m/z 449.2 ([M+NH₄]⁺); MS (ES) m/z 880.4 ([2M+NH₄]⁺); Anal. calcd for C₂₇H₂₉NO₄: C:75.15 H:6.77 N:3.25 Found: C:74.28 H:6.69 N:3.19.

### Example 150

### Methyl (2S,3S)-2-cyano-2-(methoxymethyl)-3-(2-methoxyphenyl)-3-(1-naphthyl)propanoate

A 1.0 g sample of methyl (2S*,3S*)-2-cyano-3-(2-methoxyphenyl)-3-(1-naphthyl)propanoate, prepared in Example 141, was dissolved in 29 mL of a solution of EtOH and CH₂Cl₂ and was separated into its enantiomers using a Chiralpak AD (2 x 25 cm) column, eluting with hexanes:isopropyl alcohol (4:1). This yielded, after recrystallization of both enantiomers from ethyl acetate/hexanes, 320 mg of methyl (2R,3R)-2-cyano-2-(methoxymethyl)-3-(2-methoxyphenyl)-3-(1-naphthyl)propanoate (which eluted first from the column) and 250 mg of methyl (2S,3S)-2-cyano-2-(methoxymethyl)-3-(2-methoxyphenyl)-3-(1-naphthyl)propanoate (which eluted second from the column).
Methyl (2S,3S)-2-cyano-2-(methoxymethyl)-3-(2-methoxyphenyl)-3-(1-naphthyl)propanoate
mp 176-178 °C; [α]_{D}²⁵ = -300.06° (c = 1% SOLUTION, CHCl₃); ¹H NMR 500 MHz (DMSO-d₆) δ 3.27 (s, 3 H); .3.44 (d, J = 9.27 Hz, 1 H); 3.46 (s, 3 H); 4.04 (s, 3 H); 4.09 (d, J = 9.15 Hz, 1 H); 5.78 (s, 1 H); 6.79 (td, J = 0.98 Hz, 7.54 Hz, 1 H); 7.12 (td, J = 1.30 Hz, 8.54 Hz, 2 H); 7.20-7.24 (m, 1 H); 7.40-7.45 (m, 2 H); 7.55 (t, J = 7.75 Hz, 1 H); 7.79-7.87 (m, 3 H); 8.03 (d, J = 7.20 Hz, 1 H); MS (ES) m/z 390.1 ([M+H]⁺); MS (ES) m/z 407.1 ([M+NH₄]⁺); MS (ES) m/z 796.3 ([2M+NH₄]⁺); Anal. calcd for C₂₄H₂₃NO₄: C:74.02 H:5.95 N:3.60 Found: C:73.87 H:6.06 N:3.54.

### Example 151

### Methyl (2R,3R)-2-cyano-2-(methoxymethyl)-3-(2-methoxyphenyl)-3-(1-naphthyl)propanoate

mp 175.6-177.6 °C; [α]_{D}²⁵ = +308.06° (c = 1% SOLUTION, CHCl₃)
¹H NMR 500 MHz (DMSO-d₆) δ 3.26 (s, 3 H); 3.45 (d, J = 14.52 Hz, 1 H); 3.46 (s, 3 H); 4.04 (s, 3 H); 4.09 (d, J = 9.28 Hz, 1 H); 5.78 (s, 1 H); 6.79 (td, J = 1.09 Hz, 7.50 Hz, 1 H); 7.12 (td, J = 1.26 Hz, 8.02 Hz, 2 H); 7.20-7.24 (m, 1 H); 7.40-7.45 (m, 2 H); 7.55 (dd, J = 7.45 Hz, 8.06 Hz, 1H); 7.78-7.87 (m, 3 H); 8.03 (d, J = 7.20 Hz, 1H); MS (ES) m/z 390.2 ([M+H]⁺); MS (ES) m/z 407.2 ([M+NH₄]⁺); MS (ES) m/z 796.3 ([2M+NH₄]⁺); Anal. calcd for C₂₄H₂₃NO₄: C:74.02 H:5.95 N:3.60 Found: C:73.94 H:5.97 N:3.51.

### Example 152

### Methyl (2S*,3S*)-2-(4-bromobenzyl)-2-cyano-3-(2-methoxyphenyl)-3-(1-naphthyl)propanoate

This compound was prepared in 62% yield according to Example 141 using 4-bromobenzyl bromide (525 mg, 2.1 mmol). The cooling bath was removed after the addition of the 4-bromobenzyl bromide. The crude material was recrystallized from ethyl acetate/hexanes to yield the title compound as white crystals (640 mg, 62%).
mp 218-219 °C;
¹H NMR 500 MHz (DMSO-d₆) δ 2.85 (d, J = 13.42 Hz, 1 H); 3.32 (s, 3H); 3.53 (d, J = 13.54 Hz, 1 H); 4.05 (s, 3 H); 6.00 (s, 1 H); 6.84 (dt, J = 4.03 Hz, 14.03 Hz, 1 H); 7.00 (d, J = 8.42 Hz, 2 H); 7.15 (d, J = 8.30 Hz, 1H); 7.21 (dd, J = 1.58 Hz, 7.69 Hz, 1 H); 7.24-7.28 (m, 1H); 7.42-7.49 (m, 4 H); 7.53 (t, J = 7.81 Hz, 1 H); 7.81 (d, J = 8.17 Hz, 1 H); 7.84-7.87 (m, 1 H); 7.92-7.94 (m, 1 H); 7.99 (d, J = 7.20 Hz, 1 H); MS (ES) m/z 514.1 ([M+H]⁺); MS (ES) m/z 516.1 ([M+2+H]⁺); Anal. calcd for C₂₉H₂₄BrNO₃: C:67.71 H:4.70 N:2.72 Found: C:67.53 H:4.76 N:2.66.

### Example 153

### Methyl (2S*,3S*)-2-cyano-3-(2-methoxyphenyl)-3-(1-naphthyl)-2-{[3'-(trifluoromethyl)-1,1'-biphenyl-4-yl]methyl}propanoate

A mixture of methyl (2S*,3S*)-2-(4-bromobenzyl)-2-cyano-3-(2-methoxyphenyl)-3-(1-naphthyl)propanoate (257 mg, 0.50 mmol), 3-trifluoromethylphenylboronic acid (104 mg, 0.55 mmol), and K₃PO₄ (159 mg, 0.75 mmol) was purged with nitrogen. Then Pd(PPh₃)₄ (6 mol%, 34.6 mg, 30 mmol) was added followed by 1,4-dioxane (4 mL). The reaction mixture was heated at 80-90 °C for three hours after which the reaction was cooled to room temperature. The reaction was poured into water and extracting with ethyl acetate (2x 50 mL). The combined organic layers were dried over Na₂SO₄ and filtered. The solvent was removed *in vacuo* to give a black oil that was flash chromatographed on SiO₂ (15% ethyl acetate in hexanes) then recrystallized from ethyl acetate/hexanes to yield the title compound as white crystals (195 mg, 67%).
mp 202-204.1 °C; ¹H NMR 500 MHz (DMSO-d₆) δ 2.93 (d, J = 13.54 Hz, 1 H); 3.34 (s, 3 H); 3.62 (d, J = 13.54 Hz, 1 H); 4.07 (s, 3 H); 6.05 (s, 1H); 6.86 (td, J = 0.93 Hz, 7.53 Hz, 1 H); 7.17-7.19 (m, 3 H); 7.23-7.30 (m, 2 H); 7.43-7.49 (m, 2 H); 7.53 (t, J = 7.75 Hz, 1 H); 7.63-7.70 (m, 4 H); 7.82 (d, J = 8.30 Hz, 1 H); 7.84-7.88 (m, 1H); 7.93-7.97 (m, 3 H); 8.02 (d, J = 7.20 Hz, 1H); MS m/z 580 ([M+H]⁺); MS m/z 597 ([M+H₂O]); Anal. calcd for C₃₆H₂₈F₃NO₃: C:74.60 H:4.87 N:2.42 Found: C:74.69 H:5.01 N:2.41

### Example 154

### tert-Butyl (2S*,3S*)-2-[(benzyloxy)methyl]-2-cyano-3-(2-methoxyphenyl)-3-(1-naphthyl)propanoate

The title compound was prepared in 43% yield according to Example 146 using benzyloxymethyl chloride (783 mg, 1.16 mL, 5.0 mmol, 75% purity). The crude product was flash chromatographed on SiO₂ (10% ethyl acetate in petroleum ether to 15% ethyl acetate in petroleum ether) then recrystallized from ethyl acetate to yield the title compound as white crystals (1.26 g, 60%).
mp 166-170 °C; ¹H NMR 500 MHz (DMSO-d₆) δ 0.96 (s, 9 H); 3.54 (d, J = 9.00 Hz, 1 H); 4.00 (s, 3 H); 4.13 (d, J = 9.15 Hz, 1 H); 4.51 (d, J = 11.9 Hz, 1H); 4.62 (d, J = 12.05 Hz, 1 H); 5.75 (s, 1 H); 6.84 (t, J = 7.48 Hz, 1 H); 7.13 (d, J = 8.39 Hz, 1 H); 7.24-7.27 (m, 2 H); 7.30-7.34 (m, 3 H); 7.36-7.40 (m, 2 H); 7.46-7.48 (m, 2 H); 7.62 (t, J = 7.78 Hz, 1 H); 7.83-7.91 (m, 3 H); 8.14 (d, J = 7.17 Hz, 1 H); MS (ES) m/z 525.2 ([M+NH₄]⁺); Anal. calcd for C₃₃H₃₃NO₄: C:78.08 H:6.55 N:2.76 Found: C:77.75 H:6.57 N:2.77.

### Example 155

### Methyl (2R,3R)-2-cyano-3-(2-methoxyphenyl)-3-(1-naphthyl)-2-(pyridin-3-ylmethyl)propanoate

A 140 mg sample of methyl (2S*,3S*)-2-cyano-3-(2-methoxyphenyl)-3-(1-naphthyl)-2-(pyridin-3-ylmethyl)propanoate, prepared in Example 145, was dissolved in of a solution of CH₂Cl₂ and hexanes:isopropyl alcohol (4:1) and was separated into its enantiomers using a Chiralpak AD (5 x 25 cm) column, eluting with hexanes:isopropyl alcohol (4:1). There yielded 48 mg of methyl (2R,3R)-2-cyano-3-(2-methoxyphenyl)-3-(1-naphthyl)-2-(pyridin-3-ylmethyl)propanoate (which eluted first from the column) and 60 mg of methyl (2S,3S)-2-cyano-3-(2-methoxyphenyl)-3-(1-naphthyl)-2-(pyridin-3-ylmethyl)propanoate (which eluted second from the column)
mp 193 °C dec.; [α]_{D}²⁵ = -311.22° (c = 1% solution, CHCl₃); ee = 91.02%; ¹H NMR 500 MHz (DMSO-d₆) δ 2.94 (d, J = 13.66 Hz, 1 H); 3.30 (s, 3 H); 3.59 (d, J = 13.79 Hz, 1 H); 4.06 (s, 3 H); 6.04 (s, 1 H); 6.85 (td, J = 0.94 Hz, 7.53 Hz, 1 H); 7.16 (dd, J = 0.86 Hz, 8.30 Hz, 1 H); 7.20-7.34 (m, 3 H); 7.42-7.55 (m, 4 H); 7.81-7.87 (m, 2 H); 7.93-7.99 (m, 2 H); 8.26 (d, J = 1.71 Hz, 1 H); 8.47 (dd, J = 1.59 Hz, 4.76 Hz, 1 H).

### Example 156

### Methyl (2S,3S)-2-cyano-3-(2-methoxyphenyl)-3-(1-naphthyl)-2-(pyridin-3-ylmethyl)propanoate

[α]_{D}²⁵ = +336.8° (c = 1% SOLUTION, CHCl₃); mp 193 °C dec.;
ee = 94.56%; ¹H NMR 500 MHz (DMSO-d₆) δ 2.94 (d, J = 13.66 Hz, 1 H); 3.30 (s, 3 H); 3.59 (d, J = 13.79 Hz, 1 H); 4.06 (s, 3 H); 6.04 (s, 1 H); 6.85 (td, J = 0.94 Hz, 7.53 Hz, 1 H); 7.16 (dd, J = 0.86 Hz, 8.30 Hz, 1 H); 7.20-7.34 (m, 3 H); 7.42-7.55 (m, 4 H); 7.81-7.87 (m, 2 H); 7.93-7.99 (m, 2 H); 8.26 (d, J = 1.71 Hz, 1 H); 8.47 (dd, J = 1.59 Hz, 4.76 Hz, 1 H).

### Example 157

### Methyl (2S*)-2-cyano-4-(dimethylamino)-2-[(S*)-(2-methoxyphenyl)(1-naphthyl)methyl]butanoate

To a suspension of NaH (410 mg, 10.25 mmol, 60% dispersion in mineral oil) in dry DMF (25 mL), cooled to 0 °C was added, in one portion, methyl 2-cyano-3-(2-methoxyphenyl)-3-(1-naphthyl)propanoate (1.725 g, 5.0 mmol). The reaction mixture was stirred at 0 °C for one hour. Then 2-dimethylaminoethyl chloride hydrochloride (756 mg, 5.25 mmol) was added and the mixture was stirred at room temperature overnight. Then the reaction mixture was heated at 100 °C for six hours after which an additional amount of NaH (180 mg, 7.5 mmol, 60% dispersion in mineral oil) was added. The reaction mixture was stirred overnight at room temperature and additional 2-dimethylaminoethyl chloride hydrochloride (720 mg, 5.00 mmol) was added. The reaction mixture was heated at 100 °C for 3.5 hours. The reaction mixture was cooled to room temperature and worked up as in Example 141. The residue, was flash chromatographed on SiO₂ (10% isopropanol in ethyl acetate) to yield the title compound as a pink solid. This solid is recrystallized from hexanes/ethyl acetate to give the title compound as peach crystals (375 mg, 18%).
mp 142-144.5 °C;
¹H NMR 500 MHz (DMSO-d₆) δ 1.6-1.69 (m, 1 H); 2.04 (s, 6 H); 2.20-2.29 (m, 1 H); 2.32-2.45 (m, 2 H); 3.39 (s, 3 H); 4.02 (s, 3 H); 5.79 (s, 1 H); 6.78 (td, J = 0.93 Hz, 7.50 Hz, 1 H); 7.10 (dd, J = 0.73 Hz, 8.18 Hz, 1 H); 7.16-7.22 (m, 2 H); 7.41-7.43 (m, 2 H); 7.54 (t, J = 7.81 Hz, 1 H); 7.79-7.85 (m, 2 H); 7.89-7.91 (m, 1 H); 8.08 (d, J = 7.20 Hz, 1 H); MS (ES) m/z 417.07 ([M+H]⁺); MS (ES) m/z 833.25 ([2M+H]⁺); Anal. calcd for C₂₆H₂₈N₂O₃: C:74.98 H:6.78 N:6.73 Found: C:74.48 H:7.06 N:7.49

### Example 158

### Methyl (2S,3S)-2-cyano-2-[(2-methoxyethoxy)methyl]-3-(2-methoxyphenyl)-3-(1-naphthyl)propanoate

### Example 159

### Methyl (2R,3R)-2-cyano-2-[(2-methoxyethoxy)methyl]-3-(2-methoxyphenyl)-3-(1-naphthyl)propanoate

A 150 mg sample of methyl (2S*,3S*)-2-cyano-2-[(2-methoxyethoxy)methyl]-3-(2-methoxyphenyl)-3-(1-naphthyl)propanoate, prepared in Example 144, was dissolved in 6 mL of a solution of ethanol/acetonitrile 5:1 and was separated into its enantiomers using a Chiralpak AS (2 x 25 cm) column, eluting with hexanes:isopropyl alcohol (9:1). There yielded 14 mg of methyl (2S,3S)-2-cyano-2-(methoxymethyl)-3-(2-methoxyphenyl)-3-(1-naphthyl)propanoate as a colorless oil (which eluted first from the column) and 23 mg of methyl (2R,3R)-2-cyano-2-(methoxymethyl)-3-(2-methoxyphenyl)-3-(1-naphthyl)propanoate as a white to colorless oil (which eluted second from the column).

### Methyl (2S,3S)-2-cyano-2-[(2-methoxyethoxy)methyl]-3-(2-methoxyphenyl)-3-(1-naphthyl)propanoate

[α]_{D}²⁵ = -369.34° (c = 1% SOLUTION, CHCl₃); ¹H NMR 500 MHz (CDCl₃) *δ* 3.30 (s, 3 H); 3.44-3.49 (m, 2 H); 3.55 (s, 3 H); 3.56-3.61 (m, 1 H); 3.65-3.70 (m, 2 H), 4.03 (s, 3 H); 4.35 (d, J = 9.52 Hz, 1 H); 5.90 (s, 1 H); 6.81 (td, J = 0.94 Hz, 7.57 Hz, 1 H); 6.90-6.92 (m, 1 H); 7.15-7.20 (m, 1 H); 7.33-7.40 (m, 3 H); 7.47 (t, J = 7.81 Hz, 1 H); 7.70-7.75 (m, 2 H); 7.90-7.93 (m, 1 H); 8.21 (d, J = 7.32 Hz, 1 H).

### Methyl (2R,3R)-2-cyano-2-[(2-methoxyethoxy)methyl]-3-(2-methoxyphenyl)-3-(1-naphthyl)propanoate

[α]_{D}²⁵ = +249.38° (c = 1% SOLUTION, CHCl₃); ¹H NMR 500 MHz (DMSO-d₆) δ 3.17 (s, 3 H); 3.36 (t, J = 4.51 Hz, 2 H); 3.46 (s, 3 H); 3.48-3.53 (m, 3 H); 4.02 (s, 3 H); 4.20 (d, J = 9.40 Hz, 1 H); 5.76 (s, 1 H); 6.78 (td, J = 3.17 Hz, 7.53 Hz, 1 H); 7.10-7.13 (m, 2 H); 7.21 (td, J = 1.59 Hz, 7.81 Hz, 1 H); 7.40-7.44 (m, 2 H); 7.55 (t, J = 7.75 Hz, 1 H); 7.78-7.86 (m, 3 H); 8.02 (d, J = 7.08 Hz, 1 H).

### Example 160, Part 1

### Methyl (2E)-2-cyano-3-quinolin-4-ylacrylate or methyl (2E)-2-cyano-3-(quinolin-4-yl)prop-2-enoate

To a stirred solution of 4-quinoline carboxaldehyde (1.58 g, 10 mmol) in ethanol (20 mL) was added methyl cyanoacetate (882 µ, 10 mmol). The reaction was allowed to stir at ambient temperature for 16 h, whereupon a solid had precipitated from the solution. The mixture was cooled and then filtered via vacuum filtration and the filtrate was rinsed several times with chilled ethanol resulting in the recovery of 2.24 g (94%) of the title compound.
mp 162-163.6 °C;
¹H NMR (400 MHz, DMSO-d₆) δ ppm 3.94 (s, 3 H) 7.74 (ddd, *J*=8.34, 6.92, 1.29 Hz, 1 H) 7.89 (m, 2 H) 8.08 (dd, *J*=8.54, 0.78 Hz, 1 H) 8.15 (dd, *J*=8.54, 0.52 Hz, 1 H) 9.09 (d, *J*=1.03 Hz, 1 H) 9.10 (d, *J*=4.40 Hz, 1 H)
MS (ESI) *m*/*z* 239 ([M+H]⁺);HRMS: calcd for C₁₄H₁₀N₂O₂, 238.0742 (M), 239.08151 ([M+H]⁺); found (ESI+), 239.08069

### Example 160, Part 2

Methyl 2-cyano-3-(2-methoxyphenyl)-3-(quinolin-4-yl)propanoate To a stirred cooled (0 C) solution of methyl (2*E*)-2-cyano-3-(quinolin-4-yl)prop-2-enoate (1.8 g, 7.6 mmol) was dissolved in anhydrous THF (20 mL) was added a 1M THF solution of 2-methoxyphenyl magnesium bromide (8 mL) under an atmosphere of nitrogen. Upon addition of the Grignard reagent the reaction was allowed to return to room temperature. After 2 hours, the reaction was quenched with a saturated aqueous solution of ammonium chloride. The organic phase was removed and the aqueous phase was extracted with ethyl acetate. The organic phases were combined, dried (Na₂SO₄), filtered, and concentrated. The crude residue was further purified by flash column chromatography on a Biotage® 40 Mi column, eluting with a gradient of 30-50%, methyl tert-butylether in hexane and afforded 1.44 g (55%) of the diastereomeric mixture.
mp 169.3-170.7 °C;
HRMS: calcd for C₂₁H₁₈N₂O₃, 346.1317 (M), 347.13902 ([M+H]⁺); found (ESI_FT), 347.13838

### Example 160, Part 3a

Methyl 2-cyano-3-(2-methoxyphenyl)-2-methyl-3-(quinolin-4-yl)propanoate Sodium hydride (48 mg, 1.2 mmol, 60 % mineral oil dispersion) was added to a stirred cooled (0 C) solution of methyl 2-cyano-3-(2-methoxyphenyl)-3-(quinolin-4-yl)propanoate (347 mg, 1.0 mmol) in anhydrous DMF (10 mL). The mixture was allowed to warm to room temperature and after 0.5 hour, iodomethane (170 mg, 1.2 mmol) was added. The mixture continued to stir for an additional 16 hours. LCMS analysis of the reaction mixture indicated that two products were formed at the expense of the starting material. Water was added and the aqueous phase was extracted with ethyl acetate resulting in the isolation of the less polar product. The more polar product remained in the aqueous phase, to which aqueous HCl was added until pH 4. The acidified aqueous phase was extracted with dichloromethane/isopropyl alcohol (3:1) resulting in the extraction of the more polar product, which was determined to be the alkylated acid (185 mg, 53%). The less polar material was purified by flash column chromatography on a Biotage® 40 Mi column, affording 158 mg (44%).
mp 192-193.9 °C.
¹H NMR (400 MHz, DMSO-d₆) δ ppm 1.67 (s, 3 H) 3.60 (s, 3 H) 4.06 (s, 3 H) 5.83 (s, 1 H) 6.85 (td, *J*=7.54, 1.04 Hz, 1 H) 7.12 (dd, *J*=7.80, 1.56 Hz, 1 H) 7.20 (dd, *J*=8.32, 1.04 Hz, 1 H) 7.30 (m, 1 H) 7.56 (ddd, *J*=8.45, 7.02, 1.43 Hz, 1 H) 7.71 (ddd, *J*=8.38, 6.95, 1.30 Hz, 1 H) 7.91 (m, 2 H) 8.01 (m, 1 H) 8.99 (d, *J*=4.68 Hz, 1 H)
MS (ESI) *m*/*z* 361 ([M+H]⁺);
HRMS: calcd for C₂₂H₂₀N₂O₃, 360.1474 (M), 361.15467 ([M+H]⁺); found (ESI_FT), 361.15449

### Example 160, Part 3b

Methyl 2-cyano-3-(2-methoxyphenyl)-2-methyl-3-(quinolin-4-yl)propanoate The title compound can also be prepared by treating a THF solution of 2-cyano-3-(2-methoxyphenyl)-3-(quinolin-4-yl)propanoate (347 mg, 1.0 mmol) with a 0.5 M solution potassium hexamethyldisilamide (2.5 mmol) in toluene. After 1 hour, iodomethane (156 mg, 1.1 mmol) was added and the mixture continued to stir for an additional 3 hours. Water was added and the aqueous phase was extracted with ethyl acetate. The organic phase was dried (Na₂SO₄), filtered, concentrated, and applied to a Biotage® 40 Mi column, eluting with a gradient of 30-50%, methyl tert-butylether in hexane and afforded 348 mg (97%) as a solid.

### Example 161, Part 1

2-Cyano-3-(2-methoxyphenyl)-2-methyl-3-quinolin-4-ylpropanoic acid To a stirred slurry of methyl (2*E*)-2-cyano-3-(2-methoxyphenyl)-3-(quinolin-4-yl)prop-2-enoate (208 mg, 0.572 mmol) in methanol was added aqueous 50% sodium hydroxide (50 mg, 0.63 mmol). The mixture was heated (50 °C) for 16 hours and become homogenous. The mixture was concentrated *in vacuo*, water was added and the solution was neutralized with aqueous 2N HCl until acidic resulting in the formation of a white precipitate from solution. The solid was collected by suction filtration (200 mg, 91%).
¹H NMR (400 MHz, DMSO-d₆) δ ppm 1.65 (s, 3 H) 4.06 (s, 3 H) 5.84 (s, 1 H) 6.85 (td, *J*=7.50, 1.03 Hz, 1 H) 7.13 (dd, *J*=7.76, 1.81 Hz, 1 H) 7.20 (dd, *J*=8.54, 1.03 Hz, 1 H) 7.30 (ddd, *J*=8.60, 7.05, 1.68 Hz, 1 H) 7.67 (ddd, *J*=8.47, 7.05, 1.03 Hz, 1 H) 7.82 (ddd, *J*=8.34, 7.05, 1.16 Hz, 1 H) 8.00 (d, *J*=8.02 Hz, 1 H) 8.11 (dd, *J*=8.41, 0.91 Hz, 1 H) 8.13 (d, *J*=4.91 Hz, 1 H) 9.14 (d, *J*=4.91 Hz, 1 H)
MS (ESI) *m*/*z* 347 ([M+H]⁺); MS (ESI) *m*/*z* 345 ([M-H]⁻); HRMS: calcd for C₂₁H₁₈N₂O₃ HCl 382.1084 (M), 347.13902 ([M+H]⁺); found (ESI_FT), 347.13753;

### Example 161, Part 2

### 3-(2-Methoxyphenyl)-2-methyl-3-quinolin-4-yl-2-({4-[3-(trifluoromethyl)phenyl]piperidin-1-yl}carbonyl)propanenitrile

Oxalyl chloride (103 µL, 1.17 mmol) was added to a stirred suspension of 2-cyano-3-(2-methoxyphenyl)-2-methyl-3-quinolin-4-ylpropanoic acid (90 mg, 0.235 mmol) in dichloromethane (2.5 mL) followed by the addition of a 0.25M dichloromethane solution of DMF (20 µL). After 3 hours the volatiles were removed *in vacuo* and the resulting solid dissolved in dichloromethane (2mL). The solution containing the activated acid was added to a solution containing 4-[3-(trifluoromethyl)phenyl]piperidinium hydrochloride (126 mg, 0.47 mmol) and triethylamine (98 µL, 0.705 mmol) in dichloromethane (2mL). The mixture was allowed to stir at room temperature for 16 hours. The volume of the dichloromethane was reduced (~50%) and the reaction mixture was applied directly to a Biotage® (40Mi) column for flash column chromatography (1-2.5% methanol in dichloromethane) resulting in the isolation of 90 mg (97%) of a solid that was recrystallization from ethanol.
mp 163-165 °C; ¹H NMR (400 MHz, DMSO-d₆) δ ppm 1.47 (d, J=19.92 Hz, 2 H) 1.66 (s, 3 H) 1.88 (d, *J*=11.12 Hz, 2 H) 2.97 (t, *J*=11.77 Hz, 4 H) 4.06 (s, 3 H) 4.40 (br m, 1 H) 6.04 (s, 1 H) 6.82 (td, *J*=7.57, 1.16 Hz, 1 H) 7.09 (dd, *J*=7.76, 1.55 Hz, 1 H) 7.18 (dd, *J*=8.54, 1.03 Hz, 1 H) 7.28 (ddd, *J*=8.47, 7.05, 1.81 Hz, 1 H) 7.51 (ddd, *J*=8.47, 7.05, 1.29 Hz, 2 H) 7.58 (s, 3 H) 7.67 (ddd, *J*=8.34, 6.92, 1.29 Hz, 1 H) 7.96 (m, 3 H) 8.96 (d, *J*=4.66 Hz, 1 H)
MS (ESI) *m*/*z* 558 ([M+H]⁺); HRMS: calcd for C₃₃H₃₀F₃N₃O₂, 557.2290 (M), 558.23629 ([M+H]⁺); found (ESI+), 558.23505;
Anal. Calcd for C₃₃H₃₀F₃N₃O₂: C, 71.08; H, 5.42; N, 7.54. Found: C, 70.93; H, 5.21; N, 7.45.

### Example 162

### Methyl (RR,SS)-2-cyano-2-(methoxymethyl)-3-(2-methoxyphenyl)-3-quinolin-4-ylpropanoate

The title compound was prepared from sodium hydride (261 mg, 6.52 mmol, 60 % mineral oil dispersion), methyl 2-cyano-3-(2-methoxyphenyl)-3-(quinolin-4-yl)propanoate (2.052 g, 5.92 mmol), anhydrous DMF (30 mL), and methyl chloromethyl ether (495 µL, 6.52 mmol) according to the procedure and in the same manner as described in Example 160, part 3a. The product was purified from a Biotage® (40Mi) column for flash column chromatography (30-50% methyl *tert*-butyl ether in hexane) resulting in the isolation of 986 mg (43%) of a white solid.
mp 199.6-201.0 °C;
¹H NMR (400 MHz, DMSO-d₆) δ ppm 3.31 (s, 3 H) 3.50 (d, *J*=9.10 Hz, 1 H) 3.57 (s, 3 H) 4.08 (s, 3 H) 4.11 (d, *J*=9.36 Hz, 1 H) 5.84 (s, 1 H) 6.86 (td, *J*=7.54, 1.30 Hz, 1 H) 7.18 (td, *J*=8.19, 1.30 Hz, 2 H) 7.31 (ddd, *J*=8.51, 7.21, 1.69 Hz, 1 H) 7.57 (ddd, *J*=8.45, 7.02, 1.17 Hz, 1 H) 7.72 (ddd, *J*=8.32, 6.89, 1.17 Hz, 1 H) 7.85 (d, *J*=8.32 Hz, 1 H) 7.96 (d, *J*=4.42 Hz, 1 H) 8.01 (dd, *J*=8.45, 0.91 Hz, 1 H) 9.01 (d, *J*=4.68 Hz, 1 H)
MS (ESI) *m*/*z* 391 ([M+H]⁺); HRMS: calcd for C₂₃H₂₂N₂O₄, 390.1580 (M), 391.16524 ([M+H]⁺); found (ESI_FT), 391.16473;
Anal. Calcd for C₂₃H₂₂N₂O₄: C, 70.75; H, 5.68; N, 7.17. Found: C, 70.57; H, 5.27; N, 7.04

### Example 163, Part 1

### Methyl (2E)-2-cyano-3-quinolin-3-ylacrylate or methyl (2E)-2-cyano-3-(quinolin-3-yl)prop-2-enoate

The title compound was prepared from 3-quinoline carboxaldehyde (1.58 g, 10 mmol) and methyl cyanoacetate (882 µL, 10 mmol) in ethanol (20 mL) according to the procedure and in the same manner as described in Example 160, part 1 resulting in the recovery of 2.27 g (95%) of a yellow solid.
mp 180-182.5 °C; ¹H NMR (400 MHz, DMSO-d₆) δ ppm 3.92 (s, 3 H) 7.74 (ddd, *J*=8.15, 6.85, 1.03 Hz, 1 H) 7.95 (ddd, *J*=8.47, 6.92, 1.42 Hz, 1 H) 8.11 (ddd, *J*=7.57, 2.39, 1.42 Hz, 2 H) 8.64 (s, 1 H) 9.05 (d, *J*=2.33 Hz, 1 H) 9.40 (d, J=2.33 Hz, 1 H)
MS (ESI) *m*/*z* 239 ([M+H]⁺); HRMS: calcd for C₁₄H₁₀N₂O₂, 238.0742 (M), 239.08151 ([M+H]⁺); found (ESI+), 239.08088;
Anal. Calcd for C₁₄H₁₀N₂O₂: C, 70.58; H, 4.23; N, 11.76. Found: C, 70.51; H, 4.32; N, 11.77.

### Example 163, Part 2

### Methyl (2E)-2-cyano-3-(2-methoxyphenyl)-3-(quinolin-3-yl)propanoate

The title compound was prepared from methyl (2*E*)-2-cyano-3-(quinolin-3-yl)prop-2-enoate (1.8 g, 7.6 mmol), anhydrous THF (20 mL), and 1 M THF solution of 2-methoxyphenyl magnesium bromide (8 mL) according to the procedure and in the same manner as described in Example 160, part 2. The diastereomeric product was isolated from a Biotage® (40Mi) column for flash column chromatography (30-50 % methyl tert-butylether in hexane) resulting in the isolation of 2.60 g (99%) of a white solid.
mp 140.2-143 °C;
HRMS: calcd for C₂₁H₁₈N₂O₃, 346.1317 (M), 347.13902 ([M+H]⁺); found (ESI_FT), 347.13869

### Example 163, Part 3

### Methyl (SS,RR)-2-cyano-3-(2-methoxyphenyl)-2-methyl-3-(quinolin-3-yl)propanoate

The title compound was prepared from 2-cyano-3-(2-methoxyphenyl)-3-(quinolin-3-yl)propanoate (850 mg, 2.45 mmol), 0.5 M solution potassium hexamethyldisilamide (5.4 mL, 2.7 mmol) in toluene, and iodomethane (160 mg, 2.58 mmol) according to the procedure and in the same manner as described in Example 160, part 3b. The crude product was applied to a Biotage® 40 Mi column for flash column chromatography, eluting with a gradient of 30-50%, methyl tert-butylether in hexane and afforded 626 mg (71%) as a solid.
mp 127.7-131 °C; ¹H NMR (400 MHz, DMSO-d₆) δ ppm 1.62 (s, 3 H) 3.63 (s, 3 H) 3.86 (s, 3 H) 5.31 (s, 1 H) 7.04 (td, *J*=7.54, 1.30 Hz, 1 H) 7.12 (dd, *J*=8.32, 1.04 Hz, 1 H) 7.35 (ddd, *J*=8.58, 7.02, 1.56 Hz, 1 H) 7.63 (m, 2 H) 7.77 (ddd, *J*=8.45, 6.89, 1.30 Hz, 1 H) 8.02 (m, 2 H) 8.39 (d, *J*=2.60 Hz, 1 H) 8.74 (d, *J*=2.34 Hz, 1 H)
MS (ESI) *m*/*z* 361 ([M+H]⁺); HRMS: calcd for C₂₂H₂₀N₂O₃, 360.1474 (M), 361.15467 ([M+H]⁺); found (ESI_FT), 361.15490;
Anal. Calcd for C₂₂H₂₀N₂O₃: C, 73.32; H, 5.59; N, 7.77. Found: C, 73.08; H, 5.41; N, 7.59.

### Example 164, Part 1

### 2-Cyano-3-(2-methoxyphenyl)-2-methyl-3-quinolin-3-ylpropanoic acid

The title compound was prepared from methyl 2-cyano-3-(2-methoxyphenyl)-2-methyl-3-(quinolin-3-yl)propanoate (500 mg, 1.39 mmol) and 50% aqueous sodium hydroxide (61 mg, 1.52 mmol) according to the procedure and in the same manner as described in Example 160, part 4. The product was recovered after suction filtration affording 427 mg (80 %) of a white solid.
¹H NMR (400 MHz, DMSO-d₆) δ ppm 1.60 (s, 3 H) 3.85 (s, 3 H) 5.34 (s, 1 H) 7.04 (td, *J*=7.63, 1.03 Hz, 1 H) 7.12 (dd, *J*=8.54, 1.03 Hz, 1 H) 7.35 (m, 1 H) 7.64 (dd, *J*=7.76, 1.55 Hz, 1 H) 7.74 (ddd, *J*=8.21, 7.05, 1.03 Hz, 1 H) 7.89 (ddd, *J*=8.41, 6.98, 1.42 Hz, 1 H) 8.14 (dd, *J*=12.54, 7.89 Hz, 2 H) 8.69 (d, *J*=2.07 Hz, 1 H) 8.91 (d, *J*=2.33 Hz, 1 H)
MS (ESI) *m*/*z* 347 ([M+H]⁺);
MS (ESI) *m*/*z* 345 ([M-H]⁻);
HRMS: calcd for C₂₁H₁₈N₂O₃ HCl, 382.1084 (M), 347.13902 ([M+H]⁺); found (ESI_FT), 347.13774.

### Example 164, Part 2

### 3-(2-Methoxyphenyl)-2-methyl-3-quinolin-3-yl-2-({4-[3-(trifluoromethyl)phenyl] piperidin-1-yl}carbonyl)propanenitrile

The title compound was prepared from oxalyl chloride (114 µL, 1.3 mmol), 2-cyano-3-(2-methoxyphenyl)-2-methyl-3-quinolin-3-ylpropanoic acid (100 mg, 0.26 mmol), 4-[3-(trifluoromethyl)phenyl]piperidinium hydrochloride (76 mg, 0.29 mmol), and triethylamine (109 µL, 0.78 mmol)) according to the procedure and in the same manner as described in Example 160, part 5. The title compound was isolated after purification on a Biotage® 40 Mi column for flash column chromatography, eluting with a gradient of 30-50%, methyl tert-butylether in hexane and afforded 626 mg (71%) as a solid.
¹H NMR (400 MHz, DMSO-d₆) δ ppm 1.59 (s, 3 H) 1.86 (br m, 3 H) 2.97 (br tt, *J*=12.00, 3.14 Hz, 1 H) 3.34 (br s, 2 H) 3.88 (s, 3 H) 4.42 (br s, 3 H) 5.68 (s, 1H) 7.00 (td, *J*=7.50, 1.03 Hz, 1 H) 7.13 (dd, *J*=8.28, 1.03 Hz, 1 H) 7.34 (td, *J*=7.82, 1.68 Hz, 1 H) 7.48 (dd, *J*=7.76, 1.55 Hz, 1 H) 7.59 (m, 5 H) 7.74 (ddd, *J*=8.41, 6.98, 1.42 Hz, 1 H) 7.99 (m, 2 H) 8.38 (d, *J*=2.33 Hz, 1 H) 8.74 (d, *J*=2.33 Hz, 1 H)
MS (ESI) *m*/*z* 558 ([M+H]⁺); HRMS: calcd for C₃₃H₃₀F₃N₃O₂, 557.2290 (M), 558.23629 ([M+H]⁺); found (ESI_FT), 558.23579.

### Example 165

### Methyl (2S*,3R*)-2-cyano-2-(methoxymethyl)-3-(2-methoxyphenyl)-3-quinolin -3-ylpropanoate

The title compound was prepared from sodium hydride (137 mg, 3.43 mmol, 60 % mineral oil dispersion), methyl 2-cyano-3-(2-methoxyphenyl)-3-(quinolin-3-yl)propanoate (1.08 g, 3.12 mmol), anhydrous DMF (30 mL), and methyl chloromethyl ether (260 µL, 3.43 mmol) according to the procedure and in the same manner as described in Example 160, part 3a. The product was purified from a Biotage® (40Mi) column for flash column chromatography (30-50% methyl tert-butyl ether in hexane) resulting in the isolation of 392 mg (32%) of an amorphous solid.
¹H NMR (400 MHz, DMSO-d₆) δ ppm 3.29 (s, 3 H) 3.57 (d, *J*=9.36 Hz, 1 H) 3.59 (s, 3 H) 3.87 (s, 3 H) 3.92 (d, *J*=9.36 Hz, 1 H) 5.32 (s, 1 H) 7.04 (td, *J*=7.54, 1.04 Hz, 1 H) 7.11 (dd, *J*=8.32, 1.04 Hz, 1 H) 7.35 (ddd, *J*=8.58, 7.02, 1.56 Hz, 1 H) 7.65 (m, *J*=8.25, 8.25, 7.28, 1.43 Hz, 2 H) 7.78 (ddd, *J*=8.38, 6.95, 1.56 Hz, 1 H) 8.02 (m, 2 H) 8.41 (d, *J*=2.34 Hz, 1 H) 8.74 (d, *J*=2.34 Hz, 1H)
MS (ESI) *m*/*z* 391 ([M+H]⁺);
HRMS: calcd for C₂₃H₂₂N₂O₄, 390.1580 (M), 391.16524 ([M+H]⁺); found (ESI_FT), 391.16464

### Example 166, Part 1

### tert-Butyl 2-cyano-3-(quinolin-4-yl)prop-2-enoate

To a stirred suspension of 4-quinoline carboxaldehyde (628 mg, 4.0 mmol) in ethanol (20 mL) was added *tert*-butyl cyanoacetate (564 mg, 4.0 mmol). The mixture stirred for 10 days and became homogenous. The ethanol was removed *in vacuo* and the resulting oil was purified by passing through a Biotage® (40Mi) column for flash column chromatography (50% methyl tert-butylether in hexane) resulting in the isolation of 1.0 g (89%) of a white solid that contained an impurity (17%). The title compound was carried on without further purification.
MS (ESI) *m*/*z* 281 ([M+H]⁺);

### Example 166, Part 2

### tert-Butyl 2-cyano-3-(2-methoxyphenyl)-3-quinolin-4-ylpropanoate

The title compound was prepared from *tert*-butyl 2-cyano-3-(quinolin-4-yl)prop-2-enoate (900 mg, 2.66 mmolwith 17 % impurity), 2-methoxypehnyl magnesium bromide (3.5 mL. 3.5 mmol), and anhydrous THF (10 mL) according to the procedure and in the same manner as described in Example 160, part 2. Isolation from flash column chromatography on a Biotage® (40Mi) column for flash column chromatography (50% methyl tert-butylether in hexane) afforded 426 mg (41%) of an oil.
MS (ESI) *m*/*z* 389 ([M+H]⁺);
MS (ESI) *m*/*z* 387 ([M-H]⁻);

### Example 166, Part 3

### tert-Butyl 2-cyano-3-(2-methoxyphenyl)-2-(pyridin-3-ylmethyl)-3-quinolin-4-ylpropanoate

A solution of *tert*-butyl 2-cyano-3-(2-methoxyphenyl)-3-quinolin-4-ylpropanoate (20 mg, 0.51 mmol) in anhydrous DMF (1 mL) was added to a stirred slurry of sodium hydride (51 mg, 60% mineral oil dispersion) in anhydrous DMF (1 mL). After 1 hour at room temperature, 3-picolyl chloride hydrochloride (101 mg, 0.62 mmol) was added as a solid in one portion. Water was added and the resulting solid was collected by filtration. The solid was recrystallized from ethanol and afforded 194 mg (79%) of the title compound.
mp 225-228 °C; ¹H NMR (400 MHz, CHLOROFORM-d) δ ppm 1.01 (s, 9 H) 2.97 (d, *J*=13.71 Hz, 1 H) 3.57 (d, *J*=13.71 Hz, 1 H) 4.10 (s, 3 H) 6.13 (s, 1 H) 6.95 (td, *J*=7.63, 1.03 Hz, 1 H) 7.02 (dd, *J*=8.28, 0.78 Hz, 1 H) 7.28 (m, 2 H) 7.51 (m, 2 H) 7.67 (m, 2 H) 8.08 (ddd, *J*=10.93, 8.60, 0.91 Hz, 2 H) 8.18 (d, *J*=4.66 Hz, 1 H) 8.48 (d, *J*=2.07 Hz, 1 H) 8.55 (dd, *J*=4.79, 1.68 Hz, 1 H) 8.93 (d, *J*=4.66 Hz, 1H)
MS (ESI) *m*/*z* 480 ([M+H]⁺);
HRMS: calcd for C₃₀H₂₉N₃O₃, 479.2209 (M), 480.22817 ([M+H]⁺); found (ESI+), 480.22753;

### Example 167 part 1

### (RR,SS)-2-Cyano-2-[(2-methoxyethoxy)methyl]-3-(2-methoxyphenyl)-3-(1-naphthyl)propanoic acid

Methyl (*RR,SS*)-2-cyano-2-[(2-methoxyethoxy)methyl]-3-(2-methoxyphenyl)-3-(1-naphthyl)propanoate (6.0 g, 13.8 mmol) was suspended in a 10% aqueous methanolic solution (100 mL) and potassium hydroxide (4.7 g, 83 mmol) was added. The mixture was heated (35 °C) while being stirred (22 h). The reaction was neutralized upon the addition of 2 N hydrochloric acid (42 mL) followed by the addition of water (75 mL) resulting in the precipitation of a white solid. The solid was collected by suction filtration and dried affording 4.62 g (79%).
mp 224-225 °C; ¹H NMR (400 MHz, DMSO-d₆) δppm 3.22 (s, 3 H) 3.42 (t, *J*=4.66 Hz, 2 H) 3.54 (d, *J*=9.31, 1 H) 3.59 (m, 2 H) 4.07 (s, 3 H) 4.24 (d, *J*=9.31 Hz, 1 H) 5.78 (s, 1 H) 6.82 (td, *J*=7.57, 1.16 Hz, 1 H) 7.16 (ddd, *J*=13.06, 8.15, 1.29 Hz, 2 H) 7.24 (m, 1 H) 7.46 (td, *J*=6.60, 3.36 Hz, 2 H) 7.60 (m, 1 H) 7.87 (m, 3 H) 7.90 (d, *J*=3.36 Hz, 1 H) 8.19 (d, *J*=7.24 Hz, 1 H)
MS (ESI) *m*/*z* 420 ([M+H]⁺);

### Example 167 part 2

### (RR,SS)-2-[(2-Methoxyethoxy)methyl]-3-(2-methoxyphenyl)-3-(1-naphthyl)-2-[(4-pyrimidin-2-ylpiperazin-1-yl)carbonyl]propanenitrile

Oxalyl chloride (636 µL, 7.3 mmol) was added to a stirred slurry of (RR,SS)-2-cyano-2-[(2-methoxyethoxy)methyl]-3-(2-methoxyphenyl)-3-(1-naphthyl)propanoic acid (420 mg, 1.0 mmol) followed by the addition of a 0.25 M solution of DMF in dichloromethane (40 µL). The mixture was allowed to stir for 16 h at room temperature to afford a clear homogeneous solution. The volatile components were removed *in vacuo* and the resulting solid was dissolved in anhydrous dichloromethane and reconcentrated (3x). The solid was dissolved in anhydrous dichloromethane and added to a dichloromethane solution containing 4-(pyrimidin-2-yl)piperazinyl dihydrochloride (261 mg, 1.1 mmol) and triethylamine (488 µL, 3.5 mmol). The mixture stirred at ambient temperature for 16 hours. The organic phase was washed with 5% aqueous sodium bicarbonate, dried (Na₂SO₄), filtered, concentrated, and purified from a Biotage® (40Mi) column for flash column chromatography (30-100% methyl tert-butylether in hexane) afforded 323 mg (57%). mp 205.5-206.8 °C; ¹H NMR (400 MHz, DMSO-d₆) δ ppm 3.20 (s, 3 H) 3.43 (t, *J*=4.53 Hz, 2 H) 3.60 (m, 5 H) 3.76 (s, 6 H) 4.06 (s, 3 H) 4.20 (d, *J*=9.31 Hz, 1 H) 6.08 (s, 1H) 6.68 (t, *J*=4.79 Hz, 1 H) 6.79 (td, *J*=7.50, 1.29 Hz, 1H) 7.13 (ddd, *J*=15.39, 8.15, 1.29 Hz, 2 H) 7.25 (ddd, *J*=8.47, 7.18, 1.68 Hz, 1 H) 7.42 (m, 2 H) 7.55 (dd, *J*=8.28, 7.50 Hz, 1 H) 7.83 (m, 3 H) 8.02 (d, *J*=7.24 Hz, 1 H) 8.39 (d, *J*=4.66 Hz, 2 H)
MS (ESI) *m*/*z* 566 ([M+H]⁺); HRMS: calcd for C₃₃H₃₅N₅O₄, 565.2689 (M), 566.27618 ([M+H]⁺); found (ESI_FT), 566.27482;

### Example 168

### (RR,SS)-2-[(2-Methoxyethoxy)methyl]-3-(2-methoxyphenyl)-3-(1-naphthyl)-2-({4-[3-(trifluoromethyl)phenyl]piperidin-1-yl}carbonyl)propanenitrile

The title compound was prepared from oxalyl chloride (636 µL, 7.3 mmol), (RR,SS)-2-cyano-2-[(2-methoxyethoxy)methyl]-3-(2-methoxyphenyl)-3-(1-naphthyl)propanoic acid (420 mg, 1.0 mmol), 4-[3-(trifluoromethyl)phenyl]piperidinium hydrochloride (292 mg, 1.1 mmol), and triethylamine (488 µL, 3.5 mmol)) according to the procedure and in the same manner as described in Example 167 to afford 531 mg, (84%).
mp 86-88 °C; ¹H NMR (400 MHz, DMSO-d₆) δppm 1.78 (br s, 3 H) 2.92 (br t, *J*=11.12 Hz, 2 H) 3.18 (s, 3 H) 3.32 (s, 3 H) 3.44 (m, 2 H) 3.60 (m, 4 H) 4.05 (s, 3 H) 4.22 (d, *J*=9.31 Hz, 1 H) 6.09 (s, 1 H) 6.80 (t, *J*=7.11 Hz, 1H) 7.14 (dd, *J*=8.41, 0.91 Hz, 2 H) 7.24 (td, *J*=7.76, 1.81 Hz, 1 H) 7.44 (m, 4 H) 7.58 (m, 3 H) 7.86 (m, 3 H) 8.09 (s, 1 H)
MS (ESI) *m*/*z* 631 ([M+H]⁺); HRMS: calcd for C₃₇H₃₇F₃N₂O₄, 630.2705 (M), 631.27782 ([M+H]⁺); found (ESI_FT), 631.27872;

### Example 169

### (RR, SS)-N-(tert-Butyl)-2-cyano-2-[(2-methoxyethoxy)methyl]-3-(2-methoxyphenyl)-3-(1-naphthyl)propanamide

Oxalyl chloride (436 µL, 5.0 mmol) was added to a stirred slurry of (*RR*,*SS*)-2-cyano-2-[(2-methoxyethoxy)methyl]-3-(2-methoxyphenyl)-3-(1-naphthyl)propanoic acid (420 mg, 1.0 mmol) followed by the addition of a 0.25 M solution of DMF in dichloromethane (20 µL). The mixtures was allowed to stir for 16 h at room temperature to afford a clear homogeneous solution. The volatile components were removed *in vacuo* and the resulting solid was dissolved in anhydrous dichloromethane and reconcentrated (3x). The solid was dissolved in anhydrous acetonitrile (10 mL) and divided into two equal portions. The first portion was added to a solution containing *tert*-butylamine (106 µL, 1mmol) and triethylamine (140 µL, 1 mmol) in dichloromethane and the solution was mixed for 16 hours. The volume was reduced and the residue was dissolved in dichloromethane and applied directly to a Biotage® (40Mi) column for flash column chromatography (30-50% methyl tert-butylether in hexane) afforded 199 mg (84%).
mp 182.6-183 °C; ¹H NMR (400 MHz, DMSO-d₆) δ ppm 0.92 (s, 9 H) 3.23 (s, 3 H) 3.42 (t, *J*=4.68 Hz, 2 H) 3.57 (m, 3 H) 4.05 (s, 3 H) 4.24 (d, *J*=9.88 Hz, 1 H) 5.85 (s, 1 H) 6.82 (td, *J*=7.54, 1.04 Hz, 1 H) 6.94 (s, 1 H) 7.11 (dd, *J*=8.32, 0.78 Hz, 1 H) 7.23 (m, 2 H) 7.44 (m, 2 H) 7.55 (dd, *J*=8.19, 7.41 Hz, 1 H) 7.81 (d, *J*=8.32 Hz, 1 H) 7.86 (m, 1 H) 7.95 (m, 1 H) 8.11 (d, *J*=7.28 Hz, 1 H); MS (ESI) *m*/*z* 475 ([M+H]⁺); HRMS: calcd for C₂₉H₃₄N₂O₄, 474.2519 (M), 475.25914 ([M+H]⁺); found (ESI_FT), 475.25834;
Anal. Calcd for C₂₉H₃₄N₂O₄: C, 73.39; H, 7.22; N, 5.90. Found: C, 73.54; H, 7.25; N, 5.73.

### Example 170

### (RR,SS)-2-Cyano-N-(2,2-dimethylpropyl)-2-[(2-methoxyethoxy)methyl]-3-(2-methoxyphenyl)-3-(1-naphthyl)propanamide

The title compound was prepared from the second portion of acid chloride described in Example 169, neopentylamine (118 µL, 1 mmol), and triethylamine (140 µL, 1 mmol) according to the procedure and in the same manner as described in Example 7 to afford 244 mg, (99%).
mp 172.1-173 °C; ¹H NMR (400 MHz, DMSO-d₆) δ ppm 0.50 (s, 9 H) 2.62 (dd, *J*=12.99, 5.98 Hz, 1 H) 2.80 (dd, *J*=12.99, 6.76 Hz, 1 H) 3.21 (s, 3 H) 3.40 (t, *J*=4.68 Hz, 2 H) 3.54 (m, 3 H) 4.07 (s, 3 H) 4.28 (d, *J*=9.88 Hz, 1 H) 5.90 (s, 1 H) 6.80 (td, *J*=7.54, 1.30 Hz, 1 H) 7.12 (dd, *J*=8.32, 1.04 Hz, 1H) 7.21 (m, 2 H) 7.43 (td, *J*=6.50, 3.38 Hz, 2 H) 7.51 (dd, *J*=8.06, 7.28 Hz, 1 H) 7.83 (m, 2 H) 7.93 (td, *J*=6.50, 3.12 Hz, 1 H) 7.98 (t, *J*=6.24 Hz, 1 H) 8.17 (d, *J*=7.28 Hz, 1 H); MS (ESI) *m*/*z* 489 ([M+H]⁺);
HRMS: calcd for C₃₀H₃₆N₂O₄, 488.2675 (M), 489.27479 ([M+H]⁺); found (ESI_FT), 489.27274;

### Example 171

### (RR,SS)-2-[(2-methoxyethoxy)methyl]-3-(2-methoxyphenyl)-3-(1-naphthyl)-2-[(4-pyrazin-2-ylpiperazin-1-yl)carbonyl]propanenitrile

The title compound was prepared from oxalyl chloride (218 µL, 2.5 mmol), (*RR*,*SS*)-2-cyano-2-[(2-methoxyethoxy)methyl]-3-(2-methoxyphenyl)-3-(1-naphthyl)propanoic acid (209 mg, 0.5 mmol), 4-(pyrazin-2-yl)piperazine (84 mg, 0.51 mmol), and triethylamine (140 µL, 1 mmol) according to the procedure and in the same manner as described in Example 167 part 2 to afford 227 mg (80%).
mp 198-200 °C;
¹H NMR (400 MHz, DMSO-d₆) δppm 3.19 (s, 3 H) 3.32 (s, 3 H) 3.43 (m, 3 H) 3.59 (m, 5 H) 3.61 (s, 2 H) 4.06 (s, 3 H) 4.19 (d, *J*=9.36 Hz, 1 H) 6.08 (s, 1 H) 6.79 (td, *J*=7.54, 1.04 Hz, 1 H) 7.12 (ddd, *J*=19.56, 8.12, 1.17 Hz, 2 H) 7.25 (ddd, *J*=8.58, 7.02, 1.56 Hz, 1 H) 7.42 (m, 2 H) 7.56 (m, 1 H) 7.83 (m, 3 H) 7.88 (d, *J*=2.60 Hz, 1 H) 8.01 (d, *J*=7.28 Hz, 1 H) 8.11 (dd, J=2.60, 1.30 Hz, 1 H) 8.33 (d, *J*=1.30 Hz, 1 H); MS (ESI) *m*/*z* 566 ([M+H]⁺); HRMS: calcd for C₃₃H₃₅N₅O₄, 565.2689 (M), 566.27618 ([M+H]⁺); found (ESI_FT), 566.27454;
Anal. Calcd for C₃₃H₃₅N₅O₄: C, 70.07; H, 6.24; N, 12.38. Found: C, 69.89; H, 6.51; N, 12.36.

### Example 172

### (RR,SS)-3-[4-(1H-indol-3-yl)-3,6-dihydropyridin-1(2H)-yl]-2-[(2-methoxyethoxy)methyl]-2-[(S*)-(2-methoxyphenyl)(1-naphthyl)methyl]-3-oxopropanenitrile

The title compound was prepared from oxalyl chloride (218 µL, 2.5 mmol), (*RR,SS*)-2-cyano-2-[(2-methoxyethoxy)methyl]-3-(2-methoxyphenyl)-3-(1-naphthyl)propanoic acid (209 mg, 0.5 mmol), 3-(1,2,3,6-tetrahydro-4-pyridinyl)-1*H-*indole (101 mg, 0.51 mmol), and triethylamine (140 µL, 1 mmol) according to the procedure and in the same manner as described in Example 167 part 2 to afford 223 mg (74%).
¹H NMR (400 MHz, DMSO-d₆) δ ppm 2.54 (s, 2 H) 3.15 (s, 3 H) 3.32 (s, 2 H) 3.39 (s, 3 H) 3.57 (m, 3 H) 3.64 (d, *J*=9.36 Hz, 1 H) 4.04 (s, 3 H) 4.19 (d, *J*=9.36 Hz, 1 H) 6.10 (s, 2 H) 6.80 (td, *J*=7.54, 1.04 Hz, 1 H) 7.05 (ddd, *J*=7.99, 7.08, 1.30 Hz, 1 H) 7.13 (m, 3 H) 7.25 (m, 1 H) 7.42 (m, 5 H) 7.77 (d, *J*=8.32 Hz, 1 H) 7.83 (m, 3 H) 8.01 (d, *J*=7.28 Hz, 1 H) 11.18 (s, 1 H); MS (ESI) *m*/*z* 600 ([M+H]⁺);
HRMS: calcd for C₃₈H₃₇N₃O₄, 599.2784 (M), 600.28569 ([M+H]⁺); found (ESI_FT), 600.28542.

### Example 173

### tert-Butyl (RR,SS)-2-cyano-2-[(2-methoxyethoxy)methyl]-3-(2-methoxyphenyl)-3-(1-naphthyl)propanoate

To a stirred slurry of (*RR*,*SS*)-2-cyano-2-[(2-methoxyethoxy)methyl]-3-(2-methoxyphenyl)-3-(1-naphthyl)propanoic acid (209 mg, 0.5 mmol) in anhydrous THF (2mL) and cyclohexane (1mL) was added tert-butyl 2,2,2-trichloroacetimidate (179 µL, 1 mmol) followed by borontrifluoride etherate (10 µL). After 16 hours the reaction was quenched with solid sodium bicarbonate, filtered, concentrated and purified using a Biotage® (40Mi) column for flash column chromatography (30-50% methyl tert-butylether in hexane) afforded 171 mg (72%).
mp 137.3-143.8 °C; ¹H NMR (400 MHz, DMSO-d₆) δ ppm 1.01 (s, 9 H) 3.23 (s, 3 H) 3.42 (t, *J*=4.55 Hz, 2 H) 3.59 (m, 3 H) 4.07 (s, 3 H) 4.19 (d, *J*=9.62 Hz, 1 H) 5.77 (s, 1 H) 6.84 (td, *J*=7.54, 1.30 Hz, 1 H) 7.15 (dd, *J*=8.84, 1.04 Hz, 1 H) 7.26 (td, *J*=7.34, 1.69 Hz, 2 H) 7.48 (m, 2 H) 7.62 (dd, *J*=8.19, 7.41 Hz, 1 H) 7.89 (m, 3 H) 8.13 (d, *J*=6.76 Hz, 1 H); MS (ESI) *m*/*z* 493 ([M+NH4]⁺);
MS (ESI) *m*/*z* 420 ([M+H-C3H8]⁺);
HRMS: calcd for C₂₉H₃₃NO₅, 475.2359 (M), 476.24315 ([M+H]⁺); found (ESI_FT), 476.24454;
Anal. Calcd for C₂₉H₃₃NO₅: C, 73.24; H, 6.99; N, 2.95. Found: C, 73.16; H, 7.13; N, 2.86.

### Example 174

### (RR,SS)-3-(2-methoxyphenyl)-3-(1-naphthyl)-2-(pyridin-3-ylmethyl)-2-[(4-pyrimidin-2-ylpiperazin-1-yl)carbonyl]propanenitrile

Oxalyl chloride (436 µL, 5.0 mmol) was added to a stirred slurry of (*RR,SS*)-2-cyano-3-(2-methoxyphenyl)-3-(1-naphthyl)-2-(pyridin-3-ylmethyl)propanoic acid (459 mg, 1.0 mmol) followed by the addition of a 0.25 M solution of DMF in dichloromethane (20 µL). The mixture was allowed to stir at room temperature for 2 hours to afford a clear homogeneous solution. The volatile components were removed *in vacuo* and the resulting solid was suspended in anhydrous dichloromethane and reconcentrated (3x). The solid was resuspended in dichloromethane and a slurry containing 4-(pyrimidin-2-yl)piperazinyl dihydrochloride (261 mg, 1.1 mmol) and triethylamine (488 µL, 3.5 mmol). The mixture stirred at ambient temperature for 16 hours. The organic phase was washed with 5% aqueous sodium bicarbonate, dried (Na₂SO₄), filtered, concentrated, and purified from a Biotage® (40Mi) column for flash column chromatography (0-1 % methanol in ethyl acetate) afforded 300 mg (52%).
mp 227-229 °c; ¹H NMR (400 MHz, DMSO-d₆) δ ppm 2.99 (d, *J*=13.45 Hz, 2 H) 3.19 (s, 2 H) 3.43 (s, 2 H) 3.60 (s, 2 H) 3.72 (d, *J*=13.45 Hz, 1 H) 4.07 (s, 4 H) 6.36 (s, 1 H) 6.65 (t, *J*=4.79 Hz, 1 H) 6.89 (t, *J*=7.11 Hz, 1 H) 7.19 (d, *J*=7.76 Hz, 1 H) 7.30 (td, *J*=7.89, 1.55 Hz, 2 H) 7.37 (dd, *J*=7.76, 4.91 Hz, 1 H) 7.47 (m, 2 H) 7.59 (m, 2 H) 7.85 (m, 2 H) 7.99 (m, 2 H) 8.36 (m, 3 H) 8.50 (dd, *J*=4.66, 1.55 Hz, 1 H); MS (ESI) *m*/*z* 569. ([M+H]⁺); HRMS: calcd for C₃₅H₃₂N₆O₂, 568.2587 (M), 569.26595 ([M+H]⁺); found (ESI_FT), 569.2662;

### Example 175

### (RR,SS)-N-(tert-butyl)-2-cyano-3-(2-methoxyphenyl)-3-(1-naphthyl)-2-(pyridin-3-ylmethyl)propanamide

The title compound was prepared from oxalyl chloride (436 µL, 5.0 mmol), (*RR,SS*)-2-cyano-3-(2-methoxyphenyl)-3-(1-naphthyl)-2-(pyridin-3-ylmethyl)propanoic acid (229 mg, 0.5 mmol), *tert*-butylamine (106 µL, 1 mmol), and triethylamine (140 µL, 1 mmol) according to the procedure and in the same manner as described in Example 167 part 2 to afford 230 mg (97%).
mp 291.3-293 °C; ¹H NMR (400 MHz, DMSO-d₆) δ ppm 0.72 (s, 9 H) 2.83 (d, *J*=13.77 Hz, 1 H) 3.83 (d, *J*=13.77 Hz, 1 H) 4.08 (s, 3 H) 6.14 (s, 1 H) 6.89 (td, *J*=7.54, 1.04 Hz, 1 H) 7.06 (br s, 1 H) 7.17 (dd, *J*=8.32, 1.04 Hz, 1 H) 7.27 (m, 1 H) 7.32 (ddd, *J*=7.80, 4.68, 0.78 Hz, 1 H) 7.40 (dd, *J*=7.80, 1.56 Hz, 1 H) 7.49 (m, 3 H) 7.58 (m, 1 H) 7.81 (d, *J*=8.32 Hz, 1 H) 7.87 (m, 1 H) 8.10 (dd, *J*=7.28, 0.78 Hz, 1 H) 8.13 (d, *J*=8.58 Hz, 1 H) 8.36 (dd, *J*=2.34, 0.52 Hz, 1 H) 8.47 (dd, *J*=4.81, 1.69 Hz, 1 H); MS (ESI) *m*/*z* 478 ([M+H]⁺); HRMS: calcd for C₃₁H₃₁N₃O₂, 477.2416 (M), 478.24891 ([M+H]⁺); found (ESI_FT), 478.24761.

### Example 176

### (RR,SS)-2-cyano-N-(2,2-dimethylpropyl)-3-(2-methoxyphenyl)-3-(1-naphthyl)-2-(pyridin-3-ylmethyl)propanamide

The title compound was prepared from oxalyl chloride (436 µL, 5.0 mmol), (*RR,SS*)-2-cyano-3-(2-methoxyphenyl)-3-(1-naphthyl)-2-(pyridin-3-ylmethyl)propanoic acid (229 mg, 0.5 mmol), neopentylamine (118 µL, 1 mmol), and triethylamine (140 µL, 1 mmol) according to the procedure and in the same manner as described in Example 167 part 2 to afford 200 mg (82%).
mp 193.6-194 °C; ¹H NMR (400 MHz, DMSO-d₆) δ ppm 0.20 (s, 9 H) 2.46 (m, 1 H) 2.72 (dd, *J*=12.99, 7.02 Hz, 1 H) 2.82 (d, *J*=13.77 Hz, 1 H) 3.32 (s, 1 H) 3.81 (d, *J*=13.77 Hz, 1 H) 4.10 (s, 3 H) 6.18 (s, 1 H) 6.87 (td, *J*=7.54, 1.04 Hz, 1 H) 7.18 (dd, *J*=8.32, 1.04 Hz, 1 H) 7.30 (m, 3 H) 7.48 (m, 3 H) 7.80 (d, *J*=8.32 Hz, 1 H) 7.86 (m, 1 H) 8.06 (t, *J*=6.24 Hz, 1 H) 8.13 (m, 2 H) 8.28 (d, *J*=1.56 Hz, 1 H) 8.46 (dd, *J*=4.81, 1.69 Hz, 1 H); MS (ESI) *m*/*z* 492 ([M+H]⁺); MS (ESI) *mlz* 490 ([M-H]⁻); HRMS: calcd for C₃₂H₃₃N₃O₂, 491.2573 (M), 492.26456 ([M+H]⁺); found (ESI_FT), 492.2642

### Example 177

### (RR,SS)-3-(2-methoxyphenyl)-3-(1-naphthyl)-2-[(4-pyrazin-2-ylpiperazin-1-yl)carbonyl]-2-(pyridin-3-ylmethyl)propanenitrile

The title compound was prepared from oxalyl chloride (218 µL, 2.5 mmol), (*RR*,*SS*)-2-cyano-3-(2-methoxyphenyl)-3-(1-naphthyl)-2-(pyridin-3-ylmethyl)propanoic acid (229 mg, 0.5 mmol), 4-(pyrazin-2-yl)piperazine (84 mg, 0.51 mmol), and triethylamine (140 µL, 1 mmol) according to the procedure and in the same manner as described in Example 167 part 2 to afford 148 mg (52%).
mp 247-248 °C; ¹H NMR (400 MHz, DMSO-d₆) δppm 2.98 (d, *J*=13.51 Hz, 1 H) 3.25 (s, 6 H) 3.71 (d, *J*=12.99 Hz, 2 H) 4.07 (s, 4 H) 6.36 (s, 1 H) 6.89 (td, *J*=7.54, 1.04 Hz, 1 H) 7.19 (d, *J*=7.80 Hz, 1 H) 7.34 (m, 3 H) 7.47 (m, 2 H) 7.58 (m, 2 H) 7.85 (m, 3 H) 7.99 (dd, *J*=19.88, 7.41 Hz, 2 H) 8.05 (s, 1 H) 8.17 (s, 1 H) 8.38 (d, *J*=1.82 Hz, 1 H) 8.49 (dd, *J*=4.81, 1.69 Hz, 1 H); MS (ESI) *m*/*z* 569 ([M+H]⁺); HRMS: calcd for C₃₅H₃₂N₆O₂, 568.2587 (M), 569.26595 ([M+H]⁺); found (ESI_FT), 569.26613.

### Example 178

### (RR,SS)-3-[4-(1H-indol-3-yl)-3,6-dihydropyridin-1(2H)-yl]-2-[(2-methoxyphenyl)(1 - naphthyl)methyl]-3-oxo-2-(pyridin-3-ylmethyl)propanenitrile

The title compound was prepared from oxalyl chloride (218 µL, 2.5 mmol), (*RR*,*SS*)-2-cyano-3-(2-methoxyphenyl)-3-(1-naphthyl)-2-(pyridin-3-ylmethyl)propanoic acid (229 mg, 0.5 mmol), 3-(1,2,3,6-tetrahydro-4-pyridinyl)-1*H-*indole (101 mg, 0.51 mmol), and triethylamine (140 µL, 1 mmol) according to the procedure and in the same manner as described in Example 167 part 2 to afford 230 mg (76%)..
mp 270 °C decomp.; MS (ESI) *m*/*z* 603 ([M+H]⁺); MS (ESI) *m*/*z* 601 ([M-H]⁻); HRMS: calcd for C₄₀H₃₄N₄O₂. 602.26818 (M), 603.27546 ([M+H]⁺); found (ESI_FT), 603.27355

### Example 179

### tert-Butyl (RR,SS)-2-cyano-3-(2-methoxyphenyl)-3-(1-naphthyl)-2-(pyridin-3-ylmethyl)propanoate

To a stirred slurry of (*RR*,*SS*)-2-cyano-3-(2-methoxyphenyl)-3-(1-naphthyl)-2-(pyridin-3-ylmethyl)propanoic acid (229 mg, 0.5 mmol) in anhydrous THF (2mL) and cyclohexane (1 mL) was added *tert*-butyl 2,2,2-trichloroacetimidate (179 µL, 1 mmol) followed by borontrifluoride etherate (10 µL). After 16 hours the reaction was quenched with solid sodium bicarbonate, filtered, concentrated and purified using a Biotage® (40Mi) column for flash column chromatography (2-4% methanol in dichloromethane) afforded 209 mg (88%).
mp 236.3-239 °C; ¹H NMR (400 MHz, DMSO-d₆) δ ppm 0.82 (s, 9 H) 2.93 (d, *J*=13.77 Hz, 1 H) 3.55 (d, *J*=13.77 Hz, 1 H) 4.07 (s, 3 H) 5.99 (s, 1 H) 6.87 (td, *J*=7.54, 1.30 Hz, 1 H) 7.17 (dd, *J*=8.58, 1.04 Hz, 1 H) 7.30 (m, 3 H) 7.48 (m, *J*=15.30, 8.28, 6.95, 1.43 Hz, 2 H) 7.57 (m, 2 H) 7.83 (d, *J*=8.32 Hz, 1 H) 7.87 (m, 1 H) 7.99 (d, *J*=8.32 Hz, 1 H) 8.08 (d, *J*=7.02 Hz, 1 H) 8.34 (d, *J*=1.56 Hz, 1 H) 8.48 (dd, *J*=4.94, 1.56 Hz, 1 H); MS (ESI) *m*/*z* 479 ([M+H]⁺); HRMS: calcd for C₃₁H₃₀N₂O₃, 478.2256 (M), 479.23292 ([M+H]⁺); found (ESI_FT), 479.23116.

### Example 180

### (RR,SS)-N-(tert-butyl)-2-cyano-2-(methoxymethyl)-3-(2-methoxyphenyl)-3-(1-naphthyl)propanamide

Oxalyl chloride (308 µL, 3.5 mmol) was added to a stirred slurry of (RR,SS)-2-cyano-2-(methoxymethyl)-3-(2-methoxyphenyl)-3-(1-naphthyl)propanoic acid (265 mg, 0.71 mmol) in dichloromethane followed by the addition of a 0.25 M solution of DMF in dichloromethane (20 µL). The mixture was allowed to stir for 16 h at room temperature to afford a clear homogeneous solution. The volatile components were removed *in vacuo* and the resulting solid was dissolved in anhydrous dichloromethane and reconcentrated (3x). The solid was dissolved in anhydrous dichloromethane (6 mL) and divided into two equal portions. The first portion was added to a solution containing tert-butylamine (116 µL, 1.05 mmol) and triethylamine (197 µL, 1.4 mmol) in dichloromethane and the solution was mixed for 16 hours. The volume was reduced and the residue was dissolved in dichloromethane and applied directly to a Biotage® (40Mi) column for flash column chromatography (30-50% methyl tert-butylether in hexane) afforded 122 mg (80%).
mp 180.9-183.4 °C; ¹H NMR (400 MHz, DMSO-d₆) δ ppm 0.89 (s, 9 H) 3.29 (s, 3 H) 3.41 (d, *J*=9.62 Hz, 1 H) 4.05 (s, 3 H) 4.17 (d, *J*=9.88 Hz, 1 H) 5.86 (s, 1 H) 6.82 (td, *J*=7.54, 1.04 Hz, 1 H) 6.99 (s, 1 H) 7.12 (dd, *J*=8.32, 1.04 Hz, 1 H) 7.24 (m, 2 H) 7.44 (m, 2 H) 7.55 (dd, *J*=8.19, 7.41 Hz, 1 H) 7.85 (m, 2 H) 7.96 (m, 1 H) 8.12 (d, *J*=7.28 Hz, 1 H); MS (ESI) *m*/*z* 431 ([M+H]⁺); HRMS: calcd for C₂₇H₃₀N₂O₃, 430.2256 (M), 431.23292 ([M+H]⁺); found (ESI_FT), 431.23303; Anal. Calcd for C₂₇H_{3O}N₂O₃: C, 75.32; H, 7.02; N, 6.51. Found: C, 75.29; H, 7.00; N, 6.47.

### Example 181

### (2S*,3S*)-2-cyano-N-(2,2-dimethylpropyl)-2-(methoxymethyl)-3-(2-methoxyphenyl)-3-(1-naphthyl)propanamide

The title compound was prepared from (*RR*,*SS*)-2-cyano-2-(methoxymethyl)-3-(2-methoxyphenyl)-(1-naphthyl)propanoic acid, neopentylamine (124 µL, 1.05 mmol), and triethylamine (197 µL, 1.4 mmol) according to the procedure and in the same manner as described in Example 180 to afford 128 mg, (82%).
mp 184.9-185.5 °C; ¹H NMR (400 MHz, DMSO-d₆) δ ppm 0.46 (s, 9 H) 2.62 (dd, *J*=12.99, 5.98 Hz, 1 H) 2.80 (dd, *J*=12.99, 6.76 Hz, 1 H) 3.26 (s, 3 H) 3.37 (d, *J*=9.10 Hz, 1 H) 4.07 (s, 3 H) 4.18 (d, *J*=9.36 Hz, 1 H) 5.91 (s, 1 H) 6.81 (s, 1 H) 7.13 (dd, *J*=8.32, 1.04 Hz, 1 H) 7.22 (m, 2 H) 7.43 (m, 2 H) 7.51 (dd, *J*=8.19, 7.41 Hz, 1 H) 7.84 (m, 2 H) 7.94 (m, 1 H) 8.01 (t, *J*=6.24 Hz, 1 H) 8.17 (d, *J*=7.28 Hz, 1 H); MS (ESI) *m*/*z* 445 ([M+H]⁺); HRMS: calcd for C₂₈H₃₂N₂O₃, 444.2413 (M), 445.24857 ([M+H]⁺); found (ESI_FT), 445.24759; Anal. Calcd for C₂₈H₃₂N₂O₃: C, 75.65; H, 7.26; N, 6.30. Found: C, 75.46; H, 7.28; N, 6.26.

### Example 182

### (2S)-3-[4-(1H-indol-3-yl)-3,6-dihydropyridin-1(2H)-yl]-2-[(S)-(2-methoxyphenyl)(1-naphthyl)methyl]-2-methyl-3-oxopropanenitrile

(2*S*,3*S*)-2-Cyano-3-(2-methoxyphenyl)-2-methyl-3-(1-naphthyl)propanoic acid (0.34 g, 1.0 mmol), 1-hydroxybenzotriazole (0.27 g, 2.0 mmol), and 1-(3-dimethyl aminopropyl)-3-ethylcarbodiimide (0.38 g, 2.0 mmol) were combined in DMF (100 ml) and stirred at room temperature for 1 hour. To this added 3-(1,2,3,6-tetrahydropyridin-4-yl)-1H-indole (0.2 g, 1.0 mmol) and the mixture was stirred further at room temperature for 15 hours. The mixture was then concentrated and partitioned between ethyl acetate and saturated aqueous NaHCO₃. The separated organic layer was washed with water, dried over anhydrous Na₂SO₄, and concentrated under *vacuo*. The residue was chromatographed on Biotage (FlasH40i, ethyl acetate/hexane, 2/8) to give an off-white solid as the expected product The solid product was recrystallized from ethyl acetate and minimum amount of hexane to afford the title compound as white crystals.
mp 229-231 °C; MS (ESI) *m*/*z* 526 ([M+H]⁺); MS (ESI) *m*/*z* 524 ((M-H]⁻); Anal. Calcd for C₃₅H₃₁N₃O₂0.30 H₂O: C, 79.16; H, 6.00; N, 7.91. Found: C, 79.20; H, 5.96; N, 7.83

### Example 183

### (2S,3S)-3-(2-methoxyphenyl)-2-methyl-3-(1-naphthyl)-2-{[4-(2-oxo-2,3-dihydro-1H-benzimidazol-1-yl)piperidin-1-yl]carbonyl}propanenitrile

The title compound was prepared according to Example 182 using 4-(2-keto-1-benzimidazolinyl)piperidine. The crude product was purified on Biotage (FlasH40i, ethyl acetate/hexane, 4/6) and the expected product was recrystallized from ethyl acetate and minimum amount of hexane to afford the title compound as a white solid.
mp 197-200 °C; MS (ESI) *mlz* 545 ([M+H]⁺); MS (ESI) *m*/*z* 543 ([M-H]⁻); Anal. Calcd for C₃₄H₃₂N₄O₃ H₂O: C, 72.58; H, 6.09; N, 9.96. Found: C, 72.71; H, 6.29; N, 9.61

### Example 184

### (2S,3S)-3-(2-methoxyphenyl)-2-methyl-3-(1-naphthyl)-2-[(4-oxo-1-phenyl-1,3,8-triazaspiro[4.5]dec-8-yl)carbonyl]propanenitrile

The title compound was prepared according to Example 182 using 1-phenyl-1,3,8-triazaspiro[4.5]decan-4-one. The crude product was purified on Biotage (FlasH40i, ethyl acetate/hexane, 4/6) to give a desired product as a white solid and the solid was recrystallized from ethyl acetate and a minimum amount of mixed hexane and ether to afford the title compound as a white solid.
mp 176 - 178 °C; MS (ESI) *mlz* 559 ([M+H]⁺); MS (ESI) *m*/*z* 557 ([M-H]⁻);
Anal. Calcd for C₃₅H₃₄N₄O₃ 0.40 H₂O: C, 74.29; H, 6.20; N, 9.90. Found: C, 74.58; H, 6.60; N, 9.68

### Example 185

### (2S)-3-{3-hydroxy-4-[3-(trifluoromethyl)phenyl]piperidin-1-yl}-2-[(S)-(2-methoxyphenyl)(1-naphthyl)methyl]-2-methyl-3-oxopropanenitrile

The title compound was prepared according to Example 182 using 4-[3-(trifluoromethyl)phenyl]-3-piperidinol. The crude product was purified on Biotage (FlasH40i, ethyl acetate/hexane, 4/6) to give a desired product as solid and the solid was recrystallized from ethyl acetate and a minimum amount of hexane to afford the title compound as a white solid.
mp 259 °C; MS (ESI) *m*/*z* 573 ([M+H]⁺); Anal. Calcd for C₃₄H₃₁F₃N₂O₃: C, 71.32; H, 5.46; N, 4.89. Found: C, 71.18; H, 5.59; N, 4.83

### Example 186

### (2S)-3-[4-(4-chlorophenyl)-4-hydroxypiperidin-1-yl]-2-[(S)-(2-methoxyphenyl)(1-naphthyl)methyl]-2-methyl-3-oxopropanenitrile

The title compound was prepared according to Example 182 using 4-(4-chlorophenyl)-3-piperidinol. The crude product was purified on Biotage high pressure liquid chromatography using 10-50-70% methyl t-butyl ether in hexane to give a desired product as a white solid. The solid was recrystallized from CH₂Cl₂ and a minimum amount of hexane to afford the title compound as a white solid.
mp 202-204 °C; MS (ESI) *m*/*z* [M+H]+= (539); Anal. Calcd for C₃₃H₃₁ClN₂O₃: C, 73.53; H, 5.80; N, 5.20. Found: C, 73.34; H, 5.67; N, 5.00

### Example 187

### (2S)-3-[4-(1H-indol-3-yl)piperidin-1-yl]-2-[(S)-(2-methoxyphenyl)(1-naphthyl)methyl]-2-methyl-3-oxopropanenitrile

The title compound was prepared according to Example 182 using 3-piperidin-4-yl-1H-indole. The crude product was purified by Biotage high pressure liquid chromatography using 10-50-70% methyl t-butyl ether in hexane to give a desired product as a white solid. The solid was recrystallized from CH₂Cl₂ and a minimum amount of hexane to afford the title compound as a white solid.
mp 267-268 °C; MS (ESI) *mlz* [M+H]+= (528); MS (ESI) *m*/*z* [M-H]-= (526); Anal. Calcd for C₃₅H₃₃N₃O_{2 ˙} 0.40 H₂O: C, 78.60; H, 6.37; N, 7.86. Found: C, 78.47; H, 6.02; N, 7.65

### Example 188

### (2S)-3-(1,4-dioxa-8-azaspiro[4.5]dec-8-yl)-2-[(S)-(2-methoxyphenyl)(1-naphthyl)methyl- 2-methyl-3-oxopropanenitrile

The title compound was prepared according to Example 182 using 1,4-dioxa-8-azaspiro(4,5)decane. The crude product was purified by Biotage high pressure liquid chromatography using 10-50-70% methyl t-butyl ether in hexane to give a desired product as a white solid. The solid was recrystallized from CH₂Cl₂ and a minimum amount of hexane to afford the title compound as a white solid.
mp 217 °C; MS (ESI) *m*/*z* 471 ([M+H]⁺); Anal. Calcd for C₂₉H₃₀N₂O₄: C, 74.02; H, 6.43; N, 5.95. Found: C, 73.87; H, 6.20; N, 5.90

### Example 189

### (2S)-3-(8-fluoro-1,3,4,5-tetrahydro-2H-pyrido[4,3-b]indol-2-yl)-2-[(S)-(2-methoxy phenyl)(1-naphthyl)methyl]-2-methyl-3-oxopropanenitrile

The title compound was prepared according to Example 182 using 6-fluoro-1,2,3,4-tetrahydropyrido[4,3-b]indole. The crude product was purified by Biotage high pressure liquid chromatography using 10-50-70% methyl t-butyl ether in hexane to give a desired product as a fluffy white solid. The solid was recrystallized from CH₂Cl₂ and a minimum amount of mixed hexane and ether to afford the title compound as a white solid.
mp 172-174 °C;
MS (ESI) *m*/*z* [M+H]+= (518); MS (ESI) *m*/*z* [M-H]-= (516);
Anal. Calcd for C₃₃H₂₈FN₃O₂: C, 76.58; H, 5.45; N, 8.12. Found: C, 76.53; H, 5.44; N, 7.81

### Example 190

### 2S,3S)-3-(2-methoxyphenyl)-2-methyl-3-(1-naphthyl)-2-(1,3,4,9-tetrahydro-2H-beta-carbolin-2-ylcarbonyl)propanenitrile

The title compound was prepared according to Example 182 using 1,2,3,4-tetrahydro-9H-pyrido[3,4-b]indole. The crude product was purified by Biotage high pressure liquid chromatography using 10-50-70% methyl t-butyl ether in hexane to give a desired product as a fluffy white solid. The solid was recrystallized from CH₂Cl₂ and a minimum amount of mixed hexane and ether to afford the title compound as a white solid.
mp 212-213 °C; MS (ESI) *m*/*z* 500 ([M+H]⁺); MS (ESI) *m*/*z* 498 ([M-H]⁻); Anal. Calcd for C₃₃H₂₉N₃O₂: C, 79.33; H, 5.85; N, 8.41. Found: C, 79.23; H, 5.83; N, 8.29

### Example 191

### (2S,3S)-3-(2-methoxyphenyl)-2-methyl-3-(1-naphthyl)-2-[(4-oxopiperidin-1-yl)carbonyl] propanenitrile

The title compound was prepared according to Example 182 using 4-piperidone monohydrate hydrochloride. The crude product was purified by Biotage high pressure liquid chromatography using 10-50-70% methyl t-butyl ether in hexane to give a desired product as a white solid. The solid was recrystallized from ether and a minimum amount of hexane to afford the title compound as a white solid.
mp 209-210 °C;
MS (ESI) *m*/*z* 426 ([M+H]⁺); Anal. Calcd for C₂₇H₂₆N₂O₃: C, 76.03; H, 6.14; N, 6.57. Found: C, 75.66; H, 5.98; N, 6.39

### Example 192

### (2S,3S)-3-(2-methoxyphenyl)-2-methyl-3-(1-naphthyl)-2-[(4-pyrimidin-2-ylpiperazin-1-yl)carbonyl]propanenitrile

The title compound was prepared according to Example 182 using 1-(2-pyrimidyl)-piperazine. The crude product was purified by Biotage high pressure liquid chromatography using 10-50-70% methyl t-butyl ether in hexane to give a desired product as a white solid. The solid was recrystallized from CH₂Cl₂ and a minimum amount of hexane to afford the title compound as a white solid.
mp 153-154 °C; MS (ESI) *m*/*z* 492 ([M+H]⁺); Anal. Calcd for C₃₀H₂₉N₅O₂: C, 73.30; H, 5.95; N, 14.25. Found: C, 72.95; H, 5.87; N, 14.26

### Example 193

### (2S)-3-[4-(4-bromophenyl)-4-hydroxypiperidin-1-yl]-2-[(S)-(2-methoxyphenyl)(1-naphthyl)methyl]-2-methyl-3-oxopropanenitrile

The title compound was prepared according to Example 182 using 4-(4-bromophenyl)-4-piperidinol. The crude product was purified by Biotage high pressure liquid chromatography using 10-50-70% methyl t-butyl ether in hexane to give a desired product as a white solid. The solid was recrystallized from ethyl acetate and a minimum amount of mixed hexane and ether to afford the title compound as a white solid.
mp 206-208 °C; MS (ESI) *m*/*z* 583/585 ([M+H]⁺); Anal. Calcd for C₃₃H₃₁BrN₂O₃: C, 67.93; H, 5.35; N, 4.80. Found: C, 67.89; H, 5.22; N, 4.72

### Example 194

### (2S,3S)-3-(2-methoxyphenyl)-2-methyl-3-(1-naphthyl)-2-[(4-phenyl-4-propionyl piperidin-1-yl)carbonyl]propanenitrile

The title compound was prepared according to Example 182 using 4-phenyl-4-propionylpiperidine hydrochloride. The crude product was purified by Biotage high pressure liquid chromatography using 10-50-70% methyl t-butyl ether in hexane to give a desired product as a white solid. The solid was recrystallized from ether and a minimum amount of hexane to afford the title compound as a white solid.
mp 126-128 °C; MS (ESI) *m*/*z* 531 ([M+H]⁺); Anal. Calcd for C₃₆H₃₆N₂O₃: C, 79.38; H, 6.66; N, 5.14. Found: C, 79.67; H, 6.62; N, 5.00

### Example 195

### (2S)-3-(4-acetyl-4-phenylpiperidin-1-yl)-2-[(S)-(2-methoxyphenyl)(1-naphthyl)methyl]-2-methyl-3-oxopropanenitrile

The title compound was prepared according to Example 182 using 4-acetyl-4-phenylpiperidine hydrochloride. The crude product was purified by Biotage high pressure liquid chromatography using 10-50-70% methyl t-butyl ether in hexane to give a desired product as a white solid. The solid was recrystallized from ether and a minimum amount of hexane to afford the title compound as a white solid.
mp 134-135 °C; MS (ESI) *m*/*z* 545 ([M+H]⁺); Anal. Calcd for C₃₅H₃₄N₂O₃: C, 79.22; H, 6.46; N, 5.28. Found: C, 79.29; H, 6.75; N, 5.09

### Example 196

### (2S)-3-(4-cyclohexylpiperazin-1-yl)-2-[(S)-(2-methoxyphenyl)(1-naphthyl)methyl]-2-methyl-3-oxopropanenitrile

The title compound was prepared according to Example 182 using 4-cyclohexylpiperazine. The crude product was purified by Biotage high pressure liquid chromatography using 10-50-70% methyl t-butyl ether in hexane to give a desired product as a white solid. The solid was recrystallized from ether and a minimum amount of hexane to afford the title compound as a white solid.
mp 137-138 °C; MS (ESI) *m*/*z* 496 ([M+H]⁺); Anal. Calcd for C₃₂H₃₇N₃O₂: C, 77.54; H, 7.52; N, 8.48. Found: C, 77.76; H, 7.50; N, 8.40

### Example 197

### (2S)-3-[4-(2-methoxyethyl)piperazin-1-yl]-2-[(S)-(2-methoxyphenyl)(1-naphthyl)methyl]-2-methyl-3-oxopropanenitrile

The title compound was prepared according to Example 182 using 1-(2-methoxy-ethyl)-piperazine. The crude product was purified by Biotage high pressure liquid chromatography using 10-50-70% methyl t-butyl ether in hexane to give a desired product as a white solid. The solid was recrystallized from CH₂Cl₂ and a minimum amount of mixed hexane and ether to afford the title compound as a white solid.
mp 144 °C; MS (ESI) *m*/*z* 472 ([M+H]⁺); Anal. Calcd for C₂₉H₃₃N₃O₃: C, 73.86; H, 7.05; N, 8.91. Found: C, 73.42; H, 7.05; N, 8.86

### Example 198

### (2S,3S)-3-(2-methoxyphenyl)-2-methyl-2-{[4-(2-morpholin-4-yl-2-oxoethyl)piperazin-1-yl]carbonyl}-3-(1-naphthyl)propanenitrile

The title compound was prepared according to Example 182 using piperazino-acetic acid morpholine. The concentrated reaction mixture was treated with CH₂Cl₂ to precipitate a white solid. The solid was then filtered off and washed with CH₂Cl₂ to afford the title compound as a white solid.
mp 201 °C; ¹H NMR (DMSO-d₆): δ1.43 (s, 3H, CH3), δ2.64 (t, 4H, 2NCH2CH2O), δ3.07 (t, 4H, 2NCH2CH2NC=ON), δ3.44 (t, 2H, OCH2CH2N), δ3.49 (t, 2H, OCH2CH2N), δ3.58 (4H, 2NCH2CH2NC=ON), δ3.98 (s, 3H, OCH3), δ5.87 (s, 1 H, CH), δ6.72-δ8.20 (11H, aromatic Hs); Anal. Calcd for C₃₂H₃₆N₄O₄ 1.60 H₂O: C, 67.49; H, 6.94; N, 9.84. Found: C, 67.23; H, 7.20; N, 10.49; HRMS: calcd for C₃₂H₃₆N₄O₄, 541.28094 ([M+H]⁺); found (ESI+), 541.27891

### Example 199

### (2S)-3-[4-(2-furoyl)piperazin-1-yl]-2-[(S)-(2-methoxyphenyl)(1-naphthyl)methyl]-2-methyl-3-oxopropanenitrile

The title compound was prepared according to Example 182 using 1-(2-furoyl)-piperazine. The concentrated reaction mixture was treated with CH₂Cl₂ to precipitate a white solid. The solid was then filtered off and washed with CH₂Cl₂ to afford the title compound as a white solid.
mp 193 °C;
¹H NMR (DMSO-d₆): δ1.44 (s, 3H, CH3), δ3.06 (t, 4H, 2NCH2CH2N), δ3.76 (s, 4H, 2NCH2CH2N), δ4.03 (s, 3H, OCH3), δ5.68 (s, 1 H, CH), δ6.67 (dd, 1 H, CHCHO), δ7.01 (dd, 1H, CHCO), δ7.84 (d, 1H, CHO), δ6.73-δ8.24 (11H, aromatic Hs);
Anal. Calcd for C₃₁H₂₉N₃O₄ 2.00 H₂O: C, 68.49; H, 6.12; N, 7.73. Found: C, 68.75;
H, 6.09; N, 7.95
HRMS: calcd for C₃₁H₂₉N₃O₄, 508.22309 ([M+H]⁺); found (ESI+), 508.22313

### Example 200

### (RR,SS)-2-(methoxymethyl)-3-(2-methoxyphenyl)-3-(1-naphthyl)-2-[(4-pyrimidin-2-ylpiperazin-1-yl)carbonyl]propanenitrile

The title compound was prepared according to Example 182 using 1-(2-pyrimidyl)-piperazine and (*RR*,*SS*)-2-(methoxymethyl)-2-cyano-3-(2-methoxyphenyl)-3-(1-naphthyl) propanoic acid. The crude product was purified by Biotage (FlasH40i, ethyl acetate/hexane, 3/7, 1/1) to give a desired product as a solid, which was recrystallized from CH₂Cl₂ and a minimum amount of ether to afford the title compound as a white solid.
mp 288 -289 °C; MS (ESI) *m*/*z* 522 ([M+H]⁺); Anal. Calcd for C₃₁H₃₁N₅O₃ ˙ 0.25 H₂O: C, 70.77; H, 6.03; N, 13.31. Found: C, 70.85; H, 5.80; N, 13.11

### Example 201

### (2S,3S)-3-(2-methoxyphenyl)-2-methyl-3-(1-naphthyl)-2-{[4-(2-oxo-2-pyrrolidin-1-ylethyl)piperazin-1-yl]carbonyl}propanenitrile

The title compound was prepared according to Example 182 using piperazino-acetic acid pyrrolidine. The crude product was purified by Biotage (FlasH40i, ethyl acetate/hexane, 9/1) to give a desired product as a solid, which was recrystallized from CH₂Cl₂ and a minimum amount of mixed hexane and ether to afford the title compound as a white solid.
mp 203 °C; MS (ESI) *m*/*z* 525 ([M+H]⁺); Anal. Calcd for C₃₂H₃₆N₄O₃ 0.50 H₂O: C, 72.02; H; 6.99; N, 10.50. Found: C, 72.03; H, 6.86; N, 10.46

### Example 202

### (2S)-3-{4-[3-(dimethylamino)propyl]piperazin-1-yl}-2-[(S)-(2-methoxyphenyl)(1-naphthyl)methyl]-2-methyl-3-oxopropanenitrile

The title compound was prepared according to Example 182 using 1-(3-dimethylaminopropyl)-piperazine. The crude product was purified by Biotage (FlasH40i, ethyl acetate/MeOH/NH₄OH, 90/10/0.1) to give a desired product as a solid, which was recrystallized from CH₂Cl₂ and a minimum amount of ether to afford the title compound as a white solid.
mp 78-80 °C; MS (ESI) *m*/*z* 499 ([M+H]⁺); HRMS: calcd for C₃₁H₃₈N₄O₂, 499.30676 ([M+H]⁺); found (ESI+), 499.30559

### Example 203

### (2S,3S)-3-(2-methoxyphenyl)-2-methyl-2-[(4-morpholin-4-ylpiperidin-1-yl)carbonyl]-3-(1-naphthyl)propanenitrile

The title compound was prepared according to Example 182 using 4-(4-morpholinyl)-piperidine. The crude product was purified by Biotage (FlasH40i, CH₂Cl₂/MeOH, 9.5/0.5, 9/1)) to give a desired product as a solid, which was recrystallized from CH₂Cl₂ and a minimum amount of hexane and ether to afford the title compound as a white solid.
mp 209-210 °C; MS (ESI) *m*/*z* 499 ([M+H]⁺); Anal. Calcd for C₃₁H₃₅N₃O_{3 ˙} 0.25 H₂O: C, 74.15; H, 7.13; N, 8.37. Found: C, 74.18; H, 7.37; N, 8.23

### Example 204

### 3-{4-[3-(dimethylamino)propyl]piperazin-1-yl}-2-(methoxymethyl)-2-[(2-methoxyphenyl)(1-naphthyl)methyl]-3-oxopropanenitrile

The title compound was prepared according to Example 182 using 1-(3-dimethylaminopropyl)-piperazine and (*RR,SS*)-2-(methoxymethyl)-2-cyano-3-(2-methoxy phenyl)-3-(1-naphthyl) propanoic acid. The crude product was purified by Biotage (FlasH40i, CH₂Cl₂/MeOH, 9/1, 8/2)) to give a desired product as a solid, which was recrystallized from CH₂Cl₂ and a minimum amount of ether to afford the title compound as a white solid.
mp 174-175 °C; MS (ESI) *m*/*z* 529 ([M+H]⁺); Anal. Calcd for C₃₂H₄₀N₄O₃: C, 72.70; H, 7.63; N, 10.60. Found: C, 70.19; H, 7.25; N, 11.84

### Example 205

### (2S*)-2-[(benzyloxy)methyl]-3-{4-[3-(dimethylamino)propyl]piperazin-1-yl}-2-[(R*)-(2-methoxyphenyl)(1-naphthyl)methyl]-3-oxopropanenitrile

The title compound was prepared according to Example 182 using 1-(3-dimethylaminopropyl)-piperazine and (*RR*,*SS*)-2-[(benzyloxy)methyl]-2-cyano-3-(2-methoxyphenyl)-3-(1-naphthyl)propanoic acid. The crude product was purified by Biotage (FlasH40i, CH₂Cl₂/MeOH, 9/1, 8/2)) to give a desired product as an oil, which was triturated with CH₂Cl₂ and a minimum amount of ether to afford the title compound as a white solid. mp 192-193°C; MS (ESI) *m*/*z* 605 ([M+H]⁺); HRMS:
calcd for C₃₈H₄₄N₄O₃, 605.34862 ([M+H]⁺); found (ESI+), 605.34834

### Example 206

### 3-[4-(2-methoxyethyl)piperazin-1-yl]-2-(methoxymethyl)-2-[(2-methoxyphenyl)(1-naphthyl)methyl]-3-oxopropanenitrile

The title compound was prepared according to Example 182 using 1-(2-methoxy-ethyl)-piperazine and (*RR*,*SS*)-2-(methoxymethyl)-2-cyano-3-(2-methoxy phenyl)-3-(1-naphthyl) propanoic acid. The crude product was purified by Biotage (FlasH40i, CH₂Cl₂/MeOH, 9.75/0.25)) to give a desired product as a solid, which was recrystallized from CH₂Cl₂ and a minimum amount of ether to afford the title compound as a white solid.
mp 156-158 °C; MS (ESI) *m*/*z* 502 ([M+H]⁺); HRMS: calcd for C₃₀H₃₅N₃O₄, 502.27004 ([M+H]⁺); found (ESI+), 502.26953

### Example 207

### 2-[(benzyloxy)methyl]-3-[4-(2-methoxyethyl)piperazin-1-yl]-2-[(2-methoxyphenyl)(1-naphthyl)methyl]-3-oxopropanenitrile

The title compound was prepared according to Example 182 using 1-(2-methoxy-ethyl)-piperazine and (*RR,SS*)-2-[(benzyloxy)methyl]-2-cyano-3-(2-methoxy phenyl)-3-(1-naphthyl) propanoic acid. The crude product was purified by Biotage (FlasH40i, CH₂Cl₂/MeOH, 9.75/0.25)) to give a desired product as a solid, which was recrystallized from CH₂Cl₂ and a minimum amount of ether to afford the title compound as a white solid.
mp 140-141°C; MS (ESI) *m*/*z* 578 ([M+H]⁺); HRMS: calcd for C₃₆H₃₉N₃O₄, 578.30134 ([M+H]⁺); found (ESI_FT), 578.3001

### Example 208

### 2-[(benzyloxy)methyl]-3-(2-methoxyphenyl)-2-{[4-(2-morpholin-4-ylethyl)piperazin-1-yl]carbonyl}-3-(1-naphthyl)propanenitrile

The title compound was prepared according to Example 182 using 1-(2-morpholinoethyl)-piperazine and (*RR,SS*)-2-[(benzyloxy)methyl]-2-cyano-3-(2-methoxy phenyl)-3-(1-naphthyl) propanoic acid. The crude product was purified by Biotage (FlasH40i, CH₂Cl₂/MeOH, 9.75/0.25)) to give a desired product as a solid, which was recrystallized from CH₂Cl₂ and a minimum amount of ether to afford the title compound as a white solid.
mp 162 °C; MS (ESI) *m*/*z* 633 ([M+H]⁺); HRMS: calcd for C₃₉H₄₄N₄O₄, 633.34354 ([M+H]⁺); found (ESI_FT), 633.34249

### Example 209

### (RR,SS)-3-(4-cyclohexylpiperazin-1-yl)-2-(methoxymethyl)-2-[(2-methoxyphenyl)(1-naphthyl)methyl]-3-oxopropanenitrile

(*RR*,*SS*)-2-Cyano-2-(methoxymethyl)-3-(2-methoxyphenyl}-3-(1-naphthyl)propionic acid (0.19 g, 0.5 mmol) was stirred in CH₂Cl₂ at 0 °C in an ice-bath. To this added oxalyl chloride (0.26 ml, 3.0 mmol) dropwise and a catalytic amount of DMF. The reaction mixture was further stirred for an hour until the mixture was clear. The (*RR,SS*)-2-cyano-2-(methoxymethyl)-3-(2-methoxyphenyl)-3-(1-naphthyl) propionic acid chloride was then evaporated to dryness. To this added 1-cyclohexyl-piperazine (0.084 g, 0.5. mmol) and triethylamine (0.08 ml, 0.6 mmol) in CH₂Cl₂ dropwise with stirring at 0 °C in an ice-bath. The reaction mixture was further stirred at 0 °C for 2 hours and concentrated for chromatography on Biotage (FlasH40i, ethyl acetate/hexane, 7/3, ethyl acetate) to give a desired product as a solid. The solid was recrystallized from methylene chloride and a minimum amount of ether to afford the title compound as a white solid.
mp 206 °C; MS (ESI) *m*/*z* 526 ([M+H]⁺); Anal. Calcd for C₃₃H₃₉N₃O₃: C, 75.40; H, 7.48; N. 7.99. Found: C, 75.28; H, 7.76; N, 7.92.

### Example 210

### (2S,3S)-2-(methoxymethyl)-3-(2-methoxyphenyl)-3-(1-naphthyl)-2-[(4-pyrimidin-2-ylpiperazin-1-yl)carbonyl]propanenitrile

(*RR,SS*)-2-(methoxymethyl)-3-(2-methoxyphenyl)-3-(1-naphthyl)-2-[(4-pyrimidin-2-yl piperazin-1-yl)carbonyl]propanenitrile was prepared according to Example 200 using 1-(2-pyrimidyl)-piperazine. The racemate was resolved by a chiral HPLC in the normal phase using hexane/EtOH (75/25) as a mobile phase in Whelk O-1(S, S) column at room temperature with (-) rotation of chiral detection (CD) to give a desired product. The product was recrystallized from ether and a minimum amount of hexane to afford the title compound as an off-white solid.
mp 140 °C; MS (ESI) *m*/*z* 522 ([M+H]⁺); HRMS: calcd for C₃₁H₃₁N₅O₃, 522.24997 ([M+H]⁺); found (ESI_FT), 522.24924.

### Example 211

### (2R,3R)-2-(methoxymethyl)-3-(2-methoxyphenyl)-3-(1-naphthyl)-2-[(4-pyrimidin-2-ylpiperazin-1-yl)carbonyl]propanenitrile

(*RR,SS*)-2-(methoxymethyl)-3-(2-methoxyphenyl)-3-(1-naphthyl)-2-[(4-pyrimidin-2-yl piperazin-1-yl)carbonyl]propanenitrile was prepared according to Example 200 using 1-(2-pyrimidyl)-piperazine. The racemate was resolved by a chiral HPLC in the normal phase using hexane/EtOH (75/25) as a mobile phase in Whelk O-1 (S, S) column at room temperature with (+) rotation of chiral detection (CD) to give a desired product. The product was recrystallized from ether and a minimum amount of hexane to afford the title compound as a light tan solid.
mp 136-137°C;
Optical rotation: [α]_{D}^{16.5} = +15 /EtOH
MS (ESI) *m*/*z* 522 ([M+H]⁺);
HRMS: calcd for C₃₁H₃₁N₅O₃, 522.24997 ([M+H]⁺); found (ESI_FT), 522.24943

### Example 212

### (RR,SS)-2-(methoxymethyl)-3-(2-methoxyphenyl)-3-(1-naphthyl)-2-[(4-pyrazin-2-ylpiperazin-1-yl)carbonyl]propanenitrile

The title compound was prepared according to Example 2098 using 1-(2-pyrazinyl-piperazine. The crude product was purified by Biotage (FlasH40i, ethyl acetate/hexane, 3/7, 7/3) to give a desired product as a solid, which was recrystallized from CH₂Cl₂ and a minimum amount of ether to afford the title compound as a white solid.
mp 259-260 °C;
MS (ESI) *m*/*z* 522 ([M+H]⁺);
Anal. Calcd for C₃₁H₃₁N₅O₃ · 0.20 C₄H₈O₂: C, 70.83; H, 6.09; N, 12.99. Found: C, 70.93; H, 5.94; N, 13.03

### Example 213

### (RR,SS)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile

The title compound was prepared according Example 209 using 3-(1,2,3,6-tetrahydro-pyridin-4-yl)-1H-indole. The reaction mixture was concentrated under vacuo and washed with methylene chloride several times to give a desired product as a pale yellow solid.
mp 285 °C; MS (ESI) *m*/*z* 556 ([M+H]⁺); MS (ESI) *m*/*z* 554 ([M-H]⁻); Anal. Calcd for C₃₆H₃₃N₃O₃ · 0.75 H₂O: C, 75.97; H, 6.11; N, 7.38. Found: C, 75.71; H, 6.21; N, 7.41

### Example 214

### (2S,3S)-3-(2-methoxyphenyl)-2-methyl-3-(1-naphthyl)-2-[(4-pyrazin-2-ylpiperazin-1-yl)carbonyl]propanenitrile

The title compound was prepared according to Example 209 using (2S,3S)-2-cyano-3-(2-methoxyphenyl)-2-methyl-3-(1-naphthyl)propanoic acid and 1-(2-pyrazinyl)-piperazine. The crude product was purified by Biotage (FlasH40i, ethyl acetate) to give a desired product as a solid, which was recrystallized from ether and a minimum amount of hexane in an ice-bath to afford the title compound as a white solid.
mp 143-144°C; MS (ESI) *m*/*z* 492 ([M+H]⁺); Anal. Calcd for C₃₀H₂₉N₅O₂ · 0.10 H₂O: C, 73.03; H, 5.97; N, 14.19. Found: C, 72.84; H, 5.95; N, 13.92

### Example 215

### (2S,3S)-3-(2-methoxyphenyl)-2-methyl-2-{[4-(2-morpholin-4-ylethyl)piperazin-1-yl]carbonyl}-3-(1-naphthyl)propanenitrile

The title compound was prepared according to Example 209 using (2S,3S)-2-cyano-3-(2-methoxyphenyl)-2-methyl-3-(1-naphthyl)propanoic acid and 1-(2-morpholinoethyl)-piperazine. The crude product was purified by Biotage (FlasH40i, CH₂Cl₂/MeOH, 9.5/0.5) to give a desired product as a solid, which was recrystallized from CH₂Cl₂ and a minimum amount of ether in an ice-bath to afford the title compound as a white solid.
mp 102-103 °C; MS (ESI) *m*/*z* 527 ([M+H]⁺); Anal. Calcd for C₃₂H₃₈N₄O₃ · 0.10 H₂O: C, 72.73; H, 7.29; N, 10.60. Found: C, 72.45; H, 7.29; N, 10.52

### Example 216

### (2S)-3-{4-[2-(dimethylamino)ethyl]piperazin-1-yl}-2-[(S)-(2-methoxyphenyl)(1-naphthyl)methyl]-2-methyl-3-oxopropanenitrile

The title compound was prepared according to Example 209 using (2S,3S)-2-cyano-3-(2-methoxyphenyl)-2-methyl-3-(1-naphthyl)propanoic acid and 1-(3-dimethylamino propyl)-piperazine. The crude product was purified by Biotage (FlasH40i, CH₂Cl₂/MeOH, 9.75/0.25, 9.5/0.5) to give a desired product as a solid, which was recrystallized from CH₂Cl₂ and a minimum amount of mixed ether and hexane in an ice-bath to afford the title compound as a white solid.
mp 84-85 °C; MS (ESI) *m*/*z* 485 ([M+H]⁺); HRMS: calcd for C₃₀H₃₆N₄O₂, 485.29111 ([M+H]⁺); found (ESI_FT), 485.29173

### Example 217

### (RR,SS)-2-[(benzyloxy)methyl]-3-(4-cyclohexylpiperazin-1-yl)-2-[(2-methoxyphenyl) (1-naphthyl)methyl]-3-oxopropanenitrile

The title compound was prepared according to Example 209 using (*RR*,*SS*)-2-[(benzyloxy)methyl]-2-cyano-3-(2-methoxyphenyl)-3-(1-naphthyl)propanoic acid and 1-cyclohexylpiperazine. The crude product was purified by Biotage (FlasH40i, CH2Cl2/MeOH, 9.75/0.25, 9.5/0.5) to give a desired product as a solid, which was recrystallized from CH₂Cl₂ and a minimum amount of mixed ether and hexane in an ice-bath to afford the title compound as a white solid.
mp 84 -85 °C; MS (ESI) *m*/*z* 485 ([M+H]⁺); HRMS: calcd for C₃₉H₄₃N₃O₃, 602.33772 ([M+H]⁺); found (ESI_FT), 602.33634

### Example 218

### (RR,SS)-2-[(benzyloxy)methyl]-3-(2-methoxyphenyl)-3-(1-naphthyl)-2-[(4-pyrimidin-2-ylpiperazin-1-yl)carbonyl]propanenitrile

The title compound was prepared according to Example 208 using (*RR*,*SS*)-2-[(benzyloxy)methyl]-2-cyano-3-(2-methoxyphenyl)-3-(1-naphthyl)propanoic acid and 1-(2-pyrimidyl)-piperazine. The crude product was purified by Biotage (FlasH40i, ethyl acetate/hexane, 2/8) to give a desired product as a solid, which was recrystallized from CH₂Cl₂ and a minimum amount of ether to afford the title compound as a white solid.
mp 217°C; MS (ESI) *m*/*z* 598 ([M+H]⁺); HRMS: calcd for C₃₇H₃₅N₅O₃, 598.28127 ([M+H]⁺); found (ESI_FT), 598.27985

### Example 219

### (RR,SS)-2-(methoxymethyl)-3-(2-methoxyphenyl)-2-{[4-(2-morpholin-4-ylethyl)piperazin-1-yl]carbonyl}-3-(1-naphthyl)propanenitrile

The title compound was prepared according to Example 209 using (*RR*,*SS*)-2-(methoxymethyl)-2-cyano-3-(2-methoxyphenyl)-3-(1-naphthyl)propanoic acid and 1-(2-morpholinoethyl)-piperazine. The crude product was purified by Biotage (FlasH40i, CH2Cl2/MeOH, 9.75/0.25) to give a desired product as a solid, which was recrystallized from methylene chloride and a minimum amount of ether to afford the title compound as a white solid.
mp 219-221 °C; MS (ESI) *m*/*z* 557 ([M+H]⁺); HRMS: calcd for C₃₇H₃₅N₅O₃, 557.31224 ([M+H]⁺); found (ESI_FT), 557.31308

### Example 220

### (RR,SS)-2-[(benzyloxy)methyl]-3-(2-methoxyphenyl)-3-(1-naphthyl)-2-[(4-pyrazin-2-ylpiperazin-1-yl)carbonyl]propanenitrile

The title compound was prepared according to Example 209 using (*RR*,*SS*)-2-[(benzyloxy)methyl]-2-cyano-3-(2-methoxyphenyl)-3-(1-naphthyl)propanoic acid and 1-(2-pyrazinyl)-piperazine. The crude product was purified by Biotage (FlasH40i, ethyl acetate/hexane, 3/7) to give a desired product as a solid, which was recrystallized from CH₂Cl₂ and a minimum amount of hexane to afford the title compound as a white solid.
mp 202 °C; MS (ESI) *m*/*z* 598 ([M+H]⁺); HRMS: calcd for C₃₇H₃₅N₅O₃, 578.28127 ([M+H]⁺); found (ESI_FT), 598.28125

### Example 221

### (2S,3S)-3-(4-cyclohexylpiperazin-1-yl)-2-(methoxymethyl)-2-[(2-methoxyphenyl)(1-naphthyl)methyl]-3-oxopropanenitrile

(*RR*,*SS*)-3-(4-cyclohexylpiperazin-1-yl)-2-(methoxymethyl)-2-[(2-methoxyphenyl)(1-naphthyl)methyl]-3-oxopropanenitrile was prepared according to Example 209 using 1-cyclohexyl-piperazine. The racemate was resolved by a chiral HPLC in the normal phase using hexane/EtOH (40/60) as a mobile phase in AD column at 23°C with (-) rotation of chiral detection (CD) to give the desired product. The product was recrystallized from CH2Cl2 and a minimum amount of hexane to afford the title compound as a white solid.
mp 200 °C; Optical rotation: [α]_{D}^{23.9} = -162/EtOH; MS (ESI) *m*/*z* 526 ([M+H]⁺); Anal. Calcd for C₃₃H₃₉N₃O₃· 0.75 H₂O: C, 73.51; H, 7.57; N, 7.79. Found: C, 73.4; H, 7.91; N, 7.44

### Example 222

### (2R,3R)-3-(4-cyclohexylpiperazin-1-yl)-2-(methoxymethyl)-2-[(2-methoxyphenyl)(1-naphthyl)methyl]-3-oxopropanenitrile

(*RR*,*SS*)-3-(4-cyclohexylpiperazin-1-yl)-2-(methoxymethyl)-2-[(2-methoxyphenyl)(1-naphthyl)methyl]-3-oxopropanenitrile was prepared according to Example 209 using 1-cyclohexyl-piperazine. The racemate was resolved by a chiral HPLC in the normal phase using hexane/EtOH (40/60) as a mobile phase in AD column at 23 °C with (+) rotation of chiral detection (CD) to give the desired product. The product was recrystallized from CH2Cl2 and a minimum amount of hexane to afford the title compound as a white solid.
mp 198°C; Optical rotation: [α]_{D}^{24.0} = +185/EtOH; MS (ESI) *m*/*z* 526 ([M+H]⁺); Anal. Calcd for C₃₃H₃₉N₃O₃: C, 75.4; H, 7.48; N, 7.99. Found: C, 75.1; H, 7.51; N, 7.8

### Example 223

### (2S,3S)-2-(methoxymethyl)-3-(2-methoxyphenyl)-3-(1-naphthyl)-2-[(4-pyrazin-2-ylpiperazin-1-yl)carbonyl]propanenitrile

(*RR*,*SS*)-2-(methoxymethyl)-3-(2-methoxyphenyl)-3-(1-naphthyl)-2-[(4-pyrazin-2-ylpiperazin-1-yl)carbonyl]propanenitrile was prepared according to Example 209 using 1-(2-pyrazinyl)-piperazine. The racemate was resolved by a chiral HPLC in the normal phase using 30% ACN/70% SFC CO₂ as a mobile phase in OD-H 20 x 250 mm column at 30°C with (-) rotation of chiral detection (CD) to give the desired product. The product was recrystallized from CH₂Cl₂ and a minimum amount of hexane to afford the title compound as a white solid.
mp 149 °C; Optical rotation: [α]_{D}^{23.9} = -219/EtOH; MS (ESI) *m*/*z* 522 ([M+H]⁺); Anal. Calcd for C₃₃H₃₉N₃O₃ · 0.5 H₂O: C, 70.17; H, 6.08; N, 13.20. Found: C, 70.19; H, 5.96; N, 13.03

### Example 224

### (2R,3R)-2-(methoxymethyl)-3-(2-methoxyphenyl)-3-(1-naphthyl)-2-[(4-pyrazin-2-ylpiperazin-1-yl)carbonyl]propanenitrile

(*RR*,*SS*)-2-(methoxymethyl)-3-(2-methoxyphenyl)-3-(1-naphthyl)-2-[(4-pyrazin-2-ylpiperazin-1-yl)carbonyl]propanenitrile was prepared according to Example 209 using 1-(2-pyrazinyl)-piperazine. The racemate was resolved by a chiral HPLC in the normal phase using 30% ACN/70% SFC CO₂ as a mobile phase in OD-H 20 x 250 mm column at 30°C with (+) rotation of chiral detection (CD) to give the desired product. The product was recrystallized from CH₂Cl₂ and a minimum amount of hexane to afford the title compound as a white solid.
mp 150°C; Optical rotation: [α]_{D}^{23.9} = +208/EtOH; MS (ESI) *m*/*z* 522 ([M+H]⁺); Anal. Calcd for C₃₃H₃₉N₃O₃ · 0.6 H₂O: C, 69.93; H, 6.10; N, 13.15. Found: C, 69.96; H, 5.76; N, 12.77

### Example 225

### ((2S,3S)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile

(*RR*,*SS*)-3-[4-(1*H-*indol-3-yl)-3,6-dihydropyridin-1(2*H*)-yl]-2-(methoxymethyl)-2-[(2-methoxyphenyl)(1-naphthyl)methyl]-3-oxopropanenitrile was prepared according to Example 209. The racemate was resolved by a chiral HPLC in the normal phase using hexane/CH₂Cl₂/EtOH (60/35/5) as a mobile phase in R,R-DACH-DNB 5/100 25 cm x 4.6 mm column at 23°C with (-) rotation of chiral detection (CD) to give the desired product. The product was recrystallized from CH₂Cl₂ and a minimum amount of hexane to afford the title compound as a pale yellow solid.
mp 171-173 °C; MS (ESI) *m*/*z* 554 ([M-H]⁻); Anal. Calcd for C₃₆H₃₃N₃O₃ · 0.50 C₆H₁₄ · H₂O: C, 75.95; H, 6.86; N, 6.81. Found: C, 75.62; H, 6.59; N, 6.87.

### Example 226

### ((2R,3R)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile

(*RR*,*SS*)-3-[4-(1*H*-indol-3-yl)-3,6-dihydropyridin-1(2*H*)-yl]-2-(methoxymethyl)-2-[(2-methoxyphenyl)(1-naphthyl)methyl]-3-oxopropanenitrile was prepared according to Example 209. The racemate was resolved by a chiral HPLC in the normal phase using hexane/CH₂Cl₂/EtOH (60/35/5) as a mobile phase in R,R-DACH-DNB 5/100 25cm x 4.6 mm column at 23°C with (+) rotation of chiral detection (CD) to give the desired product. The product was recrystallized from CH₂Cl₂ and a minimum amount of hexane to afford the title compound as an off-white solid.
mp 178-180 °C; MS (ESI) *m*/*z* 554 ([M-H]⁻); Anal. Calcd for C₃₆H₃₃N₃O₃ · 0.75 H₂O: C, 75.97; H, 6.11; N, 7.38. Found: C, 75.88; H, 6.45; N, 7.08.

(*RR*,*SS*)-2-cyano-3-(2-methoxyphenyl)-2-methyl-3-(1-naphthyl)propanoic acid (Example 48) may be resolved to give Example 67. The following examples show resolution to 2S, 3S (Example 48) in Examples 227, 228, 229, and 230.

### Example 227 -- Step 1

### (2S, 3S)-2-Cyano-3-(2-methoxyphenyl)-2-methyl-3-(1-naphthyl)propanoic Acid (+)-(1S, 2R)-Ephedrine Salt

(+) Ephedrine hydrochloride (11.4 g, 56.5 mmol) was liberated in a mixture of ether (200ml) and 10% sodium carbonate solution (100 ml). The aqueous phase was extracted with ether (50 ml). The combined ether extracts dried over anhydrous sodium sulfate and filtered directly into a solution of 2-cyano-3-(2-methoxyphenyl)-2-methyl-3-(1-naphthyl) propanoic acid (17.84 g, 51.65 mmol) in ether (150 ml). The mixture was concentrated *in vacuo* to a foam, which was taken up in hot methanol (200 ml), cooled to ambient temperature and diluted with ether (250 ml). The clear solution was seeded with an authentic sample of pure diastereomeric salt and left in a refrigerator overnight.

The clear supernatant was decanted off and the residue rinsed with ether. The crystalline residue was designated AA (8 g, y=30 %, 95% ee). All filtrates were kept and designated as ML A

The crystalline residue AA (8 g, 95% ee) was combined with (+)-ephedrine salt from ML B (4 g). The combined salts were crystallized from hot methanol (150 ml)/ether (200 ml) and after standing overnight gave the title compound as a white solid. (8.9 g, 99% ee). The filtrate was concentrated *in vacuo* and crystallized from methanol (75 ml)/ether (100 ml) to yield an additional amount (1.6 g, 98.8 % ee). The total yield of the (+) ephedrine salt was 10.5 g (36% of theory). The absolute configuration was assigned by single crystal X-ray diffraction.
Mp 196°C (dec); [α]_{D}²⁵= -270.1° (1% DMSO); MS (ESI) m/z 344; Anal. Calcd for C₂₂H₁₉NO₃ . C₁₀H₁₅NO: C, 75.27; H, 6.71; N, 5.49. Found: C, 75.10; H, 6.80; N, 5.39.

Chiral LC analysis were performed on the methyl ester. A sample of the salt was liberated by extraction with ether and 1 N hydrochloric acid. Organic phase was dried over magnesium sulfate, concentrated (under a stream of nitrogen) to a solid. This material was taken up in a small amount of methanol and treated with a few drops of a solution of 2M (trimethylsilyl)-diazomethane in hexane, then evaporated under a stream of nitrogen to give a solid that was submitted as such for chiral LC analysis.

### Example 227 -- Step 2

### (-)-(2S,3S)-2-Cyano-3-(2-methoxyphenyl)-2-methyl-3-(1-naphthyl)propanoic Acid

The (+) ephedrine salt prepared in Example 226 -- Step 1 was liberated to the acid by extraction with ethyl acetate/1N hydrochloric acid. The organic phase was washed with brine, dried over magnesium sulfate and filtered. The filtrate was concentrated *in vacuo* to a foam that was taken up in ether (100 mL) and treated with hexane (100 mL) to give a crystalline precipitate. The crystals were cooled in a refrigerator for 15 hours. Filtration afforded the title compound as a white crystalline solid.(5.46 g , 99.9 % ee, chemical purity:99.2 %)
mp 230-233 °C dec; [α]_{D}²⁵ = -270.0° (c = 1% solution, CHCl₃); MS (ESI) m/z 344;
Anal. Calcd for C₂₂H₁₉NO₃: C, 76.50; H, 5.54; N, 4.06. Found: C, 76.27; H, 5.47; N, 3.95.

Chiral LC analysis was performed on the methyl ester as described above.

### Example 228 -- Step 1

### (2R, 3R)-2-Cyano-3-(2-methoxyphenyl)-2-methyl-3-(1-naphthyl)propanoic Acid (-)-(1R, 2S)-Ephedrine Salt

The ML A was liberated to the acid by ether/1N hydrochloric acid extraction to give the chirally enriched starting acid (11.2g, 32.4 mmol) which was dissolved in methanol (100 ml) and a solution of (-)-(1R, 2S)-ephedrine (5.9 g, 35.7 mmol) in methanol (50 ml) was added. The clear solution was treated with ether (200 ml) and kept in a refrigerator overnight.

The crystals were filtered and washed with ether to give a white solid.(11.6g, 99.8 % ee). The filtrate, designated ML B (6g (-)-ephedrine salt) was converted to the (+)-ephedrine salt and combined with the crystalline residue AA described in Example 227 - Step 1 and used to prepare the *SS* acid.
mp 196 (dec); [α]^{D25} = +258.90° (c = 7.933MG/0.793ML, DMSO); Anal. Calcd for C22H19NO3. C10H15NO: C, 75.27; H, 6.71; N, 5.49. Found: C, 75.06; H, 6.85; N, 5.26

Chiral LC analysis were performed on the methyl ester as described in Example 227 - Step 1.

### Example 228 -- Step 2

### (-)-(2R 3R-2-Cyano-3-(2-methoxyphenyl)-2-methyl-3-(1-naphthyl)propanoic Acid

The *RR* acid was liberated from the salt as described in Example 226 -- Step 2.
mp 232-234 °C

### Example 229 -- Step 1

### (2S, 3S)-2-Cyano-3-(2-methoxyphenyl)-2-methyl-3-(1-naphthyl)propanoic Acid Cinchonidine Salt

2-Cyano-3-(2-methoxyphenyl)-2-methyl-3-(1-naphthyl) propanoic acid (20 g, 58 mmol) was dissolved in hot isopropyl acetate (1 L). To this was added (-) cinchonidine (25g, 84.9 mmol) and additional isopropyl acetate (500 mL). The solution was heated to reflux and yielded a clear solution. The solution was cooled to room temperature overnight. The solid that resulted was filtered, dried and a small sample was converted to the methyl ester for chiral HPLC analysis. The material had an ee of 78%. A second crystallization from isopropyl acetate provided 10.75g of the *SS* acid with an ee of 98.7%.
mp 155-158 °C; [α]_{D}²⁵ = -250.85° (c = 1% SOLUTION, CHCl3); MS (ESI) m/z 344; Anal. Calcd for C₂₂H₁₉NO₃. C₁₉H₂₂N₂O: C, 76.97; H, 6.46; N, 6.57. Found: C, 75.76; H, 6.63; N, 6.27.

The ee was measured by derivatization as the methyl ester as described below 5 mg of the diasteriomeric salt was dissolved in CHCl₂ (5mL). To this is added 20 mg of piperidine resin (Tetrahedron Letters, 40 (1999) 7031). The sample is mixed for five minutes, the resin is allowed to settle and the supernatant is pipetted into a THF/MeOH (4.5:0.5mL) mixture. To this is added a 2M solution of trimethylsilyl diazomethane in hexane until the yellow color is persistent. The sample is the concentrated under a nitrogen stream and is analyzed by chiral HPLC (Chiralcel OD (25x0.46 cm) using 15% H₂O/MeOH (0.01 % DEA) at 0.5mL/minute)

### Example 229 -- Step 2

### (-)-(2S, 3S)-2-Cyano-3-(2-methoxyphenyl)-2-methyl-3-(1-naphthyl)propanoic Acid

The *SS* acid was liberated from the salt as described in Example 227 Step 2.
mp 232-234 °C

### Example 230 -- Step 1

### (2S, 3S)-2-Cyano-3-(2-methoxyphenyl)-2-methyl-3-(1-naphthyl)propanoic Acid Quinidine Salt

2-Cyano-3-(2-methoxyphenyl)-2-methyl-3-(1-naphthyl) propanoic acid (30 g, 87 mmol) was dissolved in tetrohydrofuran (250 mL). This was added to a solution of quinidine (28.18g, 87 mmol) in CH₂Cl₂ (150 mL) and isopropyl acetate (200 mL). The solution was warmed to reflux to yield a clear solution, was cooled and evaporated to a solid *in vacuo.* The salt was refluxed in isopropyl acetate (1.4 L), filtered hot and cooled to room temperature. This provided 19.50 g of the diasteriomeric salt with an ee of 98.5%. A second recrystallization from isopropyl acetate provided 17.5 g of the salt with an ee 99.5%.
mp 171-172 °C; [α]_{D}²⁵ = -11.26° (c = 1% solution, CHCl₃); MS (ESI) m/z 344; Anal. Calcd for C₂₂H₁₉NO₃. C₂₀H₂₄N2O2: C, 75.31; H, 6.47; N, 6.27. Found: C, 75.01; H, 6.58; N, 6.21.

### Example 230 -- Step 2

### (-)-(2S, 3S)-2-Cyano-3-(2-methoxyphenyl)-2-methyl-3-(1-naphthyl)propanoic Acid

The *SS* acid was liberated from the salt as described in Example 227 - Step 2.
mp 232-234 °C.

### Example 231 -- Step 1

### (2S, 3S)-2-Cyano-3-(2-methoxyphenyl)-2-methyl-3-(1-naphthyl)propanoic Acid Cinchonidine Salt

2-Cyano-3-(2-methoxyphenyl)-2-methyl-3-(1-naphthyl) propanoic acid (20 g) was dissolved in isopropyl acetate (1 L). Cinchonidine (25 g) was added and 500 mL isopropyl acetate was added. The solution was warmed to reflux to yield a clear solution. The hot salt solution was filtered and cooled to room temperature. This provided the diasteriomeric salt with an ee of 78%. A second recrystallization from isopropyl acetate provided 10.75 g of the salt with an ee 98.7%
mp 171-172 °C; [α]_{D}²⁵ = -11.26° (c = 1% solution, CHCl₃); ¹H NMR (DMSO-d6): d 02-2644-PNH; MS (ESI) m/z 344.

### Example 231 -- Step 2

### (-)-(2S, 3S)-2-Cyano-3-(2-methoxyphenyl)-2-methyl-3-(1-naphthyl)propanoic Acid

The *SS* acid was liberated from the cinconidine salt as described in Example 227 -- part 2.
mp 232-234 °C

When ranges are used herein for physical properties, such as molecular weight, or chemical properties, such as chemical formulae, all combinations and subcombinations of ranges specific embodiments therein are intended to be included.

The disclosures of each patent, patent application and publication cited or described in this document are hereby incorporated herein by reference, in its entirety.

## Claims

1. Use of a compound of formula I having the structure 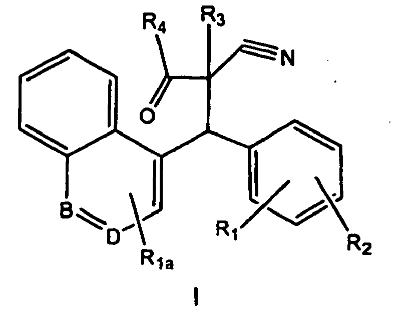 wherein
B and D are independently CH or N, provided that B and D are not both N;
R₁, R₁ₐ, R₂ are each, independently, hydrogen, halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
or R₁ and R₂ together with carbon atoms to which they are attached form a fused benzene ring, the naphthalene ring so formed being optionally substituted by halogen, alkyl of 1-6 carbon atoms, alkoxy of 1-6 carbon atoms, nitro, cyano, thioalkyl of 1-6 carbon atoms, aryl, alkylthio of 1-6 carbon atoms, CF₃, -OCF₃, -NR₅R₆, or hydroxy;
R₃ is hydrogen, alkyl of 1-6 carbon atoms, arylalkyl having 1-6 carbon atoms in the alkyl moiety, alkenyl of 2-7 carbon atoms, cycloalkylmethyl of 3-8 carbon atoms in the cycloalkyl moiety, arylalkoxyalkyl, alkoxyalkyl, dialkylaminoalkyl having 1-6 carbon atoms in the alkyl moieties, or Het-alkyl having 1-6 carbon atoms in the alkyl moiety;
R₄ is-NR₅R₆, -OR₆, 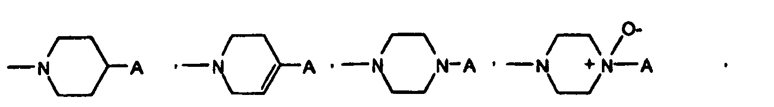 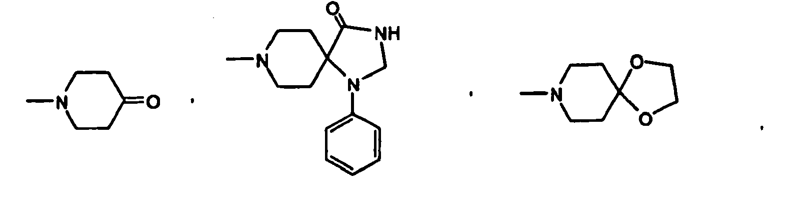 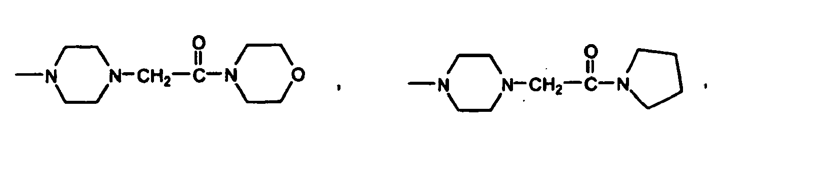 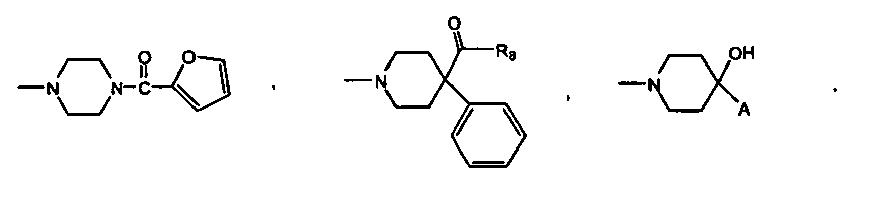 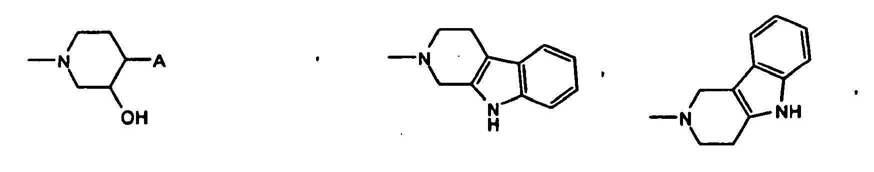 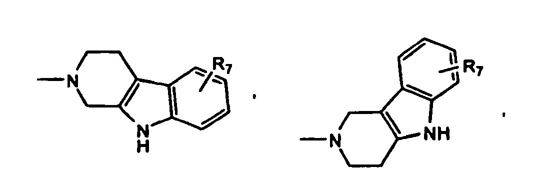
or A, wherein any phenyl ring in R₄ is optionally substituted with R₇;
R₅ and R₆ are each, independently, hydrogen, alkyl of 1-6 carbon atoms, aryl, arylalkyl having 1-6 carbon atoms in the alkyl moiety, Het-alkyl having 1-6 carbon atoms in the alkyl moiety, hydroxyalkyl of 1-6 carbon atoms, dihydroxyalkyl of 1-6 carbon atoms, or cycloalkyl of 3-8 carbon atoms;
R₇ is alkyl of 1-6 carbon atoms, alkoxy of 1-6 carbon atoms, halogen, nitro, cyano, alkylthio of 1-6 carbon atoms, thioalkyl of 1-6 carbon atoms, CF₃, or -OCF₃;
R₈ is alkyl of 1-6 carbon atoms;
A is hydrogen, cycloalkyl of 3-8 carbon atoms, alkoxyalkyl having 1-6 carbon atoms in the alkyl and alkoxy moieties, dialkylaminoalkyl having 1-6 carbon atoms in the alkyl moieties, aryl, Het, hydroxyalkyl of 1-6 carbon atoms, dihydroxyalkyl of 1-6 carbon atoms, Het-alkyl having 1-6 carbon atoms in the alkyl moiety, arylalkyl having 1-6 carbon atoms in the alkyl moiety, or 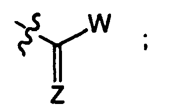
W is aryl, -Y-aryl, or Het or -Y-Het;
Y is -O- or -NH-;
Z is O or S;
Het is a saturated, unsaturated, or partially unsaturated heterocyclic ring or ring system having 4-12 ring atoms and 1-3 heteroatoms selected from N, O, or S, that may be optionally substituted with 1-3 R₇ groups;
aryl is an aromatic ring or ring system having 6-14 carbon atoms in the ring or ring system, that may be optionally substituted with 1-3 R₇ groups;
with the proviso that at least one of the R₁, R₁ₐ, or R₂ groups is not hydrogen;
or a pharmaceutically acceptable salt thereof; in the preparation of a medicament for
treating the inflammatory component of a disease.

2. Use according to claim 1, wherein said disease is selected from the group consisting of atherosclerosis, myocardial infarction, congestive heart failure, inflammatory bowel disease, arthritis, type II diabetes, and autoimmune disease.

3. Use according to claim 2, wherein said autoimmune disease is multiple sclerosis or rheumatoid arthritis..

4. Use according to any one of claims 1 to 3, wherein
R₄ is -NR₅R₆, -OR₆, 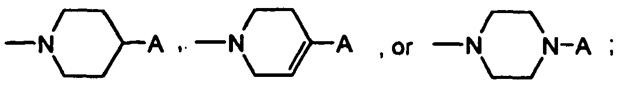 and
A is hydrogen, aryl, or Het; or
a pharmaceutically acceptable salt thereof.

5. Use according to claim 4, wherein
R₄ is 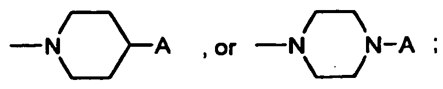 and
A is hydrogen, aryl, or Het; or
a pharmaceutically acceptable salt thereof.

6. Use according to any one of claims 1 to 3, wherein
B and D are CH.

7. Use according to any one of claims 1 to 3, in which the compound of formula I is one of the following:
ethyl 2-cyano-3-(2,6-dimethoxyphenyl)-3-(1-naphthyl)propanoate;
ethyl 2-cyano-3-(2,6-dichlorophenyl)-3-(1-naphthyl)propanoate;
ethyl 2-cyano-3-[4-(dimethylamino)phenyl]-3-(1-naphthyl)propanoate;
ethyl 2-cyano-3-(1-naphthyl)-3-[2 (trifluoromethyl) phenyl]propanoate;
ethyl 2-cyano-3-(2-isopropylphenyl)-3-(1-naphthyl)propanoate;
ethyl 2-cyano-3-(2,4-dimethoxyphenyl)-3-(1-naphthyl)propanoate;
ethyl 2-cyano-3-(2,5-dimethoxyphenyl)-3-(1-naphthyl)propanoate;
*tert*-butyl 2-cyano-3-(1-naphthyl)-3-[2-(trifluoromethyl) phenyl] propanoate;
ethyl 2-cyano-3-(2-methoxyphenyl)-3-(1-naphthyl)propanoate;
ethyl (*RR*,*SS*)-2-cyano-3-(2-methoxyphenyl)-3-(1-naphthyl)propanoate;
*tert*-butyl 2-cyano-3-(2-isopropylphenyl)-3-(1-naphthyl)propanoate;
*tert*-butyl 2-cyano-3-(2-methoxyphenyl)-3-(1-naphthyl)propanoate;
*tert*-butyl 2-cyano-3-(1-naphthyl)-3-[2-(trifluoromethoxy)phenyl] propanoate;
ethyl 2-cyano-3-(1-naphthyl)-3-(2-nitrophenyl)propanoate;
*tert*-butyl 2-cyano-3-(2,6-dimethylphenyl)-3-(1-naphthyl)propanoate;
*tert*-butyl (*RR*,*SS*)-2-cyano-3-(1-naphthyl)-3-[2-(trifluoromethyl)phenyl] propanoate;
*tert*-butyl (*RR*,*SS*)-2-cyano-3-(2-methoxyphenyl)-2-methyl-3-(1-naphthyl)propanoate;
(-) ethyl (*SS*)-2-cyano-3-(2-methoxyphenyl)-2-methyl-3-(1-naphthyl)propanoate;
(+) ethyl (*R*,*R*)-2-cyano-3-(2-methoxyphenyl)-2-methyl-3-(1-naphthyl)propanoate;
ethyl (*RR*,*SS*)-2-cyano-2-[(2-methoxyphenyl)(1-naphthyl)methyl]-4-pentenoate;
ethyl (*RR*,*SS*)-2-benzyl-2-cyano-3-(2-methoxyphenyl)-3-(1-naphthyl) propanoate;
*tert*-butyl (*RR*,*SS*)-2-cyano-3-(2-isopropylphenyl)-2-methyl-3-(1-naphthyl)propanoate;
*tert*-butyl (*RS*,*SR*)-2-cyano-2-methyl-3-(1-naphthyl)-3-[2-(trifluoromethyl)phenyl]propanoate;
*tert*-butyl (*RS*,*SR*)-2-cyano-3-(2,6-dimethylphenyl)-2-methyl-3-(1-naphthyl)propanoate;
*tert*-butyl (*RR*,*SS*)-2-benzyl-2-cyano-3-(2-methoxyphenyl)-3-(1-naphthyl)propanoate;
*tert*-butyl (*RR*, *SS*)-2-(3-chlorobenzyl)-2-cyano-3-(2-methoxyphenyl)-3-(1-naphthyl)propanoate;
*tert*-butyl (*RR*,*SS*)-2-(2-bromobenzyl)-2-cyano-3-(2-methoxyphenyl)-3-(1-naphthyl)propanoate;
*tert*-butyl (*RR*,*SS*)-2-(2-chlorobenzyl)-2-cyano-3-(2-methoxyphenyl)-3-(1-naphthyl)propanoate;
*tert*-butyl (*RR*,*SS*)-2-cyano-2-(2,6-dichlorobenzyl)-3-(2-methoxyphenyl)-3-(1-naphthyl)propanoate;
ethyl (*RR*,*SS*)-2-cyano-3-(2,4-dimethoxyphenyl)-2-methyl-3-(1-naphthyl)propanoate;
*tert*-butyl (*RS*,*SR*)-2-cyano-2-methyl-3-(1-naphthyl)-3-[2-(trifluoromethoxy)phenyl]propanoate;
ethyl 2-cyano-3-(3-methoxyphenyl)-3-(1-naphthyl)propanoate;
ethyl 2-cyano-3-(4-methylphenyl)-3-(1-naphthyl)propanoate;
ethyl 2-cyano-3-(2-methylphenyl)-3-(1-naphthyl)propanoate;
ethyl 2-cyano-3-(1-naphthyl)-3-(2-naphthyl)propanoate;
ethyl 2-cyano-3-(4-fluoro-1-naphthyl)-3-(1-naphthyl)propanoate;
ethyl 2-cyano-3-[4-(methylthio)phenyl]-3-(1-naphthyl)propanoate;
ethyl 3-[1,1'-biphenyl]-4-yl-2-cyano-3-(1-naphthyl)propanoate;
ethyl 3-[1,1'-biphenyl]-2-yl-2-cyano-3-(1-naphthyl)propanoate;
ethyl 3-(4-chlorophenyl)-2-cyano-3-(1-naphthyl)propanoate;
ethyl 2-cyano-3-[2-(methylthio)phenyl]-3-(1-naphthyl)propanoate;
ethyl-(*RR*,*SS*)- 2-cyano-3-(2-methoxyphenyl)-2-methyl-3-(1-naphthyl)propanoate;
ethyl (*RR*,*SS*)-2-cyano-2-methyl-3-[2-(methylthio)phenyl]-3-(1-naphthyl)propanoate;
(*RR*,*SS*)-2-cyano-3-(2-methoxyphenyl)-2-methyl-3-(1-naphthyl)propanoic acid;
(*RR*,*SS*)-2-cyano-3-(2-isopropylphenyl)-2-methyl-3-(1-naphthyl)propanoic acid;
(*RS*,*SR*)-2-cyano-2-methyl-3-(1-naphthyl)-3-[2-(trifluoromethyl)phenyl]propanoic acid;
(*RR*,*SS*)-2-benzyl-2-cyano-3-(2-methoxyphenyl)-3-(1-naphthyl)propanoic acid;
*tert*-butyl (*R*,*R*)-2-cyano-3-(2-isopropylphenyl)-2-methyl-3-(1-naphthyl)propanoate;
*tert*-butyl (*S*,*S*)-2-cyano-3-(2-isopropylphenyl)-2-methyl-3-(1-naphthyl)propanoate;
*tert*-butyl (*S*,*S*)-2-cyano-3-(2-methoxyphenyl)-2-methyl-3-(1-naphthyl)propanoate;
*tert*-butyl (*R*,*R*)-2-cyano-3-(2-methoxyphenyl)-2-methyl-3-(1-naphthyl)propanoate;
(*RR*,*SS*)-3-(2-methoxyphenyl)-2-methyl-3-(1-naphthyl)-2-(piperazin-1-ylcarbonyl)propanenitrile;
*RR,SS*)(3-[4-(3-chloro-2-methylphenyl)piperazin-1-yl]-2-[(2-methoxyphenyl)(1-naphthyl)methyl]-2-methyl-3-oxopropanenitrile);
(*RR*,*S*,*S*)-3-(2-methoxyphenyl)-2-methyl-2-{[4-(2-methylphenyl)piperazin-1-yl]carbonyl}-3-(1-naphthyl)propanenitrile;
(*SS*)-3-(2-methoxyphenyl)-2-methyl-2-{[4-(2-methylphenyl)-1-piperazinyl]carbonyl}-3-(1-naphthyl)propanenitrile;
(*R*,*R*)-3-(2-methoxyphenyl)-2-methyl-2-{[4-(2-methylphenyl)piperazin-1-yl]carbonyl}-3-(1-naphthyl)propanenitrile;
(*S*,*S*)-3-(2-methoxyphenyl)-2-methyl-2-{[4-(3-methylphenyl)piperazin-1-yl]carbonyl}-3-(1-naphthyl)propanenitrile;
(*S*)-3-[4-(3,5-dimethoxyphenyl)piperazin-1-yl]-2-[(*S*)-(2-methoxyphenyl)(1-naphthyl)methyl]-2-methyl-3-oxopropanenitrile;
(*S*)3-(4-indan-4-yl-piperazin-1-yl)-2-[(*S*)(2-methoxy-phenyl)-naphthalen-1-yl-methyl]-2-methyl-3-oxo-propionitrile;
(*S*,*S*)-3-(2-methoxyphenyl)-2-methyl-3-(1-naphthyl)-2-{[4-(1-naphthyl)piperazin-1-yl]carbonyl}propanenitrile;
(*S*)-3-[4-(3,4-dimethylphenyl)piperazin-1-yl]-2-[(*S*)-(2-methoxyphenyl)(1-naphthyl)methyl]-2-methyl-3-oxopropanenitrile;
(*RR*,*SS*) 3-[4-(*H*-indol-4-yl)piperazin-1-yl]-2-[(2-methoxyphenyl)(1-naphthyl)methyl]-2-methyl-3-oxopropanenitrile;
(*S*)-3-[4-(3-chlorophenyl)piperazin-1-yl]-2-[(*S*)-(2-methoxyphenyl)(1-naphthyl)methyl]-2-methyl-3-oxopropanenitrile;
(*S*)-3-[4-(2,3-dimethylphenyl)piperazin-1-yl]-2-[(*S*)-(2-methoxyphenyl)(1-naphthyl)methyl]-2-methyl-3-oxopropanenitrile;
(*S*)-3-[4-(4-chlorophenyl)piperazin-1-yl]-2-[(*S*)-(2-methoxyphenyl)(1-naphthyl)methyl]-2-methyl-3-oxopropanenitrile;
(*S*)-3-[4-(1*H*-Indol-4-yl)-piperazin-1-yl]-2-[(*S*)-(2-methoxy-phenyl)-naphthalen-1-yl-methyl]-2-methyl-3-oxo-propionitrile;
(*S*,*S*)-3-(2-methoxyphenyl)-2-methyl-3-(1-naphthyl)-2-({4-[3-(trifluoromethyl)phenyl] piperidin-1-yl}carbonyl)propanenitrile;
(*S*)-3-[4-(4-chloro-phenyl)-3,6-dihydro-2*H*-pyridin-1-yl]-2-[(*S*)-(2-methoxy-phenyl)-naphthalen-1-yl-methyl]-2-methyl-3-oxo-propionitrile;
(*S*,*S*)-3-(2-methoxy-phenyl)-2-methyl-3-naphthalen-1-yl-2-[4-(3-trifluoromethyl-phenyl)-3,6-dihydro-2*H*-pyridine-1-carbonyl]-propionitrile;
(*SS*)2-[4-(4-chloro-phenyl)-piperidine-1-carbonyl]-3-(2-methoxyphenyl)-2-methyl-3-naphthalen-1-yl-propionitrile;
(*RR*,*SS*)-3-(2-methoxyphenyl)-2-methyl-3-(1-naphthyl)-2-({4-[3-(trifluoromethyl)phenyl]piperidin-1-yl}carbonyl)propanenitrile;
(*RR*,*SS*)-3-(2-methoxyphenyl)-2-methyl-3-(1-naphthyl)-2-(piperidin-1-ylcarbonyl)propanenitrile;
(*RR*,*SS*)-2-cyano-3-(2-methoxyphenyl)-2-methyl-3-(1-naphthyl)propanamide;
(*RR*,*SS*)-2-cyano-N-ethyl-3-(2-methoxyphenyl)-2-methyl-3-(1-naphthyl)propanamide;
(*RR*,*SS*)-N-(*tert*-butyl)-2-cyano-3-(2-methoxyphenyl)-2-methyl-3-(1-naphthyl)propanamide;
(*RR*,*SS*)-2-cyano-3-(2-methoxyphenyl)-N,N,2-trimethyl-3-(1-naphthyl)propanamide;
(*RR,SS*)-2-cyano-N-methoxy-3-(2-methoxyphenyl)-N,2-dimethyl-3-(1-naphthyl)propanamide;
(*RR*,*SS*)-2-benzyl-3-[4-(3,5-dichloro-4-pyridinyl)-1-piperazinyl]-2-[(2-methoxyphenyl)(1-naphthyl)methyl]-3-oxopropanenitrile;
(*R*,*S*)-3-[4-(2,3-dimethylphenyl)piperazin-1-yl]-2-[(*R*,*S*)-(2-methoxyphenyl)(1-naphthyl)methyl]-2-methyl-3-oxopropanenitrile;
(*R*,*S*)-3-[4-(3-isopropylphenyl)piperazin-1-yl]-2-[(*R*,*S*)-(2-methoxyphenyl)(1-naphthyl)methyl]-2-methyl-3-oxopropanenitrile;
(*RR*,*SS*)-3-[4-(3,5-dichloropyridin-4-yl)piperazin-1-yl]-2-[(2-methoxyphenyl)(1-naphthyl)methyl]-2-methyl-3-oxopropanenitrile;
(2*SS*)-3-[4-(3-chloro-2-methylphenyl)piperazin-1-yl]-2-[(2-methoxyphenyl)(1-naphthyl)methyl]-2-methyl-3-oxopropanenitrile;
(*S*)-3-[4-(2-fluorophenyl)piperazin-1-yl]-2-[(*S*)-(2-methoxyphenyl)(1-naphthyl)methyl]-2-methyl-3-oxopropanenitrile;
(*S*)-3-[4-(2-chlorophenyl)piperazin-1-yl]-2-[(*S*)-(2-methoxyphenyl)(1-naphthyl)methyl]-2-methyl-3-oxopropanenitrile;
(*S*,*S*)-3-(2-methoxyphenyl)-2-([4-(3-methoxyphenyl)piperazin-1-yl]carbonyl}-2-methyl-3-(1-naphthyl)propanenitrile;
(*R*,*S*)-3-[4-(3-chloropyridin-4-yl)piperazin-1-yl]-2-[(*R*,*S*)-(2-methoxyphenyl)(1-naphthyl)methyl]-2-methyl-3-oxopropanenitrile;
(*S*)-3-[4-(2,3-dichlorophenyl)piperazin-1-yl]-2-[(*S*)-(2-methoxyphenyl)(1-naphthyl)methyl]-2-methyl-3-oxopropanenitrile;
(2*S*)-3-[4-[4-chloro-3-(trifluoromethyl)phenyl]-3,6-dihydropyridin-1(2*H*)-yl]-2-[(*S*)-(2-methoxyphenyl)(1-naphthyl)methyl]-2-methyl-3-oxopropanenitrile;
(2*S*)-3-{4-[4-chloro-3-(trifluoromethyl)phenyl]piperidin-1-yl}-2-[(*S*)-(2-methoxyphenyl)(1-naphthyl)methyl]-2-methyl-3-oxopropanenitrile;
(*RR*/*SS*)-3-(2-methoxy-phenyl)-2-methyl-3-naphthalen-1-yl-2-(4-oxy-4-o-tolyl-piperazine-1-carbonyl)-propionitrile;
(*RR*,*SS*)-3-(2-methoxyphenyl)-2-methyl-3-(1-naphthyl)-2-({4-[3-(trifluoromethoxy)phenyl]piperazin-1-yl}carbonyl)propanenitrile;
(*R*,*S*)-3-[4-(2,3-difluorophenyl)piperazin-1-yl]-2-[(*R*,*S*)-(2-methoxyphenyl)(1-naphthyl)methyl]-2-methyl-3-oxopropanenitrile;
(*R*,*S*)-3-[4-(3-fluorophenyl)piperazin-1-yl]-2-[(*R*,*S*)-(2-methoxyphenyl)(1-naphthyl)methyl]-2-methyl-3-oxopropanenitrile;
(*RR*,*SS*)-3-(2-methoxyphenyl)-2-methyl-3-(1-naphthyl)-2-[(4-pyridin-3-ylpiperazin-1-yl)carbonyl]propanenitrile;
(*RR*,*SS*)-3-[4-(2,3-dichlorophenyl)piperazin-1-yl]-2-[(2-methoxyphenyl)(1-naphthyl)methyl]-2-methyl-3-oxopropanenitrile;
(*RR*,*SS*)-3-(2-methoxyphenyl)-2-methyl-2-{[4-(3-methylphenyl)piperazin-1-yl]carbonyl}-3-(1-naphthyl)propanenitrile;
(*RR*,*SS*)-3-(2-methoxyphenyl)-2-methyl-3-(1-naphthyl)-2-(pyrrolidin-1-ylcarbonyl)propanenitrile;
(*RR*,*SS*)-3-(2-methoxyphenyl)-2-methyl-2-(morpholin-4-ylcarbonyl)-3-(1-naphthyl)propanenitrile;
(*RR*,*SS*)-3-[4-(2-hydroxyethyl)piperazin-1-yl]-2-[(2-methoxyphenyl)(1-naphthyl)methyl]-2-methyl-3-oxopropanenitrile;
(*RR*,*SS*)-3-(2,6-dimethylmorpholin-4-yl)-2-[(2-methoxyphenyl)(1-naphthyl)methyl]-2-methyl-3-oxopropanenitrile;
(*RR*,*SS*)-2-cyano-N,N-diethyl-3-(2-methoxyphenyl)-2-methyl-3-(1-naphthyl)propanamide;
(*RR*,*SS*)-2-cyano-N-[2-hydroxy-1-(hydroxymethyl)ethyl]-3-(2-methoxyphenyl)-2-methyl-3-(1-naphthyl)propanamide;
(*RR*,*SS*)-3-azetidin-1-yl-2-[(2-methoxyphenyl)(1-naphthyl)methyl]-2-methyl-3-oxopropanenitrile;
(*RR*,*SS*)-2-cyano-N,N-diisopropyl-3-(2-methoxyphenyl)-2-methyl-3-(1-naphthyl)propanamide;
(*RR*,*SS*)-3-(2-methoxyphenyl)-2-methyl-3-(1-naphthyl)-2-[(3,3,5-trimethylazepan-1-yl)carbonyl]propanenitrile;
(*RR*,*SS*)-3-(2,3-dihydro-*H*-indol-1-yl)-2-[(2-methoxyphenyl)(1-naphthyl)methyl]-2-methyl-3-oxopropanenitrile;
(*RR*,*SS*)-3-(2-methoxyphenyl)-2-methyl-3-(1-naphthyl)-2-(thiomorpholin-4-ylcarbonyl)propanenitrile;
(*RR*,*SS*)-3-azepan-1-yl-2-[(2-methoxyphenyl)(1-naphthyl)methyl]-2-methyl-3-oxopropanenitrile;
(*RR*,*SS*)-2-cyano-N-cyclohexyl-3-(2-methoxyphenyl)-N,2-dimethyl-3-(1-naphthyl)propanamide;
(*RR*,*SS*)-2-cyano-3-(2-methoxyphenyl)-N,2-dimethyl-3-(1-naphthyl)propanamide;
(*RR*,*SS*)-3-(4-benzylpiperazin-1-yl)-2-[(2-methoxyphenyl)(1-naphthyl)methyl]-2-methyl-3-oxopropanenitrile;
(*RR*,*SS*)-3-(2-methoxyphenyl)-2-methyl-2-{[4-(4-methylphenyl)piperazin-1-yl]carbonyl}-3-(1-naphthyl)propanenitrile;
(*RR*,*SS*)-N,N-dibenzyl-2-cyano-3-(2-methoxyphenyl)-2-methyl-3-(1-naphthyl)propanamide;
(*RR*,*SS*)-3-azocan-1-yl-2-[(2-methoxyphenyl)(1-naphthyl)methyl]-2-methyl-3-oxopropanenitrile;
4-chlorophenyl 4-[(*RR*,*SS*)-2-cyano-3-(2-methoxyphenyl)-2-methyl-3-(1-naphthyl)propanoyl]piperazine-1-carboxylate;
2-nitrophenyl 4-[(*RR*,*SS*)-2-cyano-3-(2-methoxyphenyl)-2-methyl-3-(1-naphthyl)propanoyl]piperazine-1-carboxylate;
4-(methoxycarbonyl)phenyl 4-[(*RR*,*SS*)-2-cyano-3-(2-methoxyphenyl)-2-methyl-3-(1-naphthyl)propanoyl]piperazine-1-carboxylate;
4-methylphenyl 4-[(*RR*,*SS*)-2-cyano-3-(2-methoxyphenyl)-2-methyl-3-(1-naphthyl)propanoyl]piperazine-1-carboxylate;
4-[(*RR*,*SS*)-2-cyano-3-(2-methoxyphenyl)-2-methyl-3-(1-naphthyl)propanoyl]-N-(2-methylphenyl)piperazine-1-carboxamide;
4-[(*RR*,*SS*)-2-cyano-3-(2-methoxyphenyl)-2-methyl-3-(1-naphthyl)propanoyl]-N-[2-(trifluoromethyl)phenyl]piperazine-1-carboxamide;
4-[(*RR*,*SS*)-2-cyano-3-(2-methoxyphenyl)-2-methyl-3-(1-naphthyl)propanoyl]-N-(3-methoxyphenyl)piperazine-1-carboxamide;
4-[(*RR*,*SS*)-2-cyano-3-(2-methoxyphenyl)-2-methyl-3-(1-naphthyl)propanoyl]-N-(4-ethoxyphenyl)piperazine-1-carboxamide;
N-(2-bromophenyl)-4-[(*RR*,*SS*)-2-cyano-3-(2-methoxyphenyl)-2-methyl-3-(1-naphthyl)propanoyl]piperazine-1-carboxamide;
4-[(*RR*,*SS*)-2-cyano-3-(2-methoxyphenyl)-2-methyl-3-(1-naphthyl)propanoyl]-N-(4-methylphenyl)piperazine-1-carboxamide;
4-fluorophenyl 4-[(*RR*,*SS*)-2-cyano-3-(2-methoxyphenyl)-2-methyl-3-(1-naphthyl)propanoyl]piperazine-1-carboxylate;
phenyl 4-[(*RR*,*SS*)-2-cyano-3-(2-methoxyphenyl)-2-methyl-3-(1-naphthyl)propanoyl]piperazine-1-carboxylate;
(*RR*, *SS*)-3-[4-(4-bromobenzoyl)piperazin-1-yl]-2-[(2-methoxyphenyl)(1-naphthyl)methyl]-2-methyl-3-oxopropanenitrile;
N-(4-chlorophenyl)-4-[(*RR*,*SS*)-2-cyano-3-(2-methoxyphenyl)-2-methyl-3-(1-naphthyl)propanoyl]piperazine-1-carboxamide;
or
a pharmaceutically acceptable salt thereof.

8. A pharmaceutical composition, comprising:
a pharmaceutical carrier; and
a compound of formula I as defined in any one of claims 1 to 7 or a pharmaceutically acceptable salt thereof.

9. A compound of formula I as defined in claim 1 wherein R₆ is hydrogen, aryl, arylalkyl having 1-6 carbon atoms in the alkyl moiety, Het-alkyl having 1-6 carbon atoms in the alkyl moiety, hydroxyalkyl of 1-6 carbon atoms, dihydroxyalkyl of 1-6 carbon atoms, or cycloalkyl of 3-8 carbon atoms; or a pharmaceutically acceptable salt thereof.

10. A compound of formula I as claimed in claim 9 wherein
R₄ is -NR₅R₆, -OR₆, 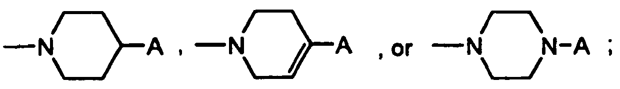 and
A is hydrogen, aryl, or Het; or
a pharmaceutically acceptable salt thereof.

11. A compound of formula I as claimed in claim 10 wherein
R₄ is 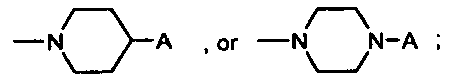 and
A is hydrogen, aryl, or Het; or
a pharmaceutically acceptable salt thereof.

12. A compound of formula I as claimed in any one of claims 9 to 11 wherein B and D are CH.

13. A compound of formula I as defined in claim 7 or a pharmaceutically acceptable salt thereof, excepting
ethyl 2-cyano-3-(4-methylphenyl)-3-(1-naphthyl)propanoate;
or
ethyl 2-cyano-3-(1-naphthyl)-3-(2-naphthyl)propanoate.

14. A compound selected from group consisting of:
methyl-(*SS*,*RR*)-2-cyano-3-(2-methoxyphenyl)-2-methyl-3-(quinolin-3-yl)propanoate;
3-(2-methoxyphenyl)-2-methyl-3-quinolin-3-yl-2-({4-[3-(trifluoromethyl)phenyl] piperidin-1-yl}carbonyl)propanenitrile;
methyl (2*S**,3*R**)-2-cyano-2-(methoxymethyl)-3-(2-methoxyphenyl)-3-quinolin-3-ylpropanoate; and
pharmaceutically acceptable salts thereof.

15. A process for preparing a compound of formula I as claimed in claim 9 which comprises steps (a)-(d) or (e), (f):
(a) reacting a compound of formula 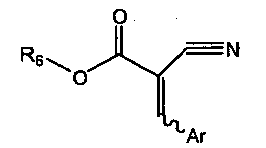 wherein R₆ is as defined in claim 9 excepting hydrogen and Ar is a group of formula (A), (B), or (C): 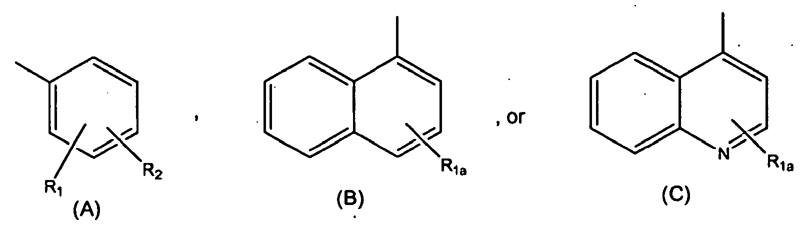 where R₁, R₂ and R₁ₐ are as defined in claim 9;
with a compound of formula Ar₁MX wherein Ar₁ is a group of formula (A) or (B) providing Ar and Ar¹ are not both of formula (A) or (B) to give a compound of formula I wherein R₄ is OR₆ where R₆ is defined in claim 9 excepting hydrogen and R₃ is hydrogen;
(b) alkylating a compound of formula: 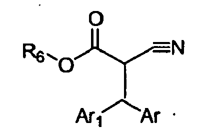 wherein R₆, Ar and Ar₁ are as defined above providing R₆ is other than hydrogen, with an alkylating agent of formula R₃L where L is a leaving group and R₃ is as defined in claim 9 excepting hydrogen to give a corresponding compound of formula I as defined in claim 9;
(c) hydrolyzing an ester of formula 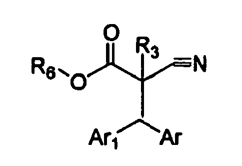 wherein Ar, Ar₁, R₃ and R₆ are as defined hereinabove providing R₆ is other than hydrogen, to give a corresponding compound of formula I wherein R₄ is OR₆ where R₆ is hydrogen;
(d) reacting an activated acid compound of formula: 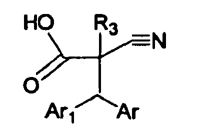 wherein Ar, Ar₁, and R₃ are as defined hereinabove, with an amine of formula HNR₅R₆, 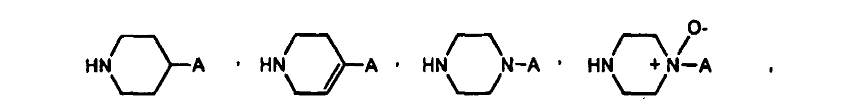 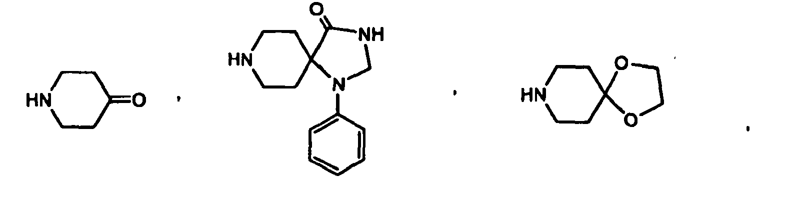 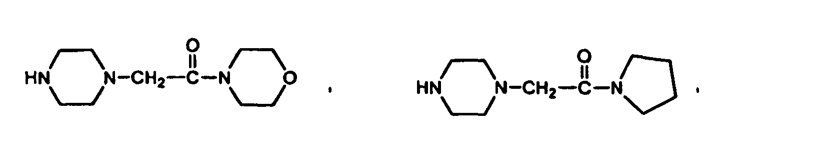 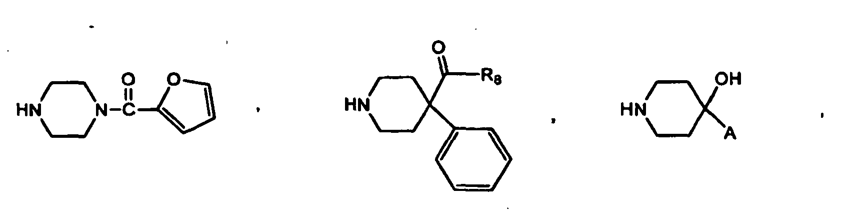 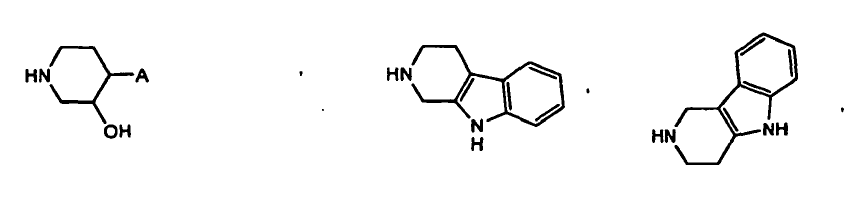 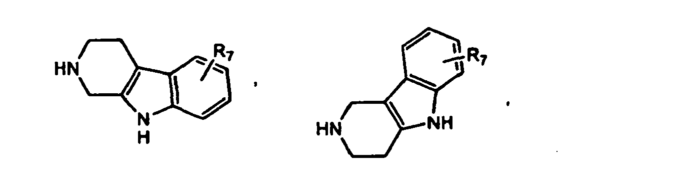 or
(e) reacting a compound of formula 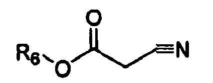 wherein R₆ is as defined hereinabove excepting hydrogen, in the presence of strong base with an halide of formula
Ar₁ArCHX
where Ar and Ar₁ are as defined herein and X is halogen to give a corresponding compound of formula I wherein R₃ is hydrogen, or
(f) converting a compound of formula I as defined in claim 1 having a reactive substituent group or site to give a different compound of formula I;
or
(g) converting a compound of formula I to a pharmaceutically acceptable salt thereof.

16. A process, comprising the steps of:
forming a ephedrine, cinchonidine, or quinidine salt of a compound of claim 9 or 14 or a pharmaceutically acceptable salt thereof; and
extracting said ephedrine, cinchonidine, or quinidine salt with solvent;
to form a substantially pure enantiomer of a compound of claim 9 or 14.

17. A compound as claimed in claim 9 selected from:
2-cyano-3,3-di(1-naphthyl)propanamide;
(RR,SS)-3-(3,4-dihydroisoquinolin-2(1H)-yl)-2-[(2-methoxyphenyl)(1-naphthyl)methyl]-2-methyl-3-oxopropanenitrile;
(2S)-3-(4-(3-bromophenyl)piperazin-1-yl]-2-[(S)-(2-methoxyphenyl)(1-naphthyl)methyl]-2-methyl-3-oxopropanenitrile;
(2S,3S)-3-(2-methoxyphenyl)-2-methyl-3-(1-naphthyl)-2-{[4-(2-oxo-2,3 dihydro-1H-indol-1-yl)piperidin-1-yl]carbonyl}propanenitrile;
(2S,3S)-3-(2-methoxyphenyl)-2-methyl-3-(1-naphthyl)-2-[(4-pyridin-4-ylpiperazin-1-yl)carbonyl]propanenitrile;
(2S,3S)-3-(2-methoxyphenyl)-2-methyl-3-(1-naphthyl)-2-{[4-(2-oxo-3,4-dihydroquinolin-1(2H)-yl)piperidin-1-yl]carbonyl}propanenitrile;
(2S)-3-[4-(4-bromophenyl)-3,6-dihydropyridin-1(2H)-yl]-2-[(S)-(2-methoxyphenyl)(1-naphthyl)methyl]-2-methyl-3-oxopropanenitrile;
(2S,3S)-3-(2-methoxyphenyl)-2-methyl-3-(1-naphthyl)-2-({4-[3-(trifluoromethyl) phenyl] piperazin-1-yl}carbonyl)propanenitrile
(2S)-3-[4-(4-fluorophenyl)-3,6-dihydropyridin-1(2H)-yl]-2-[(S)-(2-methoxyphenyl)(1-naphthyl)methyl]-2-methyl-3-oxopropanenitrile;
(2S,3S)-3-(2-methoxyphenyl)-2-{[4-(2-methoxyphenyl)piperidin-1-yl]carbonyl}-2-methyl-3-(1-naphthyl)propanenitrile;
(2S,3S)-3-(2-methoxyphenyl)-2-methyl-3-(1-naphthyl)-2-({4-[3-(trifluoromethyl) cyclohexyl]piperidin-1-yl}carbonyl)propanenitrile;
(2S)-3-{4-hydroxy-4-[3-(trifluoromethyl)phenyl]piperidin-1-yl}-2-[(S)-(2-methoxyphenyl) (1-naphthyl)methyl]-2-methyl-3-oxopropanenitrile;
2-cyano-3-(2-methoxyphenyl)-2-methyl-3-quinolin-4-ylpropanoic acid;
3-(2-methoxyphenyl)-2-methyl-3-quinolin-4-yl-2-({4-[3-(trifluoromethyl)phenyl]piperidin-1-yl}carbonyl)propanenitrile;
(RR,SS)-2-[(2-methoxyethoxy)methyl]-3-(2-methoxyphenyl)-3-(1-naphthyl)-2-[(4-pyrimidin-2-ylpiperazin-1-yl)carbonyl]propanenitrile;
(RR,SS)-2-[(2-Methoxyethoxy)methyl]-3-(2-methoxyphenyl)-3-(1-naphthyl)-2-({4-[3-(trifluoromethyl)phenyl]piperidin-1-yl}carbonyl)propanenitrile;
(RR,SS)-N-(tert-butyl)-2-cyano-2-[(2-methoxyethoxy)methyl]-3-(2-methoxyphenyl)-3-(1-naphthyl)propanamide;
(RR,SS)-2-cyano-N-(2,2-di methylpropyl)-2-[(2-methoxyethoxy)methyl]-3-(2-methoxyphenyl)-3-(1-naphthyl)propanamide;
(RR,SS)-2-[(2-methoxyethoxy)methyl]-3-(2-methoxyphenyl)-3-(1-naphthyl)-2-[(4-pyrazin-2-ylpiperazin-1-yl)carbonyl]propanenitrile;
(RR,SS)-3-[4-(1H-indol-3-yl)-3,6-dihydropyridin-1(2H)-yl]-2-[(2-methoxyethoxy)methyl]-2-[(S*)-(2-methoxyphenyl)(1-naphthyl)methyl]-3-oxopropanenitrile;
(RR,SS)-3-(2-methoxyphenyl)-2-(1-naphthyl)-2-(pyridin-3-ylmethyl)-2-[(4-pyrimidin-2-ylpiperazin-1-yl)carbonyl]propanenitrile;
(RR,SS)-N-(tert-butyl)-2-cyano-3-(2-methoxyphenyl)-3-(1-naphthyl)-2-(pyridin-3-ylmethyl)propanamide;
(RR,SS)-2-cyano-N-(2,2-dimethylpropyl)-3-(2-methoxyphenyl)-3-(1-naphthyl)-2-(pyridin-3-ylmethyl)propanamide;
(RR,SS)-3-(2-methoxyphenyl)-3-(1-naphthyl)-2-[(4-pyrazin-2-ylpiperazin-1-yl)carbonyl]-2-(pyridin-3-ylmethyl)propanenitrile;
(RR,SS)-3-[4-(1H-indol-3-yl)-3,6-dihydropyridin-1(2H)-yl]-2-[(2-methoxyphenyl)(1-naphthyl)methyl]-3-oxo-2-(pyridin-3-ylmethyl)propanenitrile;
(RR,SS)-N-(tert-butyl)-2-cyano-2-(methoxymethyl)-3-(2-methoxyphenyl)-3-(1-naphthyl)propanamide;
(2S*,3S*)-2-cyano-N-(2,2-dimethylpropyl)-2-(methoxymethyl)-3-(2-methoxyphenyl)-3-(1-naphthyl)propanamide;
(2S)-3-[4-(1H-indol-3-yl)-3,6-dihydropyridin-1(2H)-yl]-2-[(S)-(2-methoxyphenyl)(1-naphthyl)methyl]-2-methyl-3-oxopropanenitrile;
(2S,3S)-3-(2-methoxyphenyl)-2-methyl-3-(1-naphthyl)-2-{[4-(2-oxo-2,3-dihydro-1H-benzimidazol-1-yl)piperidin-1-yl]carbonyl}propanenitrile;
(2S,3S)-3-(2-methoxyphenyl)-2-methyl-3-(1-naphthyl)-2-[(4-oxo-1-phenyl-1,3,8-triazaspiro[4.5]dec-8-yl)carbonyl]propanenitrile;
(2S)-3-{3-hydroxy-4-[3-(trifluoromethyl)phenyl]piperidin-1-yl}-2-[(S)-(2-methoxyphehyl)(1-naphthyl)methyl]-2-methyl-3-oxopropanenitrile;
(2S)-3-[4-(4-chlorophenyl)-4-hydroxypiperidin-1-yl]-2-[(S)-(2-methoxyphenyl)(1-naphthyl)methyl]-2-methyl-3-oxopropanenitrile;
(2S)-3-[4-(1H-indol-3-yl)piperidin-1-yl]-2-[(S)-(2-methoxyphenyl)(1-naphthyl)methyl]-2-methyl-3-oxopropanenitrile;
(2S)-3-(1,4-dioxa-8-azaspiro[4.5]dec-8-yl)-2-[(S)-(2-methoxyphenyl)(1-naphthyl)methyl- 2-methyl-3-oxopropanenitrile;
(2S)-3-(8-fluoro-1,3,4,5-tetrahydro-2H-pyrido[4,3-b]indol-2-yl)-2-[(S)-(2-methoxyphenyl)(1-naphthyl)methyl]-2-methyl-3-oxopropanenitrile;
(2S,3S)-3-(2-methoxyphenyl)-2-methyl-3-(1-naphthyl)-2-(1,3,4,9-tetrahydro-2H-beta-carbolin-2-ylcarbonyl)propanenitrile;
(2S,3S)-3-(2-methoxyphenyl)-2-methyl-3-(1-naphthyl)-2-[(4-oxopiperidin-1-yl)carbonyl] propanenitrile;
(2S,3S)-3-(2-methoxyphenyl)-2-methyl-3-(1-naphthyl)-2-[(4-pyrimidin-2-ylpiperazin-1-yl)carbonyl]propanenitrile;
(2S)-3-[4-(4-bromophenyl)-4-hydroxypiperidin-1-yl]-2-[(S)-(2-methoxyphenyl)(1-naphthyl)methyl]-2-methyl-3-oxopropanenitrile;
(2S,3S)-3-(2-methoxyphenyl)-2-methyl-3-(1-naphthyl)-2-[(4-phenyl-4-propionylpiperidin-1-yl)carbonyl]propanenitrile;
(2S)-3-(4-acetyl-4-phenylpiperidin-1-yl)-2-[(S)-(2-methoxyphenyl)(1-naphthyl)methyl]-2-methyl-3-oxopropanenitrile;
(2S)-3-(4-cyclohexylpiperazin-1-yl)-2-[(S)-(2-methoxyphenyl)(1-naphthyl)methyl]-2-methyl-3-oxopropanenitrile;
(2S)-3-[4-(2-methoxyethyl)piperazin-1-yl]-2-[(S)-(2-methoxyphenyl)(1-naphthyl)methyl]-2-methyl-3-oxopropanenitrile;
(2S,3S)-3-(2-methoxyphenyl)-2-methyl-2-{[4-(2-morpholin-4-yl-2-oxoethyl)piperazin-1-yl]carbonyl}-3-(1-naphthyl)propanenitrile;
(2S)-3-[4-(2-furoyl)piperazin-1-yl]-2-[(S)-(2-methoxyphenyl)(1-naphthyl)methyl]-2-methyl-3-oxopropanenitrile;
(RR,SS)-2-(methoxymethyl)-3-(2-methoxyphenyl)-3-(1-naphthyl)-2-[(4-pyrimidin-2-ylpiperazin-1-yl)carbonyl]propanenitrile;
(2S,3S)-3-(2-methoxyphenyl)-2-methyl-3-(1-naphthyl)-2-{[4-(2-oxo-2-pyrrolidin-1-ylethyl)piperazin-1-yl]carbonyl}propanenitrile;
(2S)-3-{4-[3-(dimethylamino)propyl]piperazin-1-yl}-2-[(S)-(2-methoxyphenyl)(1-naphthyl)methyl]-2-methyl-3-oxopropanenitrile;
(2S,3S)-3-(2-methoxyphenyl)-2-methyl-2-[(4-morpholin-4-ylpiperidin-1-yl)carbonyl]-3-(1-naphthyl)propanenitrile;
3-{4-[3-(dimethylamino)propyl]piperazin-1-yl}-2-(methoxymethyl)-2-[(2-methoxyphenyl)(1-naphthyl)methyl]-3-oxopropanenitrile;
(2S*)-2-[(benzyloxy)methyl]-3-{4-[3-(dimethylamino)propyl]piperazin-1-yl}-2-[(R*)-(2-methoxyphenyl)(1-naphthyl)methyl]-3-oxopropanenitrile;
3-[4-(2-methoxyethyl)piperazin-1-yl]-2-(methoxymethyl)-2-[(2-methoxyphenyl)(1-naphthyl)methyl]-3-oxopropanenitrile;
2-[(benzyloxy)methyl]-3-[4-(2-methoxyethyl)piperazin-1-yl]-2-[(2-methoxyphenyl)(1-naphthyl)methyl]-3-oxopropanenitrile;
2-[(benzyloxy)methyl]-3-(2-methoxyphenyl)-2-{[4-(2-morpholin-4-ylethyl)piperazin-1-yl]carbonyl}-3-(-naphthyl)propanenitrile;
(RR,SS)-3-(4-cyclohexylpiperazin-1-yl)-2-(methoxymethyl)-2-[(2-methoxyphenyl)(1-naphthyl)methyl]-3-oxopropanenitrile;
(2S,3S)-2-(methoxymethyl)-3-(2-methoxyphenyl)-3-(1-naphthyl)-2-[(4-pyrimidin-2-ylpiperazin-1-yl)carbonyl]propanenitrile;
(2R,3R)-2-(methoxymethyl)-3-(2-methoxyphenyl)-3-(1-naphthyl)-2-[(4-pyrimidin-2-ylpiperazin-1-yl)carbonyl]propanenitrile;
(RR,SS)-2-(methoxymethyl)-3-(2-methoxyphenyl)-3-(1-naphthyl)-2-[(4-pyrazin-2-ylpiperazin-1-yl)carbonyl]propanenitrile;
(RR,SS)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile;
(2S,3S)-3-(2-methoxyphenyl)-2-methyl-3-(1-naphthyl)-2-[(4-pyrazin-2-ylpiperazin-1-yl)carbonyl]propanenitrile;
(2S,3S)-3-(2-methoxyphenyl)-2-methyl-2-{[4-(2-morpholin-4-ylethyl)piperazin-1-yl]carbonyl}-3-(1-naphthyl)propanenitrile;
(2S)-3-{4-[2-(dimethylamino)ethyl]piperazin-1-yl}-2-[(S)-(2-methoxyphenyl)(1-naphthyl)methyl]-2-methyl-3-oxopropanenitrile;
(RR,SS)-2-[(benzyloxy)methyl]-3-(4-cyclohexylpiperazin-1-yl)-2-[(2-methoxyphenyl)(1-naphthyl)methyl]-3-oxopropanenitrile;
(RR,SS)-2-[(benzyloxy)methyl]-3-(2-methoxyphenyl)-3-(1-naphthyl)-2-[(4-pyrimidin-2-ylpiperazin-1-yl)carbonyl]propanenitrile;
(RR,SS)-2-(methoxymethyl)-3-(2-methoxyphenyl)-2-{[4-(2-morpholin-4-ylethyl)piperazin-1-yl]carbonyl}-3-(1-naphthyl)propanenitrile;
(RR,SS)-2-[(benzyloxy)methyl]-3-(2-methoxyphenyl)-3-(1-naphthyl)-2-[(4-pyrazin-2-ylpiperazin-1-yl)carbonyl]propanenitrile;
(2S,3S)-3-(4-cyclohexylpiperazin-1-yl)-2-(methoxymethyl)-2-[(2-methoxyphenyl)(1-naphthyl)methyl]-3-oxopropanenitrile;
(2R,3R)-3-(4-cyclohexylpiperazin-1-yl)-2-(methoxymethyl)-2-[(2-methoxyphenyl)(1-naphthyl)methyl]-3-oxopropanenitrile;
(2S,3S)-2-(methoxymethyl)-3-(2-methoxyphenyl)-3-(1-naphthyl)-2-[(4-pyrazin-2-ylpiperazin-1-yl)carbonyl]propanenitrile;
(2R,3R)-2-(methoxymethyl)-3-(2-methoxyphenyl)-3-(1-naphthyl)-2-[(4-pyrazin-2-ylpiperazin-1-yl)carbonyl]propanenitrile;
((2S,3S)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile;
((2R,3R)-3-[4-(1H-indol-3-yl)-3,6-dihydropyridin-1(2H)-yl]-2-(methoxymethyl)-2-[(2-methoxyphenyl)(1-naphthyl)methyl]-3-oxopropanenitrile;
(-)-(2S,3S)-2-cyano-3-(2-methoxyphenyl)-2-methyl-3-(1-naphthyl)propanoic acid; and
(-)-(2R,3R)-2-cyano-3-(2-methoxyphenyl)-2-methyl-3-(1-naphthyl)propanoic acid,
or a pharmaceutically acceptable salt thereof.

## Patentansprüche

1. Verwendung einer Verbindung der Formel I mit der Struktur 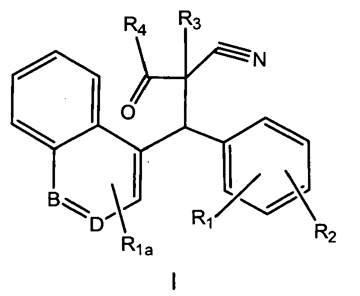 wobei
B und D unabhängig voneinander CH oder N sind, vorausgesetzt, dass B und D nicht beide N sind;
R₁, R₁ₐ, R₂ jeweils unabhängig voneinander Wasserstoff, Halogen, Alkyl mit 1-6 Kohlenstoffatomen, Alkoxy mit 1-6 Kohlenstoffatomen, Nitro, Cyano, Thioalkyl mit 1-6 Kohlenstoffatomen, Aryl, Alkylthio mit 1-6 Kohlenstoffatomen, CF₃, -OCF₃, -NR₅R₆ oder Hydroxy sind;
oder R₁ und R₂ zusammen mit Kohlenstoffatomen, an die sie gebunden sind, einen kondensierten Benzolring bilden, wobei der so gebildete Naphthalenring gegebenenfalls durch Halogen, Alkyl mit 1-6 Kohlenstoffatomen, Alkoxy mit 1-6 Kohlenstoffatomen, Nitro, Cyano, Thioalkyl mit 1-6 Kohlenstoffatomen, Aryl, Alkylthio mit 1-6 Kohlenstoffatomen, CF₃, -OCF₃, -NR₅R₆ oder Hydroxy substituiert ist;
R₃ Wasserstoff, Alkyl mit 1-6 Kohlenstoffatomen, Arylalkyl mit 1-6 Kohlenstoffatomen im Alkylrest, Alkenyl mit 2-7 Kohlenstoffatomen, Cycloalkylmethyl mit 3-8 Kohlenstoffatomen im Cycloalkylrest, Arylalkoxyalkyl, Alkoxyalkyl, Dialkylaminoalkyl mit 1-6 Kohlenstoffatomen in den Alkylresten oder Het-alkyl mit 1-6 Kohlenstoffatomen im Alkylrest ist;
R₄ ist NR₅R₆, -OR₆, 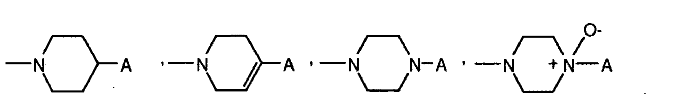 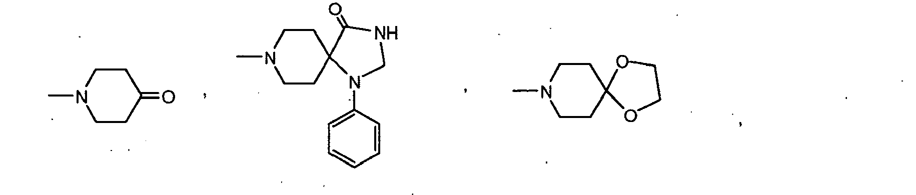 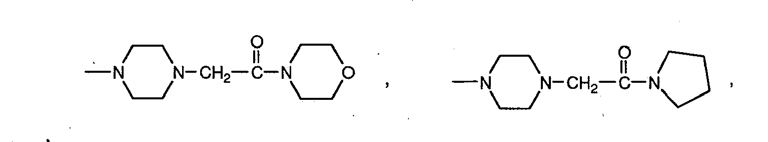 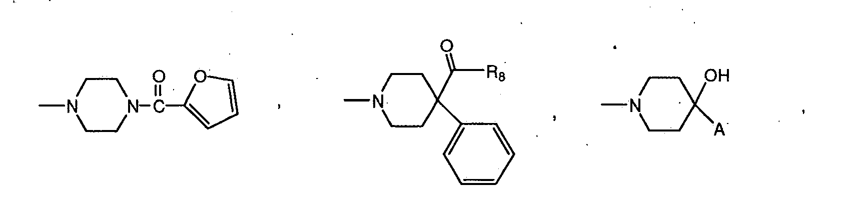 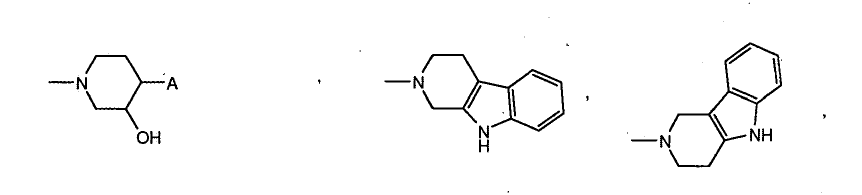 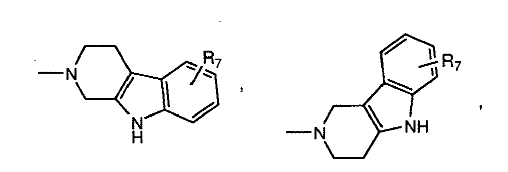 oder A, wobei irgendein Phenylring in R₄ gegebenenfalls mit R₇ substituiert ist;
R₅ und R₆ jeweils unabhängig voneinander Wasserstoff, Alkyl mit 1-6 Kohlenstoffatomen, Aryl, Arylalkyl mit 1-6 Kohlenstoffatomen im Alkylrest, Het-alkyl mit 1-6 Kohlenstoffatomen im Alkylrest, Hydroxyalkyl mit 1-6 Kohlenstoffatomen, Dihydroxyalkyl mit 1-6 Kohlenstoffatomen oder Cycloalkyl mit 3-8 Kohlenstoffatomen sind;
R₇ Alkyl mit 1-6 Kohlenstoffatomen, Alkoxy mit 1-6 Kohlenstoffatomen, Halogen, Nitro, Cyano, Alkylthio mit 1-6 Kohlenstoffatomen, Thioalkyl mit 1-6 Kohlenstoffatomen, CF₃ oder -OCF₃ ist;
R₈ Alkyl mit 1-6 Kohlenstoffatomen ist;
A Wasserstoff, Cycloalkyl mit 3-8 Kohlenstoffatomen, Alkoxyalkyl mit 1-6 Kohlenstoffatomen in den Alkyl- und Alkoxyresten, Dialkylaminoalkyl mit 1-6 Kohlenstoffatomen in den Alkylresten, Aryl, Het, Hydroxyalkyl mit 1-6 Kohlenstoffatomen, Dihydroxyalkyl mit 1-6 Kohlenstoffatomen, Het-alkyl mit 1-6 Kohlenstoffatomen im Alkylrest, Arylalkyl mit 1-6 Kohlenstoffatomen im Alkylrest oder 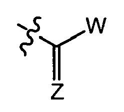 ist;
W Aryl, -Y-Aryl oder Het oder -Y-Het ist;
Y -O- oder -NH- ist;
Z O oder S ist;
Het ein gesättigter, ungesättigter oder teilweise ungesättigter heterocyclischer Ring bzw. ein gesättigtes, ungesättigtes oder teilweise ungesättigtes heterocyclisches Ringsystem mit 4-12 Ringatomen und 1-3 aus N, O oder S ausgewählten Heteroatomen ist, der bzw. das gegebenenfalls mit 1-3 R₇- Gruppen substituiert sein kann;
Aryl ein aromatischer Ring bzw. ein aromatisches Ringsystem mit 6-14 Kohlenstoffatomen im Ring bzw. Ringsystem ist, der bzw. das gegebenenfalls mit 1-3 R₇-Gruppen substituiert sein kann;
unter der Voraussetzung, dass mindestens eine der R₁-, R₁ₐ- oder R₂-Gruppen nicht Wasserstoff ist;
oder ein pharmazeutisch annehmbares Salz davon;
bei der Herstellung eines Arzneimittels zur Behandlung der entzündlichen Komponente einer Erkrankung.

2. Verwendung nach Anspruch 1, wobei die Erkrankung aus der Gruppe bestehend aus Atherosklerose, Myokardinfarkt, kongestiver Herzinsuffizienz, entzündlicher Darmerkrankung, Arthritis, Diabetes Typ II und Autoimmunkrankheit ausgewählt wird.

3. Verwendung nach Anspruch 2, wobei die Autoimmunkrankheit multiple Sklerose oder rheumatoide Arthritis ist.

4. Verwendung nach irgendeinem der Ansprüche 1 bis 3, wobei
R₄ -NR₅R₆, -OR₆, 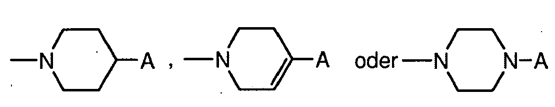 ist; und
A Wasserstoff, Aryl oder Het ist; oder
ein pharmazeutisch annehmbares Salz davon.

5. Verwendung nach Anspruch 4, wobei
R₄ 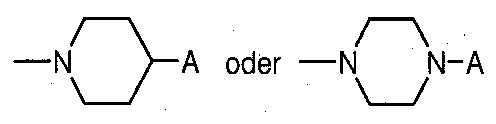 ist; und
A Wasserstoff, Aryl oder Het ist; oder
ein pharmazeutisch annehmbares Salz davon.

6. Verwendung nach irgendeinem der Ansprüche 1 bis 3, wobei B und D CH sind.

7. Verwendung nach irgendeinem der Ansprüche 1 bis 3, wobei die Verbindung der Formel I eine der folgenden ist:
Ethyl-2-cyano-3-(2,6-dimethoxyphenyl)-3-(1-naphthyl)propanoat;
Ethyl-2-cyano-3-(2,6-dichlorphenyl)-3-(1-naphthyl)propanoat;
Ethyl-2-cyano-3-[4-(dimethylamino)phenyl]-3-(1-naphthyl)propanoat;
Ethyl-2-cyano-3-(1-naphthyl)-3-[2-(trifluormethyl)-phenyl]propanoat;
Ethyl-2-cyano-3-(2-isopropylphenyl)-3-(1-naphthyl)propanoat;
Ethyl-2-cyano-3-(2,4-dimethoxyphenyl)-3-(1-naphthyl)propanoat;
Ethyl-2-cyano-3-(2,5-dimethoxyphenyl)-3-(1-naphthyl)propanoat;
*tert*-Butyl-2-cyano-3-(1-naphthyl)-3-[2-(trifluormethyl)-phenyl]propanoat;
Ethyl-2-cyano-3-(2-methoxyphenyl)-3-(1-naphthyl)propanoat;
Ethyl-(*RR*,*SS*)-2-cyano-3-(2-methoxyphenyl)-3-(1-naphthyl)propanoat;
*tert*-Butyl-2-cyano-3-(2-isopropylphenyl)-3-(1-naphthyl)propanoat;
*tert*-Butyl-2-cyano-3-(2-methoxyphenyl)-3-(1-naphthyl)propanoat;
*tert*-Butyl-2-cyano-3-(1-naphthyl)-3-[2-(trifluormethoxy)phenyl]propanoat;
Ethyl-2-cyano-3-(1-naphthyl)-3-(2-nitrophenyl)propanoat;
*tert*-Butyl-2-cyano-3-(2,6-dimethylphenyl)-3-(1-naphthyl)propanoat;
*tert*-Butyl-(*RR*,*SS*)-2-cyano-3-(1-naphthyl)-3-[2-(trifluormethyl)phenyl]propanoat;
*tert*-Butyl-(*RR*,*SS*)-2-cyano-3-(2-methoxyphenyl)-2-methyl-3-(1-naphthyl)propanoat;
(-)-Ethyl-(*SS*)-2-cyano-3-(2-methoxyphenyl)-2-methyl-3-(1-naphthyl)propanoat;
(+)-Ethyl-(*R*,*R*)-2-cyano-3-(2-methoxyphenyl)-2-methyl-3-(1-naphthyl)propanoat;
Ethyl-(*RR*,*SS*)-2-cyano-2-[(2-methoxyphenyl)(1-naphthyl)methyl]-4-pentenoat;
Ethyl-(*RR*,*SS*)-2-benzyl-2-cyano-3-(2-methoxyphenyl)-3-(1-naphthyl)-propanoat;
*tert*-Butyl-(*RR*,*SS*)-2-cyano-3-(2-isopropylphenyl)-2-methyl-3-(1-naphthyl)propanoat;
*tert*-Butyl-(*RS*,*SR*)-2-cyano-2-methyl-3-(1-naphthyl)-3-[2-(trifluormethyl)phenyl]propanoat;
*tert*-Butyl-(*RS*,*SR*)-2-cyano-3-(2,6-dimethylphenyl)-2-methyl-3-(1-naphthyl)propanoat;
*tert*-Butyl-(*RR*,*SS*)-2-benzyl-2-cyano-3-(2-methoxyphenyl)-3-(1-naphthyl)propanoat;
*tert*-Butyl-(*RR*,*SS*)-2-(3-chlorbenzyl)-2-cyano-3-(2-methoxyphenyl)-3-(1-naphthyl)propanoat;
*tert*-Butyl-(*RR*,*SS*)-2-(2-brombenzyl)-2-cyano-3-(2-methoxyphenyl)-3-(1-naphthyl)propanoat;
*tert*-Butyl-(*RR*,*SS*)-2-(2-chlorbenzyl)-2-cyano-3-(2-methoxyphenyl)-3-(1-naphthyl)propanoat;
*tert*-Butyl-(*RR,SS*)-2-cyano-2-(2,6-dichlorbenzyl)-3-(2-methoxyphenyl)-3-(1-naphthyl)propanoat;
Ethyl-(*RR,SS*)-2-cyano-3-(2,4-dimethoxyphenyl)-2-methyl-3-(1-naphthyl)propanoat;
*tert*-Butyl-(*RS,SR*)-2-cyano-2-methyl-3-(1-naphthyl)-3-[2-(trifluormethoxy)phenyl]propanoat;
Ethyl-2-cyano-3-(3-methoxyphenyl)-3-(1-naphthyl)propanoat;
Ethyl-2-cyano-3-(4-methylphenyl)-3-(1-naphthyl)propanoat;
Ethyl-2-cyano-3-(2-methylphenyl)-3-(1-naphthyl)propanoat;
Ethyl-2-cyano-3-(1-naphthyl)-3-(2-naphthyl)propanoat;
Ethyl-2-cyano-3-(4-fluor-1-naphthyl)-3-(1-naphthyl)propanoat;
Ethyl-2-cyano-3-[4-(methylthio)phenyl]-3-(1-naphthyl)propanoat;
Ethyl-3-[1,1'-biphenyl]-4-yl-2-cyano-3-(1-naphthyl)propanoat;
Ethyl-3-[1,1'-biphenyl]-2-yl-2-cyano-3-(1-naphthyl)propanoat;
Ethyl-3-(4-chlorphenyl)-2-cyano-3-(1-naphthyl)propanoat;
Ethyl-2-cyano-3-[2-(methylthio)phenyl]-3-(1-naphthyl)propanoat;
Ethyl-(*RR*,*SS*)-2-cyano-3-(2-methoxyphenyl)-2-methyl-3-(1-naphthyl)propanoat;
Ethyl-(*RR*,*SS*)-2-cyano-2-methyl-3-[2-(methylthio)phenyl]-3-(1-naphthyl)propanoat;
(*RR*,*SS*)-2-Cyano-3-(2-methoxyphenyl)-2-methyl-3-(1-naphthyl)propansäure;
(*RR*,*SS*)-2-Cyano-3-(2-isopropylphenyl)-2-methyl-3-(1-naphthyl)propansäure;
(*RS*,*SR*)-2-Cyano-2-methyl-3-(1-naphthyl)-3-[2-(trifluormethyl)phenyl]propansäure;
(*RR*,*SS*)-2-Benzyl-2-cyano-3-(2-methoxyphenyl)-3-(1-naphthyl)propansäure;
*tert*-Butyl-(*R*,*R*)-2-cyano-3-(2-isopropylphenyl)-2-methyl-3-(1-naphthyl)propanoat;
*tert*-Butyl-(*S*,*S*)-2-cyano-3-(2-isopropylphenyl)-2-methyl-3-(1-naphthyl)propanoat;
*tert*-Butyl-(*S*,*S*)-2-cyano-3-(2-methoxyphenyl)-2-methyl-3-(1-naphthyl)propanoat;
*tert*-Butyl-(*R*,*R*)-2-cyano-3-(2-methoxyphenyl)-2-methyl-3-(1-naphthyl)propanoat;
(*RR*,*SS*)-3-(2-Methoxyphenyl)-2-methyl-3-(1-naphthyl)-2-(piperazin-1-ylcarbonyl)propannitril; I;
(*RR*,*SS*)(3-[4-(3-Chlor-2-methylphenyl)piperazin-1-yl]-2-[(2-methoxyphenyl)(1-naphthyl)methyl]-2-methyl-3-oxopropannitril);
(*RR*,*S,S*)-3-(2-Methoxyphenyl)-2-methyl-2-{[4-(2-methylphenyl)piperazin-1-yl]carbonyl}-3-(1-naphthyl)propannitril;
(*SS*)-3-(2-Methoxyphenyl)-2-methyl-2-{[4-(2-methylphenyl)-1-piperazinyl]carbonyl}-3-(1-naphthyl)propannitril;
(*R*,*R*)-3-(2-Methoxyphenyl)-2-methyl-2-{[4-(2-methylphenyl)piperazin-1-yl]carbonyl}-3-(1-naphthyl)propannitril;
(*S*,*S*)-3-(2-Methoxyphenyl)-2-methyl-2-{[4-(3-methylphenyl)piperazin-1-yl]carbonyl}-3-(1-naphthyl)propannitril;
(*S*)-3-[4-(3,5-Dimethoxyphenyl)piperazin-1-yl]-2-[(*S*)-(2-methoxyphenyl)(1-naphthyl)methyl]-2-methyl-3-oxopropannitril;
(*S*)-3-(4-Indan-4-yl-piperazin-1-yl)-2-[(*S*)(2-methoxy-phenyl)-naphthalen-1-yl-methyl]-2-methyl-3-oxo-propionitril;
(*S*,*S*)-3-(2-Methoxyphenyl)-2-methyl-3-(1-naphthyl)-2-{[4-(1-naphthyl)piperazin-1-yl]carbonyl}propannitril;
(*S*)-3-[4-(3,4-Dimethylphenyl)piperazin-1-yl]-2-[(*S*)-(2-methoxyphenyl)(1-naphthyl)methyl]-2-methyl-3-oxopropannitril;
(*RR,SS*)-3-[4-(*H*-Indol-4-yl)piperazin-1-yl]-2-[(2-methoxyphenyl)(1-naphthyl)methyl]-2-methyl-3-oxopropannitril;
(*S*)-3-[4-(3-Chlorphenyl)piperazin-1-yl]-2-[(*S*)-(2-methoxyphenyl)(1-naphthyl)methyl]-2-methyl-3-oxopropannitril;
(*S*)-3-[4-(2,3-Dimethylphenyl)piperazin-1-yl]-2-[(*S*)-(2-methoxyphenyl)(1-naphthyl)methyl]-2-methyl-3-oxopropannitril; I;
(*S*)-3-[4-(4-Chlorphenyl)piperazin-1-yl]-2-[(*S*)-(2-methoxyphenyl)(1-naphthyl)methyl]-2-methyl-3-oxopropannitril;
(*S*)-3-[4-(1*H*-Indol-4-yl)-piperazin-1-yl]-2-[(*S*)-(2-methoxy-phenyl)-naphthalen-1-yl-methyl]-2-methyl-3-oxo-propionitril;
(*S,S*)-3-(2-Methoxyphenyl)-2-methyl-3-(1-naphthyl)-2-({4-[3-(trifluormethyl)phenyl]piperidin-1-yl}carbonyl)propannitril;
(*S*)-3-[4-(4-Chlorphenyl)-3,6-dihydro-2*H*-pyridin-1-yl]-2-[(*S*)-(2-methoxyphenyl)-naphthalen-1-yl-methyl]-2-methyl-3-oxo-propionitril;
(*S*,*S*)-3-(2-Methoxyphenyl)-2-methyl-3-naphthalen-1-yl-2-[4-(3-trifluormethyl-phenyl)-3,6-dihydro-2*H*-pyridin-1-carbonyl]-propionitril;
(*SS*)-2-[4-(4-Chlorphenyl)-piperidin-1-carbonyl]-3-(2-methoxy-phenyl)-2-methyl-3-naphthalen-1-yl-propionitril;
(*RR,SS*)-3-(2-Methoxyphenyl)-2-methyl-3-(1-naphthyl)-2-({4-[3-(trifluormethyl)phenyl]piperidin-1-yl}carbonyl)propannitril;
(*RR*,*SS*)-3-(2-Methoxyphenyl)-2-methyl-3-(1-naphthyl)-2-(piperidin-1-ylcarbonyl)propannitril;
(*RR*,*SS*)-2-Cyano-3-(2-methoxyphenyl)-2-methyl-3-(1-naphthyl)propanamid;
(*RR*,*SS*)-2-Cyano-N-ethyl-3-(2-methoxyphenyl)-2-methyl-3-(1-naphthyl)propanamid;
(*RR*,*SS*)-N-(*tert*-Butyl)-2-cyano-3-(2-methoxyphenyl)-2-methyl-3-(1-naphthyl)propanamid;
(*RR*,*SS*)-2-Cyano-3-(2-methoxyphenyl)-N,N,2-trimethyl-3-(1-naphthyl)propanamid;
(*RR*,*SS*)-2-Cyano-N-methoxy-3-(2-methoxyphenyl)-N,2-dimethyl-3-(1-naphthyl)propanamid;
(*RR*,*SS*)-2-Benzyl-3-[4-(3,5-dichlor-4-pyridinyl)-1-piperazinyl]-2-[(2-methoxyphenyl)(1-naphthyl)methyl]-3-oxopropannitril;
(*R*,*S*)-3-[4-(2,3-Dimethylphenyl)piperazin-1-yl]-2-[(*R*,*S*)-(2-methoxyphenyl)(1-naphthyl)methyl]-2-methyl-3-oxopropannitril;
(*R*,*S*)-3-[4-(3-Isopropylphenyl)piperazin-1-yl]-2-[(*R*,*S*)-(2-methoxyphenyl)(1-naphthyl)methyl]-2-methyl-3-oxopropannitril;
(*RR*,*SS*)-3-[4-(3,5-Dichlorpyridin-4-yl)piperazin-1-yl]-2-[(2-methoxyphenyl)(1-naphthyl)methyl]-2-methyl-3-oxopropannitril;
(2*SS*)-3-[4-(3-Chlor-2-methylphenyl)piperazin-1-yl]-2-[(2-methoxyphenyl)(1-naphthyl)methyl]-2-methyl-3-oxopropannitril;
(*S*)-3-[4-(2-Fluorphenyl)piperazin-1-yl]-2-[(*S*)-(2-methoxyphenyl)(1-naphthyl)methyl]-2-methyl-3-oxopropannitril;
(*S*)-3-[4-(2-Chlorphenyl)piperazin-1-yl]-2-[(*S*)-(2-methoxyphenyl)(1-naphthyl)methyl]-2-methyl-3-oxopropannitril;
(*S*,*S*)-3-(2-Methoxyphenyl)-2-{[4-(3-methoxyphenyl)piperazin-1-yl]carbonyl}-2-methyl-3-(1-naphthyl)propannitril;
(*R*,*S*)-3-[4-(3-Chlorpyridin-4-yl)piperazin-1-yl]-2-[(*R*,*S*)-(2-methoxyphenyl)(1-naphthyl)methyl]-2-methyl-3-oxopropannitril;
(*S*)-3-[4-(2,3-Dichlorphenyl)piperazin-1-yl]-2-[(*S*)-(2-methoxyphenyl)(1-naphthyl)methyl]-2-methyl-3-oxopropannitril;
(2*S*)-3-[4-[4-Chlor-3-(trifluormethyl)phenyl]-3,6-dihydropyridin-1(2*H*)-yl]-2-[(S)-(2-methoxyphenyl)(1-naphthyl)methyl]-2-methyl-3-oxopropannitril;
(2*S*)-3-{4-[4-Chlor-3-(trifluormethyl)phenyl]piperidin-1-yl}-2-[(*S*)-(2-methoxyphenyl)(1-naphthyl)methyl]-2-methyl-3-oxopropannitril;
(*RR*,*SS*)-3-(2-Methoxyphenyl)-2-methyl-3-naphthalen-1-yl-2-(4-oxy-4-o-tolyl-piperazin-1-carbonyl)-propionitril;
(*RR*,*SS*)-3-(2-Methoxyphenyl)-2-methyl-3-(1-naphthyl)-2-({4-[3-(trifluormethoxy)phenyl]piperazin-1-yl}carbonyl)propannitril;
(*R*,*S*)-3-[4-(2,3-Difluorphenyl)piperazin-1-yl]-2-[(R,S)-(2-methoxyphenyl)(1-naphthyl)methyl]-2-methyl-3-oxopropannitril;
(*R*,*S*)-3-[4-(3-Fluorphenyl)piperazin-1-yl]-2-[(*R*,*S*)-(2-methoxyphenyl)(1-naphthyl)methyl]-2-methyl-3-oxopropannitril;
(*RR*,*SS*)-3-(2-Methoxyphenyl)-2-methyl-3-(1-naphthyl)-2-[(4-pyridin-3-ylpiperazin-1-yl)carbonyl]propannitril;
(*RR*,*SS*)-3-[4-(2,3-Dichlorphenyl)piperazin-1-yl]-2-[(2-methoxyphenyl)(1-naphthyl)methyl]-2-methyl-3-oxopropannitril;
(*RR*,*SS*)-3-(2-Methoxyphenyl)-2-methyl-2-{[4-(3-methylphenyl)piperazin-1-yl]carbonyl}-3-(1-naphthyl)propannitril;
(*RR*,*SS*)-3-(2-Methoxyphenyl)-2-methyl-3-(1-naphthyl)-2-(pyrrolidin-1-ylcarbonyl)propannitril;
(*RR*,*SS*)-3-(2-Methoxyphenyl)-2-methyl-2-(morpholin-4-ylcarbonyl)-3-(1-naphthyl)propannitril;
(*RR*,*SS*)-3-[4-(2-Hydroxyethyl)piperazin-1-yl]-2-[(2-methoxyphenyl)(1-naphthyl)methyl]-2-methyl-3-oxopropannitril;
(*RR*,*SS*)-3-(2,6-Dimethylmorpholin-4-yl)-2-[(2-methoxyphenyl)(1-naphthyl)methyl]-2-methyl-3-oxopropannitril;
(*RR*,*SS*)-2-Cyano-N,N-diethyl-3-(2-methoxyphenyl)-2-methyl-3-(1-naphthyl)propanamid;
(*RR*,*SS*)-2-Cyano-N-[2-hydroxy-1-(hydroxymethyl)ethyl]-3-(2-methoxyphenyl)-2-methyl-3-(1-naphthyl)propanamid;
(*RR*,*SS*)-3-Azetidin-1-yl-2-[(2-methoxyphenyl)(1-naphthyl)methyl]-2-methyl-3-oxopropannitril;
(*RR*,*SS*)-2-Cyano-N,N-diisopropyl-3-(2-methoxyphenyl)-2-methyl-3-(1-naphthyl)propanamid;
(*RR*,*SS*)-3-(2-Methoxyphenyl)-2-methyl-3-(1-naphthyl)-2-[(3,3,5-trimethylazepan-1-yl)carbonyl]propannitril;
(*RR*,*SS*)-3-(2,3-Dihydro-*H*-indol-1-yl)-2-[(2-methoxyphenyl)(1-naphthyl)methyl]-2-methyl-3-oxopropannitril;
(*RR*,*SS*)-3-(2-Methoxyphenyl)-2-methyl-3-(1-naphthyl)-2-(thiomorpholin-4-ylcarbonyl)propannitril;
(*RR*,*SS*)-3-Azepan-1-yl-2-[(2-methoxyphenyl)(1-naphthyl)methyl]-2-methyl-3-oxopropannitril; I;
(*RR*,*SS*)-2-Cyano-N-cyclohexyl-3-(2-methoxyphenyl)-N,2-dimethyl-3-(1-naphthyl)propanamid;
(*RR*,*SS*)-2-Cyano-3-(2-methoxyphenyl)-N,2-dimethyl-3-(1-naphthyl)propanamid;
(*RR*,*SS*)-3-(4-Benzylpiperazin-1-yl)-2-[(2-methoxyphenyl)(1-naphthyl)methyl]-2-methyl-3-oxopropannitril; I;
(*RR*,*SS*)-3-(2-Methoxyphenyl)-2-methyl-2-{[4-(4-methylphenyl)piperazin-1-yl]carbonyl}-3-(1-naphthyl)propannitril;
(*RR*,*SS*)-N,N-Dibenzyl-2-cyano-3-(2-methoxyphenyl)-2-methyl-3-(1-naphthyl)propanamid;
(*RR*,*SS*)-3-Azocan-1-yl-2-[(2-methoxyphenyl)(1-naphthyl)methyl]-2-methyl-3-oxopropannitril;
4-Chlorphenyl-4-[(*RR*,*SS*)-2-cyano-3-(2-methoxyphenyl)-2-methyl-3-(1-naphthyl)propanoyl]piperazin-1-carboxylat;
2-Nitrophenyl-4-[(*RR*,*SS*)-2-cyano-3-(2-methoxyphenyl)-2-methyl-3-(1-naphthyl)propanoyl]piperazin-1-carboxylat;
4-(Methoxycarbonyl)phenyl-4-[(*RR*,*SS*)-2-cyano-3-(2-methoxyphenyl)-2-methyl-3-(1-naphthyl)propanoyl]piperazin-1-carboxylat;
4-Methylphenyl-4-[(*RR*,*SS*)-2-cyano-3-(2-methoxyphenyl)-2-methyl-3-(1-naphthyl)propanoyl]piperazin-1-carboxylat;
4-[(*RR*,*SS*)-2-Cyano-3-(2-methoxyphenyl)-2-methyl-3-(1-naphthyl)propanoyl]-N-(2-methylphenyl)piperazin-1-carboxamid;
4-[(*RR*,*SS*)-2-Cyano-3-(2-methoxyphenyl)-2-methyl-3-(1-naphthyl)propanoyl]-N-[2-(trifluormethyl)phenyl]piperazin-1-carboxamid;
4-[(*RR*,*SS*)-2-Cyano-3-(2-methoxyphenyl)-2-methyl-3-(1-naphthyl)propanoyl]-N-(3-methoxyphenyl)piperazin-1-carboxamid;
4-[(*RR*,*SS*)-2-Cyano-3-(2-methoxyphenyl)-2-methyl-3-(1-naphthyl)propanoyl]-N-(4-ethoxyphenyl)piperazin-1-carboxamid;
N-(2-Bromphenyl)-4-[(*RR*,*SS*)-2-cyano-3-(2-methoxyphenyl)-2-methyl-3-(1-naphthyl)propanoyl]piperazin-1-carboxamid;
4-[(*RR*,*SS*)-2-Cyano-3-(2-methoxyphenyl)-2-methyl-3-(1-naphthyl)propanoyl]-N-(4-methylphenyl)piperazin-1-carboxamid;
4-Fluorphenyl-4-[(*RR*,*SS*)-2-cyano-3-(2-methoxyphenyl)-2-methyl-3-(1-naphthyl)propanoyl]piperazin-1-carboxylat;
Phenyl-4-[(*RR*,*SS*)-2-cyano-3-(2-methoxyphenyl)-2-methyl-3-(1-naphthyl)propanoyl]piperazin-1-carboxylat;
(*RR*,*SS*)-3-[4-(4-Brombenzoyl)piperazin-1-yl]-2-[(2-methoxyphenyl)(1-naphthyl)methyl]-2-methyl-3-oxopropannitril;
N-(4-Chlorphenyl)-4-[(*RR*,*SS*)-2-cyano-3-(2-methoxyphenyl)-2-methyl-3-(1-naphthyl)propanoyl]piperazin-1-carboxamid;
oder
ein pharmazeutisch annehmbares Salz davon.

8. Pharmazeutische Zusammensetzung, umfassend:
einen pharmazeutischen Träger; und
eine Verbindung der Formel I nach irgendeinem der Ansprüche 1 bis 7 oder ein pharmazeutisch annehmbares Salz davon.

9. Verbindung der Formel I nach Anspruch 1, wobei R₆ Wasserstoff, Aryl, Arylalkyl mit 1-6 Kohlenstoffatomen im Alkylrest, Het-alkyl mit 1-6 Kohlenstoffatomen im Alkylrest, Hydroxyalkyl mit 1-6 Kohlenstoffatomen, Dihydroxyalkyl mit 1-6 Kohlenstoffatomen oder Cycloalkyl mit 3-8 Kohlenstoffatomen ist; oder ein pharmazeutisch annehmbares Salz davon.

10. Verbindung der Formel I nach Anspruch 9, wobei
R₄ -NR₅R₆, -OR₆, 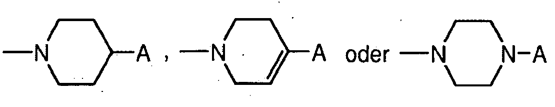 ist; und
A Wasserstoff, Aryl oder Het ist; oder
ein pharmazeutisch annehmbares Salz davon.

11. Verbindung der Formel I nach Anspruch 10, wobei
R₄ 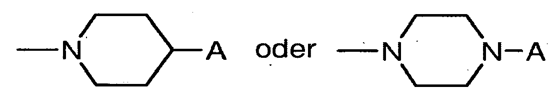 ist; und
A Wasserstoff, Aryl oder Het ist; oder
ein pharmazeutisch annehmbares Salz davon.

12. Verbindung der Formel I nach irgendeinem der Ansprüche 9 bis 11, wobei B und D CH sind.

13. Verbindung der Formel I nach Anspruch 7 oder ein pharmazeutisch annehmbares Salz davon, mit Ausnahme von
Ethyl-2-cyano-3-(4-methylphenyl)-3-(1-naphthyl)propanoat;
oder
Ethyl-2-cyano-3-(1-naphthyl)-3-(2-naphthyl)propanoat.

14. Verbindung, ausgewählt aus der Gruppe bestehend aus:
Methyl-(*SS*,*RR)*-2-cyano-3-(2-methoxyphenyl)-2-methyl-3-(chinolin-3-yl)propanoat;
3-(2-Methoxyphenyl)-2-methyl-3-chinolin-3-yl-2-({4-[3-(trifluormethyl)phenyl] piperidin-1-yl}carbonyl)propannitril;
Methyl-(2*S**,3*R**)-2-cyano-2-(methoxymethyl)-3-(2-methoxyphenyl)-3-chinolin-3-ylpropanoat; und
pharmazeutisch annehmbare Salze davon.

15. Verfahren zur Herstellung einer Verbindung der Formel I nach Anspruch 9, welches die Schritte (a)-(d) oder (e), (f) oder (g) umfasst:
(a) Reagieren einer Verbindung der Formel 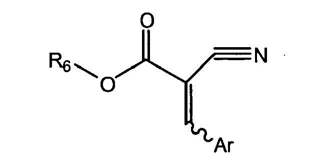 wobei R₆ wie in Anspruch 9 definiert ist mit Ausnahme von Wasserstoff und Ar eine Gruppe der Formel (A), (B) oder (C) ist: 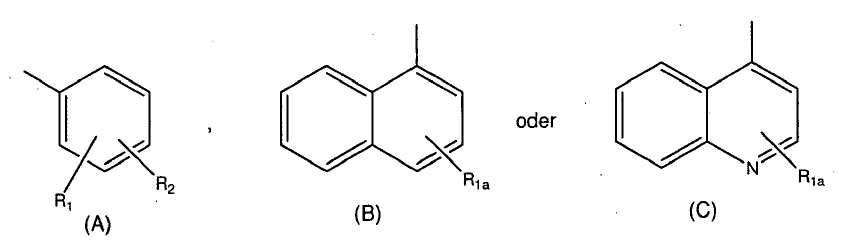 wobei R₁, R₂ und R₁ₐ wie in Anspruch 9 definiert sind;
mit einer Verbindung der Formel Ar₁MX, wobei Ar₁ eine Gruppe der Formel (A) oder (B) ist, vorausgesetzt, dass Ar und Ar₁ nicht beide die Formel (A) oder (B) aufweisen, um eine Verbindung der Formel 1 zu erhalten, bei der R₄ OR₆ ist, wobei R₆ wie in Anspruch 9 definiert ist mit Ausnahme von Wasserstoff, und R₃ Wasserstoff ist;
(b) Alkylieren einer Verbindung der Formel: 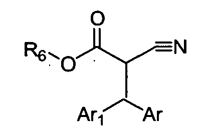 wobei R₆, Ar und Ar₁ wie vorstehend definiert sind, vorausgesetzt, dass R₆ etwas anderes als Wasserstoff ist, mit einem Alkylierungsmittel der Formel R₃L, wobei L eine Abgangsgruppe ist und R₃ wie in Anspruch 9 definiert ist mit Ausnahme von Wasserstoff, um eine entsprechende Verbindung der Formel I nach Anspruch 9 zu erhalten;
(c) Hydrolysieren eines Esters der Formel 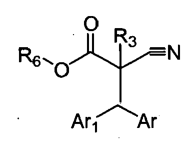 wobei Ar, Ar₁, R₃ und R₆ wie vorstehend definiert sind, vorausgesetzt, dass R₆ etwas anderes als Wasserstoff ist, um eine entsprechende Verbindung der Formel I zu erhalten, bei der R₄ OR₆ ist, wobei R₆ Wasserstoff ist;
(d) Reagieren einer aktivierten Säureverbindung der Formel: 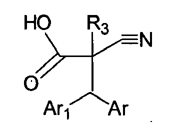 wobei Ar, Ar₁ und R₃ wie vorstehend definiert sind, mit einem Amin der Formel HNR₅R₆, 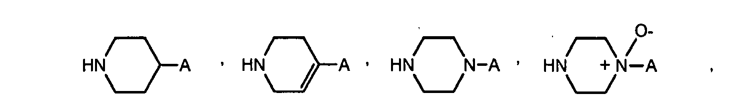 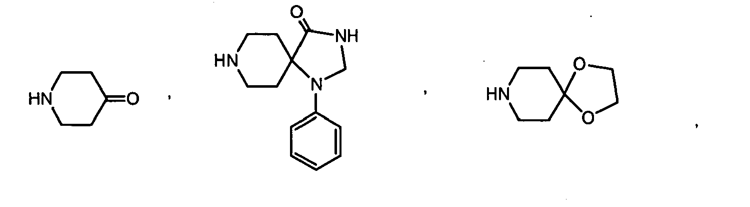 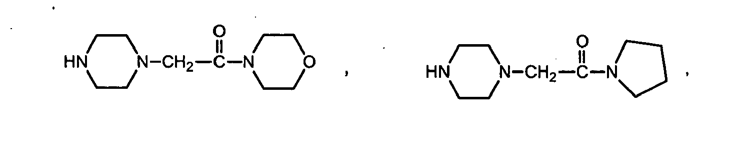 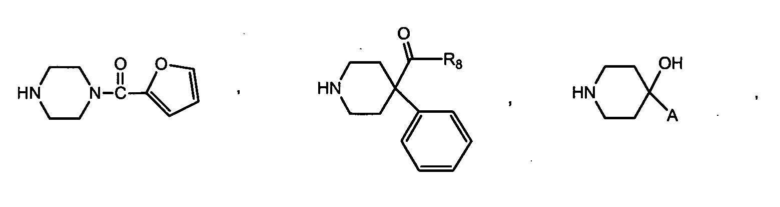 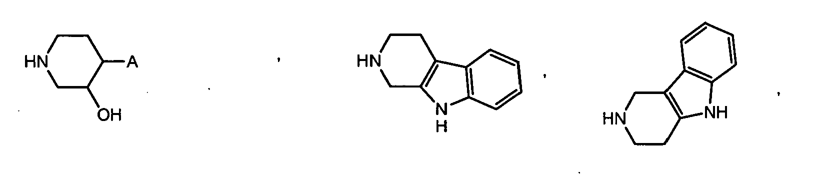 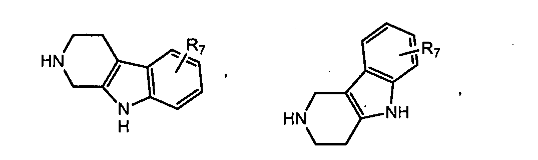 oder
(e) Reagieren einer Verbindung der Formel. 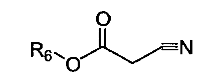 wobei R₆ wie vorstehend definiert ist mit Ausnahme von Wasserstoff, in Gegenwart einer starken Base mit einem Halogenid der Formel
Ar₁ArCHX
wobei Ar und Ar₁ wie hierin definiert sind und X ein Halogen ist, um eine entsprechende Verbindung der Formel I zu erhalten, bei der R₃ Wasserstoff ist,
oder
(f) Umwandeln einer Verbindung der Formel I nach Anspruch 1, die eine reaktive Substituentengruppe oder Stelle aufweist, um eine andere Verbindung der Formel I zu erhalten;
oder
(g) Umwandeln einer Verbindung der Formel I in ein pharmazeutisch annehmbares Salz davon.

16. Verfahren, umfassend folgende Schritte:
Bilden eines Ephedrin-, Cinchonidin- oder Chinidinsalzes einer Verbindung nach Anspruch 9 oder 14 oder eines pharmazeutisch annehmbaren Salzes davon; und
Extrahieren des Ephedrin-, Cinchonidin- oder Chinidinsalzes mit einem Lösungsmittel;
um ein im Wesentlichen reines Enantiomer einer Verbindung nach Anspruch 9 oder 14 zu bilden.

17. Verbindung nach Anspruch 9, ausgewählt aus:
2-Cyano-3,3-di(1-naphthyl)propanamid;
(*RR*,*SS*)-3-(3,4-Dihydroisochinolin-2(1*H*)-yl)-2-[(2-methoxyphenyl)(1-naphthyl)methyl]-2-methyl-3-oxopropannitril;
(2S)-3-[4-(3-Bromphenyl)piperazin-1-yl]-2-[(S)-(2-methoxyphenyl)(1-naphthyl)methyl]-2-methyl-3-oxopropannitril;
(2S,3S)-3-(2-Methoxyphenyl)-2-methyl-3-(1-naphthyl)-2-{[4-(2-oxo-2,3-dihydro-1*H*-indol-1-yl)piperidin-1-yl]carbonyl}propannitril;
(2S,3S)-3-(2-Methoxyphenyl)-2-methyl-3-(1-naphthyl)-2-[(4-pyridin-4-ylpiperazin-1-yl)carbonyl]propannitril;
(2S,3S)-3-(2-Methoxyphenyl)-2-methyl-3-(1-naphthyl)-2-{[4-(2-oxo-3,4-dihydrochinolin-1 (2*H*)-yl)piperidin-1-yl]carbonyl}propannitril;
(2S)-3-[4-(4-Bromphenyl)-3,6-dihydropyridin-1(2*H*)-yl]-2-[(S)-(2-methoxyphenyl)(1-naphthyl)methyl]-2-methyl-3-oxopropannitril;
(2S,3S)-3-(2-Methoxyphenyl)-2-methyl-3-(1-naphthyl)-2-({4-[3-(trifluormethyl)phenyl]piperazin-1-yl}carbonyl)propannitril;
(2S)-3-[4-(4-Fluorphenyl)-3,6-dihydropyridin-1(2*H*)-yl]-2-[(S)-(2-methoxyphenyl)(1-naphthyl)methyl]-2-methyl-3-oxopropannitril;
(2S,3S)-3-(2-Methoxyphenyl)-2-{[4-(2-methoxyphenyl)piperidin-1-yl]carbonyl}-2-methyl-3-(1-naphthyl)propannitril;
(2S,3S)-3-(2-Methoxyphenyl)-2-methyl-3-(1-naphthyl)-2-({4-[3-(trifluormethyl)cyclohexyl]piperidin-1-yl}carbonyl)propannitril;
(2S)-3-{4-Hydroxy-4-[3-(trifluormethyl)phenyl]piperidin-1-yl}-2-[(S)-(2-methoxyphenyl)(1-naphthyl)methyl]-2-methyl-3-oxopropannitril;
2-Cyano-3-(2-methoxyphenyl)-2-methyl-3-chinolin-4-ylpropansäure;
3-(2-Methoxyphenyl)-2-methyl-3-chinolin-4-yl-2-({4-[3-(trifluormethyl)phenyl]piperidin-1-yl}carbonyl)propannitril;
(RR,SS)-2-[(2-Methoxyethoxy)methyl]-3-(2-methoxyphenyl)-3-(1-naphthyl)-2-[(4-pyrimidin-2-ylpiperazin-1-yl)carbonyl]propannitril;
(RR,SS)-2-[(2-Methoxyethoxy)methyl]-3-(2-methoxyphenyl)-3-(1-naphthyl)-2-({4-[3-(trifluormethyl)phenyl]piperidin-1-yl}carbonyl)propannitril;
(RR,SS)-N-(*tert*-Butyl)-2-cyano-2-[(2-methoxyethoxy)methyl]-3-(2-methoxyphenyl)-3-(1-naphthyl)propanamid;
(RR,SS)-2-Cyano-N-(2,2-dimethylpropyl)-2-[(2-methoxyethoxy)methyl]-3-(2-methoxyphenyl)-3-(1-naphthyl)propanamid;
(RR,SS)-2-[(2-Methoxyethoxy)methyl]-3-(2-methoxyphenyl)-3-(1-naphthyl)-2-[(4-pyrazin-2-ylpiperazin-1-yl)carbonyl]propannitril;
(RR,SS)-3-[4-(1*H*-Indol-3-yl)-3,6-dihydropyridin-1(2*H*)-yl]-2-[(2-methoxyethoxy)methyl]-2-[(S*)-(2-methoxyphenyl)(1-naphthyl)methyl]-3-oxopropannitril;
(RR,SS)-3-(2-Methoxyphenyl)-3-(1-naphthyl)-2-(pyridin-3-ylmethyl)-2-[(4-pyrimidin-2-ylpiperazin-1-yl)carbonyl]propannitril;
(RR,SS)-N-(*tert*-Butyl)-2-cyano-3-(2-methoxyphenyl)-3-(1-naphthyl)-2-(pyridin-3-ylmethyl)propanamid;
(RR,SS)-2-Cyano-N-(2,2-dimethylpropyl)-3-(2-methoxyphenyl)-3-(1-naphthyl)-2-(pyridin-3-ylmethyl)propanamid;
(RR,SS)-3-(2-Methoxyphenyl)-3-(1-naphthyl)-2-[(4-pyrazin-2-ylpiperazin-1-yl)carbonyl]-2-(pyridin-3-ylmethyl)propannitril;
(RR,SS)-3-[4-(1*H*-Indol-3-yl)-3,6-dihydropyridin-1(2*H*)-yl]-2-[(2-methoxyphenyl)(1-naphthyl)methyl]-3-oxo-2-(pyridin-3-ylmethyl)propannitril;
(RR,SS)-N-(*tert*-Butyl)-2-cyano-2-(methoxymethyl)-3-(2-methoxyphenyl)-3-(1-naphthyl)propanamid;
(2S*,3S*)-2-Cyano-N-(2,2-dimethylpropyl)-2-(methoxymethyl)-3-(2-methoxyphenyl)-3-(1-naphthyl)propanamid;
(2S)-3-[4-(1*H*-Indol-3-yl)-3,6-dihydropyridin-1(2*H*)-yl]-2-[(S)-(2-methoxyphenyl)(1-naphthyl)methyl]-2-methyl-3-oxopropannitril;
(2S,3S)-3-(2-Methoxyphenyl)-2-methyl-3-(1-naphthyl)-2-{[4-(2-oxo-2,3-dihydro-1*H*-benzimidazol-1-yl)piperidin-1-yl]carbonyl}propannitril;
(2S,3S)-3-(2-Methoxyphenyl)-2-methyl-3-(1-naphthyl)-2-[(4-oxo-1-phenyl-1,3,8-triazaspiro[4.5]dec-8-yl)carbonyl]propannitril;
(2S)-3-{3-Hydroxy-4-[3-(trifluormethyl)phenyl]piperidin-1-yl}-2-[(S)-(2-methoxyphenyl)(1-naphthyl)methyl]-2-methyl-3-oxopropannitril;
(2S)-3-[4-(4-Chlorphenyl)-4-hydroxypiperidin-1-yl]-2-[(S)-(2-methoxyphenyl)(1-naphthyl)methyl]-2-methyl-3-oxopropannitril;
(2S)-3-[4-(1*H*-Indol-3-yl)piperidin-1-yl]-2-[(S)-(2-methoxyphenyl)(1-naphthyl)methyl]-2-methyl-3-oxopropannitril; I;
(2S)-3-(1,4-Dioxa-8-azaspiro[4.5]dec-8-yl)-2-[(S)-(2-methoxyphenyl)(1-naphthyl)methyl-2-methyl-3-oxopropannitril;
(2S)-3-(8-Fluor-1,3,4,5-tetrahydro-2*H*-pyrido[4,3-b]indol-2-yl)-2-[(S)-(2-methoxyphenyl)(1-naphthyl)methyl]-2-methyl-3-oxopropannitril;
(2S,3S)-3-(2-Methoxyphenyl)-2-methyl-3-(1-naphthyl)-2-(1,3,4,9-tetrahydro-2*H*-beta-carbolin-2-ylcarbonyl)propannitril;
(2S,3S)-3-(2-Methoxyphenyl)-2-methyl-3-(1-naphthyl)-2-[(4-oxopiperidin-1-yl)carbonyl]propannitril; I;
(2S,3S)-3-(2-Methoxyphenyl)-2-methyl-3-(1-naphthyl)-2-[(4-pyrimidin-2-ylpiperazin-1-yl)carbonyl]propannitril;
(2S)-3-[4-(4-Bromphenyl)-4-hydroxypiperidin-1-yl]-2-[(S)-(2-methoxyphenyl)(1-naphthyl)methyl]-2-methyl-3-oxopropannitril;
(2S,3S)-3-(2-Methoxyphenyl)-2-methyl-3-(1-naphthyl)-2-[(4-phenyl-4-propionylpiperidin-1-yl)carbonyl]propannitril;
(2S)-3-(4-Acetyl-4-phenylpiperidin-1-yl)-2-[(S)-(2-methoxyphenyl)(1-naphthyl)methyl]-2-methyl-3-oxopropannitril;
(2S)-3-(4-Cyclohexylpiperazin-1-yl)-2-[(S)-(2-methoxyphenyl)(1-naphthyl)methyl]-2-methyl-3-oxopropannitril;
(2S)-3-[4-(2-Methoxyethyl)piperazin-1-yl]-2-[(S)-(2-methoxyphenyl)(1-naphthyl)methyl]-2-methyl-3-oxopropannitril;
(2S,3S)-3-(2-Methoxyphenyl)-2-methyl-2-{[4-(2-morpholin-4-yl-2-oxoethyl)piperazin-1-yl]carbonyl}-3-(1-naphthyl)propannitril;
(2S)-3-[4-(2-Furoyl)piperazin-1-yl]-2-[(S)-(2-methoxyphenyl)(1-naphthyl)methyl]-2-methyl-3-oxopropannitril;
(RR,SS)-2-(Methoxymethyl)-3-(2-methoxyphenyl)-3-(1-naphthyl)-2-[(4-pyrimidin-2-ylpiperazin-1-yl)carbonyl]propannitril;
(2S,3S)-3-(2-Methoxyphenyl)-2-methyl-3-(1-naphthyl)-2-{[4-(2-oxo-2-pyrrolidin-1-ylethyl)piperazin-1-yl]carbonyl}propannitril;
(2S)-3-{4-[3-(Dimethylamino)propyl]piperazin-1-yl}-2-[(S)-(2-methoxyphenyl)(1-naphthyl)methyl]-2-methyl-3-oxopropannitril;
(2S,3S)-3-(2-Methoxyphenyl)-2-methyl-2-[(4-morpholin-4-ylpiperidin-1-yl)carbonyl]-3-(1-naphthyl)propannitril;
3-{4-[3-(Dimethylamino)propyl]piperazin-1-yl}-2-(methoxymethyl)-2-[(2-methoxyphenyl)(1-naphthyl)methyl]-3-oxopropannitril;
(2S*)-2-[(Benzyloxy)methyl]-3-{4-[3-(dimethylamino)propyl]piperazin-1-yl}-2-[(R*)-(2-methoxyphenyl)(1-naphthyl)methyl]-3-oxopropannitril;
3-[4-(2-Methoxyethyl)piperazin-1-yl]-2-(methoxymethyl)-2-[(2-methoxyphenyl)(1-naphthyl)methyl]-3-oxopropannitril;
2-[(Benzyloxy)methyl]-3-[4-(2-methoxyethyl)piperazin-1-yl]-2-[(2-methoxyphenyl)(1-naphthyl)methyl]-3-oxopropannitril;
2-[(Benzyloxy)methyl]-3-(2-methoxyphenyl)-2-{[4-(2-morpholin-4-ylethyl)piperazin-1-yl]carbonyl}-3-(1-naphthyl)propannitril;
(RR,SS)-3-(4-Cyclohexylpiperazin-1-yl)-2-(methoxymethyl)-2-[(2-methoxyphenyl)(1-naphthyl)methyl]-3-oxopropannitril;
(2S,3S)-2-(Methoxymethyl)-3-(2-methoxyphenyl)-3-(1-naphthyl)-2-[(4-pyrimidin-2-ylpiperazin-1-yl)carbonyl]propannitril;
(2R,3R)-2-(Methoxymethyl)-3-(2-methoxyphenyl)-3-(1-naphthyl)-2-[(4-pyrimidin-2-ylpiperazin-1-yl)carbonyl]propannitril;
(RR,SS)-2-(Methoxymethyl)-3-(2-methoxyphenyl)-3-(1-naphthyl)-2-[(4-pyrazin-2-ylpiperazin-1-yl)carbonyl]propannitril;
(RR,SS)-3-[4-(1*H*-Indol-3-yl)-3,6-dihydropyridin-1(2*H*)]-2-(methoxymethyl)-2-[(2-methoxyphenyl)(1-naphthyl)methyl]-3-oxopropannitril;
(2S,3S)-3-(2-Methoxyphenyl)-2-methyl-3-(1-naphthyl)-2-[(4-pyrazin-2-ylpiperazin-1-yl)carbonyl]propannitril;
(2S,3S)-3-(2-Methoxyphenyl)-2-methyl-2-{[4-(2-morpholin-4-ylethyl)piperazin-1-yl]carbonyl}-3-(1-naphthyl)propannitril;
(2S)-3-{4-[2-(Dimethylamino)ethyl]piperazin-1-yl}-2-[(S)-(2-methoxyphenyl)(1-naphthyl)methyl]-2-methyl-3-oxopropannitril;
(RR,SS)-2-[(Benzyloxy)methyl]-3-(4-cyclohexylpiperazin-1-yl)-2-[(2-methoxyphenyl)(1-naphthyl)methyl]-3-oxopropannitril;
(RR,SS)-2-[(Benzyloxy)methyl]-3-(2-methoxyphenyl)-3-(1-naphthyl)-2-[(4-pyrimidin-2-ylpiperazin-1-yl)carbonyl]propannitril;
(RR,SS)-2-(Methoxymethyl)-3-(2-methoxyphenyl)-2-{[4-(2-morpholin-4-ylethyl)piperazin-1-yl]carbonyl}-3-(1-naphthyl)propannitril;
(RR,SS)-2-[(Benzyloxy)methyl]-3-(2-methoxyphenyl)-3-(1-naphthyl)-2-[(4-pyrazin-2-ylpiperazin-1-yl)carbonyl]propannitril;
(2S,3S)-3-(4-Cyclohexylpiperazin-1-yl)-2-(methoxymethyl)-2-[(2-methoxyphenyl)(1-naphthyl)methyl]-3-oxopropannitril;
(2R,3R)-3-(4-Cyclohexylpiperazin-1-yl)-2-(methoxymethyl)-2-[(2-methoxyphenyl)(1-naphthyl)methyl]-3-oxopropannitril;
(2S,3S)-2-(Methoxymethyl)-3-(2-methoxyphenyl)-3-(1-naphthyl)-2-[(4-pyrazin-2-ylpiperazin-1-yl)carbonyl]propannitril;
(2R,3R)-2-(Methoxymethyl)-3-(2-methoxyphenyl)-3-(1-naphthyl)-2-[(4-pyrazin-2-ylpiperazin-1-yl)carbonyl]propannitril;
((2S,3S)-3-[4-(1*H*-Indol-3-yl)-3,6-dihydropyridin-1(2*H*)-yl]-2-(methoxymethyl)-2-[(2-methoxyphenyl)(1-naphthyl)methyl]-3-oxopropannitril;
((2R,3R)-3-[4-(1*H*-Indol-3-yl)-3,6-dihydropyridin-1(2*H*)-yl]-2-(methoxymethyl)-2-[(2-methoxyphenyl)(1-naphthyl)methyl]-3-oxopropannitril;
(-)-(2S,3S)-2-Cyano-3-(2-methoxyphenyl)-2-methyl-3-(1-naphthyl)propansäure; und
(-)-(2R,3R)-2-Cyano-3-(2-methoxyphenyl)-2-methyl-3-(1-naphthyl)propansäure,
oder ein pharmazeutisch annehmbares Salz davon.

## Revendications

1. Utilisation d'un composé répondant à la formule I possédant la structure 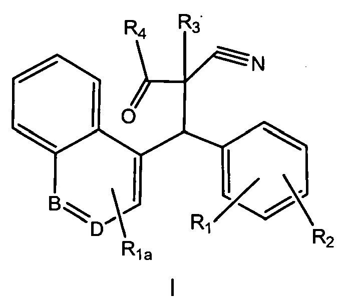 dans laquelle
B et D représentent, indépendamment l'un de l'autre, un groupe CH ou un atome d'azote, avec cette condition que B et D ne représentent pas tous les deux un atome d'azote ;
R₁, R₁ₐ, R₂ représentent, chacun indépendamment l'un de l'autre, un atome d'hydrogène, un atome d'halogène, un groupe alkyle contenant de 1 à 6 atomes de carbone, un groupe alcoxy contenant de 1 à 6 atomes de carbone, un groupe nitro, un groupe cyano, un groupe thioalkyle contenant de 1 à 6 atomes de carbone, un groupe aryle, un groupe alkylthio contenant de 1 à 6 atomes de carbone, un groupe CF₃, un groupe -OCF₃, un groupe -NR₅R₆, ou un groupe hydroxyle ;
ou bien R₁ et R₂ forment, ensemble avec les atomes de carbone auxquels ils sont fixés, un noyau benzénique condensé, le noyau naphtalène ainsi obtenu étant, de manière facultative, substitué par un atome d'halogène, un groupe alkyle contenant de 1 à 6 atomes de carbone, un groupe alcoxy contenant de 1 à 6 atomes de carbone, un groupe nitro, un groupe cyano, un groupe thioalkyle contenant de 1 à 6 atomes de carbone, un groupe aryle, un groupe alkylthio contenant de 1 à 6 atomes de carbone, un groupe CF₃, un groupe -OCF₃, un groupe -NR₅R₆, ou un groupe hydroxyle ;
R₃ représente un atome d'hydrogène, un groupe alkyle contenant de 1 à 6 atomes de carbone, un groupe arylalkyle contenant de 1 à 6 atomes de carbone dans la fraction alkyle, un groupe alcényle contenant de 2 à 7 atomes de carbone, un groupe cycloalkylméthyle contenant de 3 à 8 atomes de carbone dans la fraction cycloalkyle, un groupe arylalcoxyalkyle, un groupe alcoxyalkyle, un groupe dialkylaminoalkyle contenant de 1 à 6 atomes de carbone dans les fractions alkyle, ou un groupe Het-alkyle contenant de 1 à 6 atomes de carbone dans la fraction alkyle ;
R₄ représente un groupe -NR₅R₆, un groupe -OR₆, 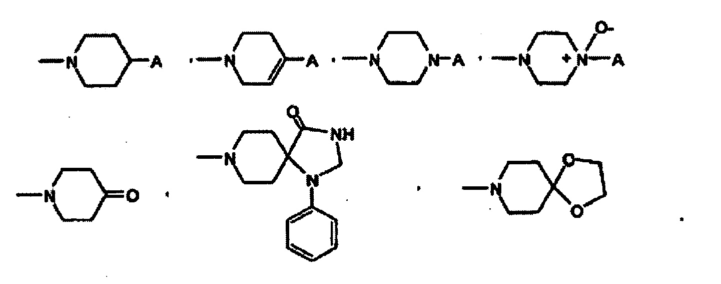 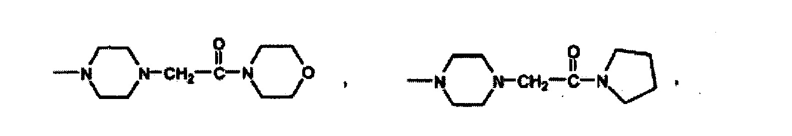 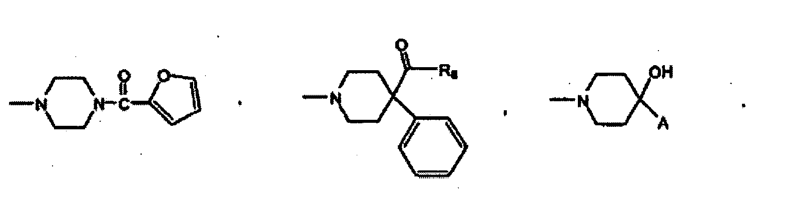 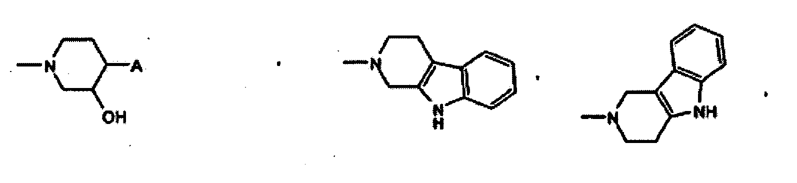 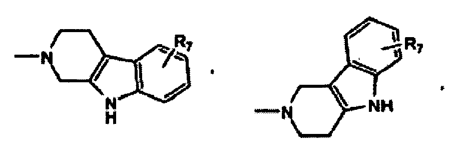
ou A, n'importe quel noyau phényle dans R₄ étant, de manière facultative, substitué par R₇:
R₅ et R₆ représentent, chacun indépendamment l'un de l'autre, un atome d'hydrogène, un groupe alkyle contenant de 1 à 6 atomes de carbone, un groupe aryle, un groupe arylalkyle contenant de 1 à 6 atomes de carbone dans la fraction alkyle, un groupe Het-alkyle contenant de 1 à 6 atomes de carbone dans la fraction alkyle, un groupe hydroxyalkyle contenant de 1 à 6 atomes de carbone, un groupe dihydroxyalkyle contenant de 1 à 6 atomes de carbone, ou un groupe cycloalkyle contenant de 3 à 8 atomes de carbone ;
R₇ représente un groupe alkyle contenant de 1 à 6 atomes de carbone, un groupe alcoxy contenant de 1 à 6 atomes de carbone, un atome d'halogène, un groupe nitro, un groupe cyano, un groupe alkylthio contenant de 1 à 6 atomes de carbone, un groupe thioalkyle contenant de 1 à 6 atomes de carbone, un groupe CF₃, ou un groupe -OCF₃ ;
R₈ représente un groupe alkyle contenant de 1 à 6 atomes de carbone ;
A représente un atome d'hydrogène, un groupe cycloalkyle contenant de 3 à 8 atomes de carbone, un groupe alcoxyalkyle contenant de 1 à 6 atomes de carbone dans les fractions alkyle et alcoxy, un groupe dialkylaminoalkyle contenant de 1 à 6 atomes de carbone dans les fractions alkyle, un groupe aryle, un groupe Het, un groupe hydroxyalkyle contenant de 1 à 6 atomes de carbone, un groupe dihydroxyalkyle contenant de 1 à 6 atomes de carbone, un groupe Het-alkyle contenant de 1 à 6 atomes de carbone dans la fraction alkyle, un groupe arylalkyle contenant de 1 à 6 atomes de carbone dans la fraction alkyle, ou un groupe 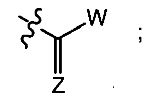
W représente un groupe aryle, un groupe -Y-aryle, ou un groupe Het ou un groupe-Y-Het ;
Y représente un atome d'oxygène ou un groupe -NH- ;
Z représente un atome d'oxygène ou un atome de soufre ;
Het représente un noyau hétérocyclique ou un système nucléaire hétérocyclique saturé, insaturé ou partiellement insaturé contenant de 4 à 12 atomes nucléaires et de 1 à 3 hétéroatomes choisis parmi un atome d'azote, un atome d'oxygène ou un atome de soufre, qui peut être, de manière facultative, substitué avec un nombre de 1 à 3 groupes R₇;
le terme « groupe aryle » représente un noyau ou un système nucléaire aromatique contenant de 6 à 14 atomes de carbone dans le noyau ou dans le système nucléaire, qui peut être, de manière facultative, substitué avec de 1 à 3 groupes R₇ ;
avec cette réserve qu'au moins un des groupes R₁, R₁ₐ, ou R₂ ne représente pas un atome d'hydrogène ;
ou d'un de ses sels pharmaceutiquement acceptables, dans la préparation d'un médicament pour traiter la composante inflammatoire d'une maladie.

2. Utilisation selon la revendication 1, dans laquelle ladite maladie est choisie parmi le groupe constitué par l'athérosclérose, l'infarctus du myocarde, l'insuffisance cardiaque congestive, une affection abdominale inflammatoire, l'arthrite, le diabète de type II et une maladie autoimmune.

3. Utilisation selon la revendication 2, dans laquelle ladite maladie autoimmune est la sclérose en plaques ou la polyarthrite rhumatoïde.

4. Utilisation selon l'une quelconque des revendications 1 à 3, dans laquelle
R₄ représente un groupe -NR₅R₆, un groupe -OR₆, 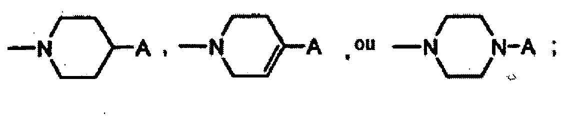 et
A représente un atome d'hydrogène, un groupe aryle ou un groupe Het ; ou
d'un de ses sels pharmaceutiquement acceptables.

5. Utilisation selon la revendication 4, dans laquelle
R₄ représente un groupe 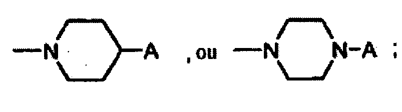 et
A représente un atome d'hydrogène, un groupe aryle ou un groupe Het ; ou
d'un de ses sels pharmaceutiquement acceptables.

6. Utilisation selon l'une quelconque des revendications 1 à 3, dans laquelle B et D représentent un groupe CH.

7. Utilisation selon l'une quelconque des revendications 1 à 3, dans laquelle le composé répondant à la formule I est un des suivants :
le 2-cyano-3-(2,6-diméthoxyphényl)-3-(1-naphtyl)propanoate d'éthyle;
le 2-cyano-3-(2,6-dichlorophényl)-3-(1-naphtyl)propanoate d'éthyle ;
le 2-cyano-3-[4-(diméthylamino)phényl]-3-(1-naphtyl)propanoate d'éthyle ;
le 2-cyano-3-(1-naphtyl)-3-[2-(trifluorométhyl)-phényl]propanoate d'éthyle ;
le 2-cyano-3-(2-isopropylphényl)-3-(1-naphtyl)propanoate d'éthyle ;
le 2-cyano-3-(2,4-diméthoxyphényl)-3-(1-naphtyl)propanoate d'éthyle ;
le 2-cyano-3-(2,5-diméthoxyphényl)-3-(1-naphtyl)propanoate d'éthyle ;
le 2-cyano-3-(1-naphtyl)-3-[2-(trifluorométhyl)phényl]propanoate de tert-butyle ;
le 2-cyano-3-(2-méthoxyphényl)-3-(1-naphtyl)propanoate d'éthyle ;
le (*RR*,*SS*)-2-cyano-3-(2-méthoxyphényl)-3-(1-naphtyl)propanoate d'éthyle ;
le 2-cyano-3-(2-isopropylphényl)-3-(1-naphtyl)propanoate de *tert-*butyle ;
le 2-cyano-3-(2-méthoxyphényl)-3-(1-naphtyl)propanoate de *tert-*butyle ;
le 2-cyano-3-(1-naphtyl)-3-[2-(trifluorométhoxy)phényl]propanoate de *tert*-butyle ;
le 2-cyano-3-(1-naphtyl)-3-(2-nitrophényl)propanoate d'éthyle ;
le 2-cyano-3-(2,6-diméthylphényl)-3-(1-naphtyl)propanoate de *tert-*butyle ;
le (*RR*,*SS*)-2-cyano-3-(1-naphtyl)-3-[2-(trifluorométhyl)phényl]-propanoate de *tert*-butyle ;
le (*RR*,*SS*)-2-cyano-3-(2-méthoxyphényl)-2-méthyl-3-(1-naphtyl)-propanoate de *ter*t-butyle ;
le (-) (*SS*)-2-cyano-3-(2-méthoxyphényl)-2-méthyl-3-(1-naphtyl)-propanoate d'éthyle ;
le (+) (*R*,*R*)-2-cyano-3-(2-méthoxyphényl)-2-méthyl-3-(1-naphtyl)propanoate d'éthyle ;
le (*RR,SS*)-2-cyano-2-[(2-méthoxyphényl)(1-naphtyl)méthyl]-4-penténoate d'éthyle ;
le (*RR*,*SS*)-2-benzyl-2-cyano-3-(2-méthoxyphényl)-3-(1-naphtyl)-propanoate d'éthyle ;
le (*RR*,*SS*)-2-cyano-3-(2-isopropylphényl)-2-méthyl-3-(1-naphtyl)propanoate de *tert*-butyle ;
le (*RS,SR*)-2-cyano-2-méthyl-3-(1-naphtyl)-3-[2-(trifluorométhyl)phényl]propanoate de *tert*-butyle ;
le (*RS*,*SR*)-2-cyano-3-(2,6-diméthylphényl)-2-méthyl-3-(1-naphtyl)propanoate de *tert*-butyle ;
le (*RR,SS*)-2-benzyl-2-cyano-3-(2-méthoxyphényl)-3-(1-naphtyl)propanoate de *tert*-butyle ;
le (*RR*,*SS*)-2-(3-chlorobenzyl)-2-cyano-3-(2-méthoxyphényl)-3-(1-naphtyl)propanoate de *tert*-butyle ;
le (*RR*,*SS*)-2-(2-bromobenzyl)-2-cyano-3-(2-méthoxyphényl)-3-(1-naphtyl)propanoate de *tert*-butyle ;
le (*RR*,*SS*)-2-(2-chlorobenzyl)-2-cyano-3-(2-méthoxyphényl)-3-(1-naphtyl)propanoate de *tert*-butyle ;
le (*RR*,*SS*)-2-cyano-2-(2,6-dichlorobenzyl)-3-(2-méthoxyphényl)-3-(1-naphtyl)propanoate de *tert*-butyle ;
le (*RR*,*SS*)-2-cyano-3-(2,4-diméthoxyphényl)-2-méthyl-3-(1-naphtyl)propanoate d'éthyle ;
le (*RS*,*SR*)-2-cyano-2-méthyl-3-(1-naphtyl)-3-[2-(trifluorométhoxy)-phényl]propanoate de *tert*-butyle ;
le 2-cyano-3-(3-méthoxyphényl)-3-(1-naphtyl)propanoate d'éthyle ;
le 2-cyano-3-(4-méthylphényl)-3-(1-naphtyl)propanoate d'éthyle ;
le 2-cyano-3-(2-méthylphényl)-3-(1-naphtyl)propanoate d'éthyle ;
le 2-cyano-3-(1-naphtyl)-3-(2-naphtyl)propanoate d'éthyle ;
le 2-cyano-3-(4-fluoro-1-naphtyl)-3-(1-naphtyl)propanoate d'éthyle;
le 2-cyano-3-[4-(méthylthio)phényl]-3-(1-naphtyl)propanoate d'éthyle ;
le 3-[1,1'-biphényl]-4-yl-2-cyano-3-(1-naphtyl)propanoate d'éthyle ;
le 3-[1,1'-biphényl]-2-yl-2-cyano-3-(1-naphtyl)propanoate d'éthyle ;
le 3-(4-chlorophényl)-2-cyano-3-(1-naphtyl)propanoate d'éthyle ;
le 2-cyano-3-[2-(méthylthio)phényl]-3-(1-naphtyl)propanoate d'éthyle ;
le (*RR*,*SS*)-2-cyano-3-(2-méthoxyphényl)-2-méthyl-3-(1-naphtyl)-propanoate d'éthyle ;
le (*RR*,*SS*)-2-cyano-2-méthyl-3-[2-(méthylthio)phényl]-3-(1-naphtyl)propanoate d'éthyle ;
l'acide (*RR*,*SS*)-2-cyano-3-(2-méthoxyphényl)-2-méthyl-3-(1-naphtyl)propandique ;
l'acide (*RR*,*SS*)-2-cyano-3-(2-isopropylphényl)-2-méthyl-3-(1-naphtyl)propanoïque;
l'acide (*RS*,*SR*)-2-cyano-2-méthyl-3-(1-naphtyl)-3-[2-(trifluorométhyl)phényl]propanoïque ;
l'acide (*RR*,*SS*)-2-benzyl-2-cyano-3-(2-méthoxyphényl)-3-(1-naphtyl)propanoïque ;
le (*R*,*R*)-2-cyano-3-(2-isopropylphényl)-2-méthyl-3-(1-naphtyl)-propanoate de *tert*-butyle ;
le (*S*,*S*)-2-cyano-3-(2-isopropylphényl)-2-méthyl-3-(1-naphtyl)-propanoate de *tert*-butyle ;
le (*S*,*S*)-2-cyano-3-(2-méthoxyphényl)-2-méthyl-3-(1-naphtyl)-propanoate de *tert*-butyle ;
le (*R*,*R*)-2-cyano-3-(2-méthoxyphényl)-2-méthyl-3-(1-naphtyl)-propanoate de *tert*-butyle ;
le (*RR*,*SS*)-3-(2-méthoxyphényl)-2-méthyl-3-(1-naphtyl)-2-(pipérazin-1-ylcarbonyl)propanenitrile ;
le (*RR*,*SS*)(3-[4-(3-chloro-2-méthylphényl)pipérazin-1-yl]-2-[(2-méthoxyphényl)(1-naphtyl)méthyl]-2-méthyl-3-oxopropanenitrile) ;
le (*RR*,*S*,*S*)-3-(2-méthoxyphényl)-2-méthyl-2-{[4-(2-méthylphényl)-pipérazin-1-yl]carbonyl}-3-(1-naphtyl)propanenitrile ;
le (*SS*)-3-(2-méthoxyphényl)-2-méthyl-2-{[4-(2-méthylphényl)-1-pipérazinyl]carbonyl}-3-(1-naphtyl)propanenitrile ;
le (*R*,*R*)-3-(2-méthoxyphényl)-2-méthyl-2-{[4-(2-méthylphényl)-pipérazin-1-yl]carbonyl}-3-(1-naphtyl)propanenitrile ;
le (*S*,*S*)-3-(2-méthoxyphényl)-2-méthyl-2-{[4-(3-méthylphényl)-pipérazin-1-yl]carbonyl}-3-(1-naphtyl)propanenitrile ;
le (*S*)-3-[4-(3,5-diméthoxyphényl)pipérazin-1-yl]-2-[(*S*)-(2-méthoxyphényl)(1-naphtyl)méthyl]-2-méthyl-3-oxopropanenitrile ;
le (*S*)3-(4-indan-4-yl-pipérazin-1-yl)-2-[(*S*)(2-méthoxy-phényl)-naphtalén-1-yl-méthyl]-2-méthyl-3-oxo-propionitrile ;
le (*S*,*S*)-3-(2-méthoxyphényl)-2-méthyl-3-(1-naphtyl)-2-{[4-(1-naphtyl)pipérazin-1-yl]carbonyl}propanenitrile ;
le (*S*)-3-[4-(3,4-diméthylphényl)pipérazin-1-yl]-2-[(*S*)-(2-méthoxyphényl)(1-naphtyl)méthyl]-2-méthyl-3-oxopropanenitrile ;
le (*RR*,*SS*)-3-[4-(*H*-indol-4-yl)pipérazin-1-yl]-2-[(2-méthoxyphényl)(1-naphtyl)méthyl]-2-méthyl-3-oxopropanenitrile ;
le (*S*)-3-[4-(3-chlorophényl)pipérazin-1-yl]-2-[(*S*)-(2-méthoxyphényl)(1-naphtyl)méthyl]-2-méthyl-3-oxopropanenitrile ;
le (*S*)-3-[4-(2,3-diméthylphényl)pipérazin-1-yl]-2-[(*S*)-(2-méthoxyphényl)(1-naphtyl)méthyl]-2-méthyl-3-oxopropanenitrile ;
le (*S*)-3-[4-(4-chlorophényl)pipérazin-1-yl]-2-[(*S*)-(2-méthoxyphényl)(1-naphtyl)méthyl]-2-méthyl-3-oxopropanenitrile ;
le (*S*)-3-[4-(1*H*-Indol-4-yl)-pipérazin-1-yl]-2-[(*S*)-(2-méthoxyphényl)-naphtalén-1-yl-méthyl]-2-méthyl-3-oxo-propionitrile ;
le (*S*,*S*)-3-(2-méthoxyphényl)-2-méthyl-3-(1-naphtyl)-2-({4-[3-(trifluorométhyl)phényl]pipéridin-1-yl}carbonyl)propanenitrile ;
le (*S*)-3-[4-(4-chloro-phényl)-3,6-dihydro-2*H*-pyridin-1-yl]-2-[(*S*)-(2-méthoxy-phényl)-naphtalén-1-yl-méthyl]-2-méthyl-3-oxo-propionitrile ;
le (*S*,*S*)-3-(2-méthoxy-phényl)-2-méthyl-3-naphtalén-1-yl-2-[4-(3-trifluorométhyl-phényl)-3,6-dihydro-2H-pyridine-1-carbonyl]-propionitrile ;
le (*SS*)2-[4-(4-chloro-phényl)-pipéridine-1-carbonyl]-3-(2-méthoxyphényl)-2-méthyl-3-naphtalén-1-yl-propionitrile ;
le (*RR*,*SS*)-3-(2-méthoxyphényl)-2-méthyl-3-(1-naphtyl)-2-({4-[3-(trifluorométhyl)phényl]pipéridin-1-yl}carbonyl)propanenitrile ;
le (*RR*,*SS*)-3-(2-méthoxyphényl)-2-méthyl-3-(1-naphtyl)-2-(pipéridin-1-ylcarbonyl)propanenitrile ;
le (*RR*,*SS*)-2-cyano-3-(2-méthoxyphényl)-2-méthyl-3-(1-naphtyl)-propanamide ;
le (*RR*,*SS*)-2-cyano-N-éthyl-3-(2-méthoxyphényl)-2-méthyl-3-(1-naphtyl)propanamide ;
le (*RR*,*SS*)-N-(*tert*-butyl)-2-cyano-3-(2-méthoxyphényl)-2-méthyl-3-(1-naphtyl)propanamide ;
le (*RR*,*SS*)-2-cyano-3-(2-méthoxyphényl)-N,N,2-triméthyl-3-(1-naphtyl)propanamide ;
le (*RR*,*SS*)-2-cyano-N-méthoxy-3-(2-méthoxyphényl)-N,2-diméthyl-3-(1-naphtyl)propanamide ;
le (*RR*,*SS*)-2-benzyl-3-[4-(3,5-dichloro-4-pyridinyl)-1-pipérazinyl]-2-[(2-méthoxyphényl)(1-naphtyl)méthyl]-3-oxopropanenitrile
le (*R*,*S*)-3-[4-(2,3-diméthylphényl)pipérazin-1-yl]-2-[(*R*,*S*)-(2-méthoxyphényl)(1-naphtyl)méthyl]-2-méthyl-3-oxopropanenitrile ;
le (*R*,*S*)-3-[4-(3-isopropylphényl)pipérazin-1-yl]-2-[(*R*,*S*)-(2-méthoxyphényl)-1-naphtyl)méthyl]-2-méthyl-3-oxopropanenitrile ;
le (*RR*,*SS*)-3-[4-(3,5-dichloropyridin-4-yl)pipérazin-1-yl]-2-[(2-méthoxyphényl)(1-naphtyl)méthyl]-2-méthyl-3-oxopropanenitrile ;
le (2*SS*)-3-[4-(3-chloro-2-méthylphényl)pipérazin-1-yl]-2-[(2-méthoxyphényl)(1-naphtyl)méthyl]-2-méthyl-3-oxopropanenitrile ;
le (*S*)-3-[4-(2-fluorophényl)pipérazin-1-yl]-2-[(*S*)-(2-méthoxyphényl)(1-naphtyl)méthyl]-2-méthyl-3-oxopropanenitrile ;
le (*S*)-3-[4-(2-chlorophényl)pipérazin-1-yl]-2-[(*S*)-(2-méthoxyphényl)(1-naphtyl)méthyl]-2-méthyl-3-oxopropanenitrile ;
le (*S*,*S*)-3-(2-méthoxyphényl)-2-{[4-(3-méthoxyphényl)pipérazin-1-yl]carbonyl}-2-méthyl-3-(1-naphtyl)propanenitrile ;
le (*R*,*S*)-3-[4-(3-chloropyridin-4-yl)pipérazin-1-yl]-2-[(*R*,*S*)-(2-méthoxyphényl)(1-naphtyl)méthyl]-2-méthyl-3-oxopropanenitrile ;
le (*S*)-3-[4-(2,3-dichlorophényl)pipérazin-1-yl]-2-[(S)-(2-méthoxyphényl)(1-naphtyl)méthyl]-2-méthyl-3-oxopropanenitrile ;
le (2*S*)-3-[4-[4-chloro-3-(trifluorométhyl)phényl]-3,6-dihydropyridin-1(2*H*)-yl]-2-[(*S*)-(2-méthoxyphényl)(1-naphtyl)méthyl]-2-méthyl-3-oxopropanenitrile ;
le (2*S*)-3-{4-[4-chloro-3-(trifluorométhyl)phényl]pipéridin-1-yl}-2-[(*S*)-(2-méthoxyphényl)(1-naphtyl)méthyl]-2-méthyl-3-oxopropanenitrile ;
le (*RR,SS*)-3-(2-méthoxyphényl)-2-méthyl-3-naphtalén-1-yl-2-(4-oxy-4-o-tolyl-pipérazine-1-carbonyl)-propionitrile ;
le (*RR*,*SS*)-3-(2-méthoxyphényl)-2-méthyl-3-(1-naphtyl)-2-({4-[3-(trifluorométhoxy)phényl]pipérazin-1-yl}carbonyl)propanenitrile ;
le (*R*,*S*)-3-[4-(2,3-difluorophényl)pipérazin-1-yl]-2-[(*R*,*S)*-(2-méthoxyphényl)(1-naphtyl)méthyl]-2-méthyl-3-oxopropanenitrile ;
le (*R*,*S*)-3-[4-(3-fluorophényl)pipérazin-1-yl]-2-[(*R*,*S*)-(2-méthoxyphényl)(1-naphtyl)méthyl]-2-méthyl-3-oxopropanenitrile ;
le (*RR*,*SS*)-3-(2-méthoxyphényl)-2-méthyl-3-(1-naphtyl)-2-[(4-pyridin-3-ylpipérazin-1-yl)carbonyl]propanenitrile ;
le (*RR*,*SS*)-3-[4-(2,3-dichlorophényl)pipérazin-1-yl]-2-[(2-méthoxyphényl)(1-naphtyl)méthyl]-2-méthyl-3-oxopropanenitrile ;
le (*RR*,*SS*)-3-(2-méthoxyphényl)-2-méthyl-2-{[4-(3-méthylphényl)-pipérazin-1-yl]carbonyl}-3-(1-naphtyl)propanenitrile ;
le (*RR*,*SS*)-3-(2-méthoxyphényl)-2-méthyl-3-(1-naphtyl)-2-(pyrrolidin-1-ylcarbonyl)propanenitrile ;
le (*RR*,*SS*)-3-(2-méthoxyphényl)-2-méthyl-2-(morpholin-4-ylcarbonyl)-3-(1-naphtyl)propanenitrile ;
le (*RR*,*SS*)-3-[4-(2-hydroxyéthyl)pipérazin-1-yl]-2-[(2-méthoxyphényl)(1-naphtyl)méthyl]-2-méthyl-3-oxopropanenitrile ;
le (*RR*,*SS*)-3-(2,6-diméthylmorpholin-4-yl)-2-[(2-méthoxyphényl)(1-naphtyl)méthyl]-2-méthyl-3-oxopropanenitrile ;
le (*RR*,*SS*)-2-cyano-N,N-diéthyl-3-(2-méthoxyphényl)-2-méthyl-3-(1-naphtyl)propanamide ;
le (*RR*,*SS*)-2-cyano-N-[2-hydroxy-1-(hydroxyméthyl)éthyl]-3-(2-méthoxyphényl)-2-méthyl-3-(1-naphtyl)propanamide ;
le (*RR*,*SS*)-3-azétidin-1-yl-2-[(2-méthoxyphényl)(1-naphtyl)-méthyl]-2-méthyl-3-oxopropanenitrile ;
le (*RR*,*SS*)-2-cyano-N,N-diisopropyl-3-(2-méthoxyphényl)-2-méthyl-3-(1-naphtyl)propanamide ;
le (*RR*,*SS*)-3-(2-méthoxyphényl)-2-méthyl-3-(1-naphtyl)-2-[(3,3,5-triméthylazépan-1-yl)carbonyl]propanenitrile ;
le (*RR*,*SS*)-3-(2,3-dihydro-*H*-indol-1-yl)-2-[(2-méthoxyphényl)(1-naphtyl)méthyl]-2-méthyl-3-oxopropanenitrile ;
le (*RR*,*SS*)-3-(2-méthoxyphényl)-2-méthyl-3-(1-naphtyl)-2-(thiomorpholin-4-ylcarbonyl)propanenitrile ;
le (*RR*,*SS*)-3-azépan-1-yl-2-[(2-méthoxyphényl)(1-naphtyl)méthyl]-2-méthyl-3-oxopropanenitrile ;
le (*RR*,*SS*)-2-cyano-N-cyclohexyl-3-(2-méthoxyphényl)-N,2-diméthyl-3-(1-naphtyl)propanamide ;
le (*RR*,*SS*)-2-cyano-3-(2-méthoxyphényl)-N,2-diméthyl-3-(1-naphtyl)propanamide ;
le (*RR*,*SS*)-3-(4-benzylpipérazin-1-yl)-2-[(2-méthoxyphényl)(1-naphtyl)méthyl]-2-méthyl-3-oxopropanenitrile ;
le (*RR*,*SS*)-3-(2-méthoxyphényl)-2-méthyl-2-{[4-(4-méthylphényl)-pipérazin-1-yl]carbonyl}-3-(1-naphtyl)propanenitrile ;
le (*RR*,*SS*)-N,N-dibenzyl-2-cyano-3-(2-méthoxyphényl)-2-méthyl-3-(1-naphtyl)propanamide ;
le (*RR*,*SS*)-3-azocan-1-yl-2-[(2-méthoxyphényl)(1-naphtyl)méthyl]-2-méthyl-3-oxopropanenitrile ;
le 4-[(*RR*,*SS*)-2-cyano-3-(2-méthoxyphényl)-2-méthyl-3-(1-naphtyl)propanoyl]pipérazine-1-carboxylate de 4-chlorophényle ;
le 4-[(*RR*,*SS*)-2-cyano-3-(2-méthoxyphényl)-2-méthyl-3-(1-naphtyl)propanoyl]pipérazine-1-carboxylate de 2-nitrophényle ;
le 4-[(*RR*,*SS*)-2-cyano-3-(2-méthoxyphényl)-2-méthyl-3-(1-naphtyl)propanoyl]pipérazine-1-carboxylate de 4-(méthoxycarbonyl)-phényle ;
le 4-[(*RR*,*SS*)-2-cyano-3-(2-méthoxyphényl)-2-méthyl-3-(1-naphtyl)propanoyl]pipérazine-1-carboxylate de 4-méthylphényle ;
le 4-[(*RR*,*SS*)-2-cyano-3-(2-méthoxyphényl)-2-méthyl-3-(1-naphtyl)propanoyl]-N-(2-méthylphényl)pipérazine-1-carboxamide ;
le 4-[(*RR*,*SS*)-2-cyano-3-(2-méthoxyphényl)-2-méthyl-3-(1-naphtyl)propanoyl]-N-[2-(trifluorométhyl)phényl]pipérazine-1-carboxamide ;
le 4-[(*RR*,*SS*)-2-cyano-3-(2-méthoxyphényl)-2-méthyl-3-(1-naphtyl)propanoyl]-N-(3-méthoxyphényl)pipérazine-1-carboxamide ;
le 4-[(*RR*,*SS*)-2-cyano-3-(2-méthoxyphényl)-2-méthyl-3-(1-naphtyl)propanoyl]-N-(4-éthoxyphényl)pipérazine-1-carboxamide ;
le N-(2-bromophényl)-4-[(*RR*,*SS*)-2-cyano-3-(2-méthoxyphényl)-2-méthyl-3-(1-naphtyl)propanoyl]pipérazine-1-carboxamide ;
le 4-[(*RR*,*SS*)-2-cyano-3-(2-méthoxyphényl)-2-méthyl-3-(1-naphtyl)propanoyl]-N-(4-méthylphényl)pipérazine-1-carboxamide ;
le 4-[(*RR*,*SS*)-2-cyano-3-(2-méthoxyphényl)-2-méthyl-3-(1-naphtyl)propanoyl]pipérazine-1-carboxylate de 4-fluorophényle ;
le 4-[(*RR*,*SS*)-2-cyano-3-(2-méthoxyphényl)-2-méthyl-3-(1-naphtyl)propanoyl]pipérazine-1-carboxylate de phényle ;
le (*RR*,*SS*)-3-[4-(4-bromobenzoyl)pipérazin-1-yl]-2-[(2-méthoxyphényl)(1-naphtyl)méthyl]-2-méthyl-3-oxopropanenitrile ;
le N-(4-chlorophényl)-4-[(*RR*,*SS*)-2-cyano-3-(2-méthoxyphényl)-2-méthyl-3-(1-naphtyl)propanoyl]pipérazine-1-carboxamide ;
ou un de leurs sels pharmaceutiquement acceptables.

8. Composition pharmaceutique, comprenant :
un support pharmaceutique ; et
un composé répondant à la formule I, tel que défini dans l'une quelconque des revendications 1 à 7 ou un de ses sels pharmaceutiquement acceptables.

9. Composé répondant à la formule I, tel que défini à la revendication 1, dans lequel R₆ représente un atome d'hydrogène, un groupe aryle, un groupe arylalkyle contenant de 1 à 6 atomes de carbone dans la fraction alkyle, un groupe Het-alkyle contenant de 1 à 6 atomes de carbone dans la fraction alkyle, un groupe hydroxyalkyle contenant de 1 à 6 atomes de carbone, un groupe dihydroxyalkyle contenant de 1 à 6 atomes de carbone, ou un groupe cycloalkyle contenant de 3 à 8 atomes de carbone, ou un de ses sels pharmaceutiquement acceptables.

10. Composé répondant à la formule I, selon la revendication 9, dans lequel
R₄ représente un groupe -NR₅R₆, un groupe -OR₆, 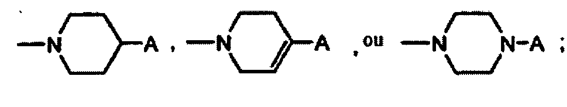 et
A représente un atome d'hydrogène, un groupe aryle ou un groupe Het ; ou
un de ses sels pharmaceutiquement acceptables.

11. Composé répondant à la formule I, selon la revendication 10, dans lequel
R₄ représente un groupe 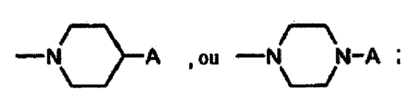 et
A représente un atome d'hydrogène, un groupe aryle ou un groupe Het ; ou
un de ses sels pharmaceutiquement acceptables.

12. Composé répondant à la formule I, selon l'une quelconque des revendications 9 à 11, dans lequel
B et D représentent un groupe CH.

13. Composé répondant à la formule I, tel que défini à la revendication 7, ou un de ses sels pharmaceutiquement acceptables à l'exception
du 2-cyano-3-(4-méthylphényl)-3-(1-naphtyl)propanoate d'éthyle ;
ou
du 2-cyano-3-(1-naphtyl)-3-(2-naphtyl)propanoate d'éthyle.

14. Composé choisi parmi le groupe constitué par :
le (*SS*,*RR*)-2-cyano-3-(2-méthoxyphényl)-2-méthyl-3-(quinolin-3-yl)-propanoate de méthyle ;
le 3-(2-méthoxyphényl)-2-méthyl-3-quinolin-3-yl-2-({4-[3-(trifluorométhyl)phényl] pipéridin-1-yl}carbonyl)propanenitrile;
le (2*S**,3*R**)-2-cyano-2-(méthoxyméthyl)-3-(2-méthoxyphényl)-3-quinolin-3-ylpropanoate de méthyle ; et
leurs sels pharmaceutiquement acceptables.

15. Procédé pour préparer un composé répondant à la formule I, selon la revendication 9, qui comprend les étapes (a)-(d) ou (e), (f) ou (g):
(a) de mise en réaction d'un composé répondant à la formule 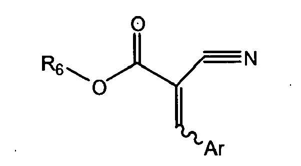 dans laquelle R₆ est tel que défini à la revendication 9, à l'exception d'un atome d'hydrogène et Ar représente un groupe répondant à la formule (A), (B), ou (C): 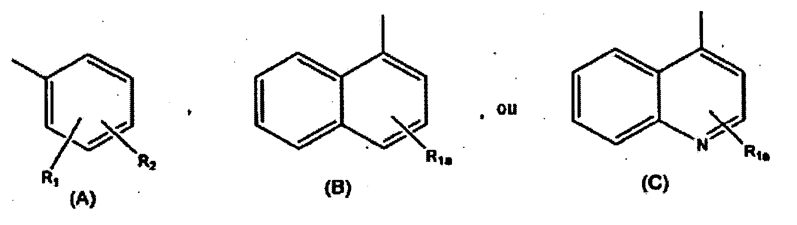 dans lesquelles R₁ ,R₂ et R₁ₐ sont tels que définis à la revendication 9; avec un composé répondant à la formule Ar₁ MX dans laquelle Ar₁ représente un groupe répondant à la formule (A) ou (B), avec cette condition que Ar et Ar₁ ne représentent pas ensemble un groupe répondant à la formule (A) ou (B), pour obtenir un groupe répondant à la formule I dans laquelle R₄ représente un groupe OR₆ où R₆ est tel que défini à la revendication 9, à l'exception d'un atome d'hydrogène, et R₃ représente un atome d'hydrogène ;
(b) d'alkylation d'un composé répondant à la formule : 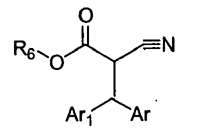 dans laquelle R₆, Ar et Ar₁ sont tels que définis ci-dessus, avec cette condition que R₆ représente autre chose qu'un atome d'hydrogène, avec un agent d'alkylation répondant à la formule R₃L dans laquelle L représente un groupe partant et R₃ est tel que défini à la revendication 9 à l'exception d'un atome d'hydrogène, pour obtenir un composé correspondant répondant à la formule I comme défini à la revendication 9 ;
(c) d'hydrolyse d'un ester répondant à la formule : 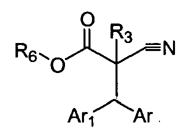 dans laquelle Ar, Ar₁, R₃ et R₆ sont tels que défini ci-dessus, avec cette condition que R₆ représente autre chose qu'un atome d'hydrogène, pour obtenir un composé correspondant répondant à la formule I dans laquelle R₄ représente un groupe OR₆ où R₆ représente un atome d'hydrogène ;
(d) de mise en réaction d'un composé acide activé répondant à la formule 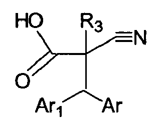 dans laquelle Ar, Ar₁, et R₃ sont tels que définis ci-dessus, avec une amine répondant à la formule HNR₅R₆, 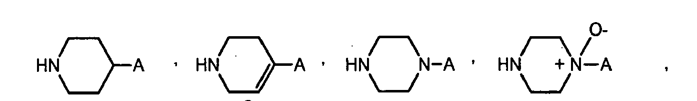 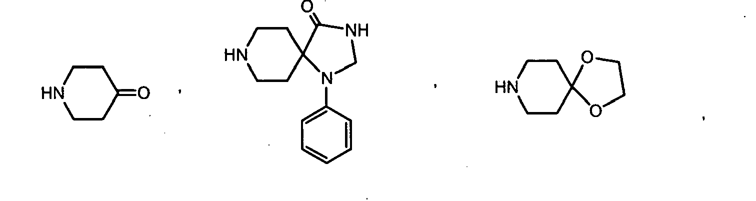 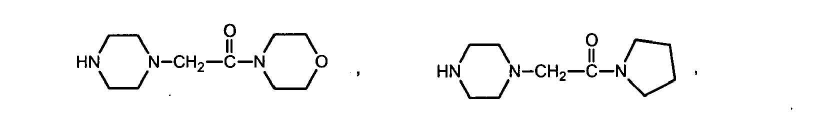 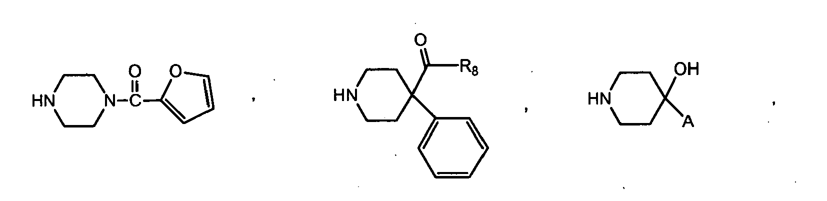 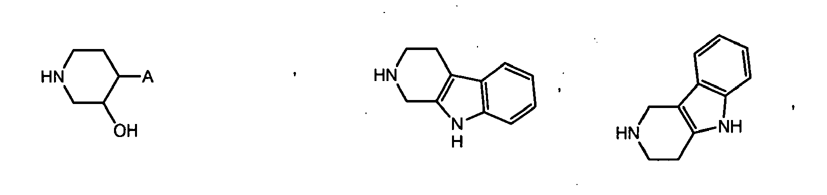 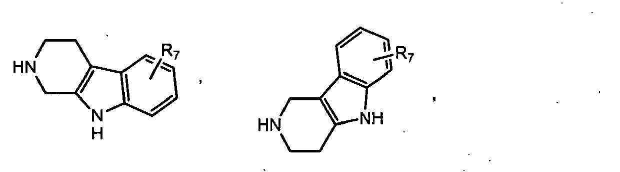 ou
(e) de mise en réaction d'un composé répondant à la formule : 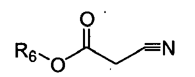 dans laquelle R₆ est tel que défini ci-dessus, à l'exception d'un atome d'hydrogène, en présence d'une base forte, avec un halogénure répondant à la formule
Ar,ArCHX
dans laquelle Ar et Ar₁ sont tels que défini en l'occurrence et X représente un atome d'halogène pour obtenir un composé correspondant répondant à la formule I dans laquelle R₃ représente un atome d'hydrogène ;
ou
(f) de conversion d'un composé répondant à la formule I tel que défini à la revendication 1 possédant un groupe ou un site réactif faisant office de substituant pour obtenir un composé différent répondant à la formule I;
ou
(g) de conversion d'un composé répondant à la formule I pour obtenir un de ses sels pharmaceutiquement acceptables.

16. Procédé comprenant les étapes :
de formation d'un sel d'éphédrine, de cinchonidine, ou de quinidine d'un composé selon la revendication 9 ou 14 ou d'un de ses sels pharmaceutiquement acceptables ; et
d'extraction dudit sel d'éphédrine, de cinchonidine, ou de quinidine dans un solvant ;
pour obtenir un énantiomère essentiellement pur d'un composé selon la revendication 9 ou 14.

17. Composé selon la revendication 9 choisi parmi :
le 2-cyano-3,3-di(1-naphtyl)propanamide ;
le (RR,SS)-3-(3,4-dihydroisoquinolin-2(1H)-yl)-2-[(2-méthoxyphényl)(1-naphtyl)méthyl]-2-méthyl-3-oxopropanenitrile ;
le (2S)-3-[4-(3-bromophényl)pipérazin-1-yl]-2-[(S)-(2-méthoxyphényl)(1-naphtyl)méthyl]-2-méthyl-3-oxopropanenitrile ;
le (2S,3S)-3-(2-méthoxyphényl)-2-méthyl-3-(1-naphtyl)-2-{[4-(2-oxo-2,3 dihydro-1H-indol-1-yl)pipéridin-1-yl]carbonyl}propanenitrile ;
le (2S,3S)-3-(2-méthoxyphényl)-2-méthyl-3-(1-naphtyl)-2-[(4-pyridin-4-ylpipérazin-1-yl)carbonyl]propanenitrile ;
le (2S,3S)-3-(2-méthoxyphényl)-2-méthyl-3-(1-naphtyl)-2-{[4-(2-oxo-3,4-dihydroquinolin-1(2H)-yl)pipéridin-1-yl]carbonyl}propanenitrile ;
le (2S)-3-[4-(4-bromophényl)-3,6-dihydropyridin-1(2H)-yl]-2-[(S)-(2-méthoxyphényl)(1-naphtyl)méthyl]-2-méthyl-3-oxopropanenitrile ;
le (2S,3S)-3-(2-méthoxyphényl)-2-méthyl-3-(1-naphtyl)-2-({4-[3-(trifluorométhyl) phényl] pipérazin-1-yl}carbonyl)propanenitrile ;
le (2S)-3-[4-(4-fluorophényl)-3,6-dihydropyridin-1(2H)-yl]-2-[(S)-(2-méthoxyphényl)(1-naphtyl)méthyl]-2-méthyl-3-oxopropanenitrile ;
le (2S,3S)-3-(2-méthoxyphényl)-2-{[4-(2-méthoxyphényl)pipéridin-1-yl]carbonyl}-2-méthyl-3-(1-naphtyl)propanenitrile ;
le (2S,3S)-3-(2-méthoxyphényl)-2-méthyl-3-(1-naphtyl)-2-({4-[3-(trifluorométhyl)cyclohexyl]pipéridin-1-yl}carbonyl)propanenitrile ;
le (2S)-3-{4-hydroxy-4-[3-(trifluorométhyl)phényl]pipéridin-1-yl}-2-[(S)-(2-méthoxyphényl) (1-naphtyl)méthyl]-2-méthyl-3-oxopropanenitrile ;
l'acide 2-cyano-3-(2-méthoxyphényl)-2-méthyl-3-quinolin-4-ylpropanoïque ;
le 3-(2-méthoxyphényl)-2-méthyl-3-quinolin-4-yl-2-({4-[3-(trifluorométhyl)phényl]pipéridin-1-yl}carbonyl)propanenitrile ;
le (RR,SS)-2-[(2-méthoxyéthoxy)méthyl]-3-(2-méthoxyphényl)-3-(1-naphtyl)-2-[(4-pyrimidin-2-ylpipérazin-1-yl)carbonyl]propanenitrile ;
le (RR,SS)-2-[(2-méthoxyéthoxy)méthyl]-3-(2-méthoxyphényl)-3-(1-naphtyl)-2-({4-[3-(trifluorométhyl)phényl]pipéridin-1-yl}carbonyl)propanenitrile ;
le (RR,SS)-N-(tert-butyl)-2-cyano-2-[(2-méthoxyéthoxy)méthyl]-3-(2-méthoxyphényl)-3-(1-naphtyl)propanamide ;
le (RR,SS)-2-cyano-N-(2,2-diméthylpropyl)-2-[(2-méthoxyéthoxy)-méthyl]-3-(2-méthoxyphényl)-3-(1-naphtyl)propanamide ;
le (RR,SS)-2-[(2-méthoxyéthoxy)méthyl]-3-(2-méthoxyphényl)-3-(1-naphtyl)-2-[(4-pyrazin-2-ylpipérazin-1-yl)carbonyl]propanenitrile ;
le (RR,SS)-3-[4-(1H-indol-3-yl)-3,6-dihydropyridin-1(2H)-yl]-2-[(2-méthoxyéthoxy)méthyl]-2-[(S*)-(2-méthoxyphényl)(1-naphtyl)méthyl]-3-oxopropanenitrile ;
le (RR,SS)-3-(2-méthoxyphényl)-3-(1-naphtyl)-2-(pyridin-3-yl-méthyl)-2-[(4-pyrimidin-2-ylpipérazin-1-yl)carbonyl]propanenitrile ;
le (RR,SS)-N-(tert-butyl)-2-cyano-3-(2-méthoxyphényl)-3-(1-naphtyl)-2-(pyridin-3-ylméthyl)propanamide ;
le (RR,SS)-2-cyano-N-(2,2-diméthylpropyl)-3-(2-méthoxyphényl)-3-(1-naphtyl)-2-(pyridin-3-ylméthyl)propanamide ;
le (RR,SS)-3-(2-méthoxyphényl)-3-(1-naphtyl)-2-[(4-pyrazin-2-ylpipérazin-1-yl)carbonyl]-2-(pyridin-3-ylméthyl)propanenitrile ;
le (RR,SS)-3-[4-(1H-indol-3-yl)-3,6-dihydropyridin-1(2H)-yl]-2-[(2-méthoxyphényl)(1-naphtyl)méthyl]-3-oxo-2-(pyridin-3-ylméthyl)propanenitrile ;
le (RR,SS)-N-(tert-butyl)-2-cyano-2-(méthoxyméthyl)-3-(2-méthoxyphényl)-3-(1-naphtyl)propanamide ;
le (2S*,3S*)-2-cyano-N-(2,2-diméthylpropyl)-2-(méthoxyméthyl)-3-(2-méthoxyphényl)-3-(1-naphtyl)propanamide ;
le (2S)-3-[4-(1H-indol-3-yl)-3,6-dihydropyridin-1(2H)-yl]-2-[(S)-(2-méthoxyphényl)(1-naphtyl)méthyl]-2-méthyl-3-oxopropanenitrile ;
le (2S,3S)-3-(2-méthoxyphényl)-2-méthyl-3-(1-naphtyl)-2-{(4-(2-oxo-2,3-dihydro-1H-benzimidazol-1-yl)pipéridin-1-yl]carbonyl}propanenitrile ;
le (2S,3S)-3-(2-méthoxyphényl)-2-méthyl-3-(1-naphtyl)-2-[(4-oxo-1-phényl-1,3,8-triazaspiro[4.5]déc-8-yl)carbonyl]propanenitrile ;
le (2S)-3-{3-hydroxy-4-[3-(trifluorométhyl)phényl]pipéridin-1-yl}-2-[(S)-(2-méthoxyphényl)(1-naphtyl)méthyl]-2-méthyl-3-oxopropanenitrile ;
le (2S)-3-[4-(4-chlorophényl)-4-hydroxypipéridin-1-yl]-2-[(S)-(2-méthoxyphényl)(1-naphtyl)méthyl]-2-méthyl-3-oxopropanenitrile ;
le (2S)-3-[4-(1H-indol-3-yl)pipéridin-1-yl]-2-[(S)-(2-méthoxyphényl)-(1-naphtyl)méthyl]-2-méthyl-3-oxopropanenitrile ;
le (2S)-3-(1,4-dioxa-8-azaspiro[4.5]déc-8-yl)-2-[(S)-(2-méthoxyphényl)(1-naphtyl)méthyl-2-méthyl-3-oxopropanenitrile ;
le (2S)-3-(8-fluoro-1,3,4,5-tetrahydro-2H-pyrido[4,3-b]indol-2-yl)-2-[(S)-(2-méthoxyphényl)(1-naphtyl)méthyl]-2-méthyl-3-oxopropanenitrile ;
le (2S,3S)-3-(2-méthoxyphényl)-2-méthyl-3-(1-naphtyl)-2-(1,3,4,9-tetrahydro-2H-β-carbolin-2-ylcarbonyl)propanenitrile ;
le (2S,3S)-3-(2-méthoxyphényl)-2-méthyl-3-(1-naphtyl)-2-[(4-oxopipéridin-1-yl)carbonyl] propanenitrile ;
le (2S,3S)-3-(2-méthoxyphényl)-2-méthyl-3-(1-naphtyl)-2-[(4-pyrimidin-2-ylpipérazin-1-yl)carbonyl]propanenitrile ;
le (2S)-3-[4-(4-bromophényl)-4-hydroxypipéridin-1-yl]-2-[(S)-(2-méthoxyphényl)(1-naphtyl)méthyl]-2-méthyl-3-oxopropanenitrile ;
le (2S,3S)-3-(2-méthoxyphényl)-2-méthyl-3-(1-naphtyl)-2-[(4-phényl-4-propionylpipéridin-1-yl)carbonyl]propanenitrile ;
le (2S)-3-(4-acetyl-4-phénylpipéridin-1-yl)-2-[(S)-(2-méthoxyphényl)(1-naphtyl)méthyl]-2-méthyl-3-oxopropanenitrile ;
le (2S)-3-(4-cyclohexylpipérazin-1-yl)-2-[(S)-(2-méthoxyphényl)(1-naphtyl)méthyl]-2-méthyl-3-oxopropanenitrile ;
le (2S)-3-[4-(2-méthoxyéthyl)pipérazin-1-yl]-2-[(S)-(2-méthoxyphényl)(1-naphtyl)méthyl]-2-méthyl-3-oxopropanenitrile ;
le (2S,3S)-3-(2-méthoxyphényl)-2-méthyl-2-{[4-(2-morpholin-4-yl-2-oxoéthyl)pipérazin-1-yl]carbonyl}-3-(1-naphtyl)propanenitrile ;
le (2S)-3-[4-(2-furoyl)pipérazin-1-yl]-2-[(S)-(2-méthoxyphényl)(1-naphtyl)méthyl]-2-méthyl-3-oxopropanenitrile ;
le (RR,SS)-2-(méthoxyméthyl)-3-(2-méthoxyphényl)-3-(1-naphtyl)-2-[(4-pyrimidin-2-ylpipérazin-1-yl)carbonyl]propanenitrile ;
le (2S,3S)-3-(2-méthoxyphényl)-2-méthyl-3-(1-naphtyl)-2-{[4-(2-oxo-2-pyrrolidin-1-yléthyl)pipérazin-1-yl]carbonyl}propanenitrile ;
le (2S)-3-{4-[3-(diméthylamino)propyl]pipérazin-1-yl}-2-[(S)-(2-méthoxyphényl)(1-naphtyl)méthyl]-2-méthyl-3-oxopropanenitrile ;
le (2S,3S)-3-(2-méthoxyphényl)-2-méthyl-2-[(4-morpholin-4-yl-pipéridin-1-yl)carbonyl]-3-(1-naphtyl)propanenitrile ;
le 3-{4-[3-(diméthylamino)propyl]pipérazin-1-yl}-2-(méthoxyméthyl)-2-[(2-méthoxyphényl)(1-naphtyl)méthyl]-3-oxopropanenitrile ;
le (2S*)-2-[(benzyloxy)méthyl]-3-{4-[3-(diméthylamino)propyl]-pipérazin-1-yl}-2-[(R*)-(2-méthoxyphényl)(1-naphtyl)méthyl]-3-oxopropanenitrile ;
le 3-[4-(2-méthoxyéthyl)pipérazin-1-yl]-2-(méthoxyméthyl)-2-[(2-méthoxyphényl)(1-naphtyl)méthyl]-3-oxopropanenitrile ;
le 2-[(benzyloxy)méthyl]-3-[4-(2-méthoxyéthyl)pipérazin-1-yl]-2-[(2-méthoxyphényl)(1-naphtyl)méthyl]-3-oxopropanenitrile ;
le 2-[(benzyloxy)méthyl]-3-(2-méthoxyphényl)-2-{[4-(2-morpholin-4-yléthyl)pipérazin-1-yl]carbonyl}-3-(1-naphtyl)propanenitrile ;
le (RR,SS)-3-(4-cyclohexylpipérazin-1-yl)-2-(méthoxyméthyl)-2-[(2-méthoxyphényl)(1-naphtyl)méthyl]-3-oxopropanenitrile ;
le (2S,3S)-2-(méthoxyméthyl)-3-(2-méthoxyphényl)-3-(1-naphtyl)-2-[(4-pyrimidin-2-ylpipérazin-1-yl)carbonyl]propanenitrile ;
le (2R,3R)-2-(méthoxyméthyl)-3-(2-méthoxyphényl)-3-(1-naphtyl)-2-[(4-pyrimidin-2-ylpipérazin-1-yl)carbonyl]propanenitrile ;
le (RR,SS)-2-(méthoxyméthyl)-3-(2-méthoxyphényl)-3-(1-naphtyl)-2-[(4-pyrazin-2-ylpipérazin-1-yl)carbonyl]propanenitrile ;
le (RR,SS)-3-[4-(1H-indol-3-yl)-3,6-dihydropyridin-1(2H)-yl]-2-(méthoxyméthyl)-2-[(2-méthoxyphényl)(1-naphtyl)méthyl]-3-oxopropanenitrile ;
le (2S,3S)-3-(2-méthoxyphényl)-2-méthyl-3-(1-naphtyl)-2-[(4-pyrazin-2-ylpipérazin-1-yl)carbonyl]propanenitrile ;
le (2S,3S)-3-(2-méthoxyphényl)-2-méthyl-2-{[4-(2-morpholin-4-yléthyl)pipérazin-1-yl]carbonyl}-3-(1-naphtyl)propanenitrile ;
le (2S)-3-{4-[2-(diméthylamino)éthyl]pipérazin-1-yl}-2-[(S)-(2-méthoxyphényl)(1-naphtyl)méthyl]-2-méthyl-3-oxopropanenitrile ;
le (RR,SS)-2-[(benzyloxy)méthyl]-3-(4-cyclohexylpipérazin-1-yl)-2-[(2-méthoxyphényl) (1-naphtyl)méthyl]-3-oxopropanenitrile ;
le (RR,SS)-2-[(benzyloxy)méthyl]-3-(2-méthoxyphényl)-3-(1-naphtyl)-2-[(4-pyrimidin-2-ylpipérazin-1-yl)carbonyl]propanenitrile ;
le (RR,SS)-2-(méthoxyméthyl)-3-(2-méthoxyphényl)-2-{[4-(2-morpholin-4-yléthyl)pipérazin-1-yl]carbonyl}-3-(1-naphtyl)propanenitrile ;
le (RR,SS)-2-[(benzyloxy)méthyl]-3-(2-méthoxyphényl)-3-(1-naphtyl)-2-[(4-pyrazin-2-ylpipérazin-1-yl)carbonyl]propanenitrile ;
le (2S,3S)-3-(4-cyclohexylpipérazin-1-yl)-2-(méthoxyméthyl)-2-[(2-méthoxyphényl)(1-naphtyl)méthyl]-3-oxopropanenitrile ;
le (2R,3R)-3-(4-cyclohexylpipérazin-1-yl)-2-(méthoxyméthyl)-2-[(2-méthoxyphényl)(1-naphtyl)méthyl]-3-oxopropanenitrile ;
le (2S,3S)-2-(méthoxyméthyl)-3-(2-méthoxyphényl)-3-(1-naphtyl)-2-[(4-pyrazin-2-ylpipérazin-1-yl)carbonyl]propanenitrile ;
le (2R,3R)-2-(méthoxyméthyl)-3-(2-méthoxyphényl)-3-(1-naphtyl)-2-[(4-pyrazin-2-ylpipérazin-1-yl)carbonyl]propanenitrile ;
le ((2S,3S)-3-[4-(1H-indol-3-yl)-3,6-dihydropyridin-1(2H)-yl]-2-(méthoxyméthyl)-2-[(2-méthoxyphényl)(1-naphtyl)méthyl]-3-oxopropanenitrile ;
le ((2R,3R)-3-[4-(1H-indol-3-yl)-3,6-dihydropyridin-1(2H)-yl]-2-(méthoxyméthyl)-2-[(2-méthoxyphényl)(1-naphtyl)méthyl]-3-oxopropanenitrile ;
l'acide (-)-(2S,3S)-2-cyano-3-(2-méthoxyphényl)-2-méthyl-3-(1-naphtyl)propanoïque ; et
l'acide (-)-(2R,3R)-2-cyano-3-(2-méthoxyphényl)-2-méthyl-3-(1-naphtyl)propanoïque ;
ou un de leurs sels pharmaceutiquement acceptables.
